# EUROPEAN PATENT SPECIFICATION

(11) **EP 2 635 567 B1**
(45) Date of publication and mention of the grant of the patent: **28.10.2015**
(21) Application number: 11788570.7
(22) Date of filing: 04.11.2011
(51) Int. Cl.: C07D 403/04, C07D 403/14, C07D 413/12, A61K 31/404, A61P 29/00

(54) **INDOLE DERIVATIVES**
INDOLDERIVATE
DÉRIVÉS D'INDOLE

(30) Priority: 05.11.2010 HU P1000598
(43) Date of publication of application: 11.09.2013
(73) Proprietor: Richter Gedeon Nyrt., 1103 Budapest (HU)
(72) Inventor: BEKE, Gyula, H-2092 Budakeszi (HU); BÉNYEI, Gyula Attila, H-1185 Budapest (HU); BORZA, István, H-1186 Budapest (HU); BOZÓ, Éva, H-1102 Budapest (HU); FARKAS, Sándor, H-1103 Budapest (HU); HORNOK, Katalin, H-1141 Budapest (HU); PAPP, Andrea, H-1027 Budapest (HU); VÁGÓ, István, H-1121 Budapest (HU); VASTAG, Mónika, H-1103 Budapest (HU)
(74) Representative: Gullerné Lados, Zsuzsanna
(86) International application number: PCT/HU2011/000104
(87) International publication number: WO 2012/059776

(56) References cited:
- WO-A1-03/066577
- WO-A1-2005/016886
- WO-A1-2010/097372
- WO-A2-2004/019868
- WO-A2-2005/085198
- FR-A1- 2 894 964

## Description

### FIELD OF THE INVENTION

The present invention relates to new indole derivatives of formula (I) and optical antipodes or racemates and/or salts thereof that serve as selective bradykinin 1 antagonists. The invention also relates to the process for producing such compounds. The invention further relates to pharmaceutical compositions comprising such compounds useful for treating or preventing disorders involving pain and inflammation. The invention also provides a method for manufacture of medicaments useful in the treatment or prevention of such disorders.

### BACKGROUND OF THE INVENTION

Kinins are endogenous peptides formed in plasma and peripheral tissues following proteolytic cleavage of kininogen precursors by kallikrein enzymes (Bhoola, Pharmacol. Rev. 1992, 44, 1-80). Their biological actions are mediated by two G-protein coupled membrane receptors, denoted B1 and B2. (Regoli and Barabe, Pharmacol. Rev. 1980, 32, 1-46, Marceau et al., Pharmacol. Rev. 1998, 50, 357-386).

The first set of kinins, bradykinin (BK) and kallidin (LysBK) involve in acute physiological process of cardiovascular, renal, nervous and immune system (Calixto et al., Pain, 2000, 87, 1-5). They preferentially act through stimulation of constitutively expressed and rapidly desensitizing B2 receptors, which are widely distributed in many tissues.

On the other hand, the second set of kinins, active carboxypeptidase metabolites of BK and LysBK, desArg⁹BK (DABK) and LysdesArg⁹BK (LysDABK) under pathological conditions activate inducible B1 receptors in several cell types of ectodermal (e.g. neurons, glias), mesodermal (e.g. endothelial cells, smooth muscle cells, blood cells, stromal cells) and endodermal (e.g. airway epithelial cells) origin (Leeb-Lundberg et al., Pharmacol. Rev. 2005, 57, 27-77). Generally B1 receptors which are rarely expressed under non-pathological conditions rapidly appear after injuries of various natures (tissue trauma, infections, *etc.)* but unlike B2 receptors elicit persistent responses due to limited desensitization and internalization with very low ligand dissociation (Couture et al., Eur.J. of Pharmacol., 2001, 429, 161-176).

Thus the B1 receptor up-regulation appears to be part of a generalized inflammatory and pain responses that includes the local co-expression (eventually up-regulation) of enzymes, receptors, autacoids, cytokines and chemokines that notoriously play key roles in the early and late responses of tissues to various types of injury. In animal models it has been demonstrated that there is a switch in dominance of function from B2 to B1 receptor in chronic inflammatory pain states. While the B2 receptor is implicated in the acute phase of the inflammatory and pain response, the B1 receptor is involved in the chronic phase of this response.

The involvement of kinin receptors in inflammation and pain transduction has been supported by the results of studies on mice lacking bradykinin B1 receptors. B1 receptor deficient mice are different from wild-type mice in sensory functions, exhibiting increased analgesic thresholds to noxious chemical and heat stimuli, drastic reduction in the accumulation of polymorphonuclear leukocytes at sites of inflammation, as well as reduction in spinal reflex (Pesquero et al., PNAS, 2000, 97, 8140-8145). Reduced nerve injury-induced neuropathic pain was also confirmed in bradykinin B1 receptor knockout mice (Ferreira et al., J. Neuroscience, 2005, 25, 2405-2412). Furthermore B1 receptor KO mice developed hyperglycemia but not hyperalgesia in response to STZ administrazion (Gabra, Reg. Pept., 2005, 127, 245-248).

Growing evidence supported a critical role of bradykinin B1 receptors in pathological states like inflammations (e.g. oedema formations, leukocyte trafficking), infections, ischemia-reperfusion lesions, cancer growth and metastasis. Involvement of B1 receptors in centrally and peripherally mediated pain like acute, chronic, neuropathic and cancer pain was also confirmed (Calixto et al., Br.J.of Pharmacol., 2004, 143, 803-818, Conley et al., Eur. J. Pharmacol,. 2005, 527, 44-51, Sevcik et al., J. Pain, 2005, 6, 771-775). The bradykinin B1 receptor provides a strategic role in development and maintenance of chronic inflammatory diseases such as asthma, diabetes, rheumatoid arthritis (Couture et al., Eur. J. Pharmacol. 2001, 429, 161-176, Gabra et al., Biol.Chem., 2006, 387, 127-143, Cassim et al., Rheumatology, 2009, 48, 490-496). Bradykinin B1 receptors could be therapeutic targets for neurological disorders like epilepsy, multiple sclerosis, Parkinson disases and Alzheimer disease (Rodi et al., Cur. Pharm. Design 2005, 11, 1313-1326, Prediger et al., Neuroscience, 2008, 151, 631-643)

Several patents and patent applications describe bradykinin B1 receptor antagonists which have different chemical structures. International patent application WO09124733 relates to substituted sulfonamide derivatives. In WO09036996 and WO09013299 disclose wide variety of small molecules with different chemical structures which have valuable properties. International patent application WO07101007 discloses aryl sulfonyl heterocycles and variables thereof. In accordance with the international patent application WO05004810 some N-arylsulfonamide derivatives are also known as bradykinin antagonists or inverse agonists useful in the treatment or prevention of symptoms such as pain and inflammation associated with the bradykinin B1 biological pathway. WO04054584 discloses 2-quinoxalinone derivatives and WO02099388 relates to benzodiazepine compounds. WO02076964 describes derivatives of N-(arylsulfonyl)beta-aminoacids comprising a substituted aminomethyl group. The compounds referred in these references may be used to modulates bradykinin receptor activity *in vivo* and *in vitro* and are useful in the treatment of conditions responsive to B1 modulation in mammals involving pain and inflammation. But neither of these prior art references discloses any compound with the scope of the class of compounds described herein below.

### SUMMARY OF THE INVENTION

We have identified a class of indole derivatives which have high affinity for bradykinin B1 receptors and selectivity over bradykinin B2 receptors providing unique role in the treatment of chronic painful and inflammatory processes and disorders. Furthermore targeting inducible receptor, which has no or minor physiological role in the homeostasis guarantees exemption from undesired side effects related to constitutive B2 receptors. The present invention relates to compounds being selective bradykinin B1 receptor antagonists and the synthesis thereof. Compounds of the present invention are useful for the treatment of disorders involving inflammation and pain.

The present invention relates to the indole derivatives of formula (I) 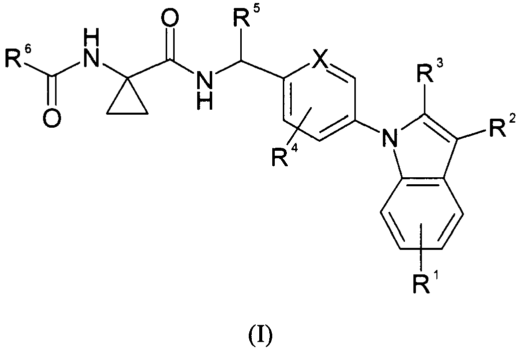 wherein
- R¹: is hydrogen atom, halogen atom or C₁-C₄-alkoxy group;
- R²: is hydrogen atom, halogen atom, C₁-C₄-alkyl group or cyano group;
- R³: is selected from (1) -COOR; (2) -CN; (3) -CONR^{a}R^{b}; 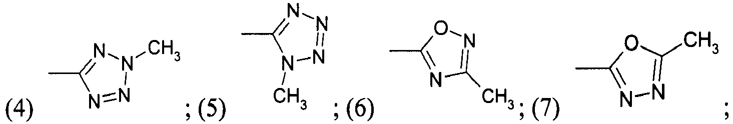 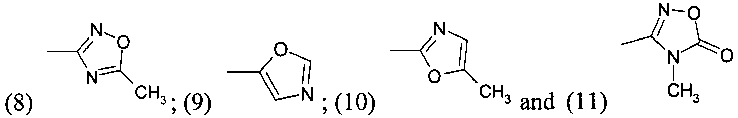
- R⁴: is hydrogen atom or halogen atom;
- R⁵: is hydrogen atom or C₁-C₄-alkyl group;
- R⁶: is selected from
(1) C₃-C₆-cycloalkyl;
(2) optionally substituted five- or six-membered aromatic ring with two nitrogen atoms;
(3) optionally substituted pyridyl;
(4) optionally substituted five-membered aromatic ring with one heteroatom selected from O or S;
(5)-CF₃; (6) -CH₂-CF₃; (7) -CH₂-CH₂-C≡CH; (8) -CH₂-CH₂-CH(CH₃)₂; 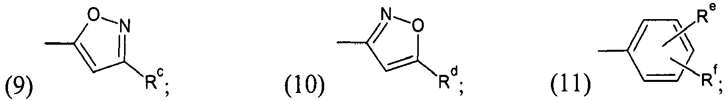 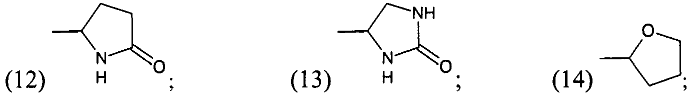 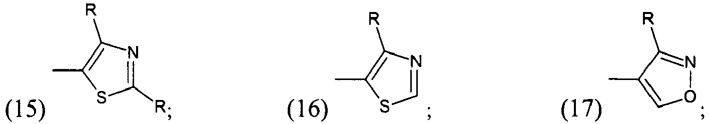 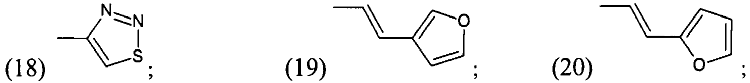 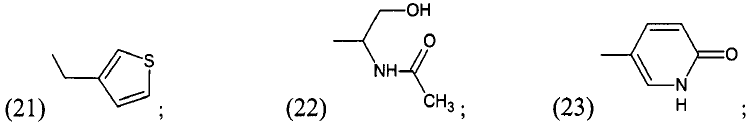 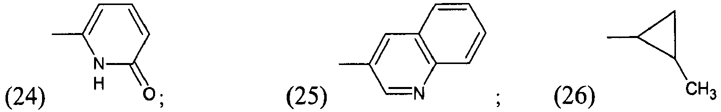 and 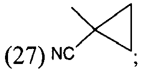
- X: is -CH- or nitrogen atom;
- R: is C₁-C₄ alkyl group;
- R^{a} and R^{b}: are independently from each other hydrogen atom; C₁-C₄ alkyl group or pyrrolidin-1-yl group;
- R^{c}: is hydrogen atom; C₁-C₄ alkyl or C₁-C₄ alkoxy group;
- R^{d}: is hydrogen atom; C₁-C₄ alkyl or C₃-C₆ cycloalkyl group;
- R^{e} and R^{f}: are independently from each other hydrogen atom; halogen atom; C₁-C₄ alkyl group; C₁-C₄ alkoxy; -CF₃; -CN or -NH₂ group;
and optical antipodes or racemates and/or salts thereof.

The invention also relates to the pharmaceutical compositions containing the compounds of formula (I) or optical antipodes or racemates and/or salts thereof as active ingredient.

Furthermore the present invention relates to the synthesis of the compounds of formula (I) and optical antipodes or racemates and/or salts thereof, the pharmaceutical compositions comprising thereof and the chemical and pharmaceutical manufacture of medicaments containing these compounds, as well as the methods of treatment with these compounds, which means administering to a mammal to be treated - including human - suffering from disorders involving pain and inflammation effective amount of compounds of formula (I) and optical antipodes or racemates and/or salts thereof of the present invention as such or as medicament.

### DETAILED DESCRIPTION OF THE INVENTION

The present invention relates to the indole derivatives of formula (I) 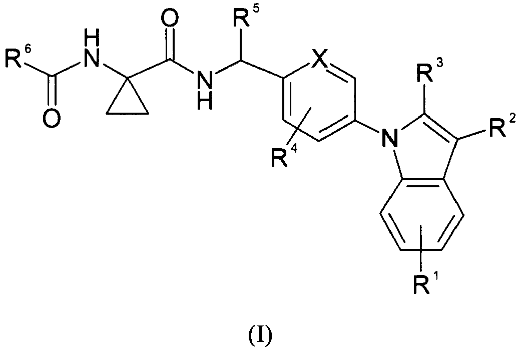 wherein
- R¹: is hydrogen atom, halogen atom or C₁-C₄-alkoxy group;
- R²: is hydrogen atom, halogen atom, C₁-C₄-alkyl group or cyano group;
- R³: is selected from (1) -COOR; (2) -CN; (3) -CONR^{a}R^{b}; 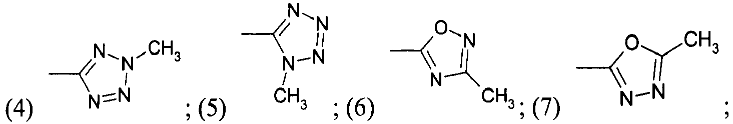 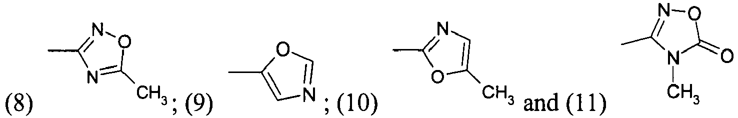
- R⁴: is hydrogen atom or halogen atom;
- R⁵: is hydrogen atom or C₁-C₄-alkyl group;
- R⁶: is selected from
(1) C₃-C₆-cycloalkyl;
(2) optionally substituted five- or six-membered aromatic ring with two nitrogen atoms;
(3) optionally substituted pyridyl;
(4) optionally substituted five-membered aromatic ring with one heteroatom selected from O or S;
(5)-CF₃; (6) -CH₂-CF₃; (7) -CH₂-CH₂-C≡CH; (8) -CH₂-CH₂-CH(CH₃)₂; 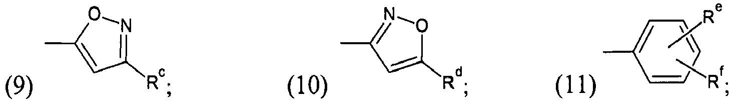 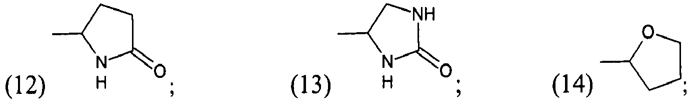 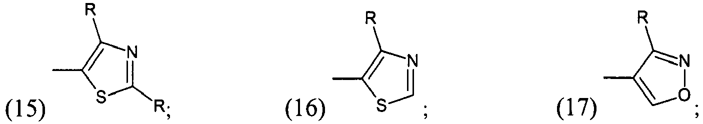 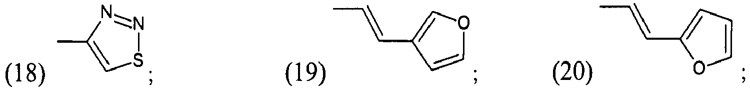 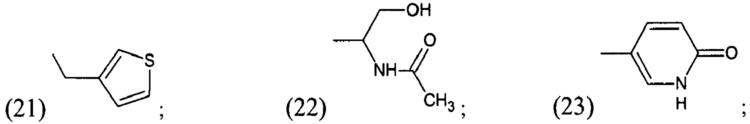 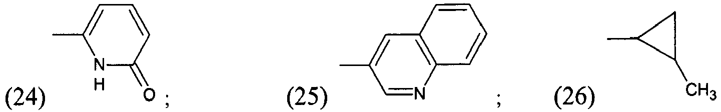 and 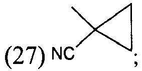
- X: is -CH- or nitrogen atom;
- R: is C₁-C₄ alkyl group;
- R^{a} and R^{b}: are independently from each other hydrogen atom; C₁-C₄ alkyl group or pyrrolidin-1-yl group;
- R^{c}: is hydrogen atom; C₁-C₄ alkyl or C₁-C₄ alkoxy group;
- R^{a}: is hydrogen atom; C₁-C₄ alkyl or C₃-C₆ cycloalkyl group;
- R^{e} and R^{f}: are independently from each other hydrogen atom; halogen atom; C₁-C₄ alkyl; C₁-C₄ alkoxy; -CF₃; -CN or -NH₂ group;
and optical antipodes or racemates and/or salts thereof.

When the meaning of R⁶ is optionally substituted six-membered aromatic ring with two nitrogen atoms the term "optionally substituted" means that the ring may be substituted by one substituent selected from halogen atom, C₁-C₄ alkyl, -CF₃ or -SCH₃ groups.

When the meaning of R⁶ is optionally substituted five-membered aromatic ring with two nitrogen atoms the term "optionally substituted" means that the ring may be substituted by C₁-C₄ alkyl group.

When the meaning of R⁶ is optionally substituted five-membered aromatic ring with one heteroatom selected from O or S the term "optionally substituted" means that the ring may be substituted by one substituent selected from C₁-C₄-alkyl or S-(C₁-C₄)-alkyl group.

When the meaning of R⁶ is optionally substituted pyridyl group the term "optionally substituted" means that it may be substituted by one or two substituent selected from halogen atom, C₁-C₄-alkyl or -CF₃ group.

The term "halogen" or "halo" as used herein alone or as a part of another group refers to chlorine, bromine, fluorine and iodine.

The term "C₁-C₄ alkyl" as used herein refers to branched or straight chain alkyl groups comprising one to four carbon atoms, including methyl, ethyl, propyl, normal- and isopropyl and different butyl groups.

The term "C₃-C₆ cycloalkyl" as used herein refers to carbocyclic groups of 3 to 6 carbons, respectively; for example, cyclopropyl, cyclobutyl, cyclopentyl and cyclohexyl.

The term "C₁-C₄ alkoxy" as used herein refers to branched or straight chain alkyl groups comprising one to four carbon atoms bonded through an oxygen atom, including but not limited to, methoxy, ethoxy, propoxy and t-butoxy.

As used herein, the term "selective Bradykinin 1 antagonist" refers to a substance for example an organic molecule that is a potent full antagonist of human B1 receptor and shows at least 50 fold selectivity over B2 receptors.

The term "mammal" as used herein refers to any members of the class "Mammalia" including, but not limited to human.

The term "salt" means nontoxic acid addition salts of the compounds of the invention which are generally prepared by reacting the base with a suitable organic or inorganic acid. Typically the salts of the present invention are pharmaceutically acceptable salts including e.g. the hydrochloride, hydrobromide salts.

Included within the scope of the present invention are all stereoisomers, geometric isomers and tautomeric forms of the compounds of formula (I), including compounds exhibiting more than one type of isomerism and mixtures of one or more thereof.

*Cis*/*trans* isomers may be separated by conventional techniques well known to those skilled in the art, for example chromatography and fractional crystallisation.

Conventional techniques for the preparation/isolaton of individual enantiomers include chiral synthesis from the suitable optically pure precursor or resolution of the racemate (or racemate of a salt or derivative) using, for example chiral high pressure liquid chromatography (HPLC).

The term "pharmaceutically acceptable" describes an ingredient that is useful in preparing a pharmaceutical composition and is generally safe, non-toxic and neither biologically nor otherwise undesirable, and includes those acceptable for veterinary use as well as human pharmaceutical use.

The term "pharmaceutically effective amount" shall mean that amount of active ingredient that will elicit the biological or medical response of a tissue, system or animal that is being sought by a researcher or clinician.

The term "pharmaceutical composition" refers to a mixture of a compound of the invention with other chemical components, such as pharmaceutically acceptable auxiliary materials, e.g. diluents or carriers. The pharmaceutical composition facilitates administration of the compound to the subject.

The term "auxiliary material" defines a chemical compound that facilitates the incorporation of a compound into cells or tissues.

As used herein, the term "treatment" means using an effective therapy to reduce, alleviate or eliminate the symptoms associated with disorders involving pain and inflammation.

As a further aspect of the present invention there is provided the synthesis of compounds of formula (I)

Compounds in accordance with the present invention were synthesized in line with the synthetic routes and schemes described below.

Accordingly, the compounds of formula (I) of the invention can be synthesized by one of the following methods:

### Method a)

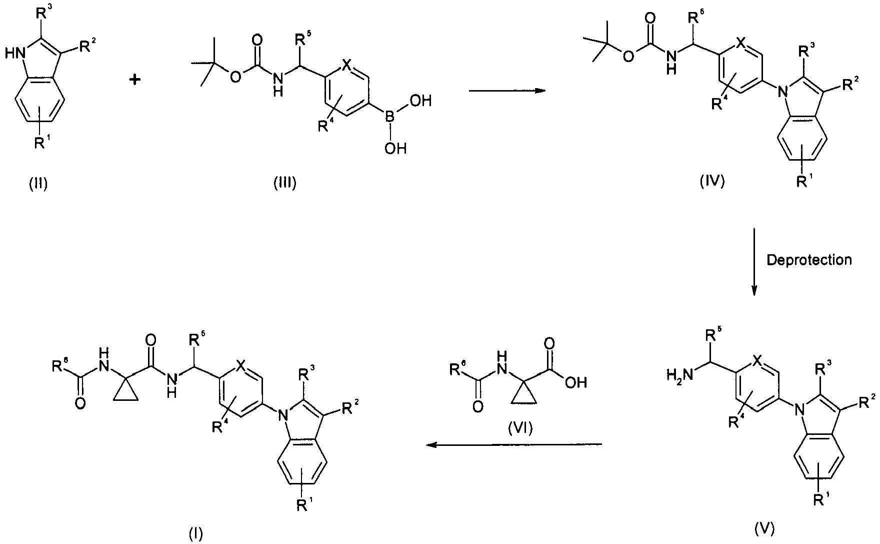

Reacting an indole derivative of formula (II) 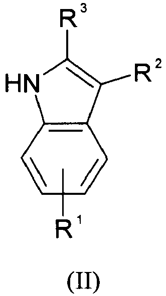
- wherein the meaning of R¹, R² and R³ is as described above for the formula (I) - with a boronic acid of formula (III) 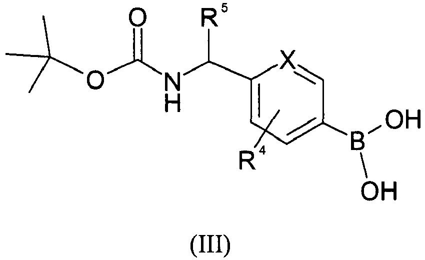
- wherein the meaning of R⁴ and R⁵ is as described above for the formula (I) - then the so obtained indole derivative of formula (IV) 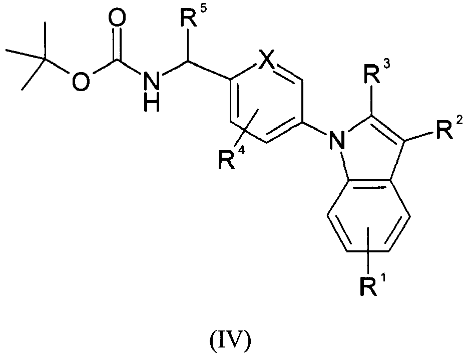
- wherein the meaning of R¹, R², R³, R⁴ and R⁵ is as described above for the formula (I) - is deprotected to yield an amine derivative of formula (V) 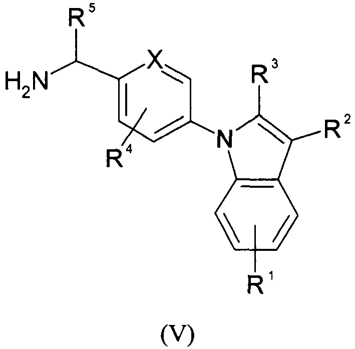
- wherein the meaning of R¹, R², R³, R⁴ and R⁵ is as described above for the formula (I) - finally the latter is reacted with an acid derivative of formula (VI) 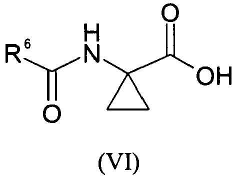
- wherein the meaning of R⁶ is as described above for the formula (I) - and the obtained indole derivative of formula (I) and optical antipodes or racemates and/or salts thereof in given case can be transformed into an other compound of formula (I) and optical antipodes or racemates and/or salts thereof by introducing new substituents and/or modifying or removing the existing ones.

### Method b)

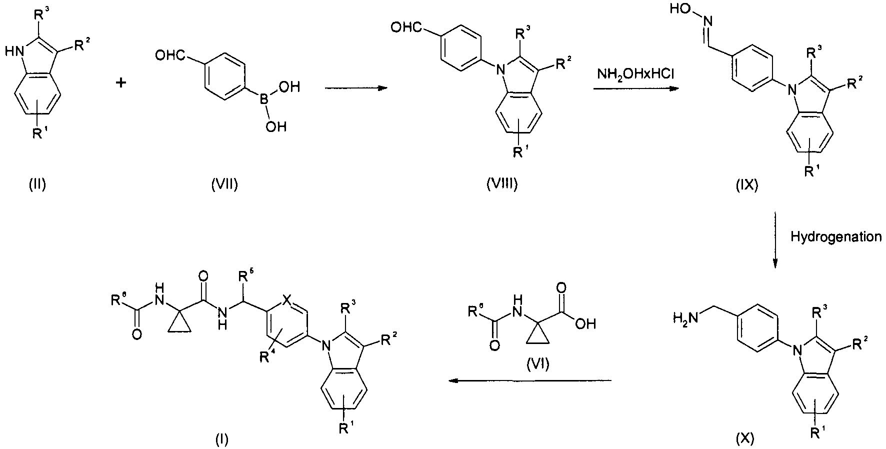

Reacting an indole derivative of formula (II) 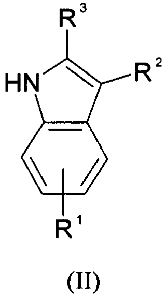
- wherein the meaning of R¹, R² and R³ is as described above for the formula (I) - with a boronic acid of formula (VII) 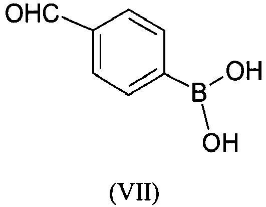 then the so obtained indole derivative of formula (VIII) 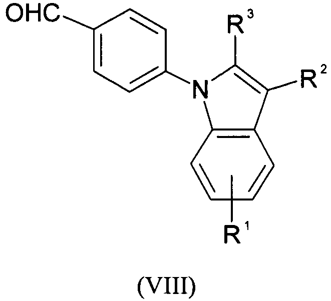
- wherein the meaning of R¹, R² and R³ is as described above for the formula (I) - is reacted with hydroxylamine hydrochloride to yield the indole derivative of formula (IX) 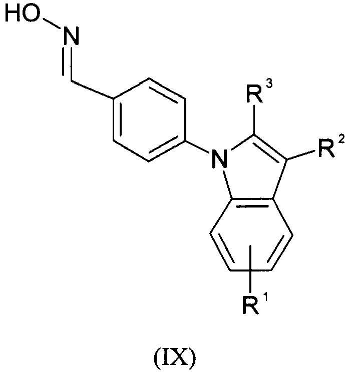 - wherein the meaning of R¹, R² and R³ is as described above for the formula (I) - the latter is hydrogenated to furnish an amine derivative of formula (X) 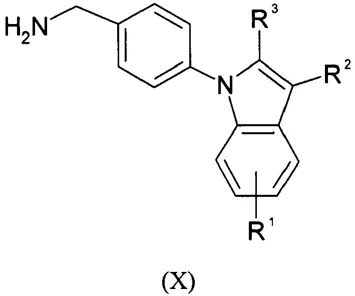
- wherein the meaning of R¹, R² and R³ is as described above for the formula (I) - finally the latter is reacted with an acid derivative of formula (VI) 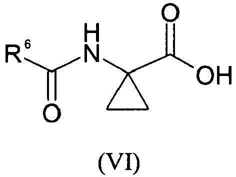
- wherein the meaning of R⁶ is as described above for the formula (I) - and the obtained indole derivative of formula (I) and optical antipodes or racemates and/or salts thereof in given case can be transformed into an other compound of formula (I) and optical antipodes or racemates and/or salts thereof by introducing new substituents and/or modifying or removing the existing ones.

### Method c)

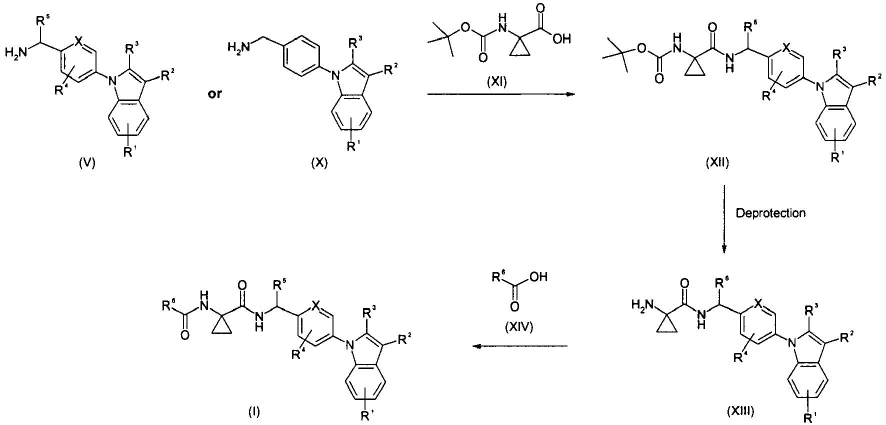

The amine derivatives of formula (V) or formula (X) - obtained according to the method described in method a) and method b) - are reacted with the amino acid derivative of formula (XI) 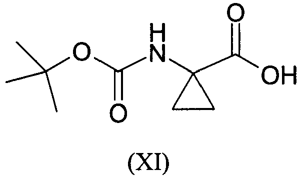 and the so obtained indole derivative of formula (XII) 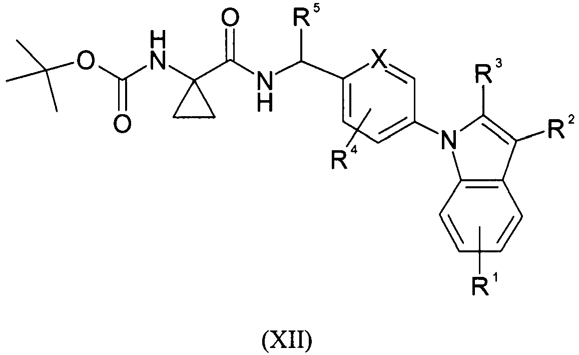
- wherein the meaning of R¹, R², R³, R⁴ and R⁵ is as described above for the formula (I) - is deprotected to yield an amine derivative of formula (XIII) 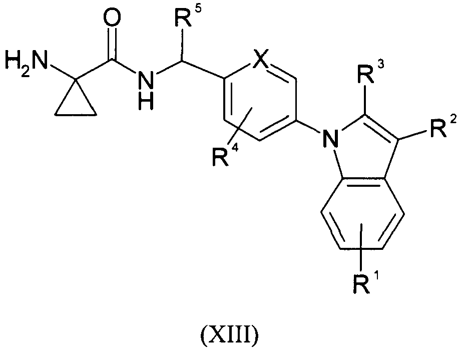
- wherein the meaning of R¹, R², R³, R⁴ and R⁵ is as described above for the formula (I) - finally the latter is reacted with an acid derivative of formula (XIV) 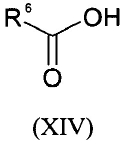
- wherein the meaning of R⁶ is as described above for the formula (I) - and the obtained indole derivative of formula (I) and optical antipodes or racemates and/or salts thereof in given case can be transformed into an other compound of formula (I) and optical antipodes or racemates and/or salts thereof by introducing new substituents and/or modifying or removing the existing ones.

The reaction of an indole derivative of formula (II) with a boronic acid of formula (III) or a boronic acid of formula (VII) is preferably carried out in a proper solvent, preferably in the presence of a copper(II) salt. The reactions are followed by thin layer chromatography. The necessary reaction time is 20-70 h. The work-up of the reaction mixture can be carried out by the following methods:
The reaction mixture is filtered through Celite and the product is isolated from the filtrate by extraction or column chromatography. The column chromatography is carried out on normal phase using Kieselgel 60 as adsorbent and different solvent systems, e.g. n-hexane/ethyl acetate, chloroform/methanol/acetic acid, dichloromethane/ethyl acetate or chloroform/acetone as eluents.

The structures of the products are determined by IR, NMR and mass spectrometry.

Deprotection of the indole derivatives of formula (IV) and formula (XII) can be carried out in a proper solvent using organic or inorganic acids, e.g. trifluoro acetic acid or hydrogen chloride.

The amide bond formations are preferably carried out by preparing an active derivative from a carboxylic acid of formula (VI), (XI) or (XIV), which is reacted with an amine of formula (V), (X) or (XIII) respectively, preferably in the presence of a base.

The transformation of a carboxylic acid into an active derivative can be carried out *in situ* during the amide bond formation in a proper solvent (e.g. *N,N-*dimethylformamide, dimethyl sulfoxide, acetonitrile, chlorinated hydrocarbons or hydrocarbons or the mixture thereof). The active derivatives can be active esters (e.g. prepared from carboxylic acid with hydroxybenztriazol (HOBt) and *N*-(3-dimethylaminopropyl)-*N*'-ethylcarbodiimide hydrochloride (EDC) or O-(7-azabenzotriazol-1-yl-*N*,*N*,*N*',*N*'-tetramethyluronium hexafluorophosphate (HATU) or O-benzotriazol-1-yl-*N*,*N*,*N*',*N*'-tetramethyluronium hexafluorophosphate (HBTU) in the presence of a base e.g. triethylamine, *N*,*N*-diisopropylethylamine). The active derivatives can be prepared at a temperature in the range of 0 °C to room temperature. A proper amine of formula (V), (X) or (XIII) is added as a base or as a salt formed with inorganic acid to the so obtained solution or suspension in the presence of a base, e.g. triethylamine, *N*,*N*-diisopropylethylamine needed for the liberation of the amine. The condensation reactions are followed by thin layer chromatography. The necessary reaction time is 6-20 h. The work-up of the reaction mixture can be carried out by different methods.
a) The reaction mixture is poured into water and the product is isolated by filtration or extraction with a proper organic solvent and in given case purified by crystallization or column chromatography. The column chromatography is carried out on normal phase using Kieselgel 60 as adsorbent and different solvent systems, e.g. n-hexane/ethyl acetate, toluene/methanol, chloroform/methanol or toluene/acetone, as eluents.
b) The reaction mixture is directly purified by column chromatography as described above to yield the pure product.

The structures of the products are determined by IR, NMR and mass spectrometry.

The obtained indole derivatives of formula (I) and optical antipodes or racemates and/or salts thereof - independently from the method of preparation - in given case can be transformed into another compound of formula (I) and optical antipodes or racemates and/or salts thereof by introducing further substituents and/or modifying and/or removing the existing ones.

For instance *N*-(*tert*-butoxycarbonyl) group can be cleaved by organic or inorganic acids (e.g. trifluoroacetic acid or hydrogen chloride).

Most of the indole derivatives of formula (II) are either commercially available or can be synthesized by different known methods. The syntheses of some new indole derivatives of formula (II) are described in the Examples. Following these procedures the other indole derivatives of formula (II) can also be prepared.

The compounds of the present invention and optical antipodes or racemates and/or salts thereof can be used as such or suitably in the form of pharmaceutical compositions.

The invention also relates to the pharmaceutical compositions containing the compounds of formula (I) or optical antipodes or racemates and/or salts thereof as active ingredient for the treatment of certain disorders associated with bradykinin B1 receptor activity.

Suitable routes of administration may, for example, include oral, rectal, transmucosal, or intestinal administration; parenteral delivery, including intramuscular, subcutaneous, intravenous, intramedullary injections, as well as intraarticular, intrathecal, direct intraventricular, intraperitoneal, intranasal, or intraocular injections and eye drops.

Alternatively, one may administer the compound in a local rather than systemic manner, for example, via injection of the compound directly in the renal or cardiac area, often in a depot or sustained release formulation. Furthermore, one may administer the drug in a targeted drug delivery system, for example, in a liposome coated with a tissue-specific antibody. The liposomes will be targeted to and taken up selectively by the organ.

The pharmaceutical compositions can be administered variety of routes and dosages forms. The compound of the invention may be administered either alone or in combination with pharmaceutically acceptable carriers, in either single or multiple doses. The dosage required to exert the therapeutical effect can vary within wide limits and will be fitted to the individual requirements in each of the particular case, depending on the stage of the disease, the condition and the bodyweight of the patient to be treated, as well as the sensitivity of the patient against the active ingredient, route of administration and number of daily treatments. The actual dose of the active ingredient to be used can safely be determined by the attending physician skilled in the art in the knowledge of the patient to be treated.

For the sake of a simple administration it is suitable if the pharmaceutical compositions comprise dosage units containing the amount of the active ingredient to be administered once, or a few multiples or a half, third or fourth part thereof. Such dosage units are e.g. tablets, which can be powdered with grooves promoting the halving or quartering of the tablet in order to exactly administer the required amount of the active ingredient.

The pharmaceutical compositions containing the active ingredient according to the present invention usually contain 0.01 to 500 mg of active ingredient in a single dosage unit. It is, of course possible that the amount of the active ingredient in some compositions exceeds the upper or lower limits defined above.

As a further aspect of the invention there is provided the pharmaceutical manufacture of medicaments containing the compounds of formula (I). or optical antipodes or racemates and/or salts thereof.

The pharmaceutical compositions of the present invention may be manufactured in a manner that is itself known, e.g., by means of conventional mixing, dissolving, granulating, dragee-making, levigating, emulsifying, encapsulating, entrapping or tabletting processes.

Pharmaceutical compositions for use in accordance with the present invention thus may be formulated in conventional manner using one or more physiologically acceptable carriers comprising excipients and auxiliaries which facilitate processing of the active compounds into preparations which can be used pharmaceutically. Proper formulation is dependent upon the route of administration chosen. Any of the well-known techniques, carriers, and excipients may be used as suitable and as understood in the art.

Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl- cellulose, sodium carboxymethylcellulose, and/or polyvinylpyrrolidone (PVP). If desired, disintegrating agents may be added, such as the cross-linked polyvinyl pyrrolidone, agar, or alginic acid or a salt thereof such as sodium alginate.

The above described ingredients and different routes of manufacture are merely representative. Other materials as well as processing techniques and the like well known in the art can also be used.

The compounds of the present invention are bradykinin receptor antagonists, in particular selective bradykinin B1 receptor antagonists, consequently are useful in the treatment or prevention of disorders involving pain and inflammation comprising the administration to a mammal to be treated an effective amount of compounds of formula (I) of the present invention as such or as medicament.

The compounds would be effective in the treatment of somatic musculo-skeletal pain and disorders including bone and joint pain and disorders (e.g. arthritis, including rheumatoid and other types of inflammatory arthritis, osteoarthritis, spondylitis and infectious arthritis, arthritis due to gout or pseudogout, autoimmune and vasculitic joint disorders as systemic lupus erythematosus, polymyalgia rheumatica, polyarthritis nodosa, osteoporosis), low-back pain, repetitive motion disorders (e.g. tenosynovitis, tendonitis, epicondylitis, bursitis, ganglion cyst, carpal tunnel syndromes, trigger finger), myofascial pain syndrome and fibromyalgia. Compounds of the present invention can additionally be used to treat inflammatory skin disorders, such as psoriasis, dermatitis and eczema, skin injuries including burning and sunburning (e.g. UV-erythema and pain), pruritus and wound.

The compounds would be effective in the treatment of visceral pain and disorders of thorax and abdomen (angina, ulcerative colitis, pancreatitis, cholecystitis, liver disease, nephritis, gastritis, appendicitis, irritable bowel syndrome, inflammatory bowel disease, Crohn's disease), visceral pain of pelvis (cystitis, hyperactive bladder, menstruation). They may be used as smooth muscle relaxants for the treatment of spasm of the gastrointestinal tract or uterus as well as for spasms and pain originating from biliary and urinary tracts. Such compounds may be used therapeutically to treat inflammatory airways disease e.g. chronic obstructive pulmonary disease (COPD), acute respiratory distress syndrome (ADRS), bronchitis, pneumonia, pleurisy and asthma. They may be used to control, restrict or reverse airways hyperreactivity in asthma, to treat intrinsic and extrinsic asthma including allergic asthma (atopic or non-atopic), occupational asthma, viral or bacterial exacerbated asthma, other non-allergic asthmas, "wheezy-infant syndrome", as well as exercise-induced bronchoconstriction. They may be effective against pneumoconiosis, including aluminosis, anthracosis, asbestosis, chalicosis, ptilosis, siderosis, silicosis, tabacosis and byssinosis. Moreover the compounds may be used for the treatment of vascular injuries, disorders and inflammatory states such as angioedema, atherosclerosis, septic shock e.g. as anti-hypovolemic and/or anti-hypotensive agents, ischemia-reperfusion injury. Compounds may be used to treat inflammatory pain of varied origins (e.g. allergic rhinitis, vasomotor rhinitis, uveitis, retinitis, gingivitis,) atopic and allergic disorders. Compounds may be used to alleviate glaucoma, dental pain and fever.

Compounds of present invention would be useful in the treatment of neuropathic pain (e.g. neuralgia, nerve injury, phantom limb pain, mononeuropathy, polyneuropathy, neuritis, and radiculitis). Compounds would be effective in the treatment of painful peripheral neuropathies like herpes zoster infection (e.g. postherpetic neuralgia), HIV-related neuropathies, nutritional deficiencies, toxins. Compounds would be effective in the treatment of cancer pain and in side effect of chemotherapy, radiation injury or surgical injury. Compounds may be used in the treatment of fibrotic disease (e.g. pulmonary fibrosis, renal fibrosis, liver fibrosis), hyperplasia (e.g. bening prostatic hyperplasia) and cancer (e.g. breast cancer). The compounds would be effective in immunological disorders (autoimmune disease, hypersensitivities, transplant rejection, immune deficiencies). Compounds would be effective in the treatment of perioperative pain (e.g. general surgery, gynecological), postoperative pain (postsurgical pain syndrome), posttraumatic pain (e.g. sprains or fracture). Compounds would be also effective in the treatment of traumatic brain injuries. Compounds of present invention may be useful analgesic agent using during general and monitored anaesthesia. Furthermore compounds would be effective in the treatment of pain and disorders associated with diabetes (e.g. diabethic neuropathy, diabethic nephropathy, diabetic vasculopathy, diabetic rethinopathy, osteopathy, post capillary resistance or diabetic symptoms associated with insulitis (e.g. hyperglycemia, diuresis, proteinurea and increased nitrite and kallikrein urinary excretion).

Additionally, they may be effective in some neurological disorders, e.g. against multiple sclerosis, Alzheimer's disease, Parkinson's disease, epilepsy, stroke, cerebral oedema, headache including cluster headache, migraine including prophylactic and acute use, as well as closed head trauma.

### Biological evaluation

### Functional assay

### Assessment of antagonist potency at B1 receptors in vitro by measurement of cytosolic calcium ion concentration with a plate reader fluorimeter in cells expressing recombinant human B1 receptors

### Cell culture

Chinese hamster ovary (CHO) cells stably expressing recombinant human B1 (CHO-B1, Euroscreen) receptors were cultured in Dulbecco's Modified Eagle's Medium (DMEM) containing 10% Fetal Calf Serum (FCS), 100 U/ml penicillin, 0.1 mg/ml streptomycin, 0.25 µg/ml amphotericin B, 1% Minimum Essential Medium Eagle (MEM), non essential amino acid solution, 600 µg/ml G418. Cells were kept at 37°C in a humidified incubator in an atmosphere of 5% CO₂/95% air and were passaged 1:4 three times a week. Cells were plated at 1.5-2.5×10⁴ cell/well on standard 96-well microplates, measurements of cytosolic calcium ion concentration ([Ca²⁺]ᵢ) were carried out 1-2 days after cell plating.

### Fluorimetric measurement of cytosolic calcium concentration

Measurements of [Ca²⁺]ᵢ were carried out on CHO-B1 cells stably expressing human B1 receptors, respectively. Cells were grown in standard 96-well microplates and before the measurement were loaded with a fluorescent Ca²⁺-sensitive dye, fluo-4/AM (2 µM): after removing the culture medium the dye was added to the cells (dissolved in assay buffer: 145 mM NaCl, 5 mM KCl, 2 mM MgCl₂, 2 mM CaCl₂, 10 mM HEPES, 20 mM D-glucose, 2 mM probenecid, 100 µl/well) and cells were incubated at 37°C in a humidified incubator in an atmosphere of 5% CO₂/95% air for 40-120 min. To stop dye loading cells were washed twice with assay buffer. After washing, various concentrations of the test compounds (diluted in extracellular medium from a DMSO stock solution, final DMSO concentration was <0.1%) or buffer were added to each well depending on the experimental setup. After incubation at 37°C for 20-25 min. baseline and agonist-evoked changes of [Ca²⁺]ᵢ were measured column by column with a plate reader fluorimeter (Fluoroskan Ascent, Labsystems). Excitation and detection of emission was carried out from the bottom of the plate. Filters used for Fluo-4: excitation filter - 485 nm, emission filter - 538 nm. The whole measurement process was performed at 37°C and was controlled by custom software. Inhibitory potency of the test compounds was assessed by measuring the reduction in the agonist-evoked [Ca²⁺]ᵢ-elevation in the presence of different concentrations of the compounds. The agonist was LysDABK for CHO-B1 cells. Agonist was applied at an EC₈₀ concentration; the EC₈₀-values were derived from daily determined dose-response curves. Fluorescence data were expressed as ΔF/F (fluorescence change normalized to baseline). All treatments on a single plate were measured in multiple wells. Data from all wells with the same treatment were averaged and the average values were used for analysis. Inhibitory potency of a compound at a single concentration point was expressed as percent inhibition of the control agonist response. Sigmoidal concentration-inhibition curves were fitted to the data (derived from at least three independent experiments) and IC₅₀-values were determined as the concentration that produces half of the maximal inhibition caused by the compound.

The examined reference compounds measured in functional and binding tests are the following:
1) 4-{2-[(2,2-diphenyl-ethyl)-amino]-5-{4-[4-[(4-methyl-1-piperazinyl)-carbonyl]-1-piperidinyl]-sulfonyl}-benzoyl}-morpholine (NVP-SAA164, Br. J. Pharmacol.144 (2005) 889-899); Kᵢ 8 nM; IC₅₀: 33 nM;
2) (R)-*N*-[2,3-dihydro-2-oxo-5-(2-phenyl-ethyl)-1-propyl-1*H*-1,4-benzodiazepin-3-yl]-N'-{4-[4-(4-pyridinyl)-1-piperazinyl]-phenyl}-urea (J. Med. Chem. 46 (2003) 1803-1806); Kᵢ 0.59 nM; IC₅₀ 1.9 nM;
3) *N*-[4-(,4'-bipiperidin)-1'-ylphenyl]-N'-[(3R)-2,3-dihydro-5-(4-methyl-phenyl)-2-oxo-1-propyl-1*H*-1,4-benzodiazepin-3-yl]-urea (J. Med. Chem. 46 (2003) 1803-1806); Kᵢ 13.4 nM; IC₅₀ 64.5 nM

The Kᵢ and IC₅₀ data measured by us for the reference compounds are in good agreement with the data given in the literature.

In Table I compounds of this invention measured in functional assay are listed below.

**Table I**

| **Number of example** | **B1 func.** | **Number of example** | **B1 func.** |
|---|---|---|---|
| 1 | +++ | 102 | +++ |
| 2 | +++ | 103 | +++ |
| 3 | +++ | 104 | +++ |
| 4 | +++ | 105 | +++ |
| 5 | +++ | 106 | +++ |
| 6 | ++ | 107 | +++ |
| 7 | +++ | 108 | +++ |
| 8 | +++ | 109 | +++ |
| 9 | +++ | 110 | +++ |
| 10 | +++ | 111 | +++ |
| 11 | +++ | 112 | +++ |
| 12 | +++ | 113 | +++ |
| 13 | +++ | 114 | +++ |
| 14 | +++ | 115 | +++ |
| 15 | +++ | 116 | ++ |
| 16 | +++ | 117 | +++ |
| 17 | +++ | 118 | +++ |
| 18 | +++ | 119 | +++ |
| 19 | +++ | 120 | +++ |
| 20 | +++ | 121 | ++ |
| 21 | +++ | 122 | ++ |
| 22 | +++ | 123 | +++ |
| 23 | +++ | 124 | ++ |
| 24 | +++ | 125 | +++ |
| 25 | ++ | 126 | ++ |
| 26 | +++ | 127 | +++ |
| 27 | +++ | 128 | +++ |
| 28 | +++ | 129 | +++ |
| 29 | +++ | 130 | +++ |
| 30 | +++ | 131 | ++ |
| 31 | +++ | 132 | +++ |
| 32 | +++ | 133 | +++ |
| 33 | + | 134 | +++ |
| 34 | ++ | 135 | ++ |
| 35 | ++ | 136 | ++ |
| 36 | +++ | 137 | +++ |
| 37 | +++ | 138 | ++ |
| 38 | ++ | 139 | +++ |
| 39 | +++ | 140 | +++ |
| 40 | +++ | 141 | +++ |
| 41 | +++ | 142 | ++ |
| 42 | +++ | 143 | +++ |
| 43 | ++ | 144 | ++ |
| 44 | +++ | 145 | ++ |
| 45 | +++ | 146 | +++ |
| 46 | +++ | 147 | ++ |
| 47 | +++ | 148 | ++ |
| 48 | +++ | 149 | ++ |
| 49 | +++ | 150 | ++ |
| 50 | +++ | 151 | + |
| 51 | +++ | 152 | ++ |
| 52 | +++ | 153 | +++ |
| 53 | +++ | 154 | +++ |
| 54 | +++ | 155 | ++ |
| 55 | +++ | 156 | +++ |
| 56 | + | 157 | ++ |
| 57 | ++ | 158 | +++ |
| 58 | +++ | 159 | +++ |
| 59 | +++ | 160 | +++ |
| 60 | +++ | 161 | ++ |
| 61 | +++ | 162 | +++ |
| 62 | +++ | 163 | +++ |
| 63 | +++ | 164 | +++ |
| 64 | +++ | 165 | +++ |
| 65 | +++ | 166 | +++ |
| 66 | +++ | 167 | ++ |
| 67 | +++ | 168 | ++ |
| 68 | +++ | 169 | +++ |
| 69 | ++ | 170 | ++ |
| 70 | +++ | 171 | ++ |
| 71 | +++ | 172 | +++ |
| 72 | +++ | 173 | ++ |
| 73 | +++ | 174 | +++ |
| 74 | +++ | 175 | ++ |
| 75 | +++ | 176 | ++ |
| 76 | +++ | 177 | +++ |
| 77 | +++ | 178 | +++ |
| 78 | +++ | 179 | ++ |
| 79 | +++ | 180 | +++ |
| 80 | +++ | 181 | +++ |
| 81 | +++ | 182 | +++ |
| 82 | +++ | 183 | ++ |
| 83 | +++ | 184 | +++ |
| 84 | +++ | 185 | +++ |
| 85 | +++ | 186 | +++ |
| 86 | +++ | 187 | ++ |
| 87 | +++ | 188 | +++ |
| 88 | +++ | 189 | +++ |
| 89 | +++ | 190 | +++ |
| 90 | +++ | 191 | +++ |
| 91 | +++ | 192 | +++ |
| 92 | +++ | 193 | +++ |
| 93 | ++ | 194 | +++ |
| 94 | +++ | 195 | +++ |
| 95 | +++ | 196 | ++ |
| 96 | +++ | 197 | +++ |
| 97 | ++ | 198 | +++ |
| 98 | ++ | 199 | +++ |
| 99 | +++ | 200 | +++ |
| 100 | +++ | 201 | +++ |
| 101 | +++ | | |

| | | | |
|---|---|---|---|
| + IC₅₀ is between 0.1 and 0.5 µM ++ IC₅₀ is between 20 and 100 nM +++ IC₅₀ < 20 nM | | | |

### Receptor binding assays

### Assessment of ligand binding affinity to B1 and B2 receptors in vitro by competitive radioligand binding assay

### 1. Human recombinant bradykinin B1 receptor binding

Binding assays were carried out on human recombinant bradykinin1 receptors (expressed in CHO cells, hB1-A5) according to the Euroscreen Technical Data Sheet (Cat.No.:ES-091). 20µg protein/tube was incubated with [3,4-prolyl-3,4-³H(N)]-[Des-Arg¹⁰] Kallidin as radioligand. Non specific binding was determined in the presence of 10 µM Lys-des-Arg⁹-Bradykinin. The final incubation volume was 250 µl. Samples were incubated for 15 min. at 25 °C then were rapidly vacuum filtered through GF/B filters presoaked for at least 1 h in 0.5 % PEI. Radioactivity was determined by liquid scintillation spectroscopy.

The ligand displacement by the compounds was determined using a minimum of seven concentrations in triplicate, and experiments were repeated at least two times. The specific radioligand binding is defined as the difference between total binding and the non-specific binding determined in the presence of an excess of unlabelled ligand. Results are expressed as a percent inhibition of specific binding obtained in the presence of RGH-478 or reference drugs. IC₅₀ values (i.e. concentration of compound giving 50% inhibition of specific binding) were calculated from concentration-displacement curves by sigmoidal fitting using GraphPad Prism Software 4.0. Kᵢ values (i.e. inhibition constants) were calculated using the Cheng-Prusoff equation. K_{D} was determined from the Scatchard plot. (Editor-in-chief: Enna, S.J. and Williams, M. Current protocols in pharmacology vol.1. John Wiley & sons Inc., 1998)

In Table II compounds of this invention measured in binding assay are listed.

**Table II**

| **Number of example** | **B1 binding** | **Number of example** | **B1 binding** |
|---|---|---|---|
| 1 | +++ | 102 | +++ |
| 2 | +++ | 103 | +++ |
| 3 | +++ | 104 | +++ |
| 4 | +++ | 105 | +++ |
| 5 | +++ | 106 | +++ |
| 6 | ++ | 107 | +++ |
| 7 | +++ | 108 | +++ |
| 8 | +++ | 109 | +++ |
| 9 | +++ | 110 | +++ |
| 10 | +++ | 111 | +++ |
| 11 | +++ | 112 | +++ |
| 12 | +++ | 113 | +++ |
| 13 | +++ | 114 | +++ |
| 14 | +++ | 115 | +++ |
| 15 | +++ | 116 | ++ |
| 16 | +++ | 117 | +++ |
| 17 | +++ | 118 | +++ |
| 18 | +++ | 119 | +++ |
| 19 | +++ | 120 | +++ |
| 20 | +++ | 121 | +++ |
| 21 | +++ | 122 | ++ |
| 22 | +++ | 123 | +++ |
| 23 | +++ | 124 | ++ |
| 24 | +++ | 125 | +++ |
| 25 | +++ | 126 | +++ |
| 26 | +++ | 127 | +++ |
| 27 | +++ | 128 | +++ |
| 28 | +++ | 129 | +++ |
| 29 | +++ | 130 | +++ |
| 30 | +++ | 131 | +++ |
| 31 | +++ | 132 | +++ |
| 32 | +++ | 133 | +++ |
| 33 | ++ | 134 | +++ |
| 34 | +++ | 135 | +++ |
| 35 | +++ | 136 | ++ |
| 36 | +++ | 137 | +++ |
| 37 | +++ | 138 | +++ |
| 38 | +++ | 139 | +++ |
| 39 | +++ | 140 | +++ |
| 40 | +++ | 141 | +++ |
| 41 | +++ | 142 | +++ |
| 42 | +++ | 143 | +++ |
| 43 | +++ | 144 | ++ |
| 44 | +++ | 145 | ++ |
| 45 | +++ | 146 | +++ |
| 46 | +++ | 147 | + |
| 47 | +++ | 148 | +++ |
| 48 | +++ | 149 | +++ |
| 49 | +++ | 150 | +++ |
| 50 | +++ | 151 | + |
| 51 | +++ | 152 | +++ |
| 52 | +++ | 153 | +++ |
| 53 | +++ | 154 | +++ |
| 54 | +++ | 155 | ++ |
| 55 | +++ | 156 | +++ |
| 56 | + | 157 | +++ |
| 57 | ++ | 158 | +++ |
| 58 | +++ | 159 | +++ |
| 59 | +++ | 160 | +++ |
| 60 | +++ | 161 | +++ |
| 61 | +++ | 162 | +++ |
| 62 | +++ | 163 | +++ |
| 63 | +++ | 164 | +++ |
| 64 | +++ | 165 | +++ |
| 65 | +++ | 166 | +++ |
| 66 | +++ | 167 | ++ |
| 67 | +++ | 168 | +++ |
| 68 | +++ | 169 | +++ |
| 69 | ++ | 170 | +++ |
| 70 | +++ | 171 | +++ |
| 71 | +++ | 172 | +++ |
| 72 | +++ | 173 | +++ |
| 73 | +++ | 174 | +++ |
| 74 | +++ | 175 | +++ |
| 75 | +++ | 176 | ++ |
| 76 | +++ | 177 | ++ |
| 77 | +++ | 178 | +++ |
| 78 | +++ | 179 | +++ |
| 79 | +++ | 180 | +++ |
| 80 | +++ | 181 | +++ |
| 81 | +++ | 182 | +++ |
| 82 | +++ | 183 | ++ |
| 83 | +++ | 184 | +++ |
| 84 | +++ | 185 | +++ |
| 85 | +++ | 186 | +++ |
| 86 | +++ | 187 | + |
| 87 | +++ | 188 | ++ |
| 88 | +++ | 189 | +++ |
| 89 | +++ | 190 | +++ |
| 90 | +++ | 191 | +++ |
| 91 | +++ | 192 | +++ |
| 92 | +++ | 193 | +++ |
| 93 | ++ | 194 | +++ |
| 94 | +++ | 195 | +++ |
| 95 | +++ | 196 | +++ |
| 96 | +++ | 197 | +++ |
| 97 | +++ | 198 | +++ |
| 98 | +++ | 199 | +++ |
| 99 | +++ | 200 | +++ |
| 100 | +++ | 201 | +++ |
| 101 | +++ | | |

| | | | |
|---|---|---|---|
| + Kᵢ is between 0.1 and 0.5 µM ++ Kᵢ is between 20 and 100 nM +++ Kᵢ < 20 nM | | | |

### 2. Human recombinant bradykinin B2 receptor binding

Binding assays were carried out on human recombinant bradykinin2 receptors (expressed in CHO-K1 cells) according to the Receptor Biology Technical Data Sheet (Cat.No.: RBHB2M) with minor modifications. 8.4 µg protein/tube was incubated with [2,3,-prolyl-3,4-³H(N)]-Bradykinin as radioligand. Non specific binding was determined in the presence of 5 µM bradykinin. The final incubation volume was 200 µl. Samples were incubated for 90 min. at +4 °C then were rapidly vacuum filtered through GF/B filters presoaked for at least 1 h in 0.5 % PEI. Radioactivity was determined by liquid scintillation spectroscopy. Compounds were tested in 5 µM test concentration.

The specific radioligand binding is defined as the difference between total binding and the non-specific binding determined in the presence of an excess of unlabelled ligand. Results are expressed as a percent inhibition of specific binding obtained in the presence of RGH-478 or reference drugs. IC₅₀ values (i.e. concentration of compound giving 50% inhibition of specific binding) were calculated from concentration-displacement curves by sigmoidal fitting using GraphPad Prism Software 4.0. Kᵢ values (i.e. inhibition constants) were calculated using the Cheng-Prusoff equation. K_{D} was determined from the Scatchard plot. (Editor-in-chief: Enna, S.J. and Williams, M. Current protocols in pharmacology vol.1. John Wiley & sons Inc., 1998)

The compounds exhibited high affinity and selectivity (>50 fold) for the human B1 receptor over the human B2 receptor according to binding assays.

The present invention will be now illustrated by the following not limiting examples.

### Reference Example 1

### 3-Fluoro-4-(tert-butoxycarbonylaminomethyl)phenylboronic acid

### a) 4-Aminomethyl-3-fluoro-phenylboronic acid

A stirred mixture of 5 g (27.33 mmol) of 3-fluoro-4-(hydroxyimino-methyl)phenylboronic acid (WO2006/38100) and 0.6 g of 10% Pd/C in 80 mL of ethanol and 4.6 mL (54.7 mmol) of concentrated hydrochloric acid was hydrogenated at room temperature for 3 h. Initial exothermic period was controlled by the immersion of the reaction flask into a water bath. The reaction mixture was filtered through Celite and the filtrate concentrated *in vacuo.* The residue was triturated with a mixture of diethyl ether and ethyl acetate, the solid was filtered off, washed with diethyl ether (2x15 mL) and dried to yield 5.47 g (97 %) of the title compound as a white amorphous solid. MS (EI) 153.1 ([M-NH]⁺).

### b) 3-Fluoro-4-(tert-butoxycarbonylaminomethyl)phenylboronic acid

To a solution of 5 g (29.6 mmol) of 4-aminomethyl-3-fluoro-phenylboronic acid in 30 mL of water and 50 mL of tetrahydrofuran a solution of 6.7 g (30.5 mmol) of ditertbutyl-dicarbonate in 15 mL of tetrahydrofuran was added followed by the addition of 15.5 mL (88.8 mmol) of *N,N-*diisopropylethylamine. The reaction mixture was stirred at room temperature for 2 h. The pH of the mixture was adjusted to 6 at 0°C by the addition of 2M hydrochloric acid solution and the organic and aqueous phases were separated. The aqueous phase was washed with 3x20 mL of ethyl acetate, the combined organic layers were washed with 10 mL of water and 2x10 mL of brine, dried over anhydrous sodium sulfate, filtered and concentrated *in vacuo.* The residue was triturated with hexane and stirred for 2 h. The solid was filtered off and dried to yield 5.44 g (68 %) of the title compound as a yellow oil. MS (EI) 292.1 [M+Na]⁺.

### Reference Example 2

### (R)-4-(1-tert-Butoxycarbonylamino-ethyl)phenylboronic acid

Under argon to a solution of 119.74 g (400 mmol) of [(*R*)-1-(4-bromo-phenyl)-ethyl]-carbamic acid *tert*-butyl ester in 1500 mL of dry tetrahydrofuran 800 mL of 2.5 M n-BuLi in hexane solution (2 mol) was added dropwise at -78°C over a period of 1.5 h. After the mixture was stirred at -78°C for 20 min, 125 mL (545 mmol) of triisopropyl borate was added dropwise over a period of 0.5 h. The so obtained mixture was allowed to warm to -20°C and stirred at this temperature for 18 h. The reaction mixture was quenched by addition of 500 mL of saturated ammonium chloride solution (pH ∼8) and allowed to warm to room temperature. The reaction mixture was extracted with ethyl acetate, the combined organic layer was washed with water, dried over anhydrous sodium sulfate, filtered and concentrated *in vacuo.* The residue was stirred with 500 mL of n-hexane overnight, then filtered and dried to yield 65.75 g (62%) of the title compound as a white solid. MS (EI) 288.2 [M+Na]⁺.

### Reference Example 3

### 6-(Tert-butoxycarbonylamino-methyl)-3-pyridineboronic acid

### a) Pinacol 6-(tert-butoxycarbonylamino-methyl)-3-pyridineboronate

To a solution of 8.7 g (38.7 mmol, dihydrochloride) of pinacol 6-(aminomethyl)-3-pyridineboronate (Bioorg. Med. Chem. Lett., 2006, 16, 1277-81) in 45 mL of water and 135 mL of tetrahydrofuran a solution of 8.5 g (38.9 mmol) of ditertbutyl-dicarbonate in 10 mL of tetrahydrofuran was added followed by the addition of 27 mL (155 mmol) of *N*,*N*-diisopropylethylamine. The reaction mixture was stirred at room temperature overnight. The organic and aqueous phases were separated, the aqueous phase was washed with 3x160 mL of ethyl acetate. The combined organic layers were washed with 80 mL of water and 80 mL of brine, dried over anhydrous sodium sulfate, filtered and concentrated *in vacuo* to yield 9.64 g (74 %) of the title compound as a yellow oil. MS (EI) 253.2 (boronic acid deriv.[252.07]H⁺).

### b) 6-(Tert-butoxycarbonylamino-methyl)-3-pyridineboronic acid

To a solution of 9.64 g (26.26 mmol) of pinacol 6-(*tert*-butoxycarbonylamino-methyl)-3-pyridineboronate in 190 mL of acetone a solution of 5.63 g (73 mmol) of ammonium acetate in 90 mL of water was added, followed by the addition of 18.8 g (87.8 mmol) of sodium periodate. The suspension was stirred at room temperature for 3 h and diluted with 180 mL of ethyl acetate. The liquid phases were decanted from the undissolved residue and the process was repeated with 20 mL of ethyl acetate. The organic and aqueous phases were separated, the aqueous phase was washed with 2x10 mL of ethyl acetate. The combined organic layers were washed with 3x30 mL of water, 2x50 mL of brine, dried over anhydrous sodium sulfate, filtered and concentrated *in vacuo.* The residue was taken up in 90 mL of water and the pH of the suspension was adjusted to 6 by the addition of 1M aqueous sodium hydroxide solution and the so obtained mixture was washed with 150 mL of ethyl acetate. The organic layer was washed with 2x50 mL of water and 2x50 mL of brine, dried over anhydrous sodium sulfate, filtered and concentrated *in vacuo.* The yellow foam obtained was triturated with a mixture of hexane and diethyl ether and the suspension was stirred overnight. The solid was filtered off, washed with hexane and dried to yield 3.55 g (48 %) of the title compound as an off-white solid. MS (EI) 253.2 (MH⁺).

### Reference Example 4

### 1-Amino-cyclopropanecarboxylic acid 4-[3-chloro-2-(2-methyl-2H-tetrazol-5-yl)-indol-1-yl]-benzylamide hydrochloride

### a) 3-Chloro-1H-indole-2-carboxylic acid

A mixture of 40.0 g (0.248 mol) of 1*H*-indole-2-carboxylic acid, 33.6 g (0.251 mol) of *N*-chlorosuccinimide in 800 mL of acetonitrile was refluxed for 5 h. The reaction mixture was cooled, concentrated *in vacuo* and the residue was stirred with 500 mL of water. The precipitated product was filtered off, washed with water and dried to yield 42.21 g (87.0 %) of the title compound. Mp: 192-193 °C.

### b) 3-Chloro-1H-indole-2-carboxylic acid amide

A mixture of 42.2 g (0.215 mol) of 3-chloro-1*H*-indole-2-carboxylic acid, 42.2 mL (0.580 mol) of thionyl chloride, 0.17 mL of *N*,*N*-dimethylformamide in 710 mL of chloroform was refluxed for 3 h. The reaction mixture was cooled to 20 °C, poured into a mixture of 420 mL of 25 % ammonia solution and 1500 g of ice, then stirred for 2h. The precipitated product was filtered off and washed with water to yield 25.29 g (60.2 %) of the title compound. Mp.: 191-192°C.

### c) 3-Chloro-1H-indole-2-carbonitrile

A mixture of 28.05 g (144 mmol) of 3-chloro-1*H*-indole-2-carboxylic acid amide, 67.36 mL (722 mmol) of phosphorus oxychloride and 525 mL of chloroform was refluxed for 3h. The reaction mixture was cooled to 20 °C, poured into a mixture of 40 mL of 37 % hydrochloric acid and 1000 g of ice, then stirred for 2 h. The organic layer was separated and the water phase was extracted with 500 mL of chloroform. The combined organic layers were washed with water and saturated sodium carbonate solution, dried over anhydrous sodium sulfate and concentrated. The residue was crystallized from a 1:1 mixture of 2-propanol and water to yield 20.89 g (82.1 %) of the title compound. Mp.: 150-154°C.

### d) 3-Chloro-2-(2-methyl-2H-tetrazol-5-yl)-1H-indole

A mixture of 23.31 g (132 mmol) of 3-chloro-1*H*-indole-2-carbonitrile, 9.32 g (143 mmol) of sodium azide and 7.95 g (148 mmol) of ammonium chloride in 240 mL of *N***,***N*-dimethylformamide was refluxed for 3.5 h. The reaction mixture was cooled to 20 °C, then 12.0 mL (192 mmol) of iodomethan was added and stirred for 16 h. The reaction mixture was poured into 500 mL of water, the precipitated product was filtered off and recrystallized from 2-propanol to yield 13.95 g (45.2 %) of the title compound. Mp.: 197-199°C.

### e) {4-[3-Chloro-2-(2-methyl-2H-tetrazol-5-yl)-indol-1-yl]-benzyl}-carbamic acid tert-butyl ester

A mixture of 5.85 g (25 mmol) of 3-chloro-2-(2-methyl-2*H*-tetrazol-5-yl)-1*H-*indole, 9.4 g (37.4 mmol) of 4-(*tert*-butoxycarbonylaminomethyl)phenylboronic acid, 7.6 g (50 mmol) of copper(II) acetate, 17.6 mL (100 mmol) of *N,N-*diisopropylethylamine, 12.2 g of 3Å molecular sieves in 200 mL of *N*,*N-*dimethylformamide was vigorously stirred at room temperature with air bubbling for 24 h, and additional 2.0 g (7.9 mmol) of 4-(*tert*-butoxycarbonylaminomethyl)-phenylboronic acid was added. The mixture was stirred at room temperature with air bubbling for 48 h, then filtered through Celite. The filtrate was *concentrated in vacuo.* The residue was submitted to flash column chromatography using Kieselgel 60 (0.015-0.040 mm) as adsorbent (Merck) and hexane:ethyl acetate = 4:1 as eluent to yield 11.8 g of the title compound as a yellow oil.

### f) 4-[3-Chloro-2-(2-methyl-2H-tetrazol-5-yl)-indol-1-yl]-benzylamine hydrochloride

The previously obtained product (∼25 mmol) was dissolved in 60 mL of 10 % hydrogen chloride in ethyl acetate and stirred at room temperature for 16 h. The precipitated product was filtered off and washed with diethyl ether to yield 9.34 g (99%) of the title compound. Mp.: 225-228 °C.

### g) 1-{4-[3-Chloro-2-(2-methyl-2H-tetrazol-5-yl)-indol-1-yl]-benzylcarbamoyl}-cyclopropyl)-carbamic acid tert-butyl ester

A mixture of 6.0 g (16 mmol) of 4-[3-chloro-2-(2-methyl-2*H*-tetrazol-5-yl)-indol-1-yl]-benzylamine hydrochloride, 3.3 g (16.4 mmol) of 1-*tert-*butoxycarbonylamino-cyclopropanecarboxylic acid, 4.8 mL (34.5 mmol) of triethylamine, 6.3 g (16.6 mmol) of HBTU [O-benzotriazol-1-yl-*N*,*N*,*N*',*N*'-tetramethyluronium hexafluorophosphate] and 75 mL of *N*,*N***-**dimethylformamide was stirred at room temperature for 24 h and concentrated *in vacuo.* The residue was submitted to flash column chromatography using Kieselgel 60 (0.015-0.040 mm) as adsorbent (Merck) and toluene:methanol = 4:1 as eluent to yield 6.42 g of the title compound as a yellow oil.

### h) 1-Amino-cyclopropanecarboxylic acid 4-[3-chloro-2-(2-methyl-2H-tetrazol-5-yl)-indol-1-yl]-benzylamide hydrochloride

The previously obtained product (∼16 mmol) was dissolved in 50 mL of 10 % hydrogen chloride in ethyl acetate and stirred at room temperature for 6 h. The reaction mixture was *concentrated in vacuo,* the residue was crystallized from diethyl ether to yield 4.22 g (62.5 %) of the title compound. Mp.: 70-80 °C.

### Reference Example 5

### 4-[2-(2-Methyl-2H-tetrazol-5-yl)-indole-yl]-benzylamine hydrochloride

### a) 2-(2H-Tetrazol-5-yl)-1H-indole

A mixture of 1.0 g (7.04 mmol) of 1*H*-indole-2-carbonitrile (I. Borza at al. Bioorg. Med. Chem. Lett. 2005, 15, 5439-5441), 1.5 g (7.28 mmol) of trimethyltin azide, and 50 mL of toluene was refluxed for 3 h. The reaction mixture was cooled to 20 °C and the precipitated product was filtered off to yield 2.22 g of tin complex of the title compound. Mp.: 236-240 °C.

### b) 4-[2-(2H-Tetrazol-5-yl)-indole-yl]-benzaldehyde

A mixture of 0.4 g (1.4 mmol) of 2-(2*H*-tetrazol-5-yl)-1*H*-indole, 0.31 g (2.06 mmol) of 4-formyl-phenylboronic acid, 0.42 g (2.77 mmol) of copper(II) acetate, 0.98 mL (5.6 mmol) of *N*,*N*-diisopropylethylamine, 0.7 g 3Å molecular sieves in 15 mL of *N*,*N*-dimethylformamide was vigorously stirred at room temperature with air bubbling for 14 h. The mixture was filtered through Celite. The filtrate was concentrated *in vacuo.* The residue was submitted to flash column chromatography using Kieselgel 60 (0.015-0.040 mm) as adsorbent (Merck) and chloroform:methanol:acetic acid = 10:1:0.1 as eluent to yield after crystallization from 2-propanol 0.12 g (19 %) of the title compound. Mp.: 210-212 °C.

### c) 4-[2-(2-Methyl-2H-tetrazol-5-yl)-indole-yl]-benzaldehyde

A mixture of 0.7 g (2.4 mmol) of 4-[2-(2*H*-tetrazol-5-yl)-indole-yl]-benzaldehyde, 0.2 mL (3.2 mmol) of iodomethane, 1.0 g (7.2 mmol) of potassium carbonate, and 50 mL of acetonitrile was refluxed for 0.5 h. The reaction mixture was cooled to 20 °C, filtered, and the filtrate was concentrated *in vacuo.* The residue was submitted to flash column chromatography using Kieselgel 60 (0.015-0.040 mm) as adsorbent (Merck) and toluene:methanol = 4:1 as eluent to yield 0.238 g (32.4 %) of the title compound (R_{f}: 0.8), and 0.21 g (28.6 %) of 4-[2-(1-methy-2*H*-tetrazol-5-yl)-indole-yl]-benzaldehyde (R_{f}: 0.7).

### d) 4-[2-(2-Methly-2H-tetrazol-5-yl)-indole-yl]-benzaldehyde oxime

A mixture of 0.238 g (0.78 mmol) of 4-[2-(2-methyl-2*H*-tetrazol-5-yl)-indole-yl]-benzaldehyde, 0.07 g (1.0 mmol) of hydroxylamine hydrochloride, 0.09 mL (1.1 mmol) of pyridine and 10 mL of ethanol was refluxed for 1 h. The reaction mixture was cooled to 20 °C, concentrated *in vacuo* and the residue was treated with water. The precipitated product was filtered off to yield 0.19 g (75.6%) of the title compound. Mp.: 159-161 °C.

### e) 4-[2-(2-Methyl-2H-tetrazol-5-yl)-indole-yl]-benzylamine hydrochloride

A mixture of 0.19 g (0.59 mmol) of 4-[2-(2-methlyl-2*H*-tetrazol-5-yl)-indole-yl]-benzaldehyde oxime, 20 mL of methanol, 1 mL of 36 % hydrochloric acid and 0.05 g of 10 % Pd/C catalyst was hydrogenated for 6 h. The catalyst was filtered off, the filtrate was concentrated *in vacuo* and the residue was treated with diethylether to yield 0.192 g (94.5 %) of the title compound. Mp.: 289-290 °C.

### Reference Example 6

### 4-[3-Chloro-2-(2-methyl-2H-tetrazol-5-yl)-indol-1-yl]-2-fluoro-benzylamine hydrochloride

The title compound was prepared from 3-chloro-2-(2-methyl-2*H*-tetrazol-5-yl)-1*H*-indole (Reference Example 4d) and 3-fluoro-4-(*tert*-butoxycarbonylamino-methyl)phenyl-boronic acid (Reference Example 1) according to the methods described in Reference Example 4e and 4f. Mp.: 268-269 °C.

### Reference Example 7

### 4-[3-Chloro-2-(5-methyl-[1,3,4]oxadiazol-2-yl)-indol-1-yl]-benzylamine hydrochloride

### a) 3-Chloro-1H-indole-2-carboxylic acid N'-acetyl-hydrazide

A mixture of 1.5 g (0.76 mmol) of 3-chloro-1*H*-indole-2-carboxylic acid (Reference Example 4a), 0.6 g (0.81 mmol) of acethydrazide, 1.2 mL (0.86 mmol) of triethylamine, 3.0 g (0.79 mmol) of HBTU and 45 mL of *N*,*N*-dimethylformamide was stirred at room temperature for 24 h, then concentrated *in vacuo.* The residue was submitted to flash column chromatography using Kieselgel 60 (0.015-0.040 mm) as adsorbent (Merck) and toluene:methanol = 4:1 as eluent to yield after crystallization from 2-propanol 1.1 g (57.0 %) of the title compound. Mp.: 253-255 °C.

### b) 3-Chloro-2-(5-methyl-[1,3,4]oxadiazol-2-yl)-1H-indole

A mixture of 1.1 g (4.37 mol) of 3-chloro-1*H*-indole-2-carboxylic acid *N*'-acetyl-hydrazide and 13.19 mL of phosphorus oxychloride was refluxed for 1 h. The reaction mixture was cooled to 20 °C, poured into 100 g of ice, stirred for 2h, then extracted with ethyl acetate. The combined organic layers were washed with water and saturated sodium carbonate solution, dried over anhydrous sodium sulfate and *concentrated in vacuo.* The residue was submitted to flash column chromatography using Kieselgel 60 (0.015-0.040 mm) as adsorbent (Merck) and toluene:methanol = 4:1 as eluent to yield after crystallization from diethyl ether 0.38 g (37.0 %) of the title compound. Mp.: 267-268 °C.

### c) 4-[3-Chloro-2-(5-methyl-[1,3,4]oxadiazol-2-yl)-indol-1-yl]-benzylamine hydrochloride

The title compound was prepared from 3-chloro-2-(5-methyl-[1,3,4]oxadiazol-2-yl)-1*H*-indole according to the methods described in Reference Example 4e and 4f. Mp.: 265-272 °C.

### Reference Example 8

### 4-[2-(1-Methyl-2H-tetrazol-5-yl)-indole-yl]-benzylamine hydrochloride

The title compound was prepared from 4-[2-(1-methy-2*H*-tetrazol-5-yl)-indole-yl]-benzaldehyde (Reference Example 5c) according to the methods described in Reference Example 5d and 5e. Mp.: 91-107 °C.

### Reference Example 9

### 1-Amino-cyclopropanecarboxylic acid 4-[3-chloro-2-(5-methyl-[1,2,4]oxadiazol-3-yl)-indol-1-yl]-benzylamide

### a) 3-Chloro-N-hydroxy-1H-indole-2-carboxamidine

A mixture of 14.95 g (84.65 mmol) of 3-chloro-1*H*-indole-2-carbonitrile (Reference Example 4c), 7.65 g (110.04 mmol) of hydroxylamine hydrochloride, 11.38 g (135.44 mmol) of sodium hydrogencarbonate and 440 mL of methanol was stirred at room temperature for 24 h. The reaction mixture was poured into 300 mL of water and stirred for 1 h, the precipitated product was filtered off, washed with water and dried to yield 17.19 g (96.8%) of the title compound as a white powder.

### b) 3-Chloro-2-(5-methyl-[1,2,4]oxadiazol-3-yl)-1H-indole

A mixture of 15.19 g (72.46 mmol) of 3-chloro-*N*-hydroxy-1*H*-indole-2-carboxamidine, 306 mL of acetic acid and 17.07 mL (180 mmol) of acetic anhydride was stirred at 90°C for 10 h. The reaction mixture was concentrated *in vacuo* and the residue was recrystallized from a 1:1 mixture of methanol and water to yield the crude product, which was recrystallized from 2-propanol to yield 12.29 g (72.6%) of the title compound.

### c) 4-[3-Chloro-2-(5-methyl-[1,2,4]oxadiazol-3-yl)-indol-1-yl]-benzylamine hydrochloride

To a stirred mixture of 10.0 g (42.7 mmol) of 3-chloro-2-(5-methyl-[1,2,4]oxadiazol-3-yl)-1*H*-indole, 21.49 g (85.5 mmol) of 4-(*tert*-butoxycarbonylamino-methyl)phenylboronic acid and 12.98 g (85.5 mmol) of copper(II) acetate in 520 mL of DMSO 29.8 mL (310 mmol) of *N*,*N*-diisopropylethylamine and 20 g of 3 Å molecular sieves were added. The reaction mixture was vigorously stirred at room temperature with air bubbling for 6 days. The mixture was filtered through Celite. The filtrate was diluted with 1000 mL of ethyl acetate and washed with 2 x 500 mL of 25% ammonium hydroxide solution. The organic layer was washed with 500 mL of 1M citric acid, dried over anhydrous sodium sulfate, filtered and concentrated *in vacuo.* The residue was dissolved in 350 mL of 10 % hydrogen chloride in ethyl acetate and stirred at room temperature for 2 h. The precipitated crude product was filtered off, washed with cold ethyl acetate and recrystallized from 2-propanol to yield 14.2 g (88.7%) of the title compound.

### d) (1-{4-[3-Chloro-2-(5-methyl-[12,4]oxadiazol-3-yl)-indol-1-yl]-benzylcarbamoyl}-cyclopropyl)-carbamic acid tert-butyl ester

A mixture of 14.0 g (37.3 mmol) of 4-[3-chloro-2-(5-methyl-[1,2,4]oxadiazol-3-yl)-indol-1-yl]-benzylamine hydrochloride, 200 mL of DMSO, 9.0 g (44.8 mmol) of 1-*tert*-butoxycarbonylamino-cyclopropanecarboxylic acid, 19.0 g (44.8 mmol) of HBTU and 13.0 mL (74.6 mmol) of *N*,*N*-diisopropylethylamine was stirred at room temperature for 2 h. The reaction mixture was poured into 2000 mL of water and stirred for 1 h. The precipitated product was filtered off, washed with water and recrystallized from a 1:1 mixture of 2-propanol and water to yield 13.2 g (67.7%) of the title compound.

### e) 1-Amino-cyclopropanecarboxylic acid 4-[3-chloro-2-(5-methyl-[1,2,4]oxadiazol-3-yl)-indol-1-yl]-benzylamide

A mixture of 29.5 g (56.5 mmol) of (1-{4-[3-chloro-2-(5-methyl-[1,2,4]oxadiazol-3-yl)-indol-1-yl]-benzylcarbamoyl}-cyclopropyl)-carbamic acid *tert-*butyl ester and 450 mL of 10 % hydrogen chloride in ethyl acetate was stirred at room temperature for 12 h. The reaction mixture was cooled and 500 mL of 20% sodium hydroxide solution was added. After stirring for 20 min the organic layer was separated, dried over anhydrous sodium sulfate, filtered and concentrated *in vacuo* to yield 23.5 g (98%) of the title compound. MS (EI) 422.2 (MH⁺).

### Reference Example 10

### 4-[3-Chloro-5-fluoro-2-(2-methyl-2H-tetrazol-5-yl)-indol-1-yl]-benzylamine hydrochloride

The title compound was prepared from 5-fluoro-1*H*-indole-2-carboxylic acid according to the methods described in Reference Example 4a-f. Mp.: 216-220 °C.

### Reference Example 11

### 4-[3,5-Dichloro-2-(2-methyl-2H-tetrazol-5-yl)-indol-1-yl]-benzylamine hydrochloride

The title compound was prepared from 5-chloro-1*H*-indole-2-carboxylic acid according to the methods described in Reference Example 4a-f. Mp.: 267-270 °C.

### Reference Example 12

### 4-[3-Chloro-5-fluoro-2-(2-methyl-2H-tetrazol-5-yl)-indol-1-yl]-2-fluoro-benzylamine hydrochloride

The title compound was prepared from 5-fluoro-1*H*-indole-2-carboxylic acid and 3-fluoro-4-(*tert*-butoxycarbonylaminomethyl)phenylboronic acid (Reference Example 1) according to the methods described in Reference Example 4a-f. Mp.: 176-178 °C.

### Reference Example 13

### 4-[3,5-Dichloro-2-(2-methyl-2H-tetrazol-5-yl)-indol-1-yl]-2-fluoro-benzylamine hydrochloride

The title compound was prepared from 5-chloro-1*H*-indole-2-carboxylic acid and 3-fluoro-4-(*tert*-butoxycarbonylaminomethyl)phenylboronic acid (Reference Example 1) according to the methods described in Reference Example 4a-f. Mp.: 186-189 °C.

### Reference Example 14

### 1-(4-Aminomethyl-phenyl)-3-chloro-1H-indole-2-carbonitrile hydrochloride

### a) [4-(3-Chloro-2-cyano-indol-1-yl)-benzyl]-carbamic acid tert-butyl ester

To a stirred mixture of 0.353 g (2.0 mmol) of 3-chloro-1*H*-indole-2-carbonitrile (Reference Example 4c), 0.753 g (3 mmol) of 4-(*tert*-butoxycarbonylaminomethyl)phenylboronic acid and 0.606 g (4 mmol) of copper(II) acetate in 24 mL of DMSO 1.4 mL (8 mmol) of *N*,*N-*diisopropylethylamine was added. The reaction mixture was vigorously stirred at room temperature with air bubbling for 6 days. The mixture was diluted with 60 mL of ethyl acetate and washed with 2 x 50 mL of 25% ammonium hydroxide solution. The organic layer was washed with 50 mL of 1M citric acid, dried over anhydrous sodium sulfate, filtered and concentrated *in vacuo.* The residue was submitted to column chromatography using Kieselgel 60 (0.015-0.040 mm) as adsorbent (Merck) and hexane:ethyl acetate = 4:1 as eluent to yield 0428 g (56 %) of the title compound. MS (EI) 404.1 [M+Na]⁺.

### b) 1-(4-Aminomethyl-phenyl)-3-chloro-1H-indole-2-carbonitrile hydrochloride

To a mixture of 0.428 g (1.121 mmol) of [4-(3-chloro-2-cyano-indol-1-yl)-benzyl]-carbamic acid *tert*-butyl ester in 12 mL of ethyl acetate 24 mL of 10 % hydrogen chloride in ethyl acetate was added and the reaction mixture was stirred at room temperature for 2 h. The mixture was diluted with diethyl ether, the precipitated product was filtered off, washed with diethyl ether and dried to yield 0.32 g (90%) of the title compound. MS (EI) 282.1 (MH⁺).

### Reference Example 15

### (R)-1-{4-[3-Chloro-2-(5-methyl-[1,2,4]oxadiazol-3-yl)-indol-1-yl]-phenyl}-ethylamine hydrochloride

### a) (R)-(1-{4-[3-Chloro-2-(5-methyl-[1,2,4]oxadiazol-3-yl)-indol-1-yl]-phenyl}-ethyl)-carbamic acid tert-butyl ester

The title compound was prepared from 3-chloro-2-(5-methyl-[1,2,4]oxadiazol-3-yl)-1*H*-indole (Reference Example 9b) and (*R*)-4-(1-*tert*-butoxycarbonylamino-ethyl)-phenylboronic acid (Reference Example 2) according to the method described in Example 1e. MS (EI) 475.2 [M+Na]⁺.

### b) (R)-1-{4-[3-Chloro-2-(5-methyl-[1,2,4]oxadiazol-3-yl)-indol-1-yl]-phenyl}-ethylamine hydrochloride

The title compound was prepared from (*R*)-(1-{4-[3-chloro-2-(5-methyl-[1,2,4]oxadiazol-3-yl)-indol-1-yl]-phenyl}-ethyl)-carbamic acid *tert*-butyl ester according to the method described in Reference Example 14b. MS (EI) 353.1 (MH⁺).

### Reference Example 16

### 1-(4-Aminomethyl-phenyl)-5-chloro-1H-indole-2-carbonitrile hydrochloride

### a) [4-(5-Chloro-2-cyano-indol-1-yl)-benzyl]-carbamic acid tert-butyl ester

The title compound was prepared from 5-chloro-1*H*-indole-2-carbonitrile (I. Borza at al. Bioorg. Med. Chem. Lett. 2005, 15, 5439-5441) according to the method described in Reference Example 14a. MS (EI) 404.1 [M+Na]⁺.

### b) 1-(4-Aminomethyl-phenyl)-5-chloro-1H-indole-2-carbonitrile hydrochloride

The title compound was prepared from [4-(5-chloro-2-cyano-indol-1-yl)-benzyl]-carbamic acid *tert*-butyl ester according to the method described in Reference Example 14b.

### Reference Example 17

### 1-Amino-cyclopropanecarboxylic acid 4-(2-cyano-5-methoxy-indol-1-yl)-benzylamide hydrochloride

### a) 1-(4-Aminomethyl-phenyl)-5-methoxy-1H-indole-2-carbonitrile hydrochloride

The title compound was prepared from 5-methoxy-1*H*-indole-2-carbonitrile (I. Borza at al. Bioorg. Med. Chem. Lett. 2005, 15, 5439-5441) according to the methods described in Reference Example 14a and 14b. MS (EI) 261. [(M-NH₂)]⁺.

### b) 1-Amino-cyclopropanecarboxylic acid 4-(2-cyano-5-methoxy-indol-1-yl)-benzylamide hydrochloride

The title compound was prepared from 1-(4-aminomethyl-phenyl)-5-methoxy-1*H*-indole-2-carbonitrile hydrochloride according to the method described in Reference Example 4g and 4h. MS (EI) 361.2 (MH⁺).

### Reference Example 18

### 1-Amino-cyclopropanecarboxylic acid 4-(2-cyano-5-fluoro-indol-1-yl)-benzylamide hydrochloride

### a) 1-(4-Aminomethyl-phenyl)-5-fluoro-1H-indole-2-carbonitrile hydrochloride

The title compound was prepared from 5-fluoro-1*H*-indole-2-carbonitrile (I. Borza at al. Bioorg. Med. Chem. Lett. 2005, 15, 5439-5441) according to the methods described in Reference Example 14a and 14b. MS (EI) 249.1 [(M-NH₂)]⁺.

### b) 1-Amino-cyclopropanecarboxylic acid 4-(2-cyano-5-fluoro-indol-1-yl)-benzylamide hydrochloride

The title compound was prepared from 1-(4-aminomethyl-phenyl)-5-fluoro-1*H-*indole-2-carbonitrile hydrochloride according to the methods described in Reference Example 4g and 4h. MS (EI) 349.2 (MH⁺).

### Reference Example 19

### 1-Amino-cyclopropanecarboxylic acid 4-[5-fluoro-2-(5-methyl-[1,2,4]oxadiazol-3-yl)-indol-1-yl]-benzylamide hydrochloride

### a) 5-Fluoro-2-(5-methyl-[1,2,4]oxadiazol-3-yl)-1H-indole

The title compound was prepared from 5-fluoro-1*H*-indole-2-carbonitrile (I.

Borza at al. Bioorg. Med. Chem. Lett. **2005,** *15*, 5439-5441) according to the methods described in Reference Example 9a and 9b.

### b) 1-Amino-cyclopropanecarboxylic acid 4-[5-fluoro-2-(5-methyl-[1,2,4]oxadiazol-3-yl)-indol-1-yl]-benzylamide hydrochloride

The title compound was prepared from 5-fluoro-2-(5-methyl-[1,2,4]oxadiazol-3-yl)-1*H*-indole according to the methods described in Reference Example 9c-e. MS (EI) 406.2 (MH⁺).

### Reference Example 20

### 1-Amino-cyclopropanecarboxylic acid 4-[3-chloro-5-methoxy-2-(3-methyl-[1,2,4]oxadiazol-5-yl)-indol-1-yl]-benzylamide hydrochloride

### a) 3-Chloro-5-methoxy-1H-indole-2-carboxylic acid

The title compound was prepared from 5-methoxy-1*H*-indole-2-carboxylic acid according to the method described in Reference Example 4a.

### b) 3-Chloro-5-methoxy-1H-indole-2-carboxylic acid methyl ester

To a stirred mixture of 24.63 g (109.3 mmol) of 3-chloro-5-methoxy-1*H*-indole-2-carboxylic acid in 1200 mL of methanol 20 mL of concentrated sulfuric acid was added and the reaction mixture was refluxed for 40h. The reaction mixture was concentrated to 300 mL and 38 g of sodium carbonate was added portion-wise to the mixture while it was cooled with ice-water. Then the mixture was diluted with 300 mL of ethyl acetate, the pH of the mixture was adjusted to 8 by addition of 40% sodium hydroxide solution. The phases were separated, the water phase was extracted with 3x100 mL of ethyl acetate, the combined organic layers were washed with water and dried over anhydrous magnesium sulfate, filtered and concentrated to yield 23.68 g (90.4%) of the title compound.

### c) 1-[4-(tert-Butoxycarbonylamino-methyl)-phenyl]-3-chloro-5-methoxy-1H-indole-2-carboxylic acid methyl ester

To a stirred mixture of 4.8 g (20 mmol) of 3-chloro-5-methoxy-1*H*-indole-2-carboxylic acid methyl ester, 7.53 g (30 mmol) of 4-(*tert*-butoxycarbonylamino-methyl)phenylboronic acid and 6.0 g (40 mmol) of copper(II) acetate in 150 mL of *N,N-*dimethylformamide 14 mL (80 mmol) of *N*,*N-*diisopropylethylamine and 10 g of 3 Å molecular sieves were added. The reaction mixture was vigorously stirred at room temperature with air bubbling for 6 days. The mixture was filtered through Celite. The filtrate was diluted with 500 mL of ethyl acetate and washed with 2 x 200 mL of 25% ammonium hydroxide solution. The organic layer was washed with 200 mL of water, dried over anhydrous magnesium sulfate, filtered and *concentrated in vacuo.* The residue was triturated with 2-propanol, the crystalline solid was filtered off and dried to yield 4.28 g (48%) of the title compound. MS (EI) 467.2 [M+Na]⁺.

### d) {4-[3-Chloro-5-methoxy-2-(3-methyl-[1,2,4]oxadiazol-5-yl)-indol-1-yl]-benzyl}-carbamic acid tert-butyl ester

A stirred mixture of 4.28 g (9.6 mmol) 1-[4-(*tert*-butoxycarbonylamino-methyl)-phenyl]-3-chloro-5-methoxy-1*H*-indole-2-carboxylic acid methyl ester, 1.07 g (14.4 mmol) *N*-hydroxy-acetamidine, 3.58 g (25.9 mmol) potassium carbonate and 100 mL of toluene was refluxed for 20 h, then filtered and the filtrate was concentrated to yield 4.23 g (94%) of the title compound. MS (EI) 491.1 [M+Na]⁺.

### e) 4-[3-Chloro-5-methoxy-2-(3-methyl-[1,2,4]oxadiazol-5-yl)-indol-1-yl]-benzylamine hydrochloride

The title compound was prepared from {4-[3-chloro-5-methoxy-2-(3-methyl-[1,2,4]oxadiazol-5-yl)-indol-1-yl]-benzyl}-carbamic acid *tert*-butyl ester according to the method described in Reference Example 14b. MS (EI) 352.1 [(M-NH₂)]⁺.

### f) (1-{4-[3-Chloro-5-methoxy-2-(3-methyl-[1,2,4]oxadiazol-5-yl)-indol-1-yl]-benzylcarbamoyl}-cyclopropyl)-carbamic acid tert-butyl ester

A mixture of 3.94 g (9.7 mmol) of 4-[3-chloro-5-methoxy-2-(3-methyl-[1,2,4]oxadiazol-5-yl)-indol-1-yl]-benzylamine hydrochloride, 2.93 g(14.7 mmol) of 1-*tert*-butoxycarbonylamino-cyclopropanecarboxylic acid, 4.43 g (11.4 mmol) of HATU [O-(7-azabenzotriazol-1-yl-*N*,*N*,*N*',*N*'-tetramethyluronium hexafluorophosphate], 7.6 g (43.7 mmol) of *N*,*N*-diisopropylethylamine, 70 mL of dichloromethane and 2 mL of *N*,*N-*dimethylformamide was stirred at room temperature for 1.5 h, then diluted with 100 mL of water and the phases were separated. The water phase was extracted with dichloromethane, the combined organic layers were dried over anhydrous magnesium sulfate, filtered and concentrated. The residue was triturated with 2-propanol, the crystalline solid was filtered off and dried to yield 4.22 g (79%) of the title compound. MS (EI) 574.2 [M+Na]⁺.

### g) 1-Amino-cyclopropanecarboxylic acid 4-[3-chloro-5-methoxy-2-(3-methyl-[1,2,4]oxadiazol-5-yl)-indol-1-yl]-benzylamide hydrochloride

The title compound was prepared from (1-{4-[3-chloro-5-methoxy-2-(3-methyl-[1,2,4]oxadiazol-5-yl)-indol-1-yl]-benzylcarbamoyl}-cyclopropyl)-carbamic acid *tert-*butyl ester according to the method described in Reference Example 14b. MS (EI) 452.2 (MH⁺).

### Reference Example 21

### 1-(4-Aminomethyl-phenyl)-3-chloro-5-methoxy-1H-indole-2-carbonitrile hydrochloride

### a) 3-Chloro-5-methoxy-1H-indole-2-carbonitrile

The title compound was prepared from 5-methoxy-1*H*-indole-2-carboxylic acid according to the methods described in Reference Example 4a-c.

### b) 1-(4-Aminomethyl-phenyl)-3-chloro-5-methoxy-1H-indole-2-carbonitrile hydrochloride

The title compound was prepared from 3-chloro-5-methoxy-1*H*-indole-2-carbonitrile according to the methods described in Reference Example 14a and 14b. MS (EI) 295.1 [(M-NH₂)]⁺.

### Reference Example 22

### 4-[5-Chloro-2-(5-methyl-[1,2,4]oxadiazol-3-yl)-indol-1-yl]-benzylamine hydrochloride

The title compound was prepared from 5-chloro-1*H*-indole-2-carbonitrile (I. Borza at al. Bioorg. Med. Chem. Lett. 2005, 15, 5439-5441) according to the methods described in Reference Example 9a-c. MS (EI) 322.1 [(M-NH₂)]⁺.

### Reference Example 23

### 1-(4-Aminomethyl-3-fluoro-phenyl)-3-chloro-1H-indole-2-carboxylic acid methyl ester hydrochloride

The title compound was prepared from 3-chloro-1*H*-indole-2-carboxylic acid methyl ester and 3-fluoro-4-(*tert*-butoxycarbonylaminomethyl)phenylboronic acid (Reference Example 1) according to the method described in Reference Example 9c. MS (EI) 333.1 (MH⁺).

### Reference Example 24

### 4-[3-Chloro-5-methoxy-2-(5-methyl-[1,2,4]oxadiazol-3-yl)-indol-1-yl]-benzylamine

### a) 5-Methoxy-1H-indole-2-carboxylic acid amide

To a suspension of 10 g (52.3 mmol) of 5-methoxy-1*H*-indole-2-carboxylic acid in 300 mL of dichloromethane and 1 mL of *N*,*N-*dimethylformamide 8.85 mL (104.6 mmol) of oxalyl chloride was added dropwise. The reaction mixture was stirred at room temperature for 3 h, than cooled in ice-bath and 56 mL of concentrated aqueous ammonium hydroxide solution was added slowly. The resulted mixture was stirred at room temperature overnight, the precipitated product was filtered off, washed with water and dried *in vacuo* to yield 9.8 g (98.5%) of the title compound. MS (EI) 191.1 (MH⁺).

### b) 3-Chloro-5-methoxy-1H-indole-2-carboxylic acid amide

To a stirred suspension of 9.9 g (52.3 mmol) of 5-methoxy-1*H*-indole-2-carboxylic acid amide in 500 mL of acetonitrile 7.68 g (57.5 mmol) of *N-*chlorosuccinimide was added. The reaction mixture was refluxed for 3 h, then cooled to room temperature. The separated crystalline solid was filtered off, washed with acetonitrile and recrystallized from 2-propanol to yield 7.96 g (68%) of the title compound. MS (EI) 225.1 (MH⁺).

### c) 3-Chloro-5-methoxy-1H-indole-2-carbonitrile

To a stirred suspension of 5.5 g (24.2 mmol) of 3-chloro-5-methoxy-1*H*-indole-2-carboxylic acid amide in 200 mL of 1,4-dioxane 17 mL (183 mmol) of phosphorus oxychloride was added. The reaction mixture was refluxed for 3 h. The resulted dark solution was cooled to room temperature and poured to crushed ice. The icy mixture was stirred for 30 min and extracted with dichloromethane. The combined organic phases were extracted with water, 10% aqueous sodium hydrogencarbonate solution and brine, dried over anhydrous magnesium sulfate, filtered and concentrated *in vacuo* to yield 3.33 g (66.6%) of the title compound. MS (EI) 207.1 (MH⁺).

### d) 3-Chloro-N-hydroxy-5-methoxy-1H-indole-2-carboxamidine

To a stirred solution of 2.2 g (10.6 mmol) of 3-chloro-5-methoxy-1*H*-indole-2-carbonitrile in 50 mL of ethanol a solution of 1.5 g (21.2 mmol) of hydroxylamine hydrochloride in 30 mL of water and a solution of 2.4 mL (17 mmol) of triethylamine in 20 mL of ethanol were added dropwise and simultaneously. The resulted mixture was refluxed 3 h, cooled to room temperature, then *concentrated in vacuo.* The residue was triturated with water, the crystalline solid was filtered off and the obtained crude product was recrystallized from a 1:1 mixture of 2-propanol and water to yield 1.6 g (63%) of the title compound. MS (EI) 241.1 (MH⁺).

### e) 3-Chloro-5-methoxy-2-(5-methyl-[1,2,4]oxadiazol-3-yl)-1H-indole

To a solution of 1.6 g (6.7 mmol) of 3-chloro-*N*-hydroxy-5-methoxy-1*H*-indole-2-carboxamidine in 40 mL of dichloromethane 2 mL (13.4 mmol) of *N*,*N-*dimethylacetamide dimethyl acetal was added and the reaction mixture was stirred at room temperature for 1 h. The reaction mixture was diluted with dichloromethane and extracted with water, dried over anhydrous magnesium sulfate, filtered through a plug of silica, the filter cake was washed with chloroform and the filtrate was concentrated *in vacuo.* The residue was recrystallized from ethyl acetate to yield 1.37 g (81%) of the title compound. MS (EI) 264.1 (MH⁺).

### f) {4-[3-Chloro-5-methoxy-2-(5-methyl-[1,2,4]oxadiazol-3-yl)-indol-1-yl]-benzyl}-carbamic acid tert-butyl ester

To a solution of 1 g (3.79 mmol) of 3-chloro-5-methoxy-2-(5-methyl-[1,2,4]oxadiazol-3-yl)-1*H*-indole in 50 mL of *N*,*N*-dimethylformamide 1.14 g (4.55 mmol) of 4-(*tert*-butoxycarbonylaminomethyl)phenylboronic acid, 1.2 g (7.58 mmol) of copper(II) acetate, 2.6 mL (15.2 mmol) of *N*,*N*-diisopropylethylamine and 2 g 3Å molecular sieves were added. The reaction mixture was vigorously stirred and bubbled with a stream of dry air at room temperature for 6 h. The mixture was filtered through Celite. The filter cake was washed with *N*,*N*-dimethylformamide and chloroform, the combined filtrates were concentrated *in vacuo.* The residue was taken up in chloroform and subsequently washed with concentrated aqueous ammonium hydroxide solution, water, 10% aqueous citric acid solution, water, saturated aqueous sodium hydrogencarbonate solution and brine, dried over anhydrous magnesium sulfate, filtered and concentrated *in vacuo.* The residue was submitted to flash column chromatography using Kieselgel 60 (0.040-0.063 mm) as absorbent (Merck) and hexane:ethyl acetate = 2:1 as eluent to yield 0.98 g (57.6%) of the title compound. MS (EI) 491.1 (M+Na)⁺.

### g) 4-[3-Chloro-5-methoxy-2-(5-methyl-[1,2,4]oxadiazol-3-yl)-indol-1-yl]-benzylamine

To an ice-cooled solution of 0.9 g (1.9 mmol) of {4-[3-chloro-5-methoxy-2-(5-methyl-[1,2,4]oxadiazol-3-yl)-indol-1-yl]-benzyl}-carbamic acid *tert*-butyl ester in 20 mL of dichloromethane 2 mL (27 mmol) of trifluoroacetic acid was added. The reaction mixture was allowed to warm to room temperature and stirred for 48 h. The reaction mixture was *concentrated in vacuo* and the residue was taken up dichloromethane and extracted with saturated aqueous sodium carbonate solution and brine, dried over magnesium sulfate, filtered and concentrated *in vacuo* to yield 0.467 g (67%) of the title compound. MS (EI) 369.1 (MH⁺).

### Reference Example 25

### 1-(4-Aminomethyl-phenyl)-3-chloro-4-fluoro-1H-indole-2-carboxylic acid methyl ester

### a) 3-Chloro-4-fluoro-1H-indole-2-carboxylic acid methyl ester

To a stirred suspension of 1.1 g (5.7 mmol) of 4-fluoro-1*H*-indole-2-carboxylic acid methyl ester in 60 mL of acetonitrile 0.836 g (6.26 mmol) of *N*-chlorosuccinimide was added. The reaction mixture was refluxed for 6 h, then cooled to room temperature. The separated crystalline solid was filtered off, washed with acetonitrile and dried *in vacuo.* The crude product was recrystallized from 2-propanol to yield 1.05g (81%) of the title compound.

### b) 1-[4-(tert-Butoxycarbonylamino-methyl)-phenyl]-3-chloro-4-fluoro-1H-indole-2-carboxylic acid methyl ester

To a solution of 0.75 g (3.3 mmol) of 3-chloro-4-fluoro-1*H*-indole-2-carboxylic acid methyl ester in 30 mL of *N*,*N-*dimethylformamide 1.1 g (4.4 mmol) of 4-(*tert-*butoxycarbonylaminomethyl)phenylboronic acid, 1.0 g (6.4 mmol) of copper(II) acetate, 2.2 mL (12.7 mmol) of *N*,*N*-diisopropylethylamine and 1.4 g of 3Å molecular sieves were added. The reaction mixture was vigorously stirred and bubbled with a stream of dry air at room temperature for 36 h. The mixture was filtered through Celite. The filter cake was washed with *N*,*N*-dimethylformamide and chloroform and the combined filtrates were concentarted *in vacuo.* The residue was taken up in chloroform and subsequently washed with concentrated aqueous ammonium hydroxide solution, water, 10% aqueous citric acid solution, water, saturated aqueous sodium hydrogencarbonate solution and brine, dried over anhydrous magnesium sulfate, filtered and concentrated *in vacuo.* The residue was submitted to flash column chromatography using Kieselgel 60 (0.040-0.063 mm) as absorbent and hexane: ethyl acetate = 2:1 as eluent to yield 0.695 g (49%) of the title compound. MS (EI) 453.2 (M+Na)⁺.

### c) 1-(4-Aminomethyl-phenyl)-3-chloro-4-fluoro-1H-indole-2-carboxylic acid methyl ester

To an ice-cooled solution of 0.18 g (0.416 mmol) of 1-[4-(*tert-*butoxycarbonylamino-methyl)-phenyl]-3-chloro-4-fluoro-1*H*-indole-2-carboxylic acid methyl ester in 10 mL of dichloromethane 0.8 mL (10.4 mmol) of trifluoroacetic acid was added. The reaction mixture was allowed to warm to room temperature and stirred for 1 h. The reaction mixture was *concentrated in vacuo* and the residue was taken up in dichloromethane and extracted with saturated aqueous sodium carbonate solution and brine, dried over magnesium sulfate, filtered and concentrated *in vacuo* to yield 0.138 g (100%) of the title compound.

### Reference Example 26

### 1-Amino-cyclopropanecarboxylic acid 4-[5-fluoro-2-(3-methyl-[1,2,4]oxadiazol-5-yl)-indol-1-yl]-benzylamide

### a) 5-Fluoro-2-(3-methyl-[1,2,4]oxadiazol-5-yl)-1H-indole

To a stirred solution of 2.70g (14.29 mmol) of 5-fluoro-1*H*-indole-2-carboxylic acid methyl ester in 150 mL of toluene 1.59 g (21.4 mmol) of *N*-hydroxy-acetamidine and 3.0 g (21.7 mmol) of potassium carbonate were added. The reaction mixture was refluxed for 24 h, then cooled and diluted with 60 mL of ethyl acetate and 60 mL of water. The mixture was separated and the organic layer was washed with water and brine, dried over anhydrous magnesium sulfate, filtered and *concentrated in vacuo* to yield 2.95 (97%) of the title compound as a white solid.

### b) {4-[5-Fluoro-2-(3-methyl-[1,2,4]oxadiazol-5-yl)-indol-1-yl]-benzyl}-carbamic acid tert-butyl ester

To a solution of 1.5 g (6.9 mmol) of 5-fluoro-2-(3-methyl-[1,2,4]oxadiazol-5-yl)-1*H*-indole in 80 mL of *N*,*N*-dimethylformamide, 2.6 g (10 mmol) of 4-(*tert-*butoxycarbonylaminomethyl)phenylboronic acid, 2.1 g (13.8 mmol) of copper(II) acetate, 4.8 mL (27.6 mmol) of *N*,*N*-diisopropylethylamine and 3 g of 3Å molecular sieves were added. The reaction mixture was vigorously stirred and bubbled with a stream of dry air at room temperature for 120 h. The mixture was filtered through Celite. The filter cake was washed with *N*,*N-*dimethylformamide and chloroform and the combined filtrates were *concentrated in vacuo.* The residue taken up in ethyl acetate and subsequently washed with concentrated aqueous ammonium hydroxide solution, water, 10% aqueous citric acid solution, water, saturated aqueous sodium hydrogencarbonate solution and brine, dried over anhydrous magnesium sulfate, filtered and concentrated *in vacuo.* The residue was submitted to flash column chromatography using Kieselgel 60 (0.040-0.063 mm) as absorbent and hexane: ethyl acetate as eluent to yield 1.456g (50%) of the title compound.

### c) (1-{4-[5-Fluoro-2-(3-methyl-[1,2,4]oxadiazol-5-yl)-indol-1-yl]-benzylcarbamoyl}-cyclopropyl)-carbamic acid tert-butyl ester

To a cooled solution of 1.456 g (3.45 mmol) of {4-[5-fluoro-2-(3-methyl-[1,2,4]oxadiazol-5-yl)-indol-1-yl]-benzyl}-carbamic acid *tert*-butyl ester in 40 mL of dichloromethane 3 mL (3.9 mmol) of trifluoroacetic acid was added. The reaction mixture was allowed to warm to room temperature and stirred for 20 h. The reaction mixture was *concentrated in vacuo* and the residue was taken up in 80 mL of dichloromethane and washed with saturated aqueous sodium carbonate solution and brine, dried over anhydrous magnesium sulfate and filtered. To the resulted stirred solution of 4-[5-fluoro-2-(3-methyl-[1,2,4]oxadiazol-5-yl)-indol-1-yl]-benzylamine 0.73 g (3.63 mmol) of 1-*tert*-butoxycarbonylamino-cyclopropanecarboxylic acid and 0.995 g (5.2 mmol) of EDC were added at 5°C. The reaction mixture was allowed to warm to room temperature and stirred overnight. The reaction mixture was washed with 15 mL of water, 2x15 mL of 5% aqueous citric acid solution, water, saturated aqueous sodium hydrogencarbonate solution and brine, dried over anhydrous magnesium sulfate, filtered and *concentrated in vacuo* to yield 1.54g (88%) of the title compound as a white foam.

### d) 1-Amino-cyclopropanecarboxylic acid 4-[5-fluoro-2-(3-methyl-[1,2,4]oxadiazol-5-yl)-indol-1-yl]-benzylamide

To a cooled solution of 1.43 g (2.83 mmol) of (1-{4-[5-fluoro-2-(3-methyl-[1,2,4]oxadiazol-5-yl)-indol-1-yl]-benzylcarbamoyl}-cyclopropyl)-carbamic acid *tert-*butyl ester in 50 mL of dichloromethane 6 mL (7.8 mmol) of trifluoroacetic acid was added. The reaction mixture was allowed to warm to room temperature and stirred for 3 h. The reaction mixture was concentrated *in vacuo,* the residue was taken up in dichloromethane and washed with saturated aqueous sodium carbonate solution and brine, dried over anhydrous magnesium sulfate, filtered and concentrated *in vacuo* to yield 1.06 g (92%) of the title compound.

### Reference Example 27

### 1-Amino-cyclopropanecarboxylic acid 4-[3,5-dichloro-2-(5-methyl-[1,2,4]oxadiazol-3-yl)-indol-1-yl]-benzylamide

### a) 4-[3,5-Dichloro-2-(5-methyl-[1,2,4]oxadiazol-3-yl)-indol-1-yl]-benzylamine

The title compound was prepared from 5-chloro-1*H*-indole-2-carboxylic acid according to the methods described in Reference Example 24a-g. MS (EI) 322.1 [(M-NH₂)]⁺.

### b) 1-Amino-cyclopropanecarboxylic acid 4-[3,5-dichloro-2-(5-methyl[1,2,4]oxadiazol-3-yl)-indol-1-yl]-benzylamide

The title compound was prepared from 4-[3,5-dichloro-2-(5-methyl-[1,2,4]oxadiazol-3-yl)-indol-1-yl]-benzylamine according to the methods described in Reference Example 9d and 9e. MS (EI) 422.2 (MH⁺).

### Reference Example 28

### 1-Amino-cyclopropanecarboxylic acid 4-[3-chloro-5-fluoro-2-(5-methyl-[1,2,4]oxadiazol-3-yl)-indol-1-yl]-benzylamide

### a) 4-[3-Chloro-5-fluoro-2-(5-methyl-[1,2,4]oxadiazol-3-yl)-indol-1-yl]-benzylamine

The title compound was prepared from 5-fluoro-1*H*-indole-2-carboxylic acid according to the methods described in Reference Example 24a-g. MS (EI) 357.1 (MH⁺).

### b) 1-Amino-cyclopropanecarboxylic acid 4-[3-chloro-5-fluoro-2-(5-methyl-[1,2,4]oxadiazol-3-yl)-indol-1-yl]-benzylamide

The title compound was prepared from 4-[3,5-dichloro-2-(5-methyl-[1,2,4]oxadiazol-3-yl)-indol-1-yl]-benzylamine according to the methods described in Reference Example 9d and 9e. MS (EI) 440.1 (MH⁺).

### Reference Example 29

### 1-Amino-cyclopropanecarboxylic acid 4-[5-methoxy-2-(5-methyl-[1,3,4]oxadiazol-2-yl)-indol-1-yl]-benzylamide

### a) 4-[5-Methoxy-2-(5-methyl-[1,3,4]oxadiazol-2-yl)-indol-1-yl]-benzylamine hydrochloride

The title compound was prepared from 5-methoxy-1*H*-indole-2-carboxylic acid according to the methods described in Reference Example 7a-c. MS (EI) 318.2 [(M-NH₂)]⁺.

### b) 1-Amino-cyclopropanecarboxylic acid 4-[5-methoxy-2-(5-methyl-[1,3,4]oxadiazol-2-yl)-indol-1-yl]-benzylamide

The title compound was prepared from 4-[5-methoxy-2-(5-methyl-[1,3,4]oxadiazol-2-yl)-indol-1-yl]-benzylamine hydrochloride according to the methods described in Reference Example 9d and 9e. MS (EI) 418.2 (MH⁺).

### Reference Example 30

### 1-Amino-cyclopropanecarboxylic acid 4-[5-methoxy-2-(3-methyl-[1,2,4]oxadiazol-5-yl)-indol-1-yl]-benzylamide

### a) 5-Methoxy-1H-indole-2-carboxylic acid methyl ester

A mixture of 9.6 g (0.050 mol) of 5-methoxy-1*H*-indole-2-carboxylic acid and 4.5 mL of concentrated sulfuric acid in 250 mL of methanol was refluxed for 6 h. The reaction mixture was cooled, *concentrated in vacuo* and 200 mL of water and 200 mL of dichloromethane was added to the residue. The organic layer was separated and the water phase was extracted with 100 mL of dichloromethane. The combined organic layers were washed with water and saturated sodium hydrogencarbonate solution, dried over sodium sulfate, concentrated and dried to yield 10.17 g (98.7 %) of the title compound. Mp: 178-181 °C.

### b) 5-Methoxy-2-(3-methyl-[1,2,4]oxadiazol-5-yl)-1H-indole

A mixture of 5.5 g (26.8 mmol) of 5-methoxy-1*H*-indole-2-carboxylic acid methyl ester, 3.0 g (40.5 mmol) of *N*-hydroxy-acetamidine and 10 g (72.3 mmol) of potassium carbonate in 150 mL of toluene was refluxed for 18 h, then additional 2.0 g (27 mmol) of *N*-hydroxy-acetamidine and 5 g (36.1 mmol) of potassium carbonate was added. The reaction mixture was refluxed for 20 h, cooled to 20 °C, filtered, the filtrate was concentrated and dried to yield 5.45 g (98.7 %) of the title compound. Mp: 141-142 °C. MS (EI) 230.2 (MH⁺).

### c) (1-{4-[5-methoxy-2-(3-methyl-[1,2,4]oxadiazol-5-yl)-indol-1-yl]-benzylcarbamoyl}-cyclopropyl)-carbamic acid tert-butyl ester

The title compound was prepared from 5-methoxy-2-(3-methyl-[1,2,4]oxadiazol-5-yl)-1*H*-indole according to the method described in Reference Example 4e-g. Mp.: 212-214 °C. MS (EI) 418.2 [(M-Boc)]⁺.

### d) 1-Amino-cyclopropanecarboxylic acid 4-[5-methoxy-2-(3-methyl-[1,2,4]oxadiazol-5-yl)-indol-1-yl]-benzylamide

A mixture of 1.56 g (3.0 mmol) of (1-{4-[5-methoxy-2-(3-methyl-[1,2,4]oxadiazol-5-yl)-indol-1-yl]-benzylcarbamoyl}-cyclopropyl)-carbamic acid *tert-*butyl ester, 15 mL of trifluoroacetic acid and 25 mL of dichloromethane was stirred at room temperature for 2 h. The reaction mixture was concentrated and 100 mL of saturated sodium hydrogencarbonate solution and 100 mL of dichloromethane was added to the residue. The organic layer was separated and the water phase was extracted with 50 mL of dichloromethane. The combined organic layers were washed with water, dried over sodium sulfate and concentrated. The residue was submitted to flash column chromatography using Kieselgel 60 (0.015-0.040 mm) as adsorbent (Merck) and toluene:methanol = 4:1 as eluent. The so obtained product was crystallized from diisopropyl ether to yield 0.99 g (78.9 %) of the title compound. Mp.: 145-146 °C. MS (EI) 418.2 (MH⁺).

### Reference Example 31

### Methyl 1-[4-(aminomethyl)phenyl]-3-chloro-1H-indole-2-carboxylate

### hydrochloride

### a) Methyl 3-chloro-1-(4-formylphenyl)-1H-indole-2-carboxylate

The title compound was prepared from methyl 3-chloro-1*H*-indole-2-carboxylate (Zeneca Limited Patent: US6288103 B1, 2001;) according to the method described in Reference Example 5b. MS (EI) 314.1 (MH⁺).

### b) Methyl 3-chloro-1-{4-[(E)-(hydroxyimino)methyl]phenyl}-1H-indole-2-carboxylate

The title compound was prepared from methyl methyl 3-chloro-1-(4-formylphenyl)-1*H*-indole-2-carboxylate according to the method described in Reference Example 5d. MS (EI) 329.1 (MH⁺).

### c) Methyl 1-[4-(aminomethyl)phenyl]-3-chloro-1H-indole-2-carboxylate hydrochloride

The title compound was prepared from methyl 3-chloro-1-{4-[(*E*)-(hydroxyimino)methyl]phenyl}-1*H*-indole-2-carboxylate according to the method described in Reference Example 5e. MS (EI) 315.1 (MH⁺).

### Reference Example 32

### 1-(4-Aminomethyl-phenyl)-3-chloro-1H-indole-2-carboxylic acid amide hydrochloride

The title compound was prepared from 3-chloro-1*H*-indole-2-carboxamide (Reference Example 4b) according to the method described in Reference Example 9c. MS (EI) 283.1 [(M-NH₂)]⁺.

### Reference Example 33

### (1R)-1-{4-[3-chloro-2-(5-methyl-1,2,4-oxadiazol-3-yl)-1H-indol-1-yl]-2-fluoro-phenyl}ethanamine hydrochloride

### a) 3-Fluoro-4-[(R)-1-(2-methyl-propane-2-sulfinylamino)-ethyl]-phenylboronic acid

Under argon to a solution of 6.42 g (19.9 mmol) of 2-methyl-propane-2-sulfinic acid [(*R*)-1-(4-bromo-2-fluoro-phenyl)-ethyl]-amide (S.D. Kuduk et al. J. Med. Chem., 2007, 50, 272-282) and 4.4 mL (30 mmol) TMEDA (tetramethylethylenediamine) in 520 mL of dry diethyl-ether 38.5 mL of 2.5 M n-butyllithium in hexane solution (39.8 mmol) was added dropwise at -78°C over a period of 1.5 h. After the mixture was stirred at -78°C for 20 min, 125 mL (545 mmol) of triisopropyl borate was added dropwise over a period of 0.5 h. The so obtained mixture was allowed to warm to room temperature and stirred at this temperature for 18 h. The reaction mixture was quenched by addition of 500 mL of saturated ammonium chloride solution (pH ∼8) and allowed to warm to room temperature. The reaction mixture was extracted with ethyl acetate, the combined organic layers were washed with 100 mL of 1 M citric acid and water, dried over anhydrous sodium sulfate, filtered and *concentrated in vacuo.* The residue was stirred with 50 mL of n-hexane overnight, then filtered and dried to yield 3.2 g (56%) of the title compound as a white solid. MS (EI) 288.1 (MH⁺).

### b) (1R)-1-{4-[3-chloro-2-(5-methyl-1,2,4-oxadiazol-3-yl)-1H-indol-1-yl]-2-fluoro-phenyl}ethanamine hydrochloride

The title compound was prepared from 3-fluoro-4-[(*R*)-1-(2-methyl-propane-2-sulfinylamino)-ethyl]-phenylboronic acid according to the method described in Reference Example 9c. MS (EI) 354.1 [(M-NH₂)]⁺.

### Reference Example 34

### 4-[3-Chloro-2-(5-methyl-[1,2,4]oxadiazol-3-yl)-indol-1-yl]-2-fluoro-benzylamine hydrochloride

The title compound was prepared from 3-fluoro-4-(*tert*-butoxycarbonyl-aminomethyl)-phenylboronic acid (Reference Example 1) according to the method described in Reference Example 9c. MS (EI) 340.1 [(M-NH₂)]⁺.

### Reference Example 35

### 1-Amino-cyclopropanecarboxylic acid 4-[3-chloro-2-(3-methyl-[1,2,4]oxadiazol-5-yl)-indol-1-yl]-benzylamide

### a) 3-Chloro-2-(3-methyl-[1,2,4]oxadiazol-5-yl)-1H-indole

The title compound was prepared from 3-chloro-1*H*-indole-2-carboxylic acid methyl ester according to the method described in Reference Example 20d. MS (EI) 234.1 (MH⁺).

### b) 4-[3-Chloro-2-(3-methyl-[1,2,4]oxadiazol-5-yl)-indol-1-yl]-benzylamine hydrochloride

The title compound was prepared from 3-chloro-2-(3-methyl-1,2,4-oxadiazol-5-yl)-1*H*-indole according to the method described in Reference Example 9c. MS (EI) 339.1 (MH⁺).

### c) (1-{4-[3-Chloro-2-(3-methyl-[1,2,4]oxadiazol-5-yl)-indol-1-yl]-benzylcarbamoyl}-cyclopropyl)-carbamic acid tert-butyl ester

The title compound was prepared from 4-[3-chloro-2-(3-methyl-[1,2,4]oxadiazol-5-yl)-indol-1-yl]-benzylamine hydrochloride according to the method described in Reference Example 9d. MS (EI) 422.2 [(M-Boc)]⁺.

### d) 1-Amino-cyclopropanecarboxylic acid 4-[3-chloro-2-(3-methyl-[1,2,4]oxadiazol-5-yl)-indol-1-yl]-benzylamide

The title compound was prepared from (1-{4-[3-chloro-2-(3-methyl-[1,2,4]oxadiazol-5-yl)-indol-1-yl]-benzylcarbamoyl}-cyclopropyl)-carbamic acid *tert-*butyl ester according to the method described in Reference Example 9e. MS (EI) 422.2 (MH⁺).

### Reference Example 36

### 1-Amino-cyclopropanecarboxylic acid 4-[2-(5-methyl-[1,2,4]oxadiazol-3-yl)-indol-1-yl]-benzylamide

### a) 2-(5-Methyl-[1,2,4]oxadiazol-3-yl)-1H-indole

The title compound was prepared from *N*-hydroxy-1*H*-indole-2-carboxamidine (Merck Sharp and Dohme Ltd. Patent: US4952587 A1, 1990 ;) according to the method described in Example 9b. MS (EI) 200.0 (MH⁺).

### b) 4-[2-(5-Methyl-[1,2,4]oxadiazol-3-yl)-indol-1-yl]-benzylamine hydrochloride

The title compound was prepared from 2-(5-methyl-[1,2,4]-oxadiazol-3-yl)-1*H-*indole according to the method described in Reference Example 9c. MS (EI) 305.2 (MH⁺).

### c) (1-{4-[2-(5-Methyl-[1,2,4]oxadiazol-3-yl)-indol-1-yl]-benzylcarbamoyl}-cyclo-propl)-carbamic acid tert-butyl ester

The title compound was prepared from 4-[2-(5-methyl-[1,2,4]oxadiazol-3-yl)-indol-1-yl]-benzylamine hydrochloride according to the method described in Reference Example 9d. MS (EI) 388.2 [(M-Boc)]⁺.

### d) 1-Amino-cyclopropanecarboxylic acid 4-[2-(5-methyl-[1,2,4]oxadiazol-3-yl)-indol-1-yl]-benzylamide

The title compound was prepared from (1-{4-[2-(5-methyl-[1,2,4]oxadiazol-3-yl)-indol-1-yl]-benzylcarbamoyl}-cyclopropyl)-carbamic acid *tert*-butyl ester according to the method described in Reference Example 9e. MS (EI) 388.2 (MH⁺).

### Reference Example 37

### 1-Amino-cyclopropanecarboxylic acid 4-[2-(3-methyl-[1,2,4]oxadiazol-5-yl)-indol-1-yl]-benzylamide

### a) 2-(3-Methyl-[1,2,4]oxadiazol-5-yl)-1H-indole

The title compound was prepared from 1*H*-indole-2-carboxylic acid methyl ester according to the method described in Reference Example 20d. MS (EI) 200.0 (MH⁺).

### b) 4-[2-(3-Methyl-[1,2,4]oxadiazol-5-yl)-indol-1-yl]-benzylamine

The title compound was prepared from 2-(3-methyl-1,2,4-oxadiazol-5-yl)-1*H-*indole according to the methods described in Reference Example 9c. MS (EI) 288.2 [(M-NH₂)]⁺.

### c) (1-{4-[2-(3-Methyl-[1,2,4]oxadiazol-5-yl)-indol-1-yl]-benzylcarbamoyl}-cyclo-propyl)-carbamic acid tert-butyl ester

The title compound was prepared from 4-[2-(3-methyl-[1,2,4]oxadiazol-5-yl)-indol-1-yl]-benzylamine hydrochloride according to the method described in Reference Example 9d. MS (EI) 388.2 [(M-Boc)]⁺.

### d) 1-Amino-cyclopropanecarboxylic acid 4-[2-(3-methyl-[1,2,4]oxadiazol-5-yl)-indol-1-yl]-benzylamide

The title compound was prepared from (1-{4-[2-(3-methyl-[1,2,4]oxadiazol-5-yl)-indol-1-yl]-benzylcarbamoyl}-cyclopropyl)-carbamic acid *tert*-butyl ester according to the method described in Reference Example 9e. MS (EI) 388.2 (MH⁺).

### Reference Example 38

### 1-Amino-cyclopropanecarboxylic acid 4-(2-cyano-indol-1-yl)-benzylamide

### a) 1-[4-(Aminomethyl)phenyl]-1H-indole-2-carbonitrile hydrochloride

The title compound was prepared from 1*H*-indole-2-carbonitrile (I. Borza at al. Bioorg. Med. Chem. Lett. 2005, 15, 5439-5441) according to the method described in Reference Example 9c. MS (EI) 231.1 [(M-NH₂)]⁺.

### b) {1-[4-(2-Cyano-indol-1-yl)-benzylcarbamoyl]-cyclopropyl}-carbamic acid tert-butyl ester

The title compound was prepared from 1-[4-(aminomethyl)phenyl]-1*H*-indole-2-carbonitrile hydrochloride according to the methods described in Reference Example 9d. MS (EI) 331.2 [(M-Boc)]⁺.

### c) 1-Amino-cyclopropanecarboxylic acid 4-(2-cyano-indol-1-yl)-benzylamide

The title compound was prepared from {1-[4-(2-cyano-indol-1-yl)-benzylcarbamoyl]-cyclopropyl}-carbamic acid *tert*-butyl ester according to the methods described in Reference Example 9e. MS (EI) 331.2 (MH⁺).

### Reference Example 39

### 4-[3,5-Difluoro-2-(5-methyl-[1,2,4]oxadiazol-3-yl)-indol-1-yl]-benzylamine hydrochloride

### a) 3,5-Difluoro-2-(5-methyl-[1,2,4]oxadiazol-3-yl)-1H-indole

To a mixture of 0.941 g (4.33 mmol) of 5-fluoro-2-(5-methyl-[1,2,4]oxadiazol-3-yl)-1*H*-indole (Reference Example 19a) and 30 mL of acetonitril 1.535 g (4.33 mmol) of Selectfluor [1-chloromethyl-4-fluoro-1,4-diazoniabicyclo[2.2.2]octane bis(tetrafluoroborate)] was added and the reaction mixture was stirred at 60°C for 18 h. The mixture was diluted with ethyl acetate, washed with saturated sodium hydrogencarbonate solution and water, dried over anhydrous sodium sulfate and concentrated. The residue was submitted to column chromatography using Kieselgel 60 (0.015-0.040 mm) as adsorbent (Merck) and hexane:ethyl acetate = 2:1 as eluent to yield 0.335 g (32.9%) of the title compound as yellow crystalline solid. MS (EI) 236.1 (MH⁺).

### b) {4-[3,5-Difluoro-2-(5-methyl-[1,2,4]oxadiazol-3-yl)-indol-1-yl]-benzyl}-carbamic acid tert-butyl ester

The title compound was prepared from 3,5-difluoro-2-(5-methyl-[1,2,4]oxadiazol-3-yl)-1*H*-indole according to the method described in Reference Example 4e. MS (EI) 463.2 [M+Na]⁺.

### c) 4-[3,5-Difluoro-2-(5-methyl-[1,2,4]oxadiazol-3-yl)-indol-1-yl]-benzylamine hydrochloride

The title compound was prepared from {4-[3,5-difluoro-2-(5-methyl-[1,2,4]oxadiazol-3-yl)-indol-1-yl]-benzyl}-carbamic acid *tert*-butyl ester according to the method described in Reference Example 4f. MS (EI) 341.1 (MH⁺).

### Example 1

### 1-(2,2,2-Trifluoro-acetylamino)-cyclopropanecarboxylic acid 4-[3-chloro-2-(2-methyl-2H-tetrazol-5-yl)-indol-1-yl]-benzylamide

To a stirred solution of 4.0 g (8.7 mmol) of 1-amino-cyclopropanecarboxylic acid 4-[3-chloro-2-(2-methyl-2*H*-tetrazol-5-yl)-indol-1-yl]-benzylamide hydrochloride (Reference Example 4) and 3.6 mL (25.8 mmol) of triethylamine in 180 mL of dichloromethane 1.36 mL (9.8 mmol) of trifluoroacetic anhydride was added dropwise below 20 °C, and the reaction mixture was stirred at room temperature for 3 h. Then 100 mL of water was added to the mixture, the organic layer was separated, dried over anhydrous sodium sulfate and *concentrated in vacuo.* The residue was submitted to flash column chromatography using Kieselgel 60 (0.015-0.040 mm) as adsorbent (Merck) and toluene:aceton = 2:1 as eluent to yield after crystallization from 2-propanol 2.2 g (48.7 %) of the title compound. MS (EI) 518.2 (MH⁺).

### Example 2

### 3-Methoxy-isoxazole-5-carboxylic acid (1-{4-[3-chloro-2-(2-methyl-2H-tetrazol-5-yl)-indol-1-yl]-benzylcarbamoyl}-cyclopropyl)-amide

A mixture of 0.16 g (0.42 mmol) of 4-[3-chloro-2-(2-methyl-2*H*-tetrazol-5-yl)-indol-1-yl]-benzylamine hydrochloride (Reference Example 4), 0.107 g (0.47 mmol) of 1-[3-methoxy-isoxazole-5-carbonyl)-amino]-cyclopropanecarboxylic acid (P.D. O'Shea et al. J. Org. Chem. 2009, 74, 4547-4553), 0.15 mL (1.07 mmol) of triethylamine, 0.192 g (0.5 mmol) of HBTU and 5 mL of *N*,*N*-dimethylformamide was stirred at room temperature for 24 h, then *concentrated in vacuo.* The residue was submitted to flash column chromatography using Kieselgel 60 (0.015-0.040 mm) as adsorbent (Merck) and toluene:methanol = 4:1 as eluent to yield after crystallization from diethylether 0.12 g (51.5 %) of the title compound. MS (EI) 548.1 (MH⁺).

### Example 3

### 3-Methoxy-isoxazole-5-carboxylic acid (1-{4-[2-(2-methyl-2H-tetrazol-5-yl)-indol-1-yl]-benzylcarbamoyl}-cyclopropyl)-amide

The title compound was prepared from 4-[2-(2-methyl-2*H*-tetrazol-5-yl)-indole-yl]-benzylamine hydrochloride (Reference Example 5) according to the method described in Example 2. MS (EI) 513.2 (MH⁺).

### Example 4

### 1-(2,2,2-Trifluoro-acetylamino)-cyclopropanecarboxylic acid 4-[3-chloro-2-(2-methyl-2H-tetrazol-5-yl)-indol-1-yl]-2-fluorobenzylamide

A mixture of 0.197 g (0.50 mmol) of 4-[3-chloro-2-(2-methyl-2*H*-tetrazol-5-yl)-indol-1-yl]-2-fluoro-benzylamine hydrochloride (Reference Example 6), 0.105 g (0.53 mmol) of 1-(2,2,2-trifluoro-acetylamino)-cyclopropanecarboxylic acid (M. R. Hickey et al. Synlett. **2005,** 255-258), 0.15 mL (1.07 mmol) of triethylamine, 0.2 g (0.53 mmol) of HBTU and 4 mL of *N*,*N*-dimethylformamide was stirred at room temperature for 24 h, then concentrated *in vacuo.* The residue was submitted to flash column chromatography using Kieselgel 60 (0.015-0.040 mm) as adsorbent (Merck) and hexane:ethyl acetate = 4:1 as eluent to yield 0.14 g (52.2 %) of the title compound. MS (EI) 536.2 (MH⁺).

### Example 5

### 3-Methoxy-N-[1-({4-[3-chloro-2-(5-methyl-1,3,4-oxadiazol-2-yl)-1H-indol-1-yl]benzyl}carbamoyl)cyclopropyl]isoxazole-5-carboxamide

The title compound was prepared from 4-[3-chloro-2-(5-methyl-[1,3,4]oxadiazol-2-yl)-indol-1-yl]-benzylamine hydrochloride (Reference Example 7) according to the method described in Example 2. MS (EI) 547.2 (MH⁺).

### Example 6

### 1-(2,2,2-Trifluoro-acetylamino)-cyclopropanecarboxylic acid 4-[3-chloro-2-(5-methyl-[1,3,4]oxadiazol-2-yl)-indol-1-yl]-benzylamide

The title compound was prepared from 4-[3-chloro-2-(5-methyl-[1,3,4]oxadiazol-2-yl)-indol-1-yl]-benzylamine hydrochloride (Reference Example 7) according to the method described in Example 4. MS (EI) 518.2 (MH⁺).

### Example 7

### 3-Methoxy-isoxazole-5-carboxylic acid (1-{4-[3-chloro-2-(1-methyl-1H-tetrazol-5-yl)-indol-1-yl]-benzylcarbamoyl}-cyclopropyl)-amide

The title compound was prepared from 4-[2-(1-methyl-2*H*-tetrazol-5-yl)-indole-yl]-benzylamine hydrochloride (Reference Example 8) according to the method described in Example 2. MS (EI) 547.1 (MH⁺).

### Example 8

### 1-(2,2,2-Trifluoro-acetylamino)-cyclopropanecarboxylic acid 4-[3-chloro-2-(1-methyl-1H-tetrazol-5-yl)-indol-1-yl]-benzylamide

The title compound was prepared from 4-[2-(1-methyl-2*H*-tetrazol-5-yl)-indole-yl]-benzylamine hydrochloride (Reference Example 8) according to the method described in Example 4. MS (EI) 518.1 (MH⁺).

### Example 9

### 3-Methoxy-isoxazole-5-carboxylic acid (1-{4-[3-chloro-2-(5-methyl-[1,2,4]oxadiazol-3-yl)-indol-1-yl]-benzylcarbamoyl}-cyclopropyl)-amide

A mixture of 23.0 g (54.5 mmol) of 1-amino-cyclopropanecarboxylic acid 4-[3-chloro-2-(5-methyl-[1,2,4]oxadiazol-3-yl)-indol-1-yl]-benzylamide (Reference Example 9), 350 mL of DMSO, 9.4 g (65.4 mmol) of 3-methoxy-isoxazole-5-carboxylic acid, 24.8 g (65.4 mmol) of HBTU and 11.4 mL (65.4 mmol) of *N*,*N-*diisopropylethylamine was stirred at room temperature for 2 h. The reaction mixture was poured into 3000 mL of water and stirred for 2 h. The precipitated product was filtered off, washed with water and recrystallized from 2-propanol to yield 22.5 g (75.4%) of the title compound. MS (EI) 547.1 (MH⁺).

### Example 10

### 3-Chloro-1-[4-({[1-(2,2,2-trifluoro-acetylamino)-cyclopropanecarbonyl]-amino}-methyl)-phenyl]-1H-indole-2-carboxylic acid methyl ester

The title compound was prepared from methyl 1-[4-(aminomethyl)phenyl]-3-chloro-1*H*-indole-2-carboxylate hydrochloride (Reference Example 31) according to the method described in Example 4. MS (EI) 494.1 (MH⁺).

### Example 11

### 3-Chloro-1-{4-[({1-[(3-methoxy-isoxazole-5-carbonyl)-amino]-cyclopropane-carbonyl}-amino)-methyl]-phenyl}-1H-indole-2-carboxylic acid methyl ester

The title compound was prepared from methyl 1-[4-(aminomethyl)phenyl]-3-chloro-1*H*-indole-2-carboxylate hydrochloride (Reference Example 31) according to the method described in Example 2. MS (EI) 523.1 (MH⁺).

### Example 12

### 1-(2,2,2-Trifluoro-acetylamino)-cyclopropanecarboxylic acid 4-[3-chloro-5-fluoro-2-(2-methyl-2H-tetrazol-5-yl)-indol-1-yl]-benzylamide

The title compound was prepared from 4-[3-chloro-5-fluoro-2-(2-methyl-2*H-*tetrazol-5-yl)-indol-1-yl]-benzylamine hydrochloride (Reference Example 10) according to the method described in Example 4. MS (EI) 536.2 (MH⁺).

### Example 13

### 3-Methoxy-isoxazole-5-carboxylic acid (1-{4-[3-chloro-5-fluoro-2-(2-methyl-2H-tetrazol-5-yl)-indol-1-yl]-benzylcarbamoyl}-cyclopropyl)-amide

The title compound was prepared from 4-[3-chloro-5-fluoro-2-(2-methyl-2*H-*tetrazol-5-yl)-indol-1-yl]-benzylamine hydrochloride (Reference Example 10) according to the method described in Example 2. MS (EI) 365.2 (MH⁺).

### Example 14

### 1-{4-[({1-[(3-Methoxy-isoxazole-5-carbonyl)-amino]-cyclopropanecarbonyl}-amino)-methyl]-phenyl}-1H-indole-2-carboxylic acid methyl ester

The title compound was prepared from 1*H*-indole-2-carboxylic acid methyl ester according to the methods described in Reference Example 5b, 5d, 5e and Example 2. MS (EI) 489.2 (MH⁺).

### Example 15

### 3-Methoxy-isoxazole-5-carboxylic acid (1-{4-[3-chloro-2-(2-methyl-2H-tetrazol-5-yl)-indol-1-yl]-2-fluorobenzylcarbamoyl}-cyclopropyl)-amide

The title compound was prepared from 4-[3-chloro-2-(2-methyl-2*H*-tetrazol-5-yl)-indol-1-yl]-2-fluoro-benzylamine hydrochloride (Reference Example 6) according to the method described in Example 2. MS (EI) 565.2 (MH⁺).

### Example 16

### 3-Methoxy-isoxazole-5-carboxylic acid (1-{4-[3-chloro-2-(1,3-oxazol-5-yl)-indol-1-yl]-benzylcarbamoyl}-cyclopropyl)-amide

### a) 3-Chloro-2-oxazol-5-yl-1H-indole

A mixture of 1.2 g (6.68 mmol) of 3-chloro-1*H*-indole-2-carbaldehyde, 1.32 g (6.76 mmol) of toluenesulfonylmethyl isocyanide, 1.2 g (8.69 mmol) of potassium carbonate and 30 mL of methanol was refluxed for 1 h. The reaction mixture was cooled to 20 °C, *concentrated in vacuo,* the residue was submitted to flash column chromatography using Kieselgel 60 (0.015-0.040 mm) as adsorbent (Merck) and hexane:ethyl acetate = 2:1 as eluent to yield 1.2 g (82 %) of the title compound. Mp.: 131-134 °C.

### b) 4-(3-Chloro-2-oxazol-5-yl-indol-yl)-benzylamine hydrochloride

The title compound was prepared from 3-chloro-2-oxazol-5-yl-1*H*-indole according to the methods described in Reference Example 4e and 4f. Mp.: 183-189 °C.

### c) 3-Methoxy-isoxazole-5-carboxylic acid (1-{4-[3-chloro-2-(1,3-oxazol-5-yl)-indol-1-yl]-benzylcarbamoyl}-cyclopropyl)-amide

The title compound was prepared from 4-(3-chloro-2-oxazol-5-yl-indol-yl)-benzylamine hydrochloride according to the method described in Example 2. MS (EI) 532.2 (MH⁺).

### Example 17

### 1-(2,2,2-Trifluoro-acetylamino)-cyclopropanecarboxylic acid 4-[3,5-dichloro-2-(2-methyl-2H-tetrazol-5-yl)-indol-1-yl]-benzylamide

The title compound was prepared from 4-[3,5-dichloro-2-(2-methyl-2*H*-tetrazol-5-yl)-indol-1-yl]-benzylamine hydrochloride (Reference Example 11) according to the method described in Example 4. MS (EI) 552.1 (MH⁺).

### Example 18

### 3-Methoxy-isoxazole-5-carboxylic acid (1-{4-[3,5-dichloro-2-(2-methyl-2H-tetrazol-5-yl)-indol-1-yl]-benzylcarbamoyl}-cyclopropyl)-amide

The title compound was prepared from 4-[3,5-dichloro-2-(2-methyl-2*H*-tetrazol-5-yl)-indol-1-yl]-benzylamine hydrochloride (Reference Example 11) according to the method described in Example 2. MS (EI) 581.1 (MH⁺).

### Example 19

### 1-(2,2,2-Trifluoro-acetylamino)-cyclopropanecarboxylic acid 4-[3-chloro-5-fluoro-2-(2-methyl-2H-tetrazol-5-yl)-indol-1-yl-2-fluoro-benzylamide

The title compound was prepared from 4-[3-chloro-5-fluoro-2-(2-methyl-2*H-*tetrazol-5-yl)-indol-1-yl]-2-fluoro-benzylamine hydrochloride (Reference Example 12) according to the method described in Example 4. MS (EI) 554.1 (MH⁺).

### Example 20

### 3-Methoxy-isoxazole-5-carboxylic acid (1-{4-[3-chloro-5fluoro-2-(2-methyl-2H-tetrazol-5-yl)-indol-1-yl]-2-fluoro-benzylcarbamoyl}-cyclopropyl)-amide

The title compound was prepared from 4-[3-chloro-5-fluoro-2-(2-methyl-2*H-*tetrazol-5-yl)-indol-1-yl]-2-fluoro-benzylamine hydrochloride (Reference Example 12) according to the method described in Example 2. MS (EI) 583.2 (MH⁺).

### Example 21

### 3-Methoxy-isoxazole-5-carboxylic acid (1-{4-[3-cyano-2-(5-methyl-[1,2,4]oxadiazol-3-yl)-indol-1-yl]-benzylcarbamoyl}-cyclopropyl)-amide

### a) 2-(5-Methyl-[1,2,4]oxadiazol-3-yl)-1H-indole-3-carbaldehyde

To a stirred mixture of 2 mL of N,N-dimethylformamide and 10 mL of dichloromethane 0.7 mL (7.5 mmol) of phosphorus oxychloride was added dropwise at 0 °C and the reaction mixture was stirred at 0 °C for 30 min. Then 1.0 g (5.0 mmol) of 2-(5-methyl-[1,2,4]oxadiazol-3-yl)-1H-indole (Reference Example 36a) in 25 mL of dichloromethane was added dropwise to the reaction mixture and stirred at room temperature for 1 h. Then 20 g of ice was added to the mixture and stirred at room temperature for 3 h. The precipitated product was filtered off to yield 1.1 g (96.5 %) of the title compound. Mp.: 228-230 °C.

### b) 2-(5-Methyl-[1,2,4]oxadiazol-3-yl)-1H-indole-3-carbonitrile

A mixture of 1.1 g (4.8 mmol) of 2-(5-methyl-[1,2,4]oxadiazol-3-yl)-1H-indole-3-carbaldehyde, 2.38 g (29.0 mmol) of sodium acetate, 2.6 mL (36.2 mmol) of nitroethane and 8 mL of acetic acid was refluxed for 8 h. The precipitated product was filtered off, washed with water and recrystallized from isopropanol to yield 0.68 g (62.9 %) of the title compound. Mp.: 209-212 °C.

### c) 1-(4-Aminomethyl-phenyl)-2-(5-methyl-[1,2,4]oxadiazol-3-yl)-1H-indole-3-carbonitrile hydrochloride

The title compound was prepared from 2-(5-methyl-[1,2,4]oxadiazol-3-yl)-1H-indole-3-carbonitrile and 4-(tert-butoxycarbonylaminomethyl)phenylboronic acid according to the method described in Reference Example 4e and 4f. Mp.: 201-204 °C.

### d) 3-Methoxy-isoxazole-5-carboxylic acid (1-{4-[3-cyano-2-(5-methyl-[1,2,4]oxadiazol-3-yl)-indol-1-yl]-benzylcarbamoyl}-cyclopropyl)-amide

The title compound was prepared from 1-(4-aminomethyl-phenyl)-2-(5-methyl-[1,2,4]oxadiazol-3-yl)-1H-indole-3-carbonitrile hydrochloride according to the method described in Example 2. MS (EI) 538.2 (MH⁺).

### Example 22

### 1-(2,2,2-Trifluoro-acetylamino)-cyclopropanecarboxylic acid 4-[3,5-dichloro-2-(2-methyl-2H-tetrazol-5-yl)-indol-1-yl]-2-fluoro-benzylamide

The title compound was prepared from 4-[3,5-dichloro-2-(2-methyl-2*H*-tetrazol-5-yl)-indol-1-yl]-2-fluoro-benzylamine hydrochloride (Reference Example 13) according to the method described in Example 4. MS (EI) 570.1 (MH⁺).

### Example 23

### 1-(2,2,2-Trifluoro-acetylamino)-cyclopropanecarboxylic acid 4-[3-chloro-2-(5-methyl-[1,2,4]oxadiazol-3-yl)-indol-1-yl]-benzylamide

The title compound was prepared from 4-[3-chloro-2-(5-methyl-[1,2,4]oxadiazol-3-yl)-indol-1-yl]-benzylamine hydrochloride (Reference Example 9c) according to the method described in Example 4. MS (EI) 518.1 (MH⁺).

### Example 24

### 5-Cyclopropyl-isoxazole-3-carboxylic acid (1-{4-[3-chloro-2-(2-methyl-2H-tetrazol-5-yl)-indol-1-yl]-benzylcarbamoyl}-cyclopropyl)-amide

A mixture of 23.0 mg (0.15 mmol) of 5-cyclopropyl-isoxazole-3-carboxylic acid, 30 µl (0.21 mmol) of triethylamine, 63.2 mg (0.18 mmol) of 1-amino-cyclopropanecarboxylic acid 4-[3-chloro-2-(2-methyl-2*H*-tetrazol-5-yl)-indol-1-yl]-benzylamide (Reference Example 4) 60.0 mg (0.16 mmol) of HATU and 1 mL of *N,N-*dimethylformamide was stirred at room temperature for 24 h. The reaction mixture was purified by column chromatography using Kieselgel 60 (Merck) as adsorbent and hexane:ethyl acetate = 4:1 as eluent to yield 26 mg (31 %) of the title compound. MS (EI) 557.2 (MH⁺).

Compounds of Table 1 were prepared from 1-amino-cyclopropanecarboxylic acid 4-[3-chloro-2-(2-methyl-2*H*-tetrazol-5-yl)-indol-1-yl]-benzylamide (Reference Example 4) according to the method described in Example 24.

**Table 1**

| **Example** | Name | MS (EI) (MH⁺) |
|---|---|---|
| **25** | 5-Methyl-pyrazine-2-carboxylic acid (1-{4-[3-chloro-2-(2-methyl-2*H*-tetrazol-5-yl)-indol-1-yl]-benzylcarbamoyl}-cyclopropyl)-amide | 542.2 |
| **26** | 5-Methyl-isoxazole-3-carboxylic acid (1-{4-[3-chloro-2-(2-methyl-2*H*-tetrazol-5-yl)-indol-1-yl]-benzylcarbamoyl}-cyclopropyl)-amide | 531.2 |
| **27** | 2-Chloro-*N*-(1-{4-[3-chloro-2-(2-methyl-2*H*-tetrazol-5-yl)-indol-1-yl]-benzylcarbamoyl}-cyclopropyl)-nicotinamide | 561.2 |
| **28** | *N*-(1-{4-[3-chloro-2-(2-methyl-2*H*-tetrazol-5-yl)-indol-1-yl]-benzylcarbamoyl}-cyclopropyl)-2-fluoro-isonicotinamide | 545.2 |
| **29** | 1-(3,3,3,-Trifluoro-propionylamino)-cyclopropanecarboxylic acid 4-[3-chloro-2-(2-methyl-2*H*-tetrazol-5-yl)-indol-1-yl]-benzylamide | 532.2 |
| **30** | *N*-(1-{4-[3-chloro-2-(2-methyl-2*H*-tetrazol-5-yl)-indol-1-yl]-benzylcarbamoyl}-cyclopropyl)-5-trifluoromethyl-nicotinamide | 595.2 |
| **31** | *N*-(1-{4-[3-chloro-2-(2-methyl-2*H*-tetrazol-5-yl)-indol-1-yl]-benzylcarbamoyl}-cyclopropyl)-3-fluoro-benzamide | 544.2 |

### Example 32

### 3-Chloro-1-[4-(1-{[1-(2,2,2-trifluoro-acetylamino)-cyclopropanecarbonyl]-amino}-ethyl)-phenyl]-1H-indole-2-carboxylic acid methylamide

### a) 3-Chloro-1H-indole-2-carboxylic acid methylamide

To a stirred suspension of 0.8 g (4.6 mmol) of 1*H*-indole-2-carboxylic acid methylamide (WO2007/53131) in 20 mL of acetonitrile 0.68 g (5 mmol) of *N-*chlorosuccinimide was added. The reaction mixture was refluxed for 6 h and *concentrated in vacuo.* The yellowish white residue was crystallised from ethanol to give 0.45 g (47 %) of the title compound as a light beige solid. MS (EI) 209.1 (MH⁺).

### b) 1-(4-Acetyl-phenyl)-3-chloro-1H-indole-2-carboxylic acid methylamide

To a stirred solution of 0.45 g (2.16 mmol) of 3-chloro-1*H*-indole-2-carboxylic acid methylamide in 10 mL of *N*,*N*-dimethylformamide 0.53 g (3.24 mmol) of 4-acetyl-phenylboronic acid, 0.79 g (4.32 mmol) of copper(II) acetate, 1.5 mL (8.6 mmol) of *N*,*N*-diisopropylethylamine and 1 g of 3Å molecular sieves were added. The reaction mixture was vigorously stirred and bubbled with a stream of dry air at room temperature for 48 h. After the reaction was complete, the mixture was filtered through Celite. The filter cake was washed with chloroform and the combined filtrates were concentrated *in vacuo.* The residue was taken up in chloroform and filtered through a plug of silica (20 g), the filter cake was washed with chloroform and the combined filtrates were concentrated *in vacuo.* The residue was crystallised from 2-propanol to yield 0.42 g (60 %) of the title compound as beige crystals. MS (EI) 327.1 (MH⁺).

### c) 1-[4-(1-Amino-ethyl)-phenyl]-3-chloro-1H-indole-2-carboxylic acid methylamide

To a solution of 0.42 g (1.29 mmol) of 1-(4-acetyl-phenyl)-3-chloro-1*H*-indole-2-carboxylic acid methylamide in a mixture of 3 mL of methanol, 3 mL of dichloromethane and 2 mL of acetonitrile 0.074 mL of acetic acid (1.29 mmol), 0.12 g (1.8 mmol) of sodium cyanoborohydride, 1 g (12.9 mmol) of ammonium acetate and 0.5 g of 3Å molecular sieves were added. The reaction mixture was stirred at room temperature overnight when a second portion of sodium cyanoborohydride (0.12 g, 1.8 mmol) was added and the mixture was stirred at room temperature for 72 h. The pH of the reaction mixture was adjusted to 5 by the addition of 3M aqueous hydrochloric acid solution and the so obtained mixture was *concentrated in vacuo.* The residue was treated with 50 mL of dichloromethane and extracted with 10 mL of water. The aqueous phase was reextracted with dichloromethane. The combined organic layers were washed with 20 mL of brine, dried over anhydrous sodium sulfate, filtered and *concentrated in vacuo.* The residue was submitted to flash column chromatography using Kieselgel 60 (0.040-0.063 mm) as adsorbent (Merck) and chloroform:methanol:ammonium hydroxide solution (25%) = 95:5:1 as eluent to yield 0.17 g (40 %) of the title compound as white vaxy solid. MS (EI) 328.1 (MH⁺).

### d) 3-Chloro-1-[4-(1-{[1-(2,2,2-trifluoro-acetylamino)-cyclopropanecarbonyl]-amino}-ethyl)-phenyl]-1H-indole-2-carboxylic acid methylamide

To a solution of 0.173 g (0.53 mmol) of 1-[4-(1-amino-ethyl)-phenyl]-3-chloro-1*H*-indole-2-carboxylic acid methylamide in 7 mL of dichloromethane 0.109 g (0.55 mmol) of 1-(2,2,2-trifluoro-acetylamino)-cyclopropanecarboxylic acid (M. R. Hickey et al. Synlett. 2005, 255-258), 0.2 g (1 mmol) of EDC [*N*-(3-dimethylaminopropyl)-N'-ethylcarbodiimide hydrochloride], 0.004 g (0.03 mmol) of hydroxybenzotriazole monohydrate and 0.44 mL (3.17 mmol) of triethylamine were added. The reaction mixture was stirred at room temperature overnight. The mixture was diluted with 100 mL of dichloromethane and washed with 2x20 mL of saturated sodium hydrogencarbonate aqueous solution, 2x20 mL of water and 20 mL brine, dried over anhydrous sodium sulfate, filtered and concentrated *in vacuo.* The residue was submitted to flash column chromatography using Kieselgel 60 (0.040-0.063 mm) as adsorbent (Merck) and chloroform:methanol = 95:5 as eluent to yield 0.19 g (73 %) of the title compound as a white amorphous solid. MS (EI) 507.1 (MH⁺).

### Example 33

### 1-(2,2,2-Trifluoro-acetylamino)-cyclopropanecarboxylic acid (1-{4-[3-chloro-2-(pyrrolidine-1-carbonyl)-indol-1-yl]-phenyl}-ethyl)-amide

### a) (3-chloro-1H-indol-2-yl)-pyrrolidin-1-yl-methanone

To a stirred suspension of 0.89 g (4.15 mmol) of (1*H*-indol-2-yl)-pyrrolidin-1-yl-methanone (US2007/21463) in 20 mL of acetonitrile 0.6 g (4.6 mmol) of *N-*chlorosuccinimide was added. The reaction mixture was refluxed for 5 h and allowed to stand at room temperature overnight. The separated crystalline solid was filtered off, washed with acetonitrile and dried to give 0.66g (66 %) of the title compound as a yellowish crystalline solid. MS (EI) 249.1 (MH⁺).

### b) 1-{4-[3-Chloro-2-(pyrrolidine-1-carbonyl)-indol-1-yl]-phenyl}-ethanone

To a solution of 0.66 g (2.65 mmol) of (3-chloro-1*H*-indol-2-yl)-pyrrolidin-1-yl-methanone in 15 mL of *N,N-*dimethylformamide 0.65 g (3.96 mmol) of 4-acetyl-phenylboronic acid 0.96 g (5.3 mmol) of copper(II) acetate, 1.9 mL (10.6 mmol) of *N*,*N*-diisopropylethylamine and 1.5 g of 3Å molecular sieves were added. The reaction mixture was vigorously stirred and bubbled with a stream of dry air at room temperature for 48 h. The mixture was filtered through Celite. The filter cake was washed with *N,N-*dimethylformamide and the combined filtrates were evaporated *in vacuo.* The residue was submitted to flash column chromatography using Kieselgel 60 (0.040-0.063 mm) as adsorbent (Merck) and chloroform:acetone = 95:5 as eluent to yield after crystallization from 2-propanol 0.33 g (34 %) of the title compound as a yellow crystalline solid. MS (EI) 367.1 (MH⁺).

### c) {1-[4-(1-Amino-ethyl)-phenyl]-3-chloro-1H-indol-2-yl}-pyrrolidin-1-yl-methanone

To a solution of 0.32 g (0.87 mmol) of 1-{4-[3-chloro-2-(pyrrolidine-1-carbonyl)-indol-1-yl]-phenyl}-ethanone in a mixture of 2 mL of methanol and 2 mL of dichloromethane 0.67 g (8.7 mmol) of ammonium acetate and 0.077 g (1.22 mmol) of sodium cyanoborohydride were added. The reaction mixture was stirred at room temperature overnight. The pH of the reaction mixture was adjusted to 5 by the addition of 3M aqueous hydrochloric acid solution and the so obtained mixture was concentrated *in vacuo.* The residue was treated with 30 mL of dichloromethane and extracted with 10 mL of water. The aqueous phase was reextracted with 10 mL of dichloromethane. The combined organic layers were washed with 10 mL of brine, dried over anhydrous sodium sulfate, filtered and *concentrated in vacuo.* The residue was submitted to flash column chromatography using Kieselgel 60 (0.040-0.063 mm) as adsorbent (Merck) and chloroform:methanol:ammonium hydroxide solution (25%) = 95:5:1 as eluent to yield 0.15 g (46 %) of the title compound as white foam. MS (EI) 368.1 (MH⁺).

### d) 1-(2,2,2-Trifluoro-acetylamino)-cyclopropanecarboxylic acid (1-{4-[3-chloro-2-(pyrrolidine-1-carbonyl)-indol-1-yl]-phenyl}-ethyl)-amide

To a solution of 0.15 g (0.4 mmol) of {1-[4-(1-amino-ethyl)-phenyl]-3-chloro-1*H*-indol-2-yl}-pyrrolidin-1-yl-methanone in 5 mL of dichloromethane 0.089 g (0.45 mmol) of 1-(2,2,2-trifluoro-acetylamino)-cyclopropanecarboxylic acid (M. R. Hickey et al. Synlett. 2005, 255-258), 0.156 g (0.82 mmol) of EDC, 0.003 g (0.02 mmol) of hydroxybenzotriazole monohydrate and 0.34 mL (2.4 mmol) of triethylamine were added. The reaction mixture was stirred at room temperature overnight. The mixture was diluted with 100 mL of dichloromethane and washed with 2x20 mL of saturated sodium hydrogencarbonate aqueous solution, 2x20 mL of water and 20 mL of brine, dried over anhydrous sodium sulfate, filtered and concentrated *in vacuo.* The residue was submitted to flash column chromatography using Kieselgel 60 (0.040-0.063 mm) as adsorbent (Merck) and chloroform:methanol = 95:5 as eluent to yield 0.14 g (66 %) of the title compound as an off-white amorphous solid. MS (EI) 547.2 (MH⁺).

### Example 34

### 3-Chloro-1-{4-[({1-[(3-methoxy-isoxazole-5-carbonyl)-amino]-cyclopropane-carbonyl}-amino)-methyl]-phenyl}-1H-indole-2-carboxylic acid ethyl ester

### a) 3-Chloro-1H-indole-2-carboxylic acid ethyl ester

To a stirred suspension of 3 g (15.86 mmol) of 1*H*-indole-2-carboxylic acid ethyl ester in 30 mL of acetonitrile 2.33 g (17.44 mmol) of *N*-chlorosuccinimide was added. The reaction mixture was refluxed for 6 h and allowed to stand at room temperature overnight. The separated crystalline solid was filtered off, washed with acetonitrile and dried to give 2.4 g (67 %) of the title compound as a yellowish crystalline solid. MS (EI) 224.1 (MH⁺).

### b) 3-Chloro-1-(4-formyl-phenyl)-1H-indole-2-carboxylic acid ethyl ester

To a solution of 1.0 g (4.47 mmol) of 3-chloro-1*H*-indole-2-carboxylic acid ethyl ester in 20 mL of *N*,*N*-dimethylformamide 1.0 g (6.7 mmol) of 4-formyl-phenylboronic acid, 1.62 g (8.94 mmol) of copper(II) acetate, 3.1 mL (17.9 mmol) of *N*,*N*-diisopropylethylamine and 2.5 g of 3Å molecular sieves were added. The reaction mixture was vigorously stirred and bubbled with a stream of dry air at room temperature for 30 h. The mixture was filtered through Celite. The filter cake was washed with *N*,*N-*dimethylformamide and dichloromethane and the combined filtrates were concentrated *in vacuo.* The residue was treated with 100 mL of brine and extracted with 4x50 mL of dichloromethane. The combined organic layers were washed with 3x50 mL of water, 50 mL of brine, dried over anhydrous sodium sulfate, filtered and concentrated *in vacuo.* The residue was submitted to flash column chromatography using Kieselgel 60 (0.040-0.063 mm) as adsorbent (Merck) and hexanes:ethyl acetate = 4:1 as eluent to yield 0.71 g (48 %) of the title compound as a yellow amorphous solid. MS (EI) 350.1 [M+Na]⁺.

### c) 3-Chloro-1-[4-(hydroxyimino-methyl)-phenyl]-1H-indole-2-carboxylic acid ethyl ester

To a solution of 0.7 g (2.13 mmol) of 3-chloro-1-(4-formyl-phenyl)-1*H*-indole-2-carboxylic acid ethyl ester in 10 mL of ethanol 0.72 g (8.54 mmol) of hydroxylamine hydrochloride and 1.5 mL (10.7 mmol) of triethylamine were added. The reaction mixture was stirred at room temperature for 1h and evaporated *in vacuo.* The residue was submitted to flash column chromatography using Kieselgel 60 (0.015-0.040 mm) as adsorbent (Merck) and hexane:ethyl acetate = 2:1 as eluent to yield 0.53 g (72 %) of the title compound as a white amorphous solid. MS (EI) 343.1 (MH⁺).

### d) 1-(4-Aminomethyl-phenyl)-3-chloro-1H-indole-2-carboxylic acid ethyl ester

A stirred mixture of 0.53 g (1.55 mmol) of 3-chloro-1-[4-(hydroxyimino-methyl)-phenyl]-1*H*-indole-2-carboxylic acid ethyl ester and 0.10 g of 10% Pd/C, 50 mL of ethanol and 10 mL of 2.44 M hydrogen chloride in ethyl acetate was hydrogenated at room temperature for 3 h. The reaction mixture was filtered through Celite and the filtrate was concentrated *in vacuo* to yield 0.56 g (100 %) of the title compound as a yellow solid. MS (EI) 312.1 ([M-NH₂]⁺).

### e) 3-Chloro-1-{4-[({1-[(3-methoxy-isoxazole-5-carbonyl)-amino]-cyclopropane-carbonyl}-amino)-methyl]-phenyl}-1H-indole-2-carboxylic acid ethyl ester

To a solution of 0.28 g (0.77 mmol) of 1-(4-aminomethyl-phenyl)-3-chloro-1*H-*indole-2-carboxylic acid ethyl ester in 5 mL of dichloromethane 0.17 g (0.85 mmol) of 1-[(3-methoxy-isoxazole-5-carbonyl)-amino]-cyclopropanecarboxylic acid (P. D. O'Shea et al. J. Org. Chem. 2009, 74, 4547-4553), 0.3 g (1.53 mmol) of EDC, 0.006 g (0.04 mmol) of hydroxybenzotriazole monohydrate and 0.86 mL (6.1 mmol) of triethylamine were added. The reaction mixture was stirred at room temperature overnight. The mixture was diluted with 100 mL of dichloromethane and washed with 2x20 mL of saturated sodium hydrogencarbonate aqueous solution, 2x20 mL of water and 20 mL of brine, dried over anhydrous sodium sulfate, filtered and concentrated *in vacuo.* The residue was submitted to flash column chromatography using Kieselgel 60 (0.040-0.063 mm) as adsorbent (Merck) and chloroform:methanol = 95:5 as eluent to yield 0.38 g (92 %) of the title compound as an off-white foam. MS (EI) 538.2 (MH⁺).

### Example 35

### 3-Chloro-1-{4-[({1-[(3-methoxy-isoxazole-5-carbonyl)-amino]-cyclopropane-carbonyl}-amino)-methyl]-phenyl}-1H-indole-2-carboxylic acid dimethylamide

### a) 3-Chloro-1-{4-[({1-[(3-methoxy-isoxazole-5-carbonyl)-amino]-cyclopropanecarbonyl}-amino)-methyl]-phenyl}-1H-indole-2-carboxylic acid

To a solution of 0.33 g (0.61 mmol) of 3-chloro-1-{4-[({1-[(3-methoxy-isoxazole-5-carbonyl)-amino]-cyclopropanecarbonyl}-amino)-methyl]-phenyl}-1*H-*indole-2-carboxylic acid ethyl ester (Example 34e) in a mixture of 1 mL of methanol, 2 mL of tetrahydrofuran and 1 mL of water 0.128 g (0.3 mmol) of lithium hydroxide monohydrate was added. The reaction mixture was stirred at room temperature overnight. The reaction mixture was diluted with 2 mL of water and the pH was adjusted to 5 by the addition of 1M aqueous hydrochloric acid solution and the so obtained mixture was extracted with 50 mL of chloroform. The organic layer was washed with 3x8 mL of water and 8 mL of brine, dried over anhydrous sodium sulfate, filtered and *concentrated in vacuo.* The residue was submitted to flash column chromatography using Kieselgel 60 (0.015-0.040 mm) as adsorbent (Merck) and chloroform:methanol:acetic acid = 90:10:1 as eluent to yield 0.23 g (74 %) of the title compound as amorphous solid. MS (EI) 509.1 (MH⁺).

### b) 3-Chloro-1-{4-[({1-[(3-methoxy-isoxazole-5-carbonyl)-amino]-cyclopropane-carbonyl}-amino)-methyl]-phenyl}-1H-indole-2-carboxylic acid dimethylamide

To a solution of 0.15 g (0.29 mmol) of 3-chloro-1-{4-[({1-[(3-methoxy-isoxazole-5-carbonyl)-amino]-cyclopropanecarbonyl}-amino)-methyl]-phenyl}-1*H-*indole-2-carboxylic acid in 5 mL of *N*,*N-*dimethylformamide 0.13 g (0.33 mmol) of HBTU, 0.048 g (0.59 mmol) of dimethylamine hydrochloride and 0.17 mL (1.18 mmol) of triethylamine were added and the reaction mixture was stirred at room temperature for 1 h. The mixture was *concentrated in vacuo.* The oily residue was triturated with 50 mL of saturated sodium hydrogencarbonate solution, the precipitated solid was filtered off, washed with saturated sodium hydrogencarbonate solution and water to yield after drying 0.1 g (66 %) of the title compound as amorphous solid. MS (EI) 536.2 (MH⁺).

### Example 36

### 1-(2,2,2-Trifluoro-acetylamino)-cyclopropanecarboxylic acid 4-[2-(2-methyl-2H-tetrazol-5-yl)-indol-1-yl]-benzylamide

The title compound was prepared from 4-[2-(2-methyl-2*H*-tetrazol-5-yl)-indole-yl]-benzylamine hydrochloride (Reference Example 5) according to the method described in Example 4. MS (EI) 484.1 (MH⁺).

### Example 37

### 3-Methoxy-isoxazole-5-carboxylic acid (1-{4-[3-chloro-5-fluoro-2-(5-methyl-[1,3,4]oxadiazol-2-yl)-indol-1-yl]-benzylcarbamoyl}-cyclopropyl)-amide

### a) 3-Chloro-5-fluoro-1H-indole-2-carboxylic acid N'-acetyl-hydrazide

To a solution of 2.62 g (12.27 mmol) of 3-chloro-5-fluoro-1*H*-indole-2-carboxylic acid (US2005/20645) in 30 mL of *N*,*N-*dimethylformamide 1.19 g (16 mmol) of acethydrazide, 5.82 g (15.3 mmol) of HBTU and 2.2 mL (15.3 mmol) of triethylamine were added and the pH of the mixture was adjusted to 8 by the addition of triethylamine. After stirring the reaction mixture for 4 h at room temperature an additional amount of HBTU (1.16 g, 3 mmol) was added and the mixture was stirred overnight at room temperature. The mixture was concentrated *in vacuo,* the residue was treated with 60 mL of saturated sodium hydrogencarbonate solution, the resultant suspension was stirred for 45 min, then filtered. The filter cake was washed consecutively with saturated sodium hydrogencarbonate solution and water and dried to yield 2.59 g (0.78 %) of the title compound as an orange amorphous solid. MS (EI) 270.1 (MH⁺).

### b) 3-Chloro-5-fluoro-2-(5-methyl-[1,3,4]oxadiazol-2-yl)-1H-indole

A stirred mixture of 2.55 g (9.45 mmol) of 3-chloro-5-fluoro-1*H*-indole-2-carboxylic acid N'-acetyl-hydrazide and 20 mL of phosphorous oxychloride was refluxed for 90 min. The cold reaction mixture was poured into 250 g of crushed ice, the precipitated solid was filtered off and thoroughly washed with water. The reddish brown solid was suspended in methanol (5 mL), stirred at room temperature for 30 min, filtered and washed with cold methanol to yield after drying 1.93 g (81 %) of the title compound as a beige solid. MS (EI) 252.1 (MH⁺).

### c) {4-[3-Chloro-5-fluoro-2-(5-methyl-[1,3,4]oxadiazol-2-yl)-indol-1-yl]-benzyl}-carbamic acid tert-butyl ester

To a solution of 1.9 g (7.55 mmol) of 3-chloro-5-fluoro-2-(5-methyl-[1,3,4]oxadiazol-2-yl)-1*H*-indole in 40 mL of *N*,*N*-dimethylformamide 2.85 g (11.33 mmol) of 4-(*tert*-butoxycarbonylamino-methyl)-boronic acid, 2.75 g (15.1 mmol) of copper(II) acetate, 5.3 mL (30.2 mmol) of *N*,*N*-diisopropylethylamine and 4 g 3Å molecular sieves were added. The reaction mixture was vigorously stirred and bubbled with a stream of dry air at room temperature for 96 h. The mixture was filtered through Celite. The filter cake was washed with *N*,*N-*dimethylformamide and chloroform and the combined filtrates were *concentrated in vacuo.* The residue was treated with 300 mL of chloroform and extracted with 2x50 mL of 25% ammonium hydroxide solution and 2x50 mL of brine, dried over anhydrous sodium sulfate, filtered and concentrated *in vacuo.* The residue was submitted to flash column chromatography using Kieselgel 60 (0.040-0.063 mm) as adsorbent (Merck) and hexane:ethyl acetate = 3:1 as eluent and rechromatographed in chloroform:acetone = 5:1 to yield 1.99 g (57 %) of the title compound as a yellow foam. MS (EI) 457.1 [M+Na]⁺.

### d) 4-[3-Chloro-5-fluoro-2-(5-methyl-[1,3,4]oxadiazol-2-yl)-indol-1-yl]-benzylamine hydrochloride

To a solution of 1.54 g (3.37 mmol) of {4-[3-chloro-5-fluoro-2-(5-methyl-[1,3,4]oxadiazol-2-yl)-indol-1-yl]-benzyl}-carbamic acid *tert*-butyl ester in 5 mL of ethyl acetate and 5 mL of anisole 30 mL of 2.44 M hydrogen chloride in ethyl acetate was added at 0 °C and the mixture was stirred at room temperature for 2h. The precipitated solid was filtered off, washed with ethyl acetate and ether and dried to yield 1.3 g (98 %) of the title compound as a beige amorphous solid. MS (EI) 340.1 ([M-NH₂]⁺).

### e) 3-Methoxy-isoxazole-5-carboxylic acid (1-{4-[3-chloro-5-fluoro-2-(5-methyl-[1,3,4]oxadiazol-2-yl)-indol-1-yl]-benzylcarbamoyl}-cyclopropyl)-amide

To a solution of 0.24 g (0.61 mmol) of 4-[3-chloro-5-fluoro-2-(5-methyl-[1,3,4]oxadiazol-2-yl)-indol-1-yl]-benzylamine in 3 mL of dichloromethane 0.145 g (0.64 mmol) of 1-[(3-methoxy-isoxazole-5-carbonyl)-amino]-cyclopropanecarboxylic acid (P.D. O'Shea et al. J. Org. Chem. 2009, 74, 4547-4553), 0.24 g (1.22 mmol) of EDC, 0.005 g (0.032 mmol) of hydroxybenzotriazole monohydrate and 0.68 mL (4.9 mmol) of triethylamine were added. The reaction mixture was stirred at room temperature overnight. The mixture was diluted with 30 mL of dichloromethane and washed with 10 mL of saturated sodium hydrogencarbonate aqueous solution and 10 mL of brine, dried over anhydrous sodium sulfate, filtered and concentrated *in vacuo.* The residue was submitted to flash column chromatography using Kieselgel 60 (0.040-0.063 mm) as adsorbent (Merck) and chloroform:acetone = 9:1 as eluent to yield 0.13 g (38 %) of the title compound as amorphous solid. MS (EI) 565.2 (MH⁺).

### Example 38

### 3-Methoxy-isoxazole-5-carboxylic acid (1-{4-[3-chloro-2-(5-methyl-oxazol-2-yl)-indol-1-yl]-benzylcarbamoyl}-cyclopropyl)-amide

### a) 3-Chloro-2-(2-oxo-propylcarbamoyl)-indole

To a suspension of 0.3 g (1.53 mmol) of 3-chloro-1*H*-indole-2-carboxylic acid (Reference Example 4a) in 20 mL of dichloromethane and 0.05 mL of *N*,*N-*dimethylformamide 0.3 mL (3 mmol) of oxalyl chloride was added. The mixture was stirred at room temperature for 1 h, then refluxed for 1 h. The mixture was concentrated *in vacuo* and redissolved in 20 mL of dichloromethane. Under inert gas atmosphere, 0.33 g (3.3 mmol) of 1-amino-propan-2-one hydrochloride and 0.9 mL (6.3 mmol) of triethylamine were added to the solution at 0°C. The suspension was stirred at room temperature for 1 h and diluted with 20 mL of dichloromethane and 10 mL of water. The solid product precipitated on standing was filtered off and dried to yield 0.19 g (50 %) of the title compound as a beige amorphous solid. MS (EI) 251.1 (MH⁺).

### b) 3-Chloro-2-(5-methyl-oxazol-2-yl)-indole

To a solution of 0.24 g (1 mmol) of Burgess-reagent in 6 mL of dry tetrahydrofuran 0.176 g (0.71 mmol) of 3-chloro-2-(2-oxo-propylcarbamoyl)-indole was added in argon athmosphere and the mixture was heated in a microwave oven (CEM) at 120 °C for 17 minutes. The mixture was diluted with 2 mL of methanol, the suspension was filtered and the filtrate was diluted with 40 mL of dichloromethane, extracted with 2x10 mL of water and 10 mL of brine, dried over anhydrous sodium sulfate, filtered and concentrated *in vacuo.* The residue was submitted to flash column chromatography using Kieselgel 60 (0.040-0.063 mm) as adsorbent (Merck) and hexane:ethyl acetate = 4:1 as eluent to yield 0.135 g (82 %) of the title compound as a yellow amorphous solid. MS (EI) 233.1 (MH⁺).

### c) {4-[3-Chloro-2-(5-methyl-oxazol-2-yl)-indol-1-yl]-benzyl}-carbamic acid tert-butyl ester

To a solution of 0.1 g (0.42 mmol) of 3-chloro-2-(5-methyl-oxazol-2-yl)-indole in 4 mL of *N*,*N*-dimethylformamide 0.16 g (0.63 mmol) of 4-(*tert-*butoxycarbonylamino-methyl)-boronic acid, 0.16 g (0.84 mmol) of copper(II) acetate, 0.3 mL (1.68 mmol) of *N*,*N*-diisopropylethylamine and 0.4 g of 3Å molecular sieves were added. The reaction mixture was vigorously stirred and bubbled with a stream of dry air at room temperature for 6 days. The mixture was filtered through Celite, the filter cake was washed with *N*,*N*-dimethylformamide and concentrated *in vacuo.* The residue was submitted to flash column chromatography using Kieselgel 60 (0.040-0.063 mm) as adsorbent (Merck) and hexane:ethyl acetate = 4:1 as eluent to yield 0.16 g (89 %) of the title compound as a yellow foam. MS (EI) 438.2 (MH⁺).

### d) 4-[3-Chloro-2-(5-methyl-oxazol-2-yl)-indol-1-yl]-benzylamine

To a solution of 0.16 g (0.37 mmol) of {4-[3-chloro-2-(5-methyl-oxazol-2-yl)-indol-1-yl]-benzyl}-carbamic acid *tert*-butyl ester in 1 mL of ethyl acetate and 1 mL of anisole 5 mL of 2.44 M hydrogen chloride in ethyl acetate was added at 0 °C and the mixture was stirred at room temperature for 90 min. The precipitated solid was filtered off, washed with ethyl acetate and ether, dried and submitted to flash column chromatography using Kieselgel 60 (0.040-0.063 mm) as adsorbent (Merck) and chloroform:methanol:ammonium hydroxide solution (25%) = 9:1:0.1 as eluent to yield 0.097 g (78 %) of the title compound as a beige amorphous solid. MS (EI) 338.2 (MH⁺).

### e) 3-Methoxy-isoxazole-5-carboxylic acid (1-{4-[3-chloro-2-(5-methyl-oxazol-2-yl)-indol-1-yl]-benzylcarbamoyl}-cyclopropyl)-amide

To a solution of 0.097 g (0.28 mmol) of 4-[3-chloro-2-(5-methyl-oxazol-2-yl)-indol-1-yl]-benzylamine in 3 mL of dichloromethane 0.068 g (0.3 mmol) of 1-[(3-methoxy-isoxazole-5-carbonyl)-amino]-cyclopropanecarboxylic acid (P. D. O'Shea et al. J. Org. Chem. 2009, 74, 4547-4553), (0.11 g (0.57 mmol) of EDC, 0.002 g (0.015 mmol) of hydroxybenzotriazole monohydrate and 0.24 mL (1.72 mmol) of triethylamine were added. The reaction mixture was stirred at room temperature for 48 h. The mixture was diluted with 20 mL of dichloromethane and washed with 2x5 mL of water and 10 mL of brine, dried over anhydrous sodium sulfate, filtered and concentrated *in vacuo.* The residue was submitted to flash column chromatography using Kieselgel 60 (0.040-0.063 mm) as adsorbent (Merck) and chloroform:methanol = 98:2 as eluent to yield after trituration with water, filtration and drying 0.046 g (29 %) of the title compound as amorphous solid. MS (EI) 546.2 (MH⁺).

### Example 39

### 3-Methoxy-isoxazole-5-carboxylic acid {1-[4-(3-chloro-2-cyano-indol-1-yl)-benzyl-carbamoyl]-cyclopropyl}-amide

The title compound was prepared from 1-(4-aminomethyl-phenyl)-3-chloro-1*H-*indole-2-carbonitrile hydrochloride (Reference Example 14) according to the method described in Example 2. MS (EI) 490.1 (MH⁺).

### Example 40

### (R)-1-(2,2,2-Trifluoro-acetylamino)-cyclopropanecarboxylic acid (1-{4-[3-chloro-2-(5-methyl-[1,2,4]oxadiazol-3-yl)-indol-1-yl]-phenyl}-ethyl)-amide

The title compound was prepared from (*R*)-1-{4-[3-chloro-2-(5-methyl-[1,2,4]oxadiazol-3-yl)-indol-1-yl]-phenyl}-ethylamine hydrochloride (Reference Example 15) according to the method described in Example 4. MS (EI) 532.1 (MH⁺).

### Example 41

### (R)-3-Methoxy-isoxazole-5-carboxylic acid [1-(1-{4-[3-chloro-2-(5-methyl-[1,2,4]oxadiazol-3-yl)-indol-1-yl]-phenyl}-ethylcarbamoyl)-cyclopropyl]-amide

The title compound was prepared from (*R*)-1-{4-[3-chloro-2-(5-methyl-[1,2,4]oxadiazol-3-yl)-indol-1-yl]-phenyl}-ethylamine hydrochloride (Reference Example 15) according to the method described in Example 2. MS (EI) 561.2 (MH⁺).

### Example 42

### 3-Propyl-isoxazole-5-carboxylic acid (1-{4-[3-chloro-2-(5-methyl-[1,2,4]oxadiazol-3-yl)-indol-1-yl]-benzylcarbamoyl}-cyclopropyl)-amide

A mixture of 0.06 g (0.13 mmol) of 1-amino-cyclopropanecarboxylic acid 4-[3-chloro-2-(5-methyl-[1,2,4]oxadiazol-3-yl)-indol-1-yl]-benzylamide hydrochloride (Reference Example 9), 0.022 g (0.142 mmol) of 3-propyl-isoxazole-5-carboxylic acid, 0.08 mL (0.575 mmol) of triethylamine, 0.054 g (0.142 mmol) of HBTU and 1.1 mL of *N*,*N*-dimethylformamide was stirred at room temperature for 2 h, then concentrated *in vacuo.* The residue was dissolved in dichloromethane and successively washed with saturated sodium hydrogencarbonate solution, water and brine, dried over anhydrous sodium sulfate and concentrated. The residue was submitted to column chromatography using Kieselgel 60 (0.015-0.040 mm) as adsorbent (Merck) and toluene:acetone = 4:1 as eluent to yield 0.04 g (55%) of the title compound as white crystals. MS (EI) 559.2 (MH⁺).

### Example 43

### 3-Methoxy-isoxazole-5-carboxylic acid {1-[4-(5-chloro-2-cyano-indol-1-yl)-benzylcarbamoyl]-cyclopropyl}-amide

The title compound was prepared from 1-(4-aminomethyl-phenyl)-5-chloro-1*H-*indole-2-carbonitrile hydrochloride (Reference Example 16) according to the method described in Example 2. MS (EI) 490.2 (MH⁺).

### Example 44

### N-{1-[4-(2-Cyano-5-methoxy-indol-1-yl)-benzylcarbamoyl]-cyclopropyl}-5-trifluoromethyl-nicotinamide

The title compound was prepared from 1-amino-cyclopropanecarboxylic acid 4-(2-cyano-5-methoxy-indol-1-yl)-benzylamide hydrochloride (Reference Example 17) and 5-trifluoromethyl-nicotinic acid according to the method described in Example 42. MS (EI) 534.2 (MH⁺).

### Example 45

### N-{1-[4-(2-Cyano-5-fluoro-indol-1-yl)-benzylcarbamoyl]-cyclopropyl}-5-trifluoromethyl-nicotinamide

The title compound was prepared from 1-amino-cyclopropanecarboxylic acid 4-(2-cyano-5-fluoro-indol-1-yl)-benzylamide hydrochloride (Reference Example 18) and 5-trifluoromethyl-nicotinic acid according to the method described in Example 42. MS (EI) 522.2 (MH⁺).

### Example 46

### N-(1-{4-[5-Fluoro-2-(5-methyl-[1,2,4]oxadiazol-3-yl)-indol-1-yl]-benzylcarbamoyl}-cyclopropyl)-5-trifluoromethyl-nicotinamide

The title compound was prepared from 1-amino-cyclopropanecarboxylic acid 4-[5-fluoro-2-(5-methyl-[1,2,4]oxadiazol-3-yl)-indol-1-yl]-benzylamide hydrochloride (Reference Example 19) and 5-trifluoromethyl-nicotinic acid according to the method described in Example 42. MS (EI) 579.2 (MH⁺).

### Example 47

### 3-Methoxy-isoxazole-5-carboxylic acid (1-{4-[5-fluoro-2-(5-methyl-[1,2,4]oxadiazol-3-yl)-indol-1-yl]-benzylcarbamoyl}-cyclopropyl)-amide

The title compound was prepared from 1-amino-cyclopropanecarboxylic acid 4-[5-fluoro-2-(5-methyl-[1,2,4]oxadiazol-3-yl)-indol-1-yl]-benzylamide hydrochloride (Reference Example 19) and 3-methoxy-isoxazole-5-carboxylic acid according to the method described in Example 42. MS (EI) 531.2 (MH⁺).

### Example 48

### 3-Methoxy-isoxazole-5-carboxylic acid {1-[4-(2-cyano-5-methoxy-indol-1-yl)-benzylcarbamoyl]-cyclopropyl}-amide

The title compound was prepared from 1-amino-cyclopropanecarboxylic acid 4-(2-cyano-5-methoxy-indol-1-yl)-benzylamide hydrochloride (Reference Example 17) and 3-methoxy-isoxazole-5-carboxylic acid according to the method described in Example 42. MS (EI) 486.2 (MH⁺).

### Example 49

### 3-Methoxy-isoxazole-5-carboxylic acid {1-[4-(2-cyano-5-fluoro-indol-1-yl)-benzylcarbamoyl]-cyclopropyl}-amide

The title compound was prepared from 1-amino-cyclopropanecarboxylic acid 4-(2-cyano-5-fluoro-indol-1-yl)-benzylamide hydrochloride (Reference Example 18) and 3-methoxy-isoxazole-5-carboxylic acid according to the method described in Example 42. MS (EI) 474.2 (MH⁺).

### Example 50

### N-(1-{4-[3-Chloro-5-methoxy-2-(3-methyl-[1,2,-4]oxadiazol-5-yl)-indol-1-yl]-benzylcarbamoyl}-cyclopropyl)-5-trifluoromethyl-nicotinamide

The title compound was prepared from 1-amino-cyclopropanecarboxylic acid 4-[3-chloro-5-methoxy-2-(3-methyl-[1,2,4]oxadiazol-5-yl)-indol-1-yl]-benzylamide hydrochloride (Reference Example 20) and 5-trifluoromethyl-nicotinic acid according to the method described in Example 42. MS (EI) 625.2 (MH⁺).

### Example 51

### 3-Methoxy-isoxazole-5-carboxylic acid (1-{4-[3-chloro-5-methoxy-2-(3-methyl-[1,2,4]oxadiazol-5-yl)-indol-1-yl]-benzylcarbamoyl}-cyclopropyl)-amide

The title compound was prepared from 1-amino-cyclopropanecarboxylic acid 4-[3-chloro-5-methoxy-2-(3-methyl-[1,2,4]oxadiazol-5-yl)-indol-1-yl]-benzylamide hydrochloride (Reference Example 20) and 3-methoxy-isoxazole-5-carboxylic acid according to the method described in Example 42. MS (EI) 577.2 (MH⁺).

### Example 52

### 3-Methoxy-isoxazole-5-carboxylic acid {1-[4-(3-chloro-2-cyano-5-methoxy-indol-1-yl)-benzylcarbamoyl]-cyclopropyl}-amide

The title compound was prepared from 1-(4-aminomethyl-phenyl)-3-chloro-5-methoxy-1*H*-indole-2-carbonitrile hydrochloride (Reference Example 21) according to the method described in Example 2. MS (EI) 520.2 (MH⁺).

### Example 53

### 3-Methoxy-isoxazole-5-carboxylic acid (1-{4-[5-chloro-2-(5-methyl-[1,2,4]oxadiazol-3-yl)-indol-1-yl]-benzylcarbamoyl}-cyclopropyl)-amide

The title compound was prepared from 4-[5-chloro-2-(5-methyl-[1,2,4]oxadiazol-3-yl)-indol-1-yl]-benzylamine hydrochloride (Reference Example 22) according to the method described in Example 2. MS (EI) 547.2 (MH⁺).

### Example 54

### 3-Chloro-1-[3-fluoro-4-({[1-(2,2,2-trifluoro-acetylamino)-cyclopropanecarbonyl]-amino}-methyl)-phenyl]-1H-indole-2-carboxylic acid methyl ester

The title compound was prepared from 1-(4-aminomethyl-3-fluoro-phenyl)-3-chloro-1*H*-indole-2-carboxylic acid methyl ester hydrochloride (Reference Example 23) according to the method described in Example 4. MS (EI) 512.1 (MH⁺).

### Example 55

### 3-Chloro-1-{3-fluoro-4-[({1-[(3-methoxy-isoxazole-5-carbonyl)-amino]-cyclo-propanecarbonyl}-amino)-methyl]-phenyl}-1H-indole-2-carboxylic acid methyl ester

The title compound was prepared from 1-(4-aminomethyl-3-fluoro-phenyl)-3-chloro-1*H*-indole-2-carboxylic acid methyl ester hydrochloride (Reference Example 23) according to the method described in Example 2. MS (EI) 541.1 (MH⁺).

### Example 56

### 3-Methoxy-isoxazole-5-carboxylic acid [1-({5-[2-(3-methyl-[1,2,4]oxadiazol-5-yl)-indol-1-yl]-pyridin-2-ylmethyl}-carbamoyl)-cyclopropyl]-amide

### a) 2-(3-Methyl-[1,2,4]oxadiazol-5-yl)-1H-indole

The title compound was prepared from 1*H*-indole-2-carboxylic acid methyl ester according to the method described in Reference Example 20d. MS (EI) 200.1 (MH⁺).

### b) {5-[2-(3-Methyl-[1,2,4]oxadiazol-5-yl)-indol-1-yl]-pyridin-2-ylmethyl}-carbamic acid tert-butyl ester

To a solution of 0.46 g (2.3 mmol) of 2-(3-methyl-[1,2,4]oxadiazol-5-yl)-1*H-*indole in 10 mL of *N,N*-dimethylformamide 0.58 g (2.3 mmol) of 6-(*tert-*butoxycarbonylamino-methyl)-3-pyridineboronic acid (Reference Example 3), 0.84 g (4.6 mmol) of copper(II) acetate, 1.6 mL (9.2 mmol) of *N,N-*diisopropylethylamine and 3 g 3Å molecular sieves were added. The reaction mixture was vigorously shaken and bubbled with a stream of dry air at room temperature for 6 days. The mixture was filtered and subsequently washed with *N,N*-dimethylformamide, concentrated ammonium hydroxide solution and *N,N-*dimethylformamide and the filtrate was concentrated *in vacuo.* The residue was submitted to flash column chromatography using Kieselgel 60 (0.040-0.063 mm) as adsorbent (Merck) and hexane:ethyl acetate = 1:1 as eluent to yield 0.16 g (17%) of the title compound. MS (EI) 406.2 (MH⁺)

### c) C-{5-[2-(3-Methyl-[1,2,4]oxadiazol-5-yl)-indol-1-yl]-pyridin-2-yl}-methylamine dihydrochloride

To a solution of 0.16 g (0.395 mmol) of {5-[2-(3-methyl-[1,2,4]oxadiazol-5-yl)-indol-1-yl]-pyridin-2-ylmethyl}-carbamic acid *tert*-butyl ester in 2 mL of ethyl acetate and 0.8 mL of anisole 5 mL of 2.44 M hydrogen chloride in ethyl acetate was added at 0 °C and the mixture was stirred at room temperature for 3 h. The precipitated solid was filtered off, washed with ethyl acetate and ether and dried to yield 0.11 g (73%) of the title compound. MS (EI) 306.2 (MH⁺)

### d) 3-Methoxy-isoxazole-5-carboxylic acid [1-({5-[2-(3-methyl-[1,2,4]oxadiazol-5-yl)-indol-1-yl]-pyridin-2-ylmethyl}-carbamoyl)-cyclopropyl]-amide

To a solution of 0.378 g (1 mmol) of C-{5-[2-(3-methyl-[1,2,4]oxadiazol-5-yl)-indol-1-yl]-pyridin-2-yl}-methylamine dihydrochloride in 14 mL of dichloromethane 0.237 g (1.05 mmol) of 1-[(3-methoxy-isoxazole-5-carbonyl)-amino]-cyclopropanecarboxylic acid (P.D. O'Shea et al. J. Org. Chem. 2009, 74, 4547-4553), 0.385 g (2 mmol) of EDC, 0.007 g (0.05 mmol) of hydroxybenzotriazole monohydrate and 1.25 mL (9 mmol) of triethylamine were added. The reaction mixture was stirred at room temperature overnight. The mixture was concentrated *in vacuo* and the residue was recrystallised from 2-propanol twice to yield 0.268 g (52%) of the title compound as a beige crystalline solid. MS (EI) 514.2 (MH⁺)

### Example 57

### 3-Methoxy-isoxazole-5-carboxylic acid [1-({5-[2-(5-methyl-[1,2,4]oxadiazol-3-yl)-indol-1-yl]-pyridin-2-ylmethyl}-carbamoyl)-cyclopropyl]-amide

### a) 2-(5-Methyl-[1,2,4]oxadiazol-3-yl)-1H-indole

The title compound was prepared from 1*H*-indole-2-carbonitrile according to the methods described in Reference Example 9a and 9b. MS (EI) 200.0 (MH⁺)

### b) {5-[2-(5-Methyl-[1,2,4]oxadiazol-3-yl)-indol-1-yl]-pyridin-2-ylmethyl}-carbamic acid tert-butyl ester

The title compound was prepared from 2-(5-methyl-[1,2,4]oxadiazol-3-yl)-1*H-*indole according to the method described in Example 56b. MS (EI) 406.2 (MH⁺)

### c) C-{5-[2-(5-Methyl-[1,2,4]oxadiazol-3-yl)-indol-1-yl]-pyridin-2-yl}-methylamine dihydrochloride

The title compound was prepared from {5-[2-(5-methyl-[1,2,4]oxadiazol-3-yl)-indol-1-yl]-pyridin-2-ylmethyl}-carbamic acid *tert*-butyl ester according to the method described in Example 56c. MS (EI) 306.2 (MH⁺)

### d) 3-Methoxy-isoxazole-5-carboxylic acid [1-({5-[2-(5-methyl-[1,2,4]oxadiazol-3-yl)-indol-1-yl]-pyridin-2-ylmethyl}-carbamoyl)-cyclopropyl]-amide

The title compound was prepared from C-{5-[2-(5-methyl-[1,2,4]oxadiazol-3-yl)-indol-1-yl]-pyridin-2-yl}-methylamine dihydrochloride according to the method described in Example 56d. MS (EI) 514.2 (MH⁺).

### Example 58

### 3-Methoxy-isoxazole-5-carboxylic acid [1-({5-[3-chloro-2-(3-methyl-[1,2,4]oxadiazol-5-yl)-indol-1-yl]-pyridin-2-ylmethyl}-carbamoyl)-cyclopropyl]-amide

### a) 3-Chloro-2-(3-methyl-[1,2,4]oxadiazol-5-yl)-1H-indole

The title compound was prepared from 3-chloro-1*H*-+indole-2-carboxylic acid methyl ester according to the method described in Reference Example 20d. MS (EI) 234.1 (MH⁺).

### b) {5-[3-Chloro-2-(3-methyl-[1,2,4]oxadiazol-5-yl)-indol-1-yl]-pyridin-2-ylmethyl}-carbamic acid tert-butyl ester

The title compound was prepared from 3-chloro-2-(3-methyl-[1,2,4]oxadiazol-5-yl)-1*H*-indole according to the method described in Example 56b. MS (EI) 440.2 (MH⁺)

### c) C-{5-[3-Chloro-2-(3-methyl-[1,2,4]oxadiazol-5-yl)-indol-1-yl]-pyridin-2-yl}-methyl-amine dihydrochloride

The title compound was prepared from {5-[3-chloro-2-(3-methyl-[1,2,4]oxadiazol-5-yl)-indol-1-yl]-pyridin-2-ylmethyl}-carbamic acid *tert*-butyl ester according to the method described in Example 56c. MS (EI) 340.1 (MH⁺)

### d) 3-Methoxy-isoxazole-5-carboxylic acid [1-({5-[3-chloro-2-(3-methyl-[1,2,4]oxadiazol-5-yl)-indol-1-yl]-pyridin-2-ylmethyl}-carbamoyl)-cyclopropyl]-amide

The title compound was prepared from C-{5-[3-chloro-2-(3-methyl-[1,2,4]oxadiazol-5-yl)-indol-1-yl]-pyridin-2-yl}-methylamine dihydrochloride according to the method described in Example 56d. MS (EI) 548.2 (MH⁺).

### Example 59

### 3-Methoxy-isoxazole-5-carboxylic acid [1-({5-[3-chloro-5-fluoro-2-(2-methyl-2H-tetrazol-5-yl)-indol-1-yl]-pyridin-2-ylmethyl}-carbamoyl)-cyclopropyl]-amide

### a) 3-Chloro-5-fluoro-2-(2-methyl-2H-tetrazol-5-yl)-1H-indole

The title compound was prepared from 5-fluoro-1*H*-indole-2-carboxylic acid according to the methods described in Reference Example 4a-4d. MS (EI) 252.1 (MH⁺).

### b) {5-[3-Chloro-5-fluoro-2-(2-methyl-2H-tetrazol-5-yl)-indol-1-yl]-pyridin-2-ylmethyl}-carbamic acid tert-butyl ester

The title compound was prepared from 3-chloro-5-fluoro-2-(2-methyl-2*H-*tetrazol-5-yl)-1*H-*indole according to the method described in Example 56b. MS (EI) 458.2 (MH⁺)

### c) C-{5-[3-Chloro-5-fluoro-2-(2-methyl-2H-tetrazol-5-yl)-indol-1-yl]-pyridin-2-yl}-methylamine trihydrochloride

The title compound was prepared from {5-[3-chloro-5-fluoro-2-(2-methyl-2*H-*tetrazol-5-yl)-indol-1-yl]-pyridin-2-ylmethyl}-carbamic acid *tert*-butyl ester according to the method described in Example 56c. MS (EI) 358.2 (MH⁺)

### d) 3-Methoxy-isoxazole-5-carboxylic acid [1-({5-[3-chloro-5-fluoro-2-(2-methyl-2H-tetrazol-5-yl)-indol-1-yl]-pyridin-2-ylmethyl}-carbamoyl)-cyclopropyl]-amide

The title compound was prepared from C-{5-[3-chloro-5-fluoro-2-(2-methyl-2*H-*tetrazol-5-yl)-indol-1-yl]-pyridin-2-yl}-methylamine trihydrochloride according to the method described in Example 56d. MS (EI) 566.2 (MH⁺).

### Example 60

### 3-Methoxy-isoxazole-5-carboxylic acid [1-({5-[3-chloro-2-(2-methyl-2H-tetrazol-5-yl)-indol-1-yl]-pyridin-2-ylmethyl}-carbamoyl)-cyclopropyl]-amide

### a) {5-[3-Chloro-2-(2-methyl-2H-tetrazol-5-yl)-indol-1-yl]-pyridin-2-ylmethyl}-carbamic acid tert-butyl ester

The title compound was prepared from 3-chloro-2-(2-methyl-2*H*-tetrazol-5-yl)-1*H*-indole (Example 1d) according to the method described in Example 56b. MS (EI) 440.2 (MH⁺)

### b) C-{5-[3-Chloro-2-(2-methyl-2H-tetrazol-5-yl)-indol-1-yl]-pyridin-2-yl}-methyl-amine trihydrochloride

The title compound was prepared from {5-[3-chloro-2-(2-methyl-2*H*-tetrazol-5-yl)-indol-1-yl]-pyridin-2-ylmethyl}-carbamic acid *tert*-butyl ester according to the method described in Example 56c. MS (EI) 340.2 (MH⁺)

### c) 3-Methoxy-isoxazole-5-carboxylic acid [1-({5-[3-chloro-2-(2-methyl-2H-tetrazol-5-yl)-indol-1-yl]-pyridin-2-ylmethyl}-carbamoyl)-cyclopropyl]-amide

The title compound was prepared from C-{5-[3-chloro-2-(2-methyl-2*H*-tetrazol-5-yl)-indol-1-yl]-pyridin-2-yl}-methylamine trihydrochloride according to the method described in Example 56d. MS (EI) 548.2 (MH⁺).

### Example 61

### 3-Methoxy-isoxazole-5-carboxylic acid [1-({5-[5-fluoro-2-(5-methyl-[1,2,4]oxadiazol-3-yl)-indol-1-yl]-pyridin-2-ylmethyl}-carbamoyl)-cyclopropyl]-amide

### a) 5-Fluoro-2-(5-methyl-[1,2,4]oxadiazol-3-yl)-1H-indole

The title compound was prepared from 5-fluoro-1*H*-indole-2-carbonitrile (I. Borza at al. Bioorg. Med. Chem. Lett. **2005,** *15*, 5439-5441) according to the methods described in Reference Example 9a and 9b. MS (EI) 218.1 (MH⁺).

### b) {5-[5-Fluoro-2-(5-methyl-[1,2,4]oxadiazol-3-yl)-indol-1-yl]-pyridin-2-ylmethyl}-carbamic acid tert-butyl ester

The title compound was prepared from 5-fluoro-2-(5-methyl-[1,2,4]oxadiazol-3-yl)-1*H*-indole according to the method described in Example 56b. MS (EI) 424.2 (MH⁺)

### c) C-{5-[5-Fluoro-2-(5-methyl-[1,2,4]oxadiazol-3-yl)-indol-1-yl]-pyridin-2-yl}-methyl-amine dihydrochloride

The title compound was prepared from {5-[5-fluoro-2-(5-methyl-[1,2,4]oxadiazol-3-yl)-indol-1-yl]-pyridin-2-ylmethyl}-carbamic acid *tert*-butyl ester according to the method described in Example 56c. MS (EI) 324.2 (MH⁺)

### d) 3-Methoxy-isoxazole-5-carboxylic acid [1-({5-[5-fluoro-2-(5-methyl-[1,2,4]oxadiazol-3-yl)-indol-1-yl]-pyridin-2-ylmethyl}-carbamoyl)-cyclopropyl]-amide

The title compound was prepared from C-{5-[5-fluoro-2-(5-methyl-[1,2,4]oxadiazol-3-yl)-indol-1-yl]-pyridin-2-yl}-methylamine dihydrochloride according to the method described in Example 56d. MS (EI) 532.2 (MH⁺).

### Example 62

### 3-Methoxy-isoxazole-5-carboxylic acid [1-({5-[3-chloro-5-fluoro-2-(5-methyl-[1,2,4]oxadiazol-3-yl)-indol-1-yl]-pyridin-2-ylmethyl}-carbamoyl)-cyclopropyl]-amide

### a) 3-Chloro-5-fluoro-1H-indole-2-carbonitrile

The title compound was prepared from 5-fluoro-1*H*-indole-2-carboxylic acid according to the methods described in Reference Example 4a-c.

### b) 3-Chloro-5-fluoro-2-(5-methyl-[1,2,4]oxadiazol-3-yl)-1H-indole

The title compound was prepared from 3-chloro-5-fluoro-1*H*-indole-2-carbonitrile according to the methods described in Reference Example 9a and 9b.

### c) {5-[3-Chloro-5-fluoro-2-(5-methyl-[1,2,4]oxadiazol-3-yl)-indol-1-yl]-pyridin-2-ylmethyl}-carbamic acid tert-butyl ester

The title compound was prepared from 3-chloro-5-fluoro-2-(5-methyl-[1,2,4]oxadiazol-3-yl)-1*H*-indole according to the method described in Example 56b. MS (EI) 458.2 (MH⁺)

### d) C-{5-[3-Chloro-5-fluoro-2-(5-methyl-[1,2,4]oxadiazol-3-yl)-indol-1-yl]-pyridin-2-yl}-methylamine dihydrochloride

The title compound was prepared from {5-[3-chloro-5-fluoro-2-(5-methyl-[1,2,4]oxadiazol-3-yl)-indol-1-yl]-pyridin-2-ylmethyl}-carbamic acid *tert*-butyl ester according to the method described in Example 56c. MS (EI) 358.1 (MH⁺)

### e) 3-Methoxy-isoxazole-5-carboxylic acid [1-({5-[3-chloro-5-fluoro-2-(5-methyl-[1,2,4]oxadiazol-3-yl)-indol-1-yl]-pyridin-2-ylmethyl}-carbamoyl)-cyclopropyl]-amide

The title compound was prepared from C-{5-[3-chloro-5-fluoro-2-(5-methyl-[1,2,4]oxadiazol-3-yl)-indol-1-yl]-pyridin-2-yl}-methylamine dihydrochloride according to the method described in Example 56d. MS (EI) 566.2 (MH⁺).

### Example 63

### 1-(2,2,2-Trifluoro-acetylamino)-cyclopropanecarboxylic acid 4-[3-chloro-5-methoxy-2-(5-methyl-[1,2,4]oxadiazol-3-yl)-indol-1-yl]-benzylamide

To a stirred and ice-cooled solution of 0.166 g (0.36 mmol) of 4-[3-chloro-5-methoxy-2-(5-methyl-[1,2,4]oxadiazol-3-yl)-indol-1-yl]-benzylamine (Reference Example 24) in 2 mL of dichloromethane 0.093 g (0.47 mmol) of 1-(2,2,2-trifluoro-acetylamino)-cyclopropanecarboxylic acid (M. R. Hickey et al. Synlett. 2005, 255-258) and 0.103 g (0.54 mmol) of EDC were added. The reaction mixture was allowed to warm to room temperature and stirred for 4 h. The reaction mixture was diluted with dichloromethane and subsequently extracted with 0.1N hydrochloric acid solution, water, saturated aqueous sodium hydrogencarbonate solution and brine, dried over anhydrous magnesium sulfate, filtered and concentrated *in vacuo.* The residue was submitted to flash chromatography using Kieselgel 60 (0.040-0.063 mm) as absorbent (Merck) and hexane: ethyl acetate = 1:1 as eluent to yield 0.129 g (65%) of the title compound. MS (EI) 548.1 (MH⁺).

### Example 64

### 3-Methoxy-isoxazole-5-carboxylic acid (1-{4-[3-chloro-5-methoxy-2-(5-methyl-[1,2,4]oxadiazol-3-yl)-indol-1-yl]-benzylcarbamoyl}-cyclopropyl)-amide

To a stirred and ice-cooled solution of 0.166 g (0.36 mmol) of 4-[3-chloro-5-methoxy-2-(5-methyl-[1,2,4]oxadiazol-3-yl)-indol-1-yl]-benzylamine (Reference Example 24) in 2 mL of dichloromethane 0.107 g (0.47 mmol) of 1-[3-methoxy-isoxazole-5-carbonyl)-amino]-cyclopropanecarboxylic acid (P.D. O'Shea et al. J. Org. Chem. 2009, 74, 4547-4553) and 0.103 g (0.54 mmol) of EDC were added. The reaction mixture was allowed to warm to room temperature and stirred for 4 h. The reaction mixture was diluted with dichloromethane and subsequently extracted with 0.1N hydrochloric acid solution, water, saturated aqueous sodium hydrogencarbonate solution and brine, dried over anhydrous magnesium sulfate, filtered and concentrated *in vacuo.* The residue was submitted to flash chromatography using Kieselgel 60 (0.040-0.063 mm) as absorbent (Merck) and hexane: ethyl acetate = 1:1 as eluent to yield 0.132 g (64%) of the title compound. MS (EI) 577.1 (MH⁺).

### Example 65

### 1-(2,2,2-Trifluoro-acetylamino)-cyclopropanecarboxylic acid 4-[3,5-dichloro-2-(5-methyl-[1,2,4]oxadiazol-3-yl)-indol-1-yl]-benzylamide

The title compound was prepared from 4-[3,5-dichloro-2-(5-methyl-[1,2,4]oxadiazol-3-yl)-indol-1-yl]-benzylamine (Reference Example 27a) according to the method described in Example 63. MS (EI) 552.1 (MH⁺).

### Example 66

### 3-Methoxy-isoxazole-5-carboxylic acid (1-{4-[3,5-dichloro-2-(5-methyl-[1,2,4]oxadiazol-3-yl)-indol-1-yl]-benzylcarbamoyl}-cyclopropyl)-amide

The title compound was prepared from 4-[3,5-dichloro-2-(5-methyl-[1,2,4]oxadiazol-3-yl)-indol-1-yl]-benzylamine (Reference Example 27a) according to the method described in Example 64. MS (EI) 581.1 (MH⁺).

### Example 67

### 3-Methoxy-isoxazole-5-carboxylic acid (1-{4-[3-chloro-5-fluoro-2-(5-methyl-[1,2,4]oxadiazol-3-yl)-indol-1-yl]-benzylcarbamoyl}-cyclopropyl)-amide

The title compound was prepared from 5-fluoro-1*H-*indole-2-carboxylic acid (Reference Example 28a) according to the method described in Example 64. MS (EI) 565.1 (MH⁺).

### Example 68

### 3-Chloro-4-fluoro-1-{4-[({1-[(3-methoxy-isoxazole-5-carbonyl)-amino]-cyclo-propanecarbonyl}-amino)-methyl]-phenyl}-1H-indole-2-carboxylic acid methyl ester

The title compound was prepared from 1-(4-aminomethyl-phenyl)-3-chloro-4-fluoro-1*H*-indole-2-carboxylic acid methyl ester (Reference Example 25) according to the method described in Example 64. MS (EI) 541 (MH⁺).

### Example 69

### 3-Chloro-4-fluoro-1-[4-({[1-(2,2,2-trifluoro-acetylamino)-cyclopropanecarbonyl]-amino}-methyl)-phenyl]-1H-indole-2-carboxylic acid methyl ester

The title compound was prepared from 1-(4-aminomethyl-phenyl)-3-chloro-4-fluoro-1*H*-indole-2-carboxylic acid methyl ester (Reference Example 25) according to the method described in Example 63. MS (EI) 512 (MH⁺).

### Example 70

### 2-Chloro-N-(1-{4-[3,5-dichloro-2-(5-methyl-[1,2,4]oxadiazol-3-yl)-indol-1-yl]-benzylcarbamoyl}-cyclopropyl)-nicotinamide

To a solution of 31.51 mg (0.2 mmol) of 2-chloro-nicotinic acid in 2 mL of dichloromethane and 0.2 mL of *N,N-*dimethylformamide 45.63 mg (0.1 mmol) of 1-amino-cyclopropanecarboxylic acid 4-[3,5-dichloro-2-(5-methyl-[1,2,4]oxadiazol-3-yl)-indol-1-yl]-benzylamide (Reference Example 27), 76 mg (0.2 mmol) of HATU and 104.6 µL (0.6 mmol) of *N,N-*diisopropylethylamine (DIPEA) were added. The mixture was shaken for 8 h at room temperature, then purified by column chromatography using n-hexane and ethyl acetate as eluent to yield 26 mg (43.6%) of the title compound. MS (EI) 597.1 (MH⁺).

Compounds of Table 2 were prepared according to the method described in Example 70.

**Table 2**

| **Example** | Name | Starting Material | MS (EI) (MH⁺) |
|---|---|---|---|
| **71** | *N*-(1-{4-[3,5-Dichloro-2-(5-methyl-[1,2,4]oxadiazol-3-yl)-indol-1-yl]-benzylcarbamoyl}-cyclopropyl)-3-fluoro-benzamide | Reference Example 27 | 579.2 |
| **72** | 5-Methyl-pyrazine-2-carboxylic acid (1-{4-[3,5-dichloro-2-(5-methyl-[1,2,4]oxadiazol-3-yl)-indol-1-yl]-benzylcarbamoyl}-cyclopropyl)-amide | Reference Example 27 | 577.1 |
| **73** | 5-Methyl-isoxazole-3-carboxylic acid (1-{4-[3,5-dichloro-2-(5-methyl-[1,2,4]oxadiazol-3-yl)-indol-1-yl]-benzylcarbamoyl}-cyclopropyl)-amide | Reference Example 27 | 566.1 |
| **74** | *N*-(1-{4-[3,5-Dichloro-2-(5-methyl-[1,2,4]oxadiazol-3-yl)-indol-1-yl]-benzylcarbamoyl}-cyclopropyl)-2-fluoro-isonicotinamide | Reference Example 27 | 580.1 |
| **75** | 5-Cyclopropyl-isoxazole-3-carboxylic acid (1-{4-[3,5-dichloro-2-(5-methyl-[1,2,4]oxadiazol-3-yl)-indol-1-yl]-benzylcarbamoyl}-cyclopropyl)-amide | Reference Example 27 | 592.1 |
| **76** | *N*-(1-{4-[3,5-Dichloro-2-(5-methyl-[1,2,4]oxadiazol-3-yl)-indol-1-yl]-benzylcarbamoyl}-cyclopropyl)-5-trifluoromethyl-nicotinamide | Reference Example 27 | 630.1 |
| **77** | 2-Methylsulfanyl-pyrimidine-5-carboxylic acid (1-{4-[3-chloro-2-(5-methyl-[1,2,4]oxadiazol-3-yl)-indol-1-yl]-benzylcarbamoyl}-cyclopropyl)-amide | Reference Example 9 | 575.2 |
| **78** | 1-(3,3,3-Trifluoro-propionylamino)-cyclopropanecarboxylic acid 4-[5-methoxy-2-(3-methyl-[1,2,4]oxadiazol-5-yl)-indol-1-yl]-benzylamide | Reference Example 30 | 528.2 |
| **79** | *N*-(1-{4-[5-Methoxy-2-(3-methyl-[1,2,4]oxadiazol-5-yl)-indol-1-yl]-benzylcarbamoyl}-cyclopropyl)-5-trifluoromethyl-nicotinamide | Reference Example 30 | 591.2 |
| **80** | 3-Methoxy-isoxazole-5-carboxylic acid (1-{4-[5-methoxy-2-(3-methyl-[1,2,4]oxadiazol-5-yl)-indol-1-yl]-benzylcarbamoyl}-cyclopropyl)-amide | Reference Example 30 | 543.2 |
| **81** | 1-(3,3,3-Trifluoro-propionylamino)-cyclopropanecarboxylic acid 4-[2-(5-methyl-[1,2,4]oxadiazol-3-yl)-indol-1-yl]-benzylamide | Reference Example 36 | 498.2 |
| **82** | *N*-(1-{4-[2-(5-Methyl-[1,2,4]oxadiazol-3-yl)-indol-1-yl]-benzylcarbamoyl}-cyclopropyl)-5-trifluoromethyl-nicotinamide | Reference Example 36 | 561.2 |
| **83** | *N*-(1-{4-[2-(3-Methyl-[1,2,4]oxadiazol-5-yl)-indol-1-yl]-benzylcarbamoyl}-cyclopropyl)-5-trifluoromethyl-nicotinamide | Reference Example 37 | 561.2 |
| **84** | *N*-{1-[4-(2-Cyano-indol-1-yl)-benzylcarbamoyl]-cyclopropyl}-5-trifluoromethyl-nicotinamide | Reference Example 38 | 504.2 |
| **85** | 2-Chloro-*N*-(1-{4-[3-chloro-5-fluoro-2-(5-methyl-[1,2,4]oxadiazol-3-yl)-indol-1-yl]-benzylcarbamoyl}-cyclopropyl)-nicotinamide | Reference Example 28 | 580.1 |
| **86** | *N*-(1-{4-[3-Chloro-5-fluoro-2-(5-methyl-[1,2,4]oxadiazol-3-yl)-indol-1-yl]-benzylcarbamoyl}-cyclopropyl)-3-fluoro-benzamide | Reference Example 28 | 563.1 |
| **87** | 5-Methyl-pyrazine-2-carboxylic acid (1-{4-[3-chloro-5-fluoro-2-(5-methyl-[1,2,4]oxadiazol-3-yl)-indol-1-yl]-benzylcarbamoyl}-cyclopropyl)-amide | Reference Example 28 | 560.2 |
| **88** | 5-Methyl-isoxazole-3-carboxylic acid (1-{4-[3-chloro-5-fluoro-2-(5-methyl-[1,2,4]oxadiazol-3-yl)-indol-1-yl]-benzylcarbamoyl}-cyclopropyl)-amide | Reference Example 28 | 549.2 |
| **89** | *N*-(1-{4-[3-Chloro-5-fluoro-2-(5-methyl-[1,2,4]oxadiazol-3-yl)-indol-1-yl]-benzylcarbamoyl}-cyclopropyl)-2-fluoro-isonicotinamide | Reference Example 28 | 564.2 |
| **90** | 5-Cyclopropyl-isoxazole-3-carboxylic acid (1-{4-[3-chloro-5-fluoro-2-(5-methyl-[1,2,4]oxadiazol-3-yl)-indol-1-yl]-benzylcarbamoyl}-cyclopropyl)-amide | Reference Example 28 | 576.2 |
| **91** | *N*-(1-{4-[3-Chloro-5-fluoro-2-(5-methyl-[1,2,4]oxadiazol-3-yl)-indol-1-yl]-benzylcarbamoyl}-cyclopropyl)-5-trifluoromethyl-nicotinamide | Reference Example 28 | 614.1 |
| **92** | *N*-(1-{4-[3-Chloro-2-(5-methyl-[1,2,4]oxadiazol-3-yl)-indol-1-yl]-benzylcarbamoyl}-cyclopropyl)-2-fluoro-3-trifluoromethyl-benzamide | Reference Example 9 | 613.2 |
| **93** | *N*-(1-{4-[3-Chloro-2-(5-methyl-[1,2,4]oxadiazol-3-yl)-indol-1-yl]-benzylcarbamoyl}-cyclopropyl)-3-fluoro-4-trifluoromethyl-benzamide | Reference Example 9 | 613.2 |
| **94** | *N*-(1-{4-[3-Chloro-2-(5-methyl-[1,2,4]oxadiazol-3-yl)-indol-1-yl]-benzylcarbamoyl}-cyclopropyl)-3-fluoro-5-trifluoromethyl-benzamide | Reference Example 9 | 613.2 |
| **95** | *N*-(1-{4-[3-Chloro-2-(5-methyl-[1,2,4]oxadiazol-3-yl)-indol-1-yl]-benzylcarbamoyl}-cyclopropyl)-2-fluoro-nicotinamide | Reference Example 9 | 546.2 |
| **96** | *N*-(1-{4-[3-Chloro-2-(5-methyl-[1,2,4]oxadiazol-3-yl)-indol-1-yl]-benzylcarbamoyl}-cyclopropyl)-2-fluoro-5-trifluoromethyl-benzamide | Reference Example 9 | 613.2 |
| **97** | *N*-(1-{4-[3-Chloro-2-(5-methyl-[1,2,4]oxadiazol-3-yl)-indol-1-yl]-benzylcarbamoyl}-cyclopropyl)-3-fluoro-isonicotinamide | Reference Example 9 | 546.2 |
| **98** | *N*-(1-{4-[3-Chloro-2-(5-methyl-[1,2,4]oxadiazol-3-yl)-indol-1-yl]-benzylcarbamoyl}-cyclopropyl)-4-fluoro-3-trifluoromethyl-benzamide | Reference Example 9 | 613.2 |
| **99** | *N*-(1-{4-[3-Chloro-2-(5-methyl-[1,2,4]oxadiazol-3-yl)-indol-1-yl]-benzylcarbamoyl}-cyclopropyl)-6-fluoro-nicotinamide | Reference Example 9 | 545.2 |
| **100** | *N*-{1-[4-(5-Chloro-2-cyano-indol-1-yl)-benzylcarbamoyl]-cyclopropyl}-5-trifluoromethyl-nicotinamide | Reference Example 16 | 538.2 |
| **101** | 1-(3,3,3-Trifluoro-propionylamino)-cyclopropanecarboxylic acid 4-[5-fluoro-2-(3-methyl-[1,2,4]oxadiazol-5-yl)-indol-1-yl]-benzylamide | Reference Example 26 | 516.2 |
| **102** | *N*-(1-{4-[5-Fluoro-2-(3-methyl-[1,2,4]oxadiazol-5-yl)-indol-1-yl]-benzylcarbamoyl}-cyclopropyl)-5-trifluoromethyl-nicotinamide | Reference Example 26 | 579.2 |
| **103** | 3-Methoxy-isoxazole-5-carboxylic acid (1-{4-[5-fluoro-2-(3-methyl-[1,2,4]oxadiazol-5-yl)-indol-1-yl]-benzylcarbamoyl}-cyclopropyl)-amide | Reference Example 26 | 531.2 |
| **104** | 2-Chloro-*N*-(1-{4-[3-chloro-2-(5-methyl-[1,2,4]oxadiazol-3-yl)-indol-1-yl]-2-fluoro-benzylcarbamoyl}-cyclopropyl)-nicotinamide | Reference Example 9 | 579.1 |
| **105** | *N*-(1-{4-[3-Chloro-2-(5-methyl-[1,2,4]oxadiazol-3-yl)-indol-1-yl]-2-fluoro-benzylcarbamoyl}-cyclopropyl)-3-fluoro-benzamide | Reference Example 9 | 562.2 |
| **106** | 5-Methyl-pyrazine-2-carboxylic acid (1-{4-[3-chloro-2-(5-methyl-[1,2,4]oxadiazol-3-yl)-indol-1-yl]-2-fluoro-benzylcarbamoyl}-cyclopropyl)-amide | Reference Example 9 | 560.2 |
| **107** | 5-Methyl-isoxazole-3-carboxylic acid (1-{4-[3-chloro-2-(5-methyl-[1,2,4]oxadiazol-3-yl)-indol-1-yl]-2-fluoro-benzylcarbamoyl}-cyclopropyl)-amide | Reference Example 9 | 549.2 |
| **108** | 1-(3,3,3-Trifluoro-propionylamino)-cyclopropanecarboxylic acid 4-[3-chloro-2-(5-methyl-[1,2,4]oxadiazol-3-yl)-indol-1-yl]-2-fluoro-benzylamide | Reference Example 9 | 550.1 |
| **109** | *N*-(1-{4-[3-Chloro-2-(5-methyl-[1,2,4]oxadiazol-3-yl)-indol-1-yl]-2-fluoro-benzylcarbamoyl}-cyclopropyl)-2-fluoro-isonicotinamide | Reference Example 9 | 563.2 |
| **110** | 5-Cyclopropyl-isoxazole-3-carboxylic acid (1-{4-[3-chloro-2-(5-methyl-[1,2,4]oxadiazol-3-yl)-indol-1-yl]-2-fluoro-benzylcarbamoyl}-cyclopropyl)-amide | Reference Example 9 | 575.2 |
| **111** | *N*-(1-{4-[3-Chloro-2-(5-methyl-[1,2,4]oxadiazol-3-yl)-indol-1-yl]-2-fluoro-benzylcarbamoyl}-cyclopropyl)-5-trifluoromethyl-nicotinamide | Reference Example 9 | 613.1 |
| **112** | 1-(3,3,3-Trifluoro-propionylamino)-cyclopropanecarboxylic acid 4-[3-chloro-2-(3-methyl-[1,2,4]oxadiazol-5-yl)-indol-1-yl]-benzylamide | Reference Example 35 | 532.2 |
| **113** | *N*-(1-{4-[3-Chloro-2-(3-methyl-[1,2,4]oxadiazol-5-yl)-indol-1-yl]-benzylcarbamoyl}-cyclopropyl)-2-fluoro-isonicotinamide | Reference Example 35 | 545.1 |
| **114** | 5-Cyclopropyl-isoxazole-3-carboxylic acid (1-{4-[3-chloro-2-(3-methyl-[1,2,4]oxadiazol-5-yl)-indol-1-yl]-benzylcarbamoyl}-cyclopropyl)-amide | Reference Example 35 | 557.2 |
| **115** | *N*-(1-{4-[3-Chloro-2-(3-methyl-[1,2,4]oxadiazol-5-yl)-indol-1-yl]-benzylcarbamoyl}-cyclopropyl)-5-trifluoromethyl-nicotinamide | Reference Example 35 | 596.2 |
| **116** | 2-Fluoro-*N*-(1-{4-[5-methoxy-2-(3-methyl-[1,2,4]oxadiazol-5-yl)-indol-1-yl]-benzylcarbamoyl}-cyclopropyl)-nicotinamide | Reference Example 30 | 541.2 |
| **117** | *N*-(1-{4-[3-Chloro-5-fluoro-2-(5-methyl-[1,2,4]oxadiazol-3-yl)-indol-1-yl]-benzylcarbamoyl}-cyclopropyl)-2-fluoro-nicotinamide | Reference Example 28 | 564.2 |
| **118** | *N*-(1-{4-[3-Chloro-5-methoxy-2-(3-methyl-[1,2,4]oxadiazol-5-yl)-indol-1-yl]-benzylcarbamoyl}-cyclopropyl)-2-fluoro-nicotinamide | Reference Example 20 | 576.2 |
| **119** | *N*-(1-{4-[3-Chloro-2-(2-methyl-2*H*-tetrazol-5-yl)-indol-1-yl]-benzylcarbamoyl}-cyclopropyl)-2-fluoro-nicotinamide | Reference Example 4 | 546.2 |
| **120** | 2-Fluoro-*N*-(1-{4-[5-fluoro-2-(5-methyl-[1,2,4]oxadiazol-3-yl)-indol-1-yl]-benzylcarbamoyl}-cyclopropyl)-nicotinamide | Reference Example 19 | 529.2 |
| **121** | 1-Methyl-1*H*-pyrazole-4-carboxylic acid (1-{4-[3-chloro-2-(5-methyl-[1,2,4]oxadiazol-3-yl)-indol-1-yl]-benzylcarbamoyl}-cyclopropyl)-amide | Reference Example 9 | 531.2 |
| **122** | 2-Oxo-imidazolidine-4-carboxylic acid (1-{4-[3-chloro-2-(5-methyl-[1,2,4]oxadiazol-3-yl)-indol-1-yl]-benzylcarbamoyl}-cyclopropyl)-amide | Reference Example 9 | 535.2 |
| **123** | 3-Ethyl-isoxazole-5-carboxylic acid (1-{4-[3-chloro-2-(5-methyl-[1,2,4]oxadiazol-3-yl)-indol-1-yl]-benzylcarbamoyl}-cyclopropyl)-amide | Reference Example 9 | 546.2 |
| **124** | 1-(3,3,3-Trifluoro-propionylamino)-cyclopropanecarboxylic acid 4-[5-methoxy-2-(5-methyl-[1,3,4]oxadiazol-2-yl)-indol-1-yl]-benzylamide | Reference Example 29 | 528.2 |
| **125** | *N*-(1-{4-[5-Methoxy-2-(5-methyl-[1,3,4]oxadiazol-2-yl)-indol-1-yl]-benzylcarbamoyl}-cyclopropyl)-5-trifluoromethyl-nicotinamide | Reference Example 29 | 591.2 |
| **126** | 3-Methoxy-isoxazole-5-carboxylic acid (1-{4-[5-methoxy-2-(5-methyl-[1,3,4]oxadiazol-2-yl)-indol-1-yl]-benzylcarbamoyl}-cyclopropyl)-amide | Reference Example 29 | 543.2 |
| **127** | 1-(3,3,3-Trifluoro-propionylamino)-cyclopropanecarboxylic acid 4-[5-chloro-2-(5-methyl-[1,2,4]oxadiazol-3-yl)-indol-1-yl]-benzylamide | Reference Example 22 | 532.2 |
| **128** | *N*-(1-{4-[5-Chloro-2-(5-methyl-[1,2,4]oxadiazol-3-yl)-indol-1-yl]-benzylcarbamoyl}-cyclopropyl)-5-trifluoromethyl-nicotinamide | Reference Example 22 | 596.2 |
| **129** | 5-Methyl-pyrazine-2-carboxylic acid (1-{4-[3-chloro-5-methoxy-2-(5-methyl-[1,2,4]oxadiazol-3-yl)-indol-1-yl]-benzylcarbamoyl}-cyclopropyl)-amide | Reference Example 24 | 572.2 |
| **130** | *N*-(1-{4-[3-Chloro-5-methoxy-2-(5-methyl-[1,2,4]oxadiazol-3-yl)-indol-1-yl]-benzylcarbamoyl}-cyclopropyl)-5-trifluoromethyl-nicotinamide | Reference Example 24 | 625.2 |
| **131** | 5-Methyl-pyrazine-2-carboxylic acid (1-{2-fluoro-4-[5-fluoro-2-(5-methyl-[1,2,4]oxadiazol-3-yl)-indol-1-yl]-benzylcarbamoyl}-cyclopropyl)-amide | Reference Example 19 | 544.2 |
| **132** | *N*-(1-{2-Fluoro-4-[5-fluoro-2-(5-methyl-[1,2,4]oxadiazol-3-yl)-indol-1-yl]-benzylcarbamoyl}-cyclopropyl)-5-trifluoromethyl-nicotinamide | Reference Example 19 | 597.2 |
| **133** | 3-Methoxy-isoxazole-5-carboxylic acid (1-{2-fluoro-4-[5-fluoro-2-(5-methyl-[1,2,4]oxadiazol-3-yl)-indol-1-yl]-benzylcarbamoyl}-cyclopropyl)-amide | Reference Example 19 | 549.2 |

### Example 134

### Isoxazole-5-carboxylic acid (1-{4-[3-chloro-2-(5-methyl-[1,2,4]oxadiazol-3-yl)-indol-1-yl]-benzylcarbamoyl}-cyclopropyl)-amide

To a solution of 12.0 mg (0.106 mmol) of isoxazole-5-carboxylic acid in 2 mL of dichloromethane and 0.2 mL of *N,N-*dimethylformamide 15 mg (0.036 mmol) of 1-amino-cyclopropanecarboxylic acid 4-[3-chloro-2-(5-methyl-[1,2,4]oxadiazol-3-yl)-indol-1-yl]-benzylamide (Reference Example 9), 40.5 mg (0.106 mmol) of HBTU and 28.8 µL (0.43 mmol) of triethylamine were added. The mixture was shaken at room temperature for 18 h, then purified by column chromatography using n-hexane and ethylacetate as eluent to yield 17 mg (91%) of the title compound. MS (EI) 517.1 (MH⁺).

Compounds of Table 3 were prepared according to the method described in Example 134.

**Table 3**

| **Example** | Name | Starting Material | MS (EI) (MH⁺) |
|---|---|---|---|
| **135** | 5-Methyl-isoxazole-3-carboxylic acid (1-{4-[3-chloro-2-(5-methyl-[1,2,4]oxadiazol-3-yl)-indol-1-yl]-benzylcarbamoyl}-cyclopropyl)-amide | Reference Example 9 | 531.2 |
| **136** | Tetrahydro-furan-2-carboxylic acid (1-{4-[3-chloro-2-(5-methyl-[1,2,4]oxadiazol-3-yl)-indol-1-yl]-benzylcarbamoyl}-cyclopropyl)-amide | Reference Example 9 | 520.2 |
| **137** | Furan-3-carboxylic acid (1-{4-[3-chloro-2-(5-methyl-[1,2,4]oxadiazol-3-yl)-indol-1-yl]-benzylcarbamoyl}-cyclopropyl)-amide | Reference Example 9 | 516.2 |
| **138** | 2-Methyl-cyclopropanecarboxylic acid (1-{4-[3-chloro-2-(5-methyl-[1,2,4]oxadiazol-3-yl)-indol-1-yl]-benzylcarbamoyl}-cyclopropyl)-amide | Reference Example 9 | 504.2 |
| **139** | 1-Pent-4-ynoylamino-cyclopropanecarboxylic acid 4-[3-chloro-2-(5-methyl-[1,2,4]oxadiazol-3-yl)-indol-1-yl]-benzylamide | Reference Example 9 | 502.2 |
| **140** | 1-(4-Methyl-pentanoylamino)-cyclopropanecarboxylic acid 4-[3-chloro-2-(5-methyl-[1,2,4]oxadiazol-3-yl)-indol-1-yl]-benzylamide | Reference Example 9 | 521.2 |
| **141** | 1*H*-Pyrazole-4-carboxylic acid (1-{4-[3-chloro-2-(5-methyl-[1,2,4]oxadiazol-3-yl)-indol-1-yl]-benzylcarbamoyl}-cyclopropyl)-amide | Reference Example 9 | 516.1 |
| **142** | 1-Cyano-cyclopropanecarboxylic acid (1-{4-[3-chloro-2-(5-methyl-[1,2,4]oxadiazol-3-yl)-indol-1-yl]-benzylcarbamoyl}-cyclopropyl)-amide | Reference Example 9 | 515.2 |
| **143** | Cyclobutanecarboxylic acid (1-{4-[3-chloro-2-(5-methyl-[1,2,4]oxadiazol-3-yl)-indol-1-yl]-benzylcarbamoyl}-cyclopropyl)-amide | Reference Example 9 | 504.2 |
| **144** | Cyclopentanecarboxylic acid (1-{4-[3-chloro-2-(5-methyl-[1,2,4]oxadiazol-3-yl)-indol-1-yl]-benzylcarbamoyl}-cyclopropyl)-amide | Reference Example 9 | 519.2 |
| **145** | Furan-2-carboxylic acid (1-{4-[3-chloro-2-(5-methyl-[1,2,4]oxadiazol-3-yl)-indol-1-yl]-benzylcarbamoyl}-cyclopropyl)-amide | Reference Example 9 | 516.1 |
| **146** | *N*-(1-{4-[3-Chloro-2-(5-methyl-[1,2,4]oxadiazol-3-yl)-indol-1-yl]-benzylcarbamoyl}-cyclopropyl)-3-cyano-benzamide | Reference Example 9 | 551.2 |
| **147** | 1-(2-Thiophen-3-yl-acetylamino)-cyclopropanecarboxylic acid 4-[3-chloro-2-(5-methyl-[1,2,4]oxadiazol-3-yl)-indol-1-yl]-benzylamide | Reference Example 9 | 547.1 |
| **148** | 1,5-Dimethyl-1*H*-pyrazole-3-carboxylic acid (1-{4-[3-chloro-2-(5-methyl-[1,2,4]oxadiazol-3-yl)-indol-1-yl]-benzylcarbamoyl}-cyclopropyl)-amide | Reference Example 9 | 544.2 |
| **149** | 4-Methyl-thiazole-5-carboxylic acid (1-{4-[3-chloro-2-(5-methyl-[1,2,4]oxadiazol-3-yl)-indol-1-yl]-benzylcarbamoyl}-cyclopropyl)-amide | Reference Example 9 | 548.1 |
| **150** | 2,4-Dimethyl-thiazole-5-carboxylic acid (1-{4-[3-chloro-2-(5-methyl-[1,2,4]oxadiazol-3-yl)-indol-1-yl]-benzylcarbamoyl}-cyclopropyl)-amide | Reference Example 9 | 562.1 |
| **151** | 1-(2-Acetylamino-3-hydroxy-propionylamino)-cyclopropanecarboxylic acid 4-[3-chloro-2-(5-methyl-[1,2,4]oxadiazol-3-yl)-indol-1-yl]-benzylamide | Reference Example 9 | 551.2 |
| **152** | 5-Methyl-1*H*-pyrazole-3-carboxylic acid (1-{4-[3-chloro-2-(5-methyl-[1,2,4]oxadiazol-3-yl)-indol-1-yl]-benzylcarbamoyl}-cyclopropyl)-amide | Reference Example 9 | 530.2 |
| **153** | 2-Chloro-*N*-(1-{4-[3-chloro-2-(5-methyl-[1,2,4]oxadiazol-3-yl)-indol-1-yl]-benzylcarbamoyl}-cyclopropyl)-nicotinamide | Reference Example 9 | 562.1 |
| **154** | *N*-(1-{4-[3-Chloro-2-(5-methyl-[1,2,4]oxadiazol-3-yl)-indol-1-yl]-benzylcarbamoyl}-cyclopropyl)-6-methyl-nicotinamide | Reference Example 9 | 542.2 |
| **155** | *N*-(1-{4-[3-Chloro-2-(5-methyl-[1,2,4]oxadiazol-3-yl)-indol-1-yl]-benzylcarbamoyl}-cyclopropyl)-2-methyl-nicotinamide | Reference Example 9 | 542.2 |
| **156** | 2-Chloro-*N*-(1-{4-[3-chloro-2-(5-methyl-[1,2,4]oxadiazol-3-yl)-indol-1-yl]-benzylcarbamoyl}-cyclopropyl)-6-methyl-nicotinamide | Reference Example 9 | 576.1 |
| **157** | 6-Bromo-pyridine-2-carboxylic acid (1-{4-[3-chloro-2-(5-methyl-[1,2,4]oxadiazol-3-yl)-indol-1-yl]-benzylcarbamoyl}-cyclopropyl)-amide | Reference Example 9 | 606.1 |
| **158** | *N*-(1-{4-[3-Chloro-2-(5-methyl-[1,2,4]oxadiazol-3-yl)-indol-1-yl]-benzylcarbamoyl}-cyclopropyl)-nicotinamide | Reference Example 9 | 527.1 |
| **159** | Quinoline-3-carboxylic acid (1-{4-[3-chloro-2-(5-methyl-[1,2,4]oxadiazol-3-yl)-indol-1-yl]-benzylcarbamoyl}-cyclopropyl)-amide | Reference Example 9 | 578.1 |
| **160** | 6-Oxo-1,6-dihydro-pyridine-3-carboxylic acid (1-{4-[3-chloro-2-(5-methyl-[1,2,4]oxadiazol-3-yl)-indol-1-yl]-benzylcarbamoyl}-cyclopropyl)-amide | Reference Example 9 | 543.2 |
| **161** | 5-Methyl-pyrazine-2-carboxylic acid (1-{4-[3-chloro-2-(5-methyl-[1,2,4]oxadiazol-3-yl)-indol-1-yl]-benzylcarbamoyl}-cyclopropyl)-amide | Reference Example 9 | 542.1 |
| **162** | 6-Oxo-1,6-dihydro-pyridine-2-carboxylic acid (1-{4-[3-chloro-2-(5-methyl-[1,2,4]oxadiazol-3-yl)-indol-1-yl]-benzylcarbamoyl}-cyclopropyl)-amide | Reference Example 9 | 543.1 |
| **163** | 1-(3,3,3-Trifluoro-propionylamino)-cyclopropanecarboxylic acid 4-[3-chloro-2-(5-methyl-[1,2,4]oxadiazol-3-yl)-indol-1-yl]-benzylamide | Reference Example 9 | 532.1 |
| **164** | *N*-(1-{4-[3-Chloro-2-(5-methyl-[1,2,4]oxadiazol-3-yl)-indol-1-yl]-benzylcarbamoyl}-cyclopropyl)-2-fluoro-isonicotinamide | Reference Example 9 | 545.1 |
| **165** | 5-Cyclopropyl-isoxazole-3-carboxylic acid (1-{4-[3-chloro-2-(5-methyl-[1,2,4]oxadiazol-3-yl)-indol-1-yl]-benzylcarbamoyl}-cyclopropyl)-amide | Reference Example 9 | 557.2 |
| **166** | Pyrimidine-5-carboxylic acid (1-{4-[3-chloro-2-(5-methyl-[1,2,4]oxadiazol-3-yl)-indol-1-yl]-benzylcarbamoyl}-cyclopropyl)-amide | Reference Example 9 | 528.2 |
| **167** | Thiophene-3-carboxylic acid (1-{4-[3-chloro-2-(5-methyl-[1,2,4]oxadiazol-3-yl)-indol-1-yl]-benzylcarbamoyl}-cyclopropyl)-amide | Reference Example 9 | 532.7 |
| **168** | 5-Oxo-pyrrolidine-2-carboxylic acid (1-{4-[3-chloro-2-(5-methyl-[1,2,4]oxadiazol-3-yl)-indol-1-yl]-benzylcarbamoyl}-cyclopropyl)-amide | Reference Example 9 | 533.7 |
| **169** | 1-(3-Furan-3-yl-acryloylamino)-cyclopropanecarboxylic acid 4-[3-chloro-2-(5-methyl-[1,2,4]oxadiazol-3-yl)-indol-1-yl]-benzylamide | Reference Example 9 | 543.0 |
| **170** | *N*-(1-{4-[3-Chloro-2-(5-methyl-[1,2,4]oxadiazol-3-yl)-indol-1-yl]-benzylcarbamoyl}-cyclopropyl)-4-cyano-benzamide | Reference Example 9 | 551.7 |
| **171** | 1-(3-Furan-2-yl-acryloylamino)-cyclopropanecarboxylic acid 4-[3-chloro-2-(5-methyl-[1,2,4]oxadiazol-3-yl)-indol-1-yl]-benzylamide | Reference Example 9 | 542.7 |
| **172** | Thiophene-2-carboxylic acid (1-{4-[3-chloro-2-(5-methyl-[1,2,4]oxadiazol-3-yl)-indol-1-yl]-benzylcarbamoyl}-cyclopropyl)-amide | Reference Example 9 | 532.6 |
| **173** | 5-Methylsulfanyl-thiophene-2-carboxylic acid (1-{4-[3-chloro-2-(5-methyl-[1,2,4]oxadiazol-3-yl)-indol-1-yl]-benzylcarbamoyl}-cyclopropyl)-amide | Reference Example 9 | 578.7 |
| **174** | [1,2,3]Thiadiazole-4-carboxylic acid (1-{4-[3-chloro-2-(5-methyl-[1,2,4]oxadiazol-3-yl)-indol-1-yl]-benzylcarbamoyl}-cyclopropyl)-amide | Reference Example 9 | 534.6 |
| **175** | *N*-(1-{4-[3-Chloro-2-(5-methyl-[1,2,4]oxadiazol-3-yl)-indol-1-yl]-benzylcarbamoyl}-cyclopropyl)-3-methoxy-benzamide | Reference Example 9 | 557.0 |
| **176** | *N*-(1-{4-[3-Chloro-2-(5-methyl-[1,2,4]oxadiazol-3-yl)-indol-1-yl]-benzylcarbamoyl}-cyclopropyl)-3-iodo-benzamide | Reference Example 9 | 652.2 |
| **177** | *N*-(1-{4-[3-Chloro-2-(5-methyl-[1,2,4]oxadiazol-3-yl)-indol-1-yl]-benzylcarbamoyl}-cyclopropyl)-3-trifluoromethyl-benzamide | Reference Example 9 | 594.2 |
| **178** | *N*-(1-{4-[3-Chloro-2-(5-methyl-[1,2,4]oxadiazol-3-yl)-indol-1-yl]-benzylcarbamoyl}-cyclopropyl)-3-fluoro-benzamide | Reference Example 9 | 544.2 |
| **179** | *N*-(1-{4-[3-Chloro-2-(5-methyl-[1,2,4]oxadiazol-3-yl)-indol-1-yl]-benzylcarbamoyl}-cyclopropyl)-3-methyl-benzamide | Reference Example 9 | 541.0 |
| **180** | *N*-(1-{4-[3-Chloro-2-(5-methyl-[1,2,4]oxadiazol-3-yl)-indol-1-yl]-benzylcarbamoyl}-cyclopropyl)-5-trifluoromethyl-nicotinamide | Reference Example 9 | 596.0 |
| **181** | 3-Methyl-isoxazole-4-carboxylic acid (1-{4-[3-chloro-2-(5-methyl-[1,2,4]oxadiazol-3-yl)-indol-1-yl]-benzylcarbamoyl}-cyclopropyl)-amide | Reference Example 9 | 531.2 |
| **182** | Quinoline-3-carboxylic acid (1-{4-[3-chloro-2-(5-methyl-[1,3,4]oxadiazol-2-yl)-indol-1-yl]-benzylcarbamoyl}-cyclopropyl)-amide | Reference Example 7 | 578.2 |
| **183** | 1-(3,3,3-Trifluoro-propionylamino)-cyclopropanecarboxylic acid 4-[3-chloro-2-(5-methyl-[1,3,4]oxadiazol-2-yl)-indol-1-yl]-benzylamide | Reference Example 7 | 532.2 |
| **184** | Pyrimidine-5-carboxylic acid (1-{4-[3-chloro-2-(5-methyl-[1,3,4]oxadiazol-2-yl)-indol-1-yl]-benzylcarbamoyl}-cyclopropyl)-amide | Reference Example 7 | 528.2 |
| **185** | 2-Methyl-pyrimidine-5-carboxylic acid (1-{4-[3-chloro-2-(5-methyl-[1,2,4]oxadiazol-3-yl)-indol-1-yl]-benzylcarbamoyl}-cyclopropyl)-amide | Reference Example 9 | 543.2 |

### Example 186

### 3-Amino-N-(1-{4-[3-chloro-2-(5-methyl-[1,2,4]oxadiazol-3-yl)-indol-1-yl]-benzylcarbamoyl}-cyclopropyl)-benzamide hydrochloride

### a) [3-(1-{4-[3-Chloro-2-(5-methyl-[1,2,4]oxadiazol-3-yl)-indol-1-yl]-benzylcarbamoyl}-cyclopropylcarbamoyl)-phenyl]-carbamic acid tert-butyl ester

The title compound was prepared from 1-amino-cyclopropanecarboxylic acid 4-[3-chloro-2-(5-methyl-[1,2,4]oxadiazol-3-yl)-indol-1-yl]-benzylamide (Reference Example 9) and 3-*tert*-butoxycarbonylamino-benzoic acid according to the method described in Example 134.

### b) 3-Amino-N-(1-{4-[3-chloro-2-(5-methyl-[1,2,4]oxadiazol-3-yl)-indol-1-yl]-benzylcarbamoyl}-cyclopropyl)-benzamide hydrochloride

The title compound was prepared from [3-(1-{4-[3-chloro-2-(5-methyl-[1,2,4]oxadiazol-3-yl)-indol-1-yl]-benzyl-carbamoyl}-cyclopropylcarbamoyl)-phenyl]-carbamic acid *tert*-butyl ester according to the method described in Reference Example 14 b. MS (EI) 542.0 (MH⁺).

### Example 187

### 3-Chloro-1-[4-({[1-(2,2,2-trifluoro-acetylamino)-cyclopropanecarbonyl]-amino}-methyl)-phenyl]-1H-indole-2-carboxylic acid amide

The title compound was prepared from 1-(4-aminomethyl-phenyl)-3-chloro-1*H-*indole-2-carboxylic acid amide (Reference Example 32) according to the method described in Example 4. MS (EI) 479.1 (MH⁺).

### Example 188

### 3-Chloro-1-{4-[({1-[(3-methoxy-isoxazole-5-carbonyl)-amino]-cyclopropane-carbonyl}-amino)-methyl]-phenyl}-1H-indole-2-carboxylic acid amide

The title compound was prepared from 1-(4-aminomethyl-phenyl)-3-chloro-1*H-*indole-2-carboxylic acid amide hydrochloride (Reference Example 32) according to the method described in Example 2. MS (EI) 508.2 (MH⁺).

### Example 189

### 1-(2,2,2-Trifluoro-acetylamino)-cyclopropanecarboxylic acid 4-[3-chloro-5-fluoro-2-(5-methyl-[1,2,4]oxadiazol-3-yl)-indol-1-yl]-benzylamide

The title compound was prepared from 4-[3-chloro-5-fluoro-2-(5-methyl-[1,2,4]oxadiazol-3-yl)-indol-1-yl]-benzylamine (Reference Example 28a) according to the method described in Example 4. MS (EI) 536.1 (MH⁺).

### Example 190

### 1-(2,2,2-Trifluoro-acetylamino)-cyclopropanecarboxylic acid ((R)-1-{4-[3-chloro-2-(5-methyl-[1,2,4]oxadiazol-3-yl)-indol-1-yl]-2-fluoro-phenyl}-ethyl)-amide

The title compound was prepared from (1*R*)-1-{4-[3-chloro-2-(5-methyl-1,2,4-oxadiazol-3-yl)-1*H*-indol-1-yl]-2-fluorophenyl}ethanamine hydrochloride (Reference Example 33) according to the method described in Example 4. MS (EI) 550.0 (MH⁺).

### Example 191

### 3-Methoxy-isoxazole-5-carboxylic acid [1-((R)-1-{4-[3-chloro-2-(5-methyl-[1,2,4]oxadiazol-3-yl)-indol-1-yl]-2-fluoro-phenyl}-ethylcarbamoyl)-cyclopropyl]-amide

The title compound was prepared from (1*R*)-1-{4-[3-chloro-2-(5-methyl-1,2,4-oxadiazol-3-yl)-1*H*-indol-1-yl]-2-fluorophenyl}ethanamine hydrochloride (Reference Example 33) according to the method described in Example 2. MS (EI) 579.2 (MH⁺).

### Example 192

### 3-Methoxy-isoxazole-5-carboxylic acid (1-{4-[3-chloro-2-(5-methyl-[1,2,4]oxadiazol-3-yl)-indol-1-yl]-2-fluoro-benzylcarbamoyl}-cyclopropyl)-amide

The title compound was prepared from 4-[3-chloro-2-(5-methyl-[1,2,4]oxadiazol-3-yl)-indol-1-yl]-2-fluoro-benzylamine hydrochloride (Reference Example 34) according to the method described in Example 2. MS (EI) 565.0 (MH⁺).

### Example 193

### 3-Methoxy-isoxazole-5-carboxylic acid (1-{4-[3-chloro-2-(3-methyl-[1,2,4]oxadiazol-5-yl)-indol-1-yl]-benzylcarbamoyl}-cyclopropyl)-amide

The title compound was prepared from 4-[3-chloro-2-(3-methyl-[1,2,4]oxadiazol-5-yl)-indol-1-yl]-benzylamine hydrochloride (Reference Example 35b) according to the method described in Example 2. MS (EI) 547.3 (MH⁺).

### Example 194

### 1-(2,2,2-Trifluoro-acetylamino)-cyclopropanecarboxylic acid 4-[3-chloro-2-(3-methyl-[1,2,4]oxadiazol-5-yl)-indol-1-yl]-benzylamide

The title compound was prepared from 4-[3-chloro-2-(3-methyl-[1,2,4]oxadiazol-5-yl)-indol-1-yl]-benzylamine hydrochloride (Reference Example 35b) according to the method described in Example 4. MS (EI) 518.2 (MH⁺).

### Example 195

### 3-Methoxy-isoxazole-5-carboxylic acid (1-{4-[2-(5-methyl-[1,2,4]oxadiazol-3-yl)-indol-1-yl]-benzylcarbamoyl}-cyclopropyl)-amide

The title compound was prepared from 1-amino-cyclopropanecarboxylic acid 4-[2-(5-methyl-[1,2,4]oxadiazol-3-yl)-indol-1-yl]-benzylamide hydrochloride (Reference Example 36b) according to the method described in Example 2. MS (EI) 513.2 (MH⁺).

### Example 196

### 3-Methoxy-isoxazole-5-carboxylic acid (1-{4-[2-(3-methyl-[1,2,4]oxadiazol-5-yl)-indol-1-yl]-benzylcarbamoyl}-cyclopropyl)-amide

The title compound was prepared from 4-[2-(3-methyl-[1,2,4]oxadiazol-5-yl)-indol-1-yl]-benzylamine hydrochloride (Reference Example 37b) according to the method described in Reference Example 2. MS (EI) 513.2 (MH⁺).

### Example 197

### 3-Methoxy-isoxazole-5-carboxylic acid (1-{4-[3-chloro-5-fluoro-2-(5-methyl-[1,2,4]oxadiazol-3-yl)-indol-1-yl]-2-fluoro-benzylcarbamoyl}-cyclopropyl)-amide

### a) 4-[3-Chloro-5-fluoro-2-(5-methyl-[1,2,4]oxadiazol-3-yl)-indol-1-yl]-2-fluoro-benzylamine hydrochloride

The title compound was prepared from 3-fluoro-4-(*tert*-butoxycarbonyl-aminomethyl)-phenylboronic acid (Reference Example 1) and 3-chloro-5-fluoro-2-(5-methyl-1,2,4-oxadiazol-3-yl)-1*H*-indole (Example 62 b) according to the methods described in Reference Example 9c. MS (EI) 358.1 [(M-NH₂)]⁺.

### b) 3-Methoxy-isoxazole-5-carboxylic acid (1-{4-[3-chloro-5-fluoro-2-(5-methyl-[1,2,4]oxadiazol-3-yl)-indol-1-yl]-2-fluoro-benzylcarbamoyl}-cyclopropyl)-amide

The title compound was prepared from 4-[3-chloro-5-fluoro-2-(5-methyl-[1,2,4]oxadiazol-3-yl)-indol-1-yl]-2-fluoro-benzylamine hydrochloride according to the method described in Example 2. MS (EI) 583.2 (MH⁺).

### Example 198

### 3-Methoxy-isoxazole-5-carboxylic acid (1-{4-[3,5-difluoro-2-(5-methyl-[1,2,4]oxadiazol-3-yl)-indol-1-yl]-benzylcarbamoyl}-cyclopropyl)-amide

The title compound was prepared from 4-[3,5-difluoro-2-(5-methyl-[1,2,4]oxadiazol-3-yl)-indol-1-yl]-benzylamine hydrochloride (Reference Example 39) according to the method described in Example 2. MS (EI) 549.2 (MH⁺).

### Example 199

### 1-(2,2,2-Trifluoro-acetylamino)-cyclopropanecarboxylic acid 4-[3,5-difluoro-2-(5-methyl-[1,2,4]oxadiazol-3-yl)-indol-1-yl]-benzylamide

The title compound was prepared from 4-[3,5-difluoro-2-(5-methyl-[1,2,4]oxadiazol-3-yl)-indol-1-yl]-benzylamine hydrochloride (Reference Example 39) according to the method described in Example 4. MS (EI) 520.2 (MH⁺).

### Example 200

### 3-Methoxy-isoxazole-5-carboxylic acid (1-{4-[5-fluoro-3-methyl-2-(5-methyl-[1,2,4]oxadiazol-3-yl)-indol-1-yl]-benzylcarbamoyl}-cyclopropyl)-amide

### a) 5-Fluoro-3-methyl-1H-indole-2-carboxylic acid amide

The title compound was prepared from 5-fluoro-3-methyl-1*H*-indole-2-carboxylic acid (US2005/222148) according to the methods described in Reference Example 4b. MS (EI) 193.2 (MH⁺).

### b) 5-Fluoro-3-methyl-1H-indole-2-carbonitrile

The title compound was prepared from 5-fluoro-3-methyl-1*H*-indole-2-carboxylic acid amide according to the method described in Reference Example 4c. MS (EI) 175.2 (MH⁺).

### c) 5-Fluoro-N-hydroxy-3-methyl-1H-indole-2-carboxamidine

The title compound was prepared from 5-fluoro-3-methyl-1*H*-indole-2-carbonitrile according to the method described in Reference Example 9a. MS (EI) 208.1 (MH⁺).

### d) 5-Fluoro-3-methyl-2-(5-methyl-[1,2,4]oxadiazol-3-yl)-1H-indole

The title compound was prepared from 5-fluoro-*N*-hydroxy-3-methyl-1*H*-indole-2-carboxamidine according to the method described in Reference Example 9b. MS (EI) 232.1 (MH⁺).

### e) {4-[5-Fluoro-3-methyl-2-(5-methyl-[1,2,4]oxadiazol-3-yl)-indol-1-yl]-benzyl}-carbamic acid tert-butyl ester

The title compound was prepared from 5-fluoro-3-methyl-2-(5-methyl-[1,2,4]oxadiazol-3-yl)-1*H*-indole according to the method described in Reference Example 24f. MS (EI) 459.2 [M+Na]⁺.

### f) 4-[5-Fluoro-3-methyl-2-(5-methyl-[1,2,4]oxadiazol-3-yl)-indol-1-yl]-benzylamine

The title compound was prepared from {4-[5-fluoro-3-methyl-2-(5-methyl-[1,2,4]oxadiazol-3-yl)-indol-1-yl]-benzyl}-carbamic acid tert-butyl ester according to the method described in Example 37d. MS (EI) 320.2 [(M-NH₂)]⁺.

### g) 3-Methoxy-isoxazole-5-carboxylic acid (1-{4-[5-fluoro-3-methyl-2-(5-methyl-[1,2,4]oxadiazol-3-yl)-indol-1-yl]-benzylcarbamoyl}-cyclopropyl)-amide

The title compound was prepared from 4-[5-fluoro-3-methyl-2-(5-methyl-[1,2,4]oxadiazol-3-yl)-indol-1-yl]-benzylamine according to the method described in Example 34e. MS (EI) 545.2 (MH⁺).

### Example 201

### 3-Methoxy-isoxazole-5-carboxylic acid (1-{4-[3-chloro-2-(4-methyl-5-oxo-4,5-dihydro-[1,2,4]oxadiazol-3-yl)-indol-1-yl]-benzylcarbamoyl}-cyclopropyl)-amide

### a) 3-(3-Chloro-1H-indol-2-yl)-4H-[1,2,4]oxadiazol-5-one

A mixture of 4.91 g (23.4 mmol) of 3-chloro-*N*-hydroxy-1*H*-indole-2-carboxamidine (Reference Example 9a) and 4.91 g (30.28 mmol) of 1,1'-carbonyldiimidazole in 49.1 mL of tetrahydrofuran was refluxed for 1.5 h. The reaction mixture was cooled, concentrated *in vacuo* and the residue was stirred with 50 mL of water. The precipitated product was filtered off, washed with water and dried to yield 4.0 g (72.5 %) of the title compound. Mp: 240-241 °C.

### b) 3-(3-Chloro-1H-indol-2-yl)-4-methyl-4H-[1,2,4]oxadiazol-5-one

A mixture of 3.69 g (15.6 mmol) of 3-(3-chloro-1*H*-indol-2-yl)-4*H-*[1,2,4]oxadiazol-5-one, 3.69 mL (59.2 mmol) of iodomethan in 240 mL of acetone was refluxed for 1 h. The reaction mixture was cooled to 20 °C, filtered and the filtrate was concentrated. The residue was submitted to flash column chromatography using Kieselgel 60 (0.015-0.040 mm) as adsorbent (Merck) and hexane : ethyl acetate = 2:1 as eluent to yield 0.4 g (10.2 %) of the title compound. MS (EI) 250.1 (MH⁺).

### c) 3-[1-(4-Aminomethyl-phenyl)-3-chloro-1H-indol-2-yl]-4-methyl-4H-[1,2,4]oxadiazol-5-one hydrochloride

The title compound was prepared from 3-(3-chloro-1*H*-indol-2-yl)-4-methyl-4*H-*[1,2,4]oxadiazol-5-one and of 4-(*tert*-butoxycarbonylamino-methyl)phenylboronic acid (Reference Example 1) according to the method described in Reference Example 9c. MS (EI) 355.1 (MH⁺).

### d) 3-Methoxy-isoxazole-5-carboxylic acid (1-{4-[3-chloro-2-(4-methyl-5-oxo-4,5-dihydro-[1,2,4]oxadiazol-3-yl)-indol-1-yl]-benzylcarbamoyl}-cyclopropyl)-amide

The title compound was prepared from 3-[1-(4-aminomethyl-phenyl)-3-chloro-1*H*-indol-2-yl]-4-methyl-4*H*-[1,2,4]oxadiazol-5-one hydrochloride and 1-[(3-methoxy-isoxazole-5-carbonyl)-amino]-cyclopropanecarboxylic acid (P. D. O'Shea et al. J. Org. Chem. 2009, 74, 4547-4553) according to the method described in Example 34e. MS (EI) 563.2 (MH⁺).

### Preparation of pharmaceutical compositions

The following formulation examples illustrate representative pharmaceutical compositions of this invention. The present invention however not limited to the following pharmaceutical compositions. The concentration of mixtures are expressed in weight percent.

### Preparation Example 1

### Preparation of pharmaceutical compositions:

### a) Tablets:

0.01-50 % of active ingredient of formula (I), 15-50 % of lactose, 15-50 % of potato starch, 5-15 % of polyvinyl pyrrolidone, 1-5 % of talc, 0.01-3 % of magnesium stearate, 1-3 % of colloid silicon dioxide and 2-7 % of ultraamylopectin were mixed, then granulated by wet granulation and pressed to tablets.

### b) Dragées, filmcoated tablets:

The tablets made according to the method described above were coated by a layer consisting of entero- or gastrosolvent film, or of sugar and talc. The dragées were polished by a mixture of beeswax and carnuba wax.

### c) Capsules:

0.01-50 % of active ingredient of formula (I), 1-5 % of sodium lauryl sulfate, 15-50 % of starch, 15-50 % of lactose, 1-3 % of colloid silicon dioxide and 0.01-3 % of magnesium stearate were thoroughly mixed, the mixture was passed through a sieve and filled in hard gelatin capsules.

### d) Suspensions:

Ingredients: 0.01-15 % of active ingredient of formula (I), 0.1-2 % of sodium hydroxide, 0.1-3 % of citric acid, 0.05-0.2 % of nipagin (sodium methyl 4-hydroxybenzoate), 0.005-0.02 % of nipasol, 0.01-0.5 % of carbopol (polyacrilic acid), 0.1-5 % of 96 % ethanol, 0.1-1 % of flavoring agent, 20-70 % of sorbitol (70 % aqueous solution) and 30-50 % of distilled water.

To solution of nipagin and citric acid in 20 ml of distilled water, carbopol was added in small portions under vigorous stirring, and the solution was left to stand for 10-12 h. Then the sodium hydroxide in 1 ml of distilled water, the aqueous solution of sorbitol and finally the ethanolic raspberry flavor were added with stirring. To this carrier the active ingredient was added in small portions and suspended with an immersing homogenizator. Finally the suspension was filled up to the desired final volume with distilled water and the suspension syrup was passed through a colloid milling equipment.

### e) Suppositories:

For each suppository 0.01-15% of active ingredient of formula (I) and 1-20% of lactose were thoroughly mixed, then 50-95% of adeps pro suppository (for example Witepsol 4) was melted, cooled to 35 °C and the mixture of active ingredient and lactose was mixed in it with homogenizator. The obtained mixture was mould in cooled forms.

### f) Lyophilized powder ampoule compositions:

A 5 % solution of mannitol or lactose was made with bidistilled water for injection use, and the solution was filtered so as to have sterile solution. A 0.01-5 % solution of the active ingredient of formula (I) was also made with bidistilled water for injection use, and this solution was filtered so as to have sterile solution. These two solutions were mixed under aseptic conditions, filled in 1 ml portions into ampoules, the content of the ampoules was lyophilized, and the ampoules were sealed under nitrogen. The contents of the ampoules were dissolved in sterile water or 0.9 % (physiological) sterile aqueous sodium chloride solution before administration.

The invention described and claimed herein is not to be limited in scope by the specific embodiments herein disclosed, since these embodiments are intended as illustrations of several aspects of the invention. Any equivalent embodiments are intended to be within the scope of this invention. Indeed, various modifications of the invention in addition to those shown and described herein will become apparent to those skilled in the art from the foregoing description. Such modifications are also intended to fall within the scope of the appended claims.

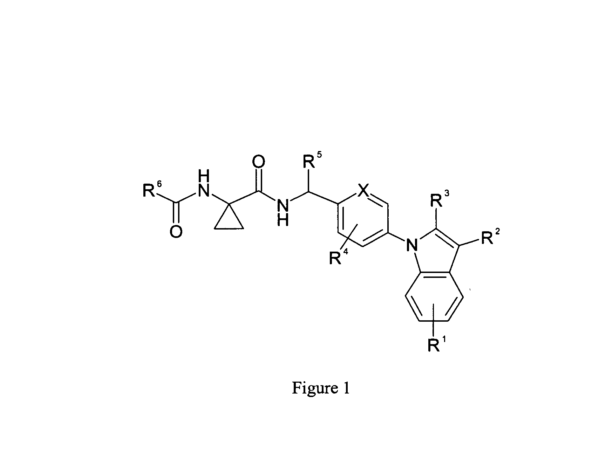

## Claims

1. Indole derivatives of formula (I) 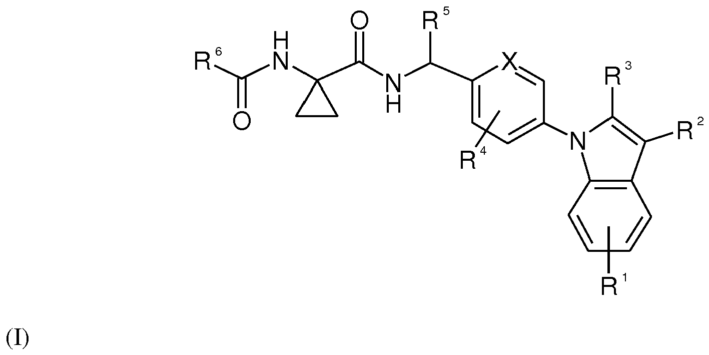 wherein
R1 is hydrogen atom, halogen atom or C1-C4-alkoxy group;
R2 is hydrogen atom, halogen atom, C1-C4-alkyl group or cyano group;
R3 is selected from (1) -COOR; (2) -CN; (3) -CONRaRb; 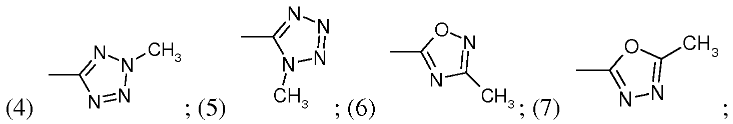 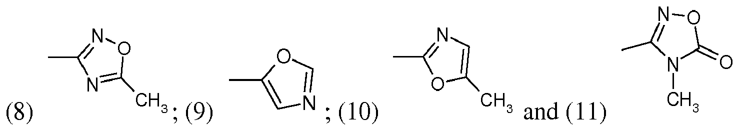
R4 is hydrogen atom or halogen atom;
R5 is hydrogen atom or C1-C4-alkyl group;
R6 is selected from
(1) C3-C6-cycloalkyl;
(2) optionally substituted five- or six-membered aromatic ring with two nitrogen atoms;
(3) optionally substituted pyridyl;
(4) optionally substituted five-membered aromatic ring with one heteroatom selected from O or S;
(5)-CF3; (6) -CH2-CF3; (7) -CH2-CH2-C=CH; (8) -CH2-CH2-CH(CH3)2 ; 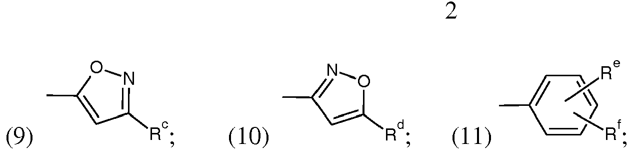 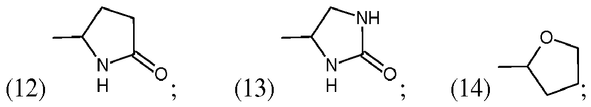 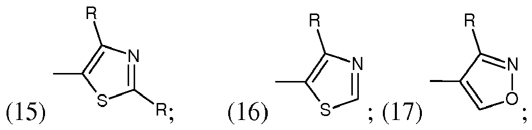 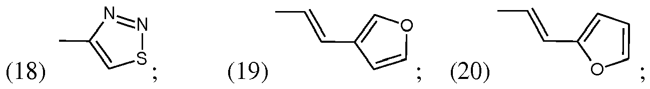 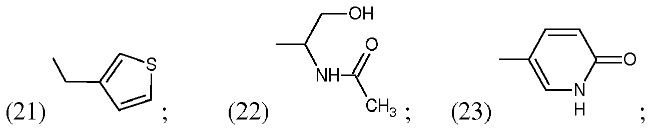 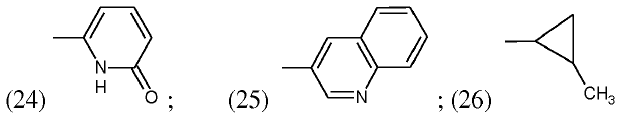 and 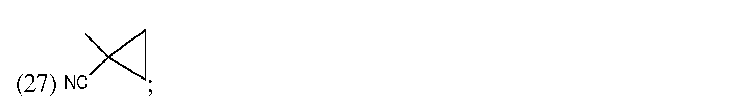
X is -CH- or nitrogen atom;
R is C1-C4 alkyl group;
Ra and Rb are independently from each other hydrogen atom; C1-C4 alkyl group or pyrrolidin-1-yl group;
Rc is hydrogen atom; C1-C4 alkyl or C1-C4 alkoxy group;
Rd is hydrogen atom; C1-C4 alkyl or C3-C6 cycloalkyl group;
Re and Rf are independently from each other hydrogen atom; halogen atom; C1-C4 alkoxy; -CF3; -CN or -NH2 group;
and optical antipodes or racemates and/or salts thereof.

2. A compound of Claim 1 selected from the group of
1-(2,2,2-Trifluoro-acetylamino)-cyclopropanecarboxylic acid 4-[3-chloro-2-(2-methyl-2H-tetrazol-5-yl)-indol-1-yl]-benzylamide
3-Methoxy-isoxazole-5-carboxylic acid (1-{4-[3-chloro-2-(2-methyl-2H-tetrazol-5-yl)-indol-1-yl]-benzylcarbamoyl}-cyclopropyl)-amide
3-Methoxy-isoxazole-5-carboxylic acid (1-{4-[2-(2-methyl-2H-tetrazol-5-yl)-indol-1-yl]-benzylcarbamoyl}-cyclopropyl)-amide
1-(2,2,2-Trifluoro-acetylamino)-cyclopropanecarboxylic acid 4-[3-chloro-2-(2-methyl-2H-tetrazol-5-yl)-indol-1-yl]-2-fluorobenzylamide
3-Methoxy-N-[1-({4-[3-chloro-2-(5-methyl-1,3,4-oxadiazol-2-yl)-1H-indol-1-yl]benzyl}carbamoyl)cyclopropyl]isoxazole-5-carboxamide
1-(2,2,2-Trifluoro-acetylamino)-cyclopropanecarboxylic acid 4-[3-chloro-2-(5-methyl-[1,3,4]oxadiazol-2-yl)-indol-1-yl]-benzylamide
3-Methoxy-isoxazole-5-carboxylic acid (1-{4-[3-chloro-2-(1-methyl-1H-tetrazol-5-yl)-indol-1-yl]-benzylcarbamoyl}-cyclopropyl)-amide
1-(2,2,2-Trifluoro-acetylamino)-cyclopropanecarboxylic acid 4-[3-chloro-2-(1-methyl-1H-tetrazol-5-yl)-indol-1-yl]-benzylamide
3-Methoxy-isoxazole-5-carboxylic acid (1-{4-[3-chloro-2-(5-methyl-[1,2,4]oxadiazol-3-yl)-indol-1-yl]-benzylcarbamoyl}-cyclopropyl)-amide
3-Chloro-1-[4-({[1-(2,2,2-trifluoro-acetylamino)-cyclopropanecarbonyl]-amino}-methyl)-phenyl]-1H-indole-2-carboxylic acid methyl ester
3-Chloro-1-{4-[({1-[(3-methoxy-isoxazole-5-carbonyl)-amino]-cyclopropanecarbonyl}-amino)-methyl]-phenyl}-1H-indole-2-carboxylic acid methyl ester
1-(2,2,2-Trifluoro-acetylamino)-cyclopropanecarboxylic acid 4-[3-chloro-5-fluoro-2-(2-methyl-2H-tetrazol-5-yl)-indol-1-yl]-benzylamide
3-Methoxy-isoxazole-5-carboxylic acid (1-{4-[3-chloro-5-fluoro-2-(2-methyl-2H-tetrazol-5-yl)-indol-1-yl]-benzylcarbamoyl}-cyclopropyl)-amide
1-{4-[({1-[(3-Methoxy-isoxazole-5-carbonyl)-amino]-cyclopropanecarbonyl}-amino)-methyl]-phenyl}-1H-indole-2-carboxylic acid methyl ester
3-Methoxy-isoxazole-5-carboxylic acid (1-{4-[3-chloro-2-(2-methyl-2H-tetrazol-5-yl)-indol-1-yl]-2-fluorobenzylcarbamoyl}-cyclopropyl)-amide
3-Methoxy-isoxazole-5-carboxylic acid (1-{4-[3-chloro-2-(1,3-oxazol-5-yl)-indol-1-yl]-benzylcarbamoyl}-cyclopropyl)-amide
1-(2,2,2-Trifluoro-acetylamino)-cyclopropanecarboxylic acid 4-[3,5-dichloro-2-(2-methyl-2H-tetrazol-5-yl)-indol-1-yl]-benzylamide
3-Methoxy-isoxazole-5-carboxylic acid (1-{4-[3,5-dichloro-2-(2-methyl-2H-tetrazol-5-yl)-indol-1-yl]-benzylcarbamoyl}-cyclopropyl)-amide
1-(2,2,2-Trifluoro-acetylamino)-cyclopropanecarboxylic acid 4-[3-chloro-5-fluoro-2-(2-methyl-2H-tetrazol-5-yl)-indol-1-yl]-2-fluoro-benzylamide
3-Methoxy-isoxazole-5-carboxylic acid (1-{4-[3-chloro-5fluoro-2-(2-methyl-2H-tetrazol-5-yl)-indol-1-yl]-2-fluoro-benzylcarbamoyl}-cyclopropyl)-amide
3-Methoxy-isoxazole-5-carboxylic acid (1-{4-[3-cyano-2-(5-methyl-[1,2,4]oxadiazol-3-yl)-indol-1-yl]-benzylcarbamoyl}-cyclopropyl)-amide
1-(2,2,2-Trifluoro-acetylamino)-cyclopropanecarboxylic acid 4-[3,5-dichloro-2-(2-methyl-2H-tetrazol-5-yl)-indol-1-yl]-2-fluoro-benzylamide
1-(2,2,2-Trifluoro-acetylamino)-cyclopropanecarboxylic acid 4-[3-chloro-2-(5-methyl-[1,2,4]oxadiazol-3-yl)-indol-1-yl]-benzylamide
5-Cyclopropyl-isoxazole-3-carboxylic acid (1-{4-[3-chloro-2-(2-methyl-2H-tetrazol-5-yl)-indol-1-yl]-benzylcarbamoyl}-cyclopropyl)-amide
5-Methyl-pyrazine-2-carboxylic acid (1-{4-[3-chloro-2-(2-methyl-2H-tetrazol-5-yl)-indol-1-yl]-benzylcarbamoyl}-cyclopropyl)-amide
5-Methyl-isoxazole-3-carboxylic acid (1-{4-[3-chloro-2-(2-methyl-2H-tetrazol-5-yl)-indol-1-yl]-benzylcarbamoyl}-cyclopropyl)-amide
2-Chloro-N-(1-{4-[3-chloro-2-(2-methyl-2H-tetrazol-5-yl)-indol-1-yl]-benzylcarbamoyl}-cyclopropyl)-nicotinamide
N-(1-{4-[3-chloro-2-(2-methyl-2H-tetrazol-5-yl)-indol-1-yl]-benzylcarbamoyl}-cyclopropyl)-2-fluoro-isonicotinamide
1-(3,3,3,-Trifluoro-propionylamino)-cyclopropanecarboxylic acid 4-[3-chloro-2-(2-methyl-2H-tetrazol-5-yl)-indol-1-yl]-benzylamide
N-(1-{4-[3-chloro-2-(2-methyl-2H-tetrazol-5-yl)-indol-1-yl]-benzylcarbamoyl}-cyclopropyl)-5-trifluoromethyl-nicotinamide
N-(1-{4-[3-chloro-2-(2-methyl-2H-tetrazol-5-yl)-indol-1-yl]-benzylcarbamoyl}-cyclopropyl)-3-fluoro-benzamide
3-Chloro-1-[4-(1-{[1-(2,2,2-trifluoro-acetylamino)-cyclopropanecarbonyl]-amino}-ethyl)-phenyl]-1H-indole-2-carboxylic acid methylamide
1-(2,2,2-Trifluoro-acetylamino)-cyclopropanecarboxylic acid (1-{4-[3-chloro-2-(pyrrolidine-1-carbonyl)-indol-1-yl]-phenyl}-ethyl)-amide
3-Chloro-1-{4-[({1-[(3-methoxy-isoxazole-5-carbonyl)-amino]-cyclopropanecarbonyl}-amino)-methyl]-phenyl}-1H-indole-2-carboxylic acid ethyl ester
3-Chloro-1-{4-[({1-[(3-methoxy-isoxazole-5-carbonyl)-amino]-cyclopropanecarbonyl}-amino)-methyl]-phenyl}-1H-indole-2-carboxylic acid dimethylamide
1-(2,2,2-Trifluoro-acetylamino)-cyclopropanecarboxylic acid 4-[2-(2-methyl-2H-tetrazol-5-yl)-indol-1-yl]-benzylamide
3-Methoxy-isoxazole-5-carboxylic acid (1-{4-[3-chloro-5-fluoro-2-(5-methyl-[1,3,4]oxadiazol-2-yl)-indol-1-yl]-benzylcarbamoyl}-cyclopropyl)-amide
3-Methoxy-isoxazole-5-carboxylic acid (1-{4-[3-chloro-2-(5-methyl-oxazol-2-yl)-indol-1-yl]-benzylcarbamoyl}-cyclopropyl)-amide
3-Methoxy-isoxazole-5-carboxylic acid {1-[4-(3-chloro-2-cyano-indol-1-yl)-benzylcarbamoyl]-cyclopropyl}-amide
(R)-1-(2,2,2-Trifluoro-acetylamino)-cyclopropanecarboxylic acid (1-{4-[3-chloro-2-(5-methyl-[1,2,4]oxadiazol-3-yl)-indol-1-yl]-phenyl}-ethyl)-amide
(R)-3-Methoxy-isoxazole-5-carboxylic acid [1-(1-{4-[3-chloro-2-(5-methyl-[1,2,4]oxadiazol-3-yl)-indol-1-yl]-phenyl}-ethylcarbamoyl)-cyclopropyl]-amide
3-Propyl-isoxazole-5-carboxylic acid (1-{4-[3-chloro-2-(5-methyl-[1,2,4]oxadiazol-3-yl)-indol-1-yl]-benzylcarbamoyl}-cyclopropyl)-amide
3-Methoxy-isoxazole-5-carboxylic acid {1-[4-(5-chloro-2-cyano-indol-1-yl)-benzylcarbamoyl]-cyclopropyl}-amide
N-{1-[4-(2-Cyano-5-methoxy-indol-1-yl)-benzylcarbamoyl]-cyclopropyl}-5-trifluoromethyl-nicotinamide
N-{1-[4-(2-Cyano-5-fluoro-indol-1-yl)-benzylcarbamoyl]-cyclopropyl}-5-trifluoromethyl-nicotinamide
N-(1-{4-[5-Fluoro-2-(5-methyl-[1,2,4]oxadiazol-3-yl)-indol-1-yl]-benzylcarbamoyl}-cyclopropyl)-5-trifluoromethyl-nicotinamide
3-Methoxy-isoxazole-5-carboxylic acid (1-{4-[5-fluoro-2-(5-methyl-[1,2,4]oxadiazol-3-yl)-indol-1-yl]-benzylcarbamoyl}-cyclopropyl)-amide
3-Methoxy-isoxazole-5-carboxylic acid {1-[4-(2-cyano-5-methoxy-indol-1-yl)-benzylcarbamoyl]-cyclopropyl}-amide
3-Methoxy-isoxazole-5-carboxylic acid {1-[4-(2-cyano-5-fluoro-indol-1-yl)-benzylcarbamoyl]-cyclopropyl}-amide
N-(1-{4-[3-Chloro-5-methoxy-2-(3-methyl-[1,2,4]oxadiazol-5-yl)-indol-1-yl]-benzylcarbamoyl}-cyclopropyl)-5-trifluoromethyl-nicotinamide
3-Methoxy-isoxazole-5-carboxylic acid (1-{4-[3-chloro-5-methoxy-2-(3-methyl-[1,2,4]oxadiazol-5-yl)-indol-1-yl]-benzylcarbamoyl}-cyclopropyl)-amide
3-Methoxy-isoxazole-5-carboxylic acid {1-[4-(3-chloro-2-cyano-5-methoxy-indol-1-yl)-benzylcarbamoyl]-cyclopropyl}-amide
3-Methoxy-isoxazole-5-carboxylic acid (1-{4-[5-chloro-2-(5-methyl-[1,2,4]oxadiazol-3-yl)-indol-1-yl]-benzylcarbamoyl}-cyclopropyl)-amide
3-Chloro-1-[3-fluoro-4-({[1-(2,2,2-trifluoro-acetylamino)-cyclopropanecarbonyl]-amino}-methyl)-phenyl]-1H-indole-2-carboxylic acid methyl ester
3-Chloro-1-{3-fluoro-4-[({1-[(3-methoxy-isoxazole-5-carbonyl)-amino]-cyclopropanecarbonyl}-amino)-methyl]-phenyl}-1H-indole-2-carboxylic acid methyl ester
3-Methoxy-isoxazole-5-carboxylic acid [1-({5-[2-(3-methyl-[1,2,4]oxadiazol-5-yl)-indol-1-yl]-pyridin-2-ylmethyl}-carbamoyl)-cyclopropyl]-amide
3-Methoxy-isoxazole-5-carboxylic acid [1-({5-[2-(5-methyl-[1,2,4]oxadiazol-3-yl)-indol-1-yl]-pyridin-2-ylmethyl}-carbamoyl)-cyclopropyl]-amide
3-Methoxy-isoxazole-5-carboxylic acid [1-({5-[3-chloro-2-(3-methyl-[1,2,4]oxadiazol-5-yl)-indol-1-yl]-pyridin-2-ylmethyl}-carbamoyl)-cyclopropyl]-amide
3-Methoxy-isoxazole-5-carboxylic acid [1-({5-[3-chloro-5-fluoro-2-(2-methyl-2H-tetrazol-5-yl)-indol-1-yl]-pyridin-2-ylmethyl}-carbamoyl)-cyclopropyl]-amide
3-Methoxy-isoxazole-5-carboxylic acid [1-({5-[3-chloro-2-(2-methyl-2H-tetrazol-5-yl)-indol-1-yl]-pyridin-2-ylmethyl}-carbamoyl)-cyclopropyl]-amide
3-Methoxy-isoxazole-5-carboxylic acid [1-({5-[5-fluoro-2-(5-methyl-[1,2,4]oxadiazol-3-yl)-indol-1-yl]-pyridin-2-ylmethyl}-carbamoyl)-cyclopropyl]-amide
3-Methoxy-isoxazole-5-carboxylic acid [1-({5-[3-chloro-5-fluoro-2-(5-methyl-[1,2,4]oxadiazol-3-yl)-indol-1-yl]-pyridin-2-ylmethyl}-carbamoyl)-cyclopropyl]-amide
1-(2,2,2-Trifluoro-acetylamino)-cyclopropanecarboxylic acid 4-[3-chloro-5-methoxy-2-(5-methyl-[1,2,4]oxadiazol-3-yl)-indol-1-yl]-benzylamide
3-Methoxy-isoxazole-5-carboxylic acid (1-{4-[3-chloro-5-methoxy-2-(5-methyl-[1,2,4]oxadiazol-3-yl)-indol-1-yl]-benzylcarbamoyl}-cyclopropyl)-amide
1-(2,2,2-Trifluoro-acetylamino)-cyclopropanecarboxylic acid 4-[3,5-dichloro-2-(5-methyl-[1,2,4]oxadiazol-3-yl)-indol-1-yl]-benzylamide
3-Methoxy-isoxazole-5-carboxylic acid (1-{4-[3,5-dichloro-2-(5-methyl-[1,2,4]oxadiazol-3-yl)-indol-1-yl]-benzylcarbamoyl}-cyclopropyl)-amide
3-Methoxy-isoxazole-5-carboxylic acid (1-{4-[3-chloro-5-fluoro-2-(5-methyl-[1,2,4]oxadiazol-3-yl)-indol-1-yl]-benzylcarbamoyl}-cyclopropyl)-amide
3-Chloro-4-fluoro-1-{4-[({1-[(3-methoxy-isoxazole-5-carbonyl)-amino]-cyclopropanecarbonyl}-amino)-methyl]-phenyl}-1H-indole-2-carboxylic acid methyl ester
3-Chloro-4-fluoro-1-[4-({[1-(2,2,2-trifluoro-acetylamino)-cyclopropanecarbonyl]-amino}-methyl)-phenyl]-1H-indole-2-carboxylic acid methyl ester
2-Chloro-N-(1-{4-[3,5-dichloro-2-(5-methyl-[1,2,4]oxadiazol-3-yl)-indol-1-yl]-benzylcarbamoyl} -cyclopropyl)-nicotinamide
N-(1-{4-[3,5-Dichloro-2-(5-methyl-[1,2,4]oxadiazol-3-yl)-indol-1-yl]-benzylcarbamoyl} -cyclopropyl)-3-fluoro-benzamide
5-Methyl-pyrazine-2-carboxylic acid (1-{4-[3,5-dichloro-2-(5-methyl-[1,2,4]oxadiazol-3-yl)-indol-1-yl]-benzylcarbamoyl}-cyclopropyl)-amide
5-Methyl-isoxazole-3-carboxylic acid (1-{4-[3,5-dichloro-2-(5-methyl-[1,2,4]oxadiazol-3-yl)-indol-1-yl]-benzylcarbamoyl}-cyclopropyl)-amide
N-(1-{4-[3,5-Dichloro-2-(5-methyl-[1,2,4]oxadiazol-3-yl)-indol-1-yl]-benzylcarbamoyl} -cyclopropyl)-2-fluoro-isonicotinamide
5-Cyclopropyl-isoxazole-3-carboxylic acid (1-{4-[3,5-dichloro-2-(5-methyl-[1,2,4]oxadiazol-3-yl)-indol-1-yl]-benzylcarbamoyl}-cyclopropyl)-amide
N-(1-{4-[3,5-Dichloro-2-(5-methyl-[1,2,4]oxadiazol-3-yl)-indol-1-yl]-benzylcarbamoyl}-cyclopropyl)-5-trifluoromethyl-nicotinamide
2-Methylsulfanyl-pyrimidine-5-carboxylic acid (1-{4-[3-chloro-2-(5-methyl-[1,2,4]oxadiazol-3-yl)-indol-1-yl]-benzylcarbamoyl}-cyclopropyl)-amide
1-(3,3,3-Trifluoro-propionylamino)-cyclopropanecarboxylic acid 4-[5-methoxy-2-(3-methyl-[1,2,4]oxadiazol-5-yl)-indol-1-yl]-benzylamide
N-(1-{4-[5-Methoxy-2-(3-methyl-[1,2,4]oxadiazol-5-yl)-indol-1-yl]-benzylcarbamoyl}-cyclopropyl)-5-trifluoromethyl-nicotinamide
3-Methoxy-isoxazole-5-carboxylic acid (1-{4-[5-methoxy-2-(3-methyl-[1,2,4]oxadiazol-5-yl)-indol-1-yl]-benzylcarbamoyl}-cyclopropyl)-amide
1-(3,3,3-Trifluoro-propionylamino)-cyclopropanecarboxylic acid 4-[2-(5-methyl-[1,2,4]oxadiazol-3-yl)-indol-1-yl]-benzylamide
N-(1-{4-[2-(5-Methyl-[1,2,4]oxadiazol-3-yl)-indol-1-yl]-benzylcarbamoyl}-cyclopropyl)-5-trifluoromethyl-nicotinamide
N-(1-{4-[2-(3-Methyl-[1,2,4]oxadiazol-5-yl)-indol-1-yl]-benzylcarbamoyl}-cyclopropyl)-5-trifluoromethyl-nicotinamide
N-{1-[4-(2-Cyano-indol-1-yl)-benzylcarbamoyl]-cyclopropyl}-5-trifluoromethyl-nicotinamide
2-Chloro-N-(1-{4-[3-chloro-5-fluoro-2-(5-methyl-[1,2,4]oxadiazol-3-yl)-indol-1-yl]-benzylcarbamoyl}-cyclopropyl)-nicotinamide
N-(1-{4-[3-Chloro-5-fluoro-2-(5-methyl-[1,2,4]oxadiazol-3-yl)-indol-1-yl]-benzylcarbamoyl}-cyclopropyl)-3-fluoro-benzamide
5-Methyl-pyrazine-2-carboxylic acid (1-{4-[3-chloro-5-fluoro-2-(5-methyl-[1,2,4]oxadiazol-3-yl)-indol-1-yl]-benzylcarbamoyl}-cyclopropyl)-amide
5-Methyl-isoxazole-3-carboxylic acid (1-{4-[3-chloro-5-fluoro-2-(5-methyl-[1,2,4]oxadiazol-3-yl)-indol-1-yl]-benzylcarbamoyl}-cyclopropyl)-amide
N-(1-{4-[3-Chloro-5-fluoro-2-(5-methyl-[1,2,4]oxadiazol-3-yl)-indol-1-yl]-benzylcarbamoyl}-cyclopropyl)-2-fluoro-isonicotinamide
5-Cyclopropyl-isoxazole-3-carboxylic acid (1-{4-[3-chloro-5-fluoro-2-(5-methyl-[1,2,4]oxadiazol-3-yl)-indol-1-yl]-benzylcarbamoyl}-cyclopropyl)-amide
N-(1-{4-[3-Chloro-5-fluoro-2-(5-methyl-[1,2,4]oxadiazol-3-yl)-indol-1-yl]-benzylcarbamoyl}-cyclopropyl)-5-trifluoromethyl-nicotinamide
N-(1-{4-[3-Chloro-2-(5-methyl-[1,2,4]oxadiazol-3-yl)-indol-1-yl]-benzylcarbamoyl}-cyclopropyl)-2-fluoro-3-trifluoromethyl-benzamide
N-(1-{4-[3-Chloro-2-(5-methyl-[1,2,4]oxadiazol-3-yl)-indol-1-yl]-benzylcarbamoyl}-cyclopropyl)-3-fluoro-4-trifluoromethyl-benzamide
N-(1-{4-[3-Chloro-2-(5-methyl-[1,2,4]oxadiazol-3-yl)-indol-1-yl]-benzylcarbamoyl}-cyclopropyl)-3-fluoro-5-trifluoromethyl-benzamide
N-(1-{4-[3-Chloro-2-(5-methyl-[1,2,4]oxadiazol-3-yl)-indol-1-yl]-benzylcarbamoyl}-cyclopropyl)-2-fluoro-nicotinamide
N-(1-{4-[3-Chloro-2-(5-methyl-[1,2,4]oxadiazol-3-yl)-indol-1-yl]-benzylcarbamoyl}-cyclopropyl)-2-fluoro-5-trifluoromethyl-benzamide
N-(1-{4-[3-Chloro-2-(5-methyl-[1,2,4]oxadiazol-3-yl)-indol-1-yl]-benzylcarbamoyl}-cyclopropyl)-3-fluoro-isonicotinamide
N-(1-{4-[3-Chloro-2-(5-methyl-[1,2,4]oxadiazol-3-yl)-indol-1-yl]-benzylcarbamoyl}-cyclopropyl)-4-fluoro-3-trifluoromethyl-benzamide
N-(1-{4-[3-Chloro-2-(5-methyl-[1,2,4]oxadiazol-3-yl)-indol-1-yl]-benzylcarbamoyl}-cyclopropyl)-6-fluoro-nicotinamide
N-{1-[4-(5-Chloro-2-cyano-indol-1-yl)-benzylcarbamoyl]-cyclopropyl}-5-trifluoromethyl-nicotinamide
1-(3,3,3-Trifluoro-propionylamino)-cyclopropanecarboxylic acid 4-[5-fluoro-2-(3-methyl-[1,2,4]oxadiazol-5-yl)-indol-1-yl]-benzylamide
N-(1-{4-[5-Fluoro-2-(3-methyl-[1,2,4]oxadiazol-5-yl)-indol-1-yl]-benzylcarbamoyl}-cyclopropyl)-5-trifluoromethyl-nicotinamide
3-Methoxy-isoxazole-5-carboxylic acid (1-{4-[5-fluoro-2-(3-methyl-[1,2,4]oxadiazol-5-yl)-indol-1-yl]-benzylcarbamoyl}-cyclopropyl)-amide
2-Chloro-N-(1-{4-[3-chloro-2-(5-methyl-[1,2,4]oxadiazol-3-yl)-indol-1-yl]-2-fluoro-benzylcarbamoyl}-cyclopropyl)-nicotinamide
N-(1-{4-[3-Chloro-2-(5-methyl-[1,2,4]oxadiazol-3-yl)-indol-1-yl]-2-fluoro-benzylcarbamoyl} -cyclopropyl)-3-fluoro-benzamide
5-Methyl-pyrazine-2-carboxylic acid (1-{4-[3-chloro-2-(5-methyl-[1,2,4]oxadiazol-3-yl)-indol-1-yl]-2-fluoro-benzylcarbamoyl}-cyclopropyl)-amide
5-Methyl-isoxazole-3-carboxylic acid (1-{4-[3-chloro-2-(5-methyl-[1,2,4]oxadiazol-3-yl)-indol-1-yl]-2-fluoro-benzylcarbamoyl}-cyclopropyl)-amide
1-(3,3,3-Trifluoro-propionylamino)-cyclopropanecarboxylic acid 4-[3-chloro-2-(5-methyl-[1,2,4]oxadiazol-3-yl)-indol-1-yl]-2-fluoro-benzylamide
N-(1-{4-[3-Chloro-2-(5-methyl-[1,2,4]oxadiazol-3-yl)-indol-1-yl]-2-fluoro-benzylcarbamoyl}-cyclopropyl)-2-fluoro-isonicotinamide
5-Cyclopropyl-isoxazole-3-carboxylic acid (1-{4-[3-chloro-2-(5-methyl-[1,2,4]oxadiazol-3-yl)-indol-1-yl]-2-fluoro-benzylcarbamoyl}-cyclopropyl)-amide
N-(1-{4-[3-Chloro-2-(5-methyl-[1,2,4]oxadiazol-3-yl)-indol-1-yl]-2-fluoro-benzylcarbamoyl}-cyclopropyl)-5-trifluoromethyl-nicotinamide
1-(3,3,3-Trifluoro-propionylamino)-cyclopropanecarboxylic acid 4-[3-chloro-2-(3-methyl-[1,2,4]oxadiazol-5-yl)-indol-1-yl]-benzylamide
N-(1-{4-[3-Chloro-2-(3-methyl-[1,2,4]oxadiazol-5-yl)-indol-1-yl]-benzylcarbamoyl}-cyclopropyl)-2-fluoro-isonicotinamide
5-Cyclopropyl-isoxazole-3-carboxylic acid (1-{4-[3-chloro-2-(3-methyl-[1,2,4]oxadiazol-5-yl)-indol-1-yl]-benzylcarbamoyl}-cyclopropyl)-amide
N-(1-{4-[3-Chloro-2-(3-methyl-[1,2,4]oxadiazol-5-yl)-indol-1-yl]-benzylcarbamoyl}-cyclopropyl)-5-trifluoromethyl-nicotinamide
2-Fluoro-N-(1-{4-[5-methoxy-2-(3-methyl-[1,2,4]oxadiazol-5-yl)-indol-1-yl]-benzylcarbamoyl}-cyclopropyl)-nicotinamide
N-(1-{4-[3-Chloro-5-fluoro-2-(5-methyl-[1,2,4]oxadiazol-3-yl)-indol-1-yl]-benzylcarbamoyl}-cyclopropyl)-2-fluoro-nicotinamide
N-(1-{4-[3-Chloro-5-methoxy-2-(3-methyl-[1,2,4]oxadiazol-5-yl)-indol-1-yl]-benzylcarbamoyl}-cyclopropyl)-2-fluoro-nicotinamide
N-(1-{4-[3-Chloro-2-(2-methyl-2H-tetrazol-5-yl)-indol-1-yl]-benzylcarbamoyl}-cyclopropyl)-2-fluoro-nicotinamide
2-Fluoro-N-(1-{4-[5-fluoro-2-(5-methyl-[1,2,4]oxadiazol-3-yl)-indol-1-yl]-benzylcarbamoyl}-cyclopropyl)-nicotinamide
1-Methyl-1H-pyrazole-4-carboxylic acid (1-{4-[3-chloro-2-(5-methyl-[1,2,4]oxadiazol-3-yl)-indol-1-yl]-benzylcarbamoyl}-cyclopropyl)-amide
2-Oxo-imidazolidine-4-carboxylic acid (1-{4-[3-chloro-2-(5-methyl-[1,2,4]oxadiazol-3-yl)-indol-1-yl]-benzylcarbamoyl}-cyclopropyl)-amide
3-Ethyl-isoxazole-5-carboxylic acid (1-{4-[3-chloro-2-(5-methyl-[1,2,4]oxadiazol-3-yl)-indol-1-yl]-benzylcarbamoyl}-cyclopropyl)-amide
1-(3,3,3-Trifluoro-propionylamino)-cyclopropanecarboxylic acid 4-[5-methoxy-2-(5-methyl-[1,3,4]oxadiazol-2-yl)-indol-1-yl]-benzylamide
N-(1-{4-[5-Methoxy-2-(5-methyl-[1,3,4]oxadiazol-2-yl)-indol-1-yl]-benzylcarbamoyl}-cyclopropyl)-5-trifluoromethyl-nicotinamide
3-Methoxy-isoxazole-5-carboxylic acid (1-{4-[5-methoxy-2-(5-methyl-[1,3,4]oxadiazol-2-yl)-indol-1-yl]-benzylcarbamoyl}-cyclopropyl)-amide
1-(3,3,3-Trifluoro-propionylamino)-cyclopropanecarboxylic acid 4-[5-chloro-2-(5-methyl-[1,2,4]oxadiazol-3-yl)-indol-1-yl]-benzylamide
N-(1-{4-[5-Chloro-2-(5-methyl-[1,2,4]oxadiazol-3-yl)-indol-1-yl]-benzylcarbamoyl}-cyclopropyl)-5-trifluoromethyl-nicotinamide
5-Methyl-pyrazine-2-carboxylic acid (1-{4-[3-chloro-5-methoxy-2-(5-methyl-[1,2,4]oxadiazol-3-yl)-indol-1-yl]-benzylcarbamoyl}-cyclopropyl)-amide
N-(1-{4-[3-Chloro-5-methoxy-2-(5-methyl-[1,2,4]oxadiazol-3-yl)-indol-1-yl]-benzylcarbamoyl}-cyc1opropyl)-5-trifluoromethyl-nicotinamide
5-Methyl-pyrazine-2-carboxylic acid (1-{2-fluoro-4-[5-fluoro-2-(5-methyl-[1,2,4]oxadiazol-3-yl)-indol-1-yl]-benzylcarbamoyl}-cyclopropyl)-amide
N-(1-{2-Fluoro-4-[5-fluoro-2-(5-methyl-[1,2,4]oxadiazol-3-yl)-indol-1-yl]-benzylcarbamoyl}-cyclopropyl)-5-trifluoromethyl-nicotinamide
3-Methoxy-isoxazole-5-carboxylic acid (1-{2-fluoro-4-[5-fluoro-2-(5-methyl-[1,2,4]oxadiazol-3-yl)-indol-1-yl]-benzylcarbamoyl}-cyclopropyl)-amide
Isoxazole-5-carboxylic acid (1-{4-[3-chloro-2-(5-methyl-[1,2,4]oxadiazol-3-yl)-indol-1-yl] -benzylcarbamoyl}-cyclopropyl)-amide
5-Methyl-isoxazole-3-carboxylic acid (1-{4-[3-chloro-2-(5-methyl-[1,2,4]oxadiazol-3-yl)-indol-1-yl]-benzylcarbamoyl}-cyclopropyl)-amide
Tetrahydro-furan-2-carboxylic acid (1-{4-[3-chloro-2-(5-methyl-[1,2,4]oxadiazol-3-yl)-indol-1-yl]-benzylcarbamoyl}-cyclopropyl)-amide
Furan-3-carboxylic acid (1-{4-[3-chloro-2-(5-methyl-[1,2,4]oxadiazol-3-yl)-indol-1-yl]-benzylcarbamoyl}-cyclopropyl)-amide
2-Methyl-cyclopropanecarboxylic acid (1-{4-[3-chloro-2-(5-methyl-[1,2,4]oxadiazol-3-yl)-indol-1-yl]-benzylcarbamoyl}-cyclopropyl)-amide
1-Pent-4-ynoylamino-cyclopropanecarboxylic acid 4-[3-chloro-2-(5-methyl-[1,2,4]oxadiazol-3-yl)-indol-1-yl]-benzylamide
1-(4-Methyl-pentanoylamino)-cyclopropanecarboxylic acid 4-[3-chloro-2-(5-methyl-[1,2,4]oxadiazol-3-yl)-indol-1-yl]-benzylamide
1H-Pyrazole-4-carboxylic acid (1-{4-[3-chloro-2-(5-methyl-[1,2,4]oxadiazol-3-yl)-indol-1-yl]-benzylcarbamoyl}-cyclopropyl)-amide
1-Cyano-cyclopropanecarboxylic acid (1-{4-[3-chloro-2-(5-methyl-[1,2,4]oxadiazol-3-yl)-indol-1-yl]-benzylcarbamoyl}-cyclopropyl)-amide
Cyclobutanecarboxylic acid (1-{4-[3-chloro-2-(5-methyl-[1,2,4]oxadiazol-3-yl)-indol-1-yl] -benzylcarbamoyl}-cyclopropyl)-amide
Cyclopentanecarboxylic acid (1-{4-[3-chloro-2-(5-methyl-[1,2,4]oxadiazol-3-yl)-indol-1-yl]-benzylcarbamoyl}-cyclopropyl)-amide
Furan-2-carboxylic acid (1-{4-[3-chloro-2-(5-methyl-[1,2,4]oxadiazol-3-yl)-indol-1-yl]-benzylcarbamoyl}-cyclopropyl)-amide
N-(1-{4-[3-Chloro-2-(5-methyl-[1,2,4]oxadiazol-3-yl)-indol-1-yl]-benzylcarbamoyl}-cyclopropyl)-3-cyano-benzamide
1-(2-Thiophen-3-yl-acetylamino)-cyclopropanecarboxylic acid 4-[3-chloro-2-(5-methyl-[1,2,4]oxadiazol-3-yl)-indol-1-yl]-benzylamide
1,5-Dimethyl-1H-pyrazole-3-carboxylic acid (1-{4-[3-chloro-2-(5-methyl-[1,2,4]oxadiazol-3-yl)-indol-1-yl]-benzylcarbamoyl}-cyclopropyl)-amide
4-Methyl-thiazole-5-carboxylic acid (1-{4-[3-chloro-2-(5-methyl-[1,2,4]oxadiazol-3-yl)-indol-1-yl]-benzylcarbamoyl}-cyclopropyl)-amide
2,4-Dimethyl-thiazole-5-carboxylic acid (1-{4-[3-chloro-2-(5-methyl-[1,2,4]oxadiazol-3-yl)-indol-1-yl]-benzylcarbamoyl}-cyclopropyl)-amide
1-(2-Acetylamino-3-hydroxy-propionylamino)-cyclopropanecarboxylic acid 4-[3-chloro-2-(5-methyl-[1,2,4]oxadiazol-3-yl)-indol-1-yl]-benzylamide
5-Methyl-1H-pyrazole-3-carboxylic acid (1-{4-[3-chloro-2-(5-methyl-[1,2,4]oxadiazol-3-yl)-indol-1-yl]-benzylcarbamoyl}-cyclopropyl)-amide
2-Chloro-N-(1-{4-[3-chloro-2-(5-methyl-[1,2,4]oxadiazol-3-yl)-indol-1-yl]-benzylcarbamoyl}-cyclopropyl)-nicotinamide
N-(1-{4-[3-Chloro-2-(5-methyl-[1,2,4]oxadiazol-3-yl)-indol-1-yl]-benzylcarbamoyl}-cyclopropyl)-6-methyl-nicotinamide
N-(1-{4-[3-Chloro-2-(5-methyl-[1,2,4]oxadiazol-3-yl)-indol-1-yl]-benzylcarbamoyl}-cyclopropyl)-2-methyl-nicotinamide
2-Chloro-N-(1-{4-[3-chloro-2-(5-methyl-[1,2,4]oxadiazol-3-yl)-indol-1-yl]-benzylcarbamoyl}-cyclopropyl)-6-methyl-nicotinamide
6-Bromo-pyridine-2-carboxylic acid (1-{4-[3-chloro-2-(5-methyl-[1,2,4]oxadiazol-3-yl)-indol-1-yl]-benzylcarbamoyl}-cyclopropyl)-amide
N-(1-{4-[3-Chloro-2-(5-methyl-[1,2,4]oxadiazol-3-yl)-indol-1-yl]-benzylcarbamoyl}-cyclopropyl)-nicotinamide
Quinoline-3-carboxylic acid (1-{4-[3-chloro-2-(5-methyl-[1,2,4]oxadiazol-3-yl)-indol-1-yl]-benzylcarbamoyl}-cyclopropyl)-amide
6-Oxo-1,6-dihydro-pyridine-3-carboxylic acid (1-{4-[3-chloro-2-(5-methyl-[1,2,4]oxadiazol-3-yl)-indol-1-yl]-benzylcarbamoyl}-cyclopropyl)-amide
5-Methyl-pyrazine-2-carboxylic acid (1-{4-[3-chloro-2-(5-methyl-[1,2,4]oxadiazol-3-yl)-indol-1-yl]-benzylcarbamoyl}-cyclopropyl)-amide
6-Oxo-1,6-dihydro-pyridine-2-carboxylic acid (1-{4-[3-chloro-2-(5-methyl-[1,2,4]oxadiazol-3-yl)-indol-1-yl]-benzylcarbamoyl}-cyclopropyl)-amide
1-(3,3,3-Trifluoro-propionylamino)-cyclopropanecarboxylic acid 4-[3-chloro-2-(5-methyl-[1,2,4]oxadiazol-3-yl)-indol-1-yl]-benzylamide
N-(1-{4-[3-Chloro-2-(5-methyl-[1,2,4]oxadiazol-3-yl)-indol-1-yl]-benzylcarbamoyl}-cyclopropyl)-2-fluoro-isonicotinamide
5-Cyclopropyl-isoxazole-3-carboxylic acid (1-{4-[3-chloro-2-(5-methyl-[1,2,4]oxadiazol-3-yl)-indol-1-yl]-benzylcarbamoyl}-cyclopropyl)-amide
Pyrimidine-5-carboxylic acid (1-{4-[3-chloro-2-(5-methyl-[1,2,4]oxadiazol-3-yl)-indol-1-yl] -benzylcarbamoyl}-cyclopropyl)-amide
Thiophene-3-carboxylic acid (1-{4-[3-chloro-2-(5-methyl-[1,2,4]oxadiazol-3-yl)-indol-1-yl]-benzylcarbamoyl}-cyclopropyl)-amide
5-Oxo-pyrrolidine-2-carboxylic acid (1-{4-[3-chloro-2-(5-methyl-[1,2,4]oxadiazol-3-yl)-indol-1-yl]-benzylcarbamoyl}-cyclopropyl)-amide
1-(3-Furan-3-yl-acryloylamino)-cyclopropanecarboxylic acid 4-[3-chloro-2-(5-methyl-[1,2,4]oxadiazol-3-yl)-indol-1-yl]-benzylamide
N-(1-{4-[3-Chloro-2-(5-methyl-[1,2,4]oxadiazol-3-yl)-indol-1-yl]-benzylcarbamoyl}-cyclopropyl)-4-cyano-benzamide
1-(3-Furan-2-yl-acryloylamino)-cyclopropanecarboxylic acid 4-[3-chloro-2-(5-methyl-[1,2,4]oxadiazol-3-yl)-indol-1-yl]-benzylamide
Thiophene-2-carboxylic acid (1-{4-[3-chloro-2-(5-methyl-[1,2,4]oxadiazol-3-yl)-indol-1-yl]-benzylcarbamoyl}-cyclopropyl)-amide
5-Methylsulfanyl-thiophene-2-carboxylic acid (1-{4-[3-chloro-2-(5-methyl-[1,2,4]oxadiazol-3-yl)-indol-1-yl]-benzylcarbamoyl}-cyclopropyl)-amide
[1,2,3]Thiadiazole-4-carboxylic acid (1-{4-[3-chloro-2-(5-methyl-[1,2,4]oxadiazol-3-yl)-indol-1-yl]-benzylcarbamoyl}-cyclopropyl)-amide
N-(1-{4-[3-Chloro-2-(5-methyl-[1,2,4]oxadiazol-3-yl)-indol-1-yl]-benzylcarbamoyl}-cyclopropyl)-3-methoxy-benzamide
N-(1-{4-[3-Chloro-2-(5-methyl-[1,2,4]oxadiazol-3-yl)-indol-1-yl]-benzylcarbamoyl}-cyclopropyl)-3-iodo-benzamide
N-(1-{4-[3-Chloro-2-(5-methyl-[1,2,4]oxadiazol-3-yl)-indol-1-yl]-benzylcarbamoyl}-cyclopropyl)-3-trifluoromethyl-benzamide
N-(1-{4-[3-Chloro-2-(5-methyl-[1,2,4]oxadiazol-3-yl)-indol-1-yl]-benzylcarbamoyl}-cyclopropyl)-3-fluoro-benzamide
N-(1-{4-[3-Chloro-2-(5-methyl-[1,2,4]oxadiazol-3-yl)-indol-1-yl]-benzylcarbamoyl}-cyclopropyl)-3-methyl-benzamide
N-(1-{4-[3-Chloro-2-(5-methyl-[1,2,4]oxadiazol-3-yl)-indol-1-yl]-benzylcarbamoyl}-cyclopropyl)-5-trifluoromethyl-nicotinamide
3-Methyl-isoxazole-4-carboxylic acid (1-{4-[3-chloro-2-(5-methyl-[1,2,4]oxadiazol-3-yl)-indol-1-yl]-benzylcarbamoyl}-cyclopropyl)-amide
Quinoline-3-carboxylic acid (1-{4-[3-chloro-2-(5-methyl-[1,3,4]oxadiazol-2-yl)-indol-1-yl]-benzylcarbamoyl}-cyclopropyl)-amide
1-(3,3,3-Trifluoro-propionylamino)-cyclopropanecarboxylic acid 4-[3-chloro-2-(5-methyl-[1,3,4]oxadiazol-2-yl)-indol-1-yl]-benzylamide
Pyrimidine-5-carboxylic acid (1-{4-[3-chloro-2-(5-methyl-[1,3,4]oxadiazol-2-yl)-indol-1-yl] -benzylcarbamoyl}-cyclopropyl)-amide
2-Methyl-pyrimidine-5-carboxylic acid (1-{4-[3-chloro-2-(5-methyl-[1,2,4]oxadiazol-3-yl)-indol-1-yl]-benzylcarbamoyl}-cyclopropyl)-amide
3-Amino-N-(1-{4-[3-chloro-2-(5-methyl-[1,2,4]oxadiazol-3-yl)-indol-1-yl]-benzylcarbamoyl}-cyclopropyl)-benzamide hydrochloride
3-Chloro-1-[4-({[1-(2,2,2-trifluoro-acetylamino)-cyclopropanecarbonyl]-amino}-methyl)-phenyl]-1H-indole-2-carboxylic acid amide
3-Chloro-1-{4-[({1-[(3-methoxy-isoxazole-5-carbonyl)-amino]-cyclopropanecarbonyl}-amino)-methyl]-phenyl}-1H-indole-2-carboxylic acid amide
1-(2,2,2-Trifluoro-acetylamino)-cyclopropanecarboxylic acid 4-[3-chloro-5-fluoro-2-(5-methyl-[1,2,4]oxadiazol-3-yl)-indol-1-yl]-benzylamide
1-(2,2,2-Trifluoro-acetylamino)-cyclopropanecarboxylic acid ((R)-1-{4-[3-chloro-2-(5-methyl-[1,2,4]oxadiazol-3-yl)-indol-1-yl]-2-fluoro-phenyl}-ethyl)-amide
3-Methoxy-isoxazole-5-carboxylic acid [1-((R)-1-{4-[3-chloro-2-(5-methyl-[1,2,4]oxadiazol-3-yl)-indol-1-yl]-2-fluoro-phenyl}-ethylcarbamoyl)-cyclopropyl]-amide
3-Methoxy-isoxazole-5-carboxylic acid (1-{4-[3-chloro-2-(5-methyl-[1,2,4]oxadiazol-3-yl)-indol-1-yl]-2-fluoro-benzylcarbamoyl}-cyclopropyl)-amide
3-Methoxy-isoxazole-5-carboxylic acid (1-{4-[3-chloro-2-(3-methyl-[1,2,4]oxadiazol-5-yl)-indol-1-yl]-benzylcarbamoyl} -cyclopropyl)-amide
1-(2,2,2-Trifluoro-acetylamino)-cyclopropanecarboxylic acid 4-[3-chloro-2-(3-methyl-[1,2,4]oxadiazol-5-yl)-indol-1-yl]-benzylamide
3-Methoxy-isoxazole-5-carboxylic acid (1-{4-[2-(5-methyl-[1,2,4]oxadiazol-3-yl)-indol-1-yl]-benzylcarbamoyl} -cyclopropyl)-amide
3-Methoxy-isoxazole-5-carboxylic acid (1-{4-[2-(3-methyl-[1,2,4]oxadiazol-5-yl)-indol-1-yl]-benzylcarbamoyl}-cyclopropyl)-amide
3-Methoxy-isoxazole-5-carboxylic acid (1-{4-[3-chloro-5-fluoro-2-(5-methyl-[1,2,4]oxadiazol-3-yl)-indol-1-yl]-2-fluoro-benzylcarbamoyl}-cyclopropyl)-amide
3-Methoxy-isoxazole-5-carboxylic acid (1-{4-[3,5-difluoro-2-(5-methyl-[1,2,4]oxadiazol-3-yl)-indol-1-yl]-benzylcarbamoyl}-cyclopropyl)-amide
1-(2,2,2-Trifluoro-acetylamino)-cyclopropanecarboxylic acid 4-[3,5-difluoro-2-(5-methyl-[1,2,4]oxadiazol-3-yl)-indol-1-yl]-benzylamide
3-Methoxy-isoxazole-5-carboxylic acid (1-{4-[5-fluoro-3-methyl-2-(5-methyl-[1,2,4]oxadiazol-3-yl)-indol-1-yl]-benzylcarbamoyl}-cyclopropyl)-amide and
3-Methoxy-isoxazole-5-carboxylic acid (1-{4-[3-chloro-2-(4-methyl-5-oxo-4,5-dihydro-[1,2,4]oxadiazol-3-yl)-indol-1-yl]-benzylcarbamoyl}-cyclopropyl)-amide and optical antipodes or racemates and/or salts thereof.

3. Process for preparing the compounds of formula (I) as claimed in Claim 1 **characterized by**
a) reacting an indole derivative of formula (II) 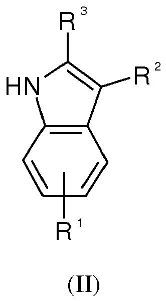
- wherein the meaning of R1, R2 and R3 is as described for the formula (I) in Claim 1 - with a boronic acid of formula (III) 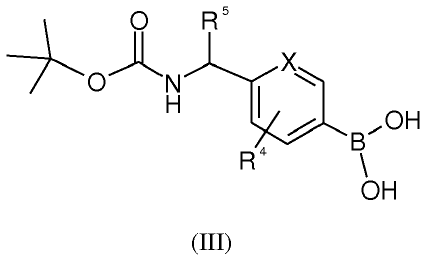
- wherein the meaning of R4 and R5 is as described for the formula (I) in Claim 1 - then the so obtained indole derivative of formula (IV) 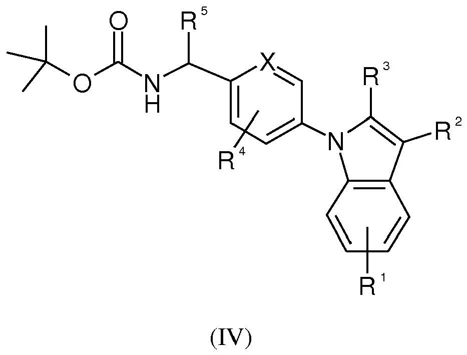
- wherein the meaning of R1, R2, R3, R4 and R5 is as described for the formula (I) in Claim 1 - is deprotected to yield an amine derivative of formula (V) 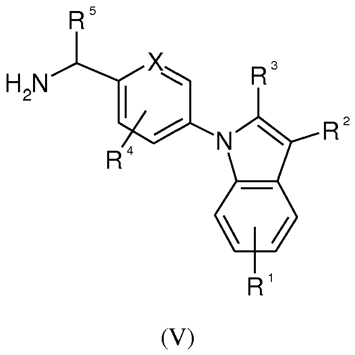
- wherein the meaning of R1, R2, R3, R4 and R5 is as described for the formula (I) in Claim 1 - finally the latter is reacted with an acid derivative of formula (VI) 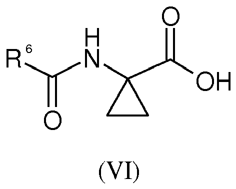
- wherein the meaning of R6 is as described for the formula (I) in Claim 1; or
b) reacting an indole derivative of formula (II) 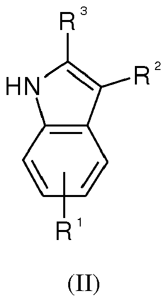
- wherein the meaning of R1, R2 and R3 is as described for the formula (I) in Claim 1 - with a boronic acid of formula (VII) 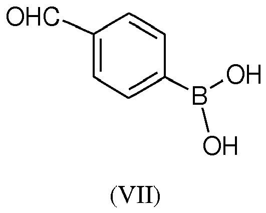 then the so obtained indole derivative of formula (VIII) 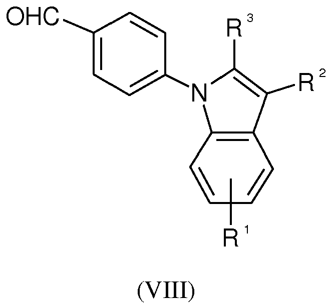
- wherein the meaning of R1, R2 and R3 is as described for the formula (I) in Claim 1 - is reacted with hydroxylamine hydrochloride to yield the indole derivative of formula (IX) 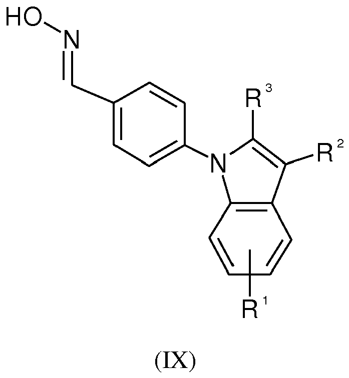
- wherein the meaning of R1, R2 and R3 is as described for the formula (I) in Claim 1 - the latter is hydrogenated to furnish an amine derivative of formula (X) 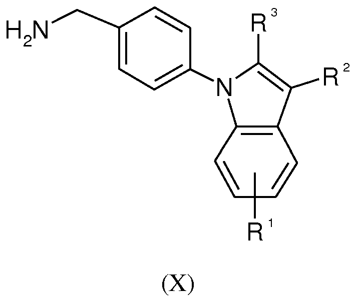
- wherein the meaning of R1, R2 and R3 is as described for the formula (I) in Claim 1 - finally the latter is reacted with an acid derivative of formula (VI) 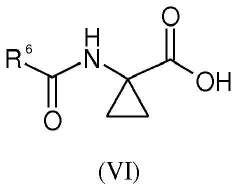
- wherein the meaning of R6 is as described for the formula (I) in Claim 1; or
c) reacting the amine derivatives of formula (V) or formula (X) - obtained according to the method described in method a) and method b) - with the amino acid derivative of formula (XI) 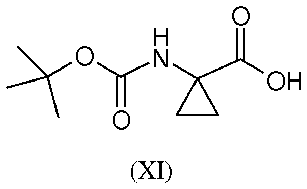 and the so obtained indole derivative of formula (XII) 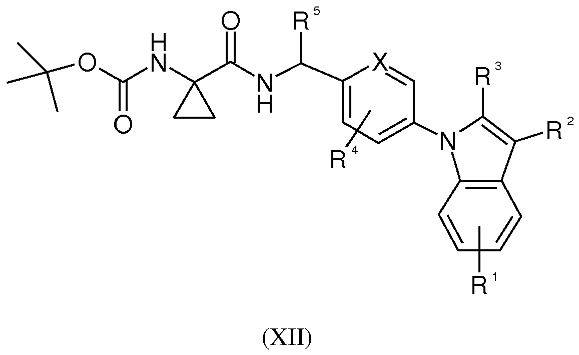
- wherein the meaning of R1, R2, R3, R4 and R5 is as described for the formula (I) in Claim 1 - is deprotected to yield an amine derivative of formula (XIII) 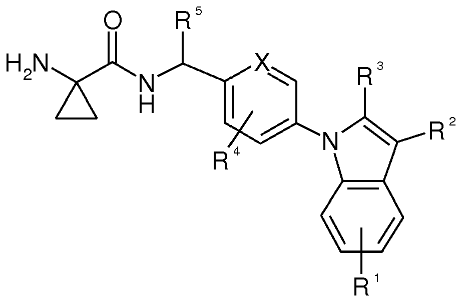 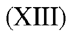
- wherein the meaning of R1, R2, R3, R4 and R5 is as described for the formula (I) in Claim 1 - finally the latter is reacted with an acid derivative of formula (XIV) 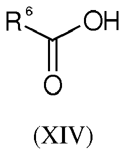
- wherein the meaning of R6 is as described for the formula (I) in Claim 1; and optionally an indole derivative of formula (I) obtained in process a) or b) or c) and optical antipodes or racemates and/or salts thereof can be transformed into an other compound of formula (I) and optical antipodes or racemates and/or salts thereof by introducing new substituents and/or modifying or removing the existing ones.

4. A pharmaceutical composition comprising a therapeutically effective amount of a compound of formula (I) as claimed in Claim 1 and optical antipodes or racemates and/or salts thereof as active ingredients and pharmaceutically acceptable auxiliary materials.

5. Process for manufacturing pharmaceutical composition having selective bradykinin B1 receptor antagonist effect **characterized by** mixing a therapeutically effective amount of a compound of formula (I) as claimed in Claim 1 and optical antipodes or racemates and/or salts thereof as active ingredients and auxiliary materials.

6. A compound of formula (I) as claimed in Claim 1 and optical antipodes or racemates and/or salt thereof for use as selective bradykinin B1 receptor antagonist.

7. A compound of formula (I) as claimed in Claim 1 and optical antipodes or racemates and/or salts thereof for use in the treatment or prevention of disorders associated with bradykinin B1 receptor activity.

8. A compound in accordance with claim 7 wherein disorder is selected from the group of pain and inflammation, including somatic musculo-skeletal pain, low-back pain, repetitive motion disorders, myofascial pain syndrome, arthritis - including osteoarthritis, rheumatoid arthritis and gout - fibromyalgia, visceral pain, inflammatory skin disorders and skin injuries, complications associated with diabetes such as neuropathy, nephropathy, vasculopathy, retinopathy, cancer pain and inflammatory bowel disease.

9. Use of a compound of formula (I) as claimed in Claim 1 or optical antipodes or racemates or pharmaceutically acceptable salt or hydrate or solvate thereof for the manufacture of a medicament for prevention and/or treatment of a condition which requires inhibition of a bradykinin receptor.

10. Use according to Claim 9 wherein the bradykinin receptor is bradykinin B1 receptor.

## Patentansprüche

1. Indol Derivate der Formel (I) 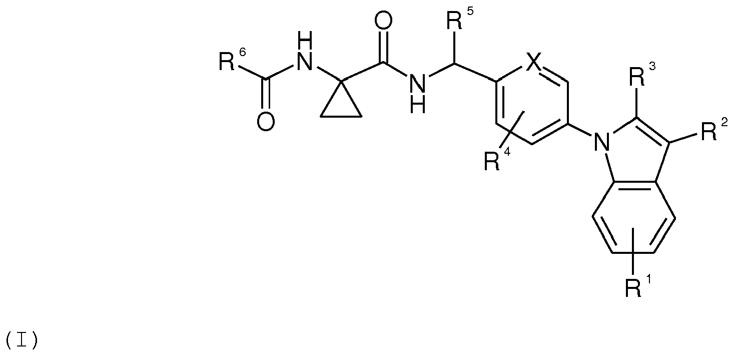 worin
R¹ ein Wasserstoffatom, Halogenatom oder C₁₋₄-Akoxygruppe ist;
R² ein Wasserstoffatom, Halogenatom, C₁₋₄-Alkylgruppe oder Cyanogruppe ist;
R³ aus (1) -COOR; (2) -CN; (3) -CONRaRb; 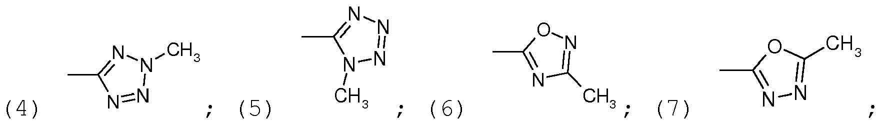 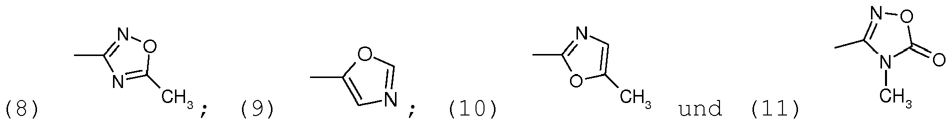 Gruppe ausgewählt ist;
R⁴ ein Wasserstoffatom oder Halogenatom ist;
R⁵ ein Wasserstoffatom oder C₁₋₄-Alkylgruppe ist;
R⁶ aus
(1) C₃₋₆-Cycloalkyl;
(2) gegebenenfalls mit zwei Stickstoffatomen substituiert fünf- oder sechs-gliediger aromatischer Ring;
(3) gegebenenfalls substituiert Pyridyl;
(4) gegebenenfalls mit einem Heteroatom, ausgewählt aus O und S, substituiert fünf-gliediger aromatischer Ring; (5)-CF₃; (6) -CH₂-CF₃; (7) -CH₂-CH₂-C≡CH; (8) -CH₂-CH₂-CH(CH₃)₂ ; 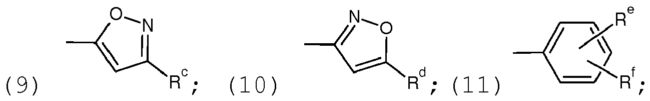 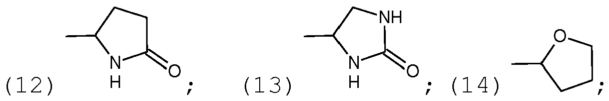 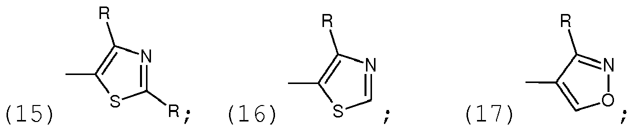 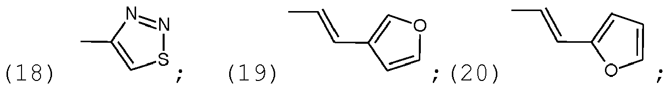 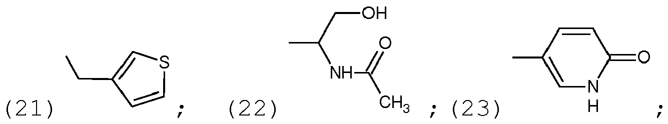 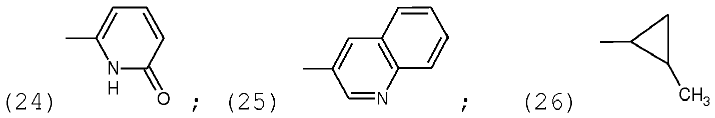 und 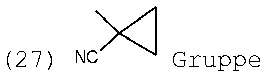 ausgewählt ist;
X -CH- oder Stickstoffatom ist;
R C₁₋₄-Alkylgruppe ist;
Ra und Rb unabhängig voneinander Wasserstoffatom; C₁₋₄-Alkylgruppe oder Pyrrolidin-1-yl Gruppe sind;
Rc Wasserstoffatom; C₁₋₄-Alkylgruppe oder C₁₋₄-Alkoxygruppe ist;
Rd Wasserstoffatom; C₁₋₄-Alkylgruppe oder C₃₋₆-Cycloalkylgruppe ist;
Re und Rf unabhängig voneinander Wasserstoffatom; Halogenatom; C₁₋₄-Alkoxygruppe; -CF₃; -CN oder -NH₂ Gruppe sind;
und optische Antipoden oder Racemate und/oder Salze davon.

2. Verbindung gemäß Anspruch 1, ausgewählt aus der Gruppe:
1-(2,2,2-Trifluor-acetylamino)-cyclopropane carbonsäure 4-[3-chlor-2-(2-methyl-2H-tetrazol-5-yl)-indol-1-yl]-benzylamid
3-Methoxy-isoxazol-5-carbonsäure (1-{4-[3-chlor-2-(2-methyl-2H-tetrazol-5-yl)-indol-1-yl]-benzylcarbamoyl}-cyclopropyl)-amid
3-Methoxy-isoxazol-5-carbonsäure (1-{4-[2-(2-methyl-2H-tetrazol-5-yl)-indol-1-yl]-benzylcarbamoyl}-cyclopropyl)-amid
1-(2,2,2-Trifluor-acetylamino)-cyclopropane carbonsäure 4-[3-chlor-2-(2-methyl-2H-tetrazol-5-yl)-indol-1-yl]-2-fluorbenzylamid
3-Methoxy-N-[1-({4-[3-chlor-2-(5-methyl-1,3,4-oxadiazol-2-yl)-1H-indol-1-yl]benzyl}carbamoyl)cyclopropyl]isoxazol-5-carboxamid
1-(2,2,2-Trifluor-acetylamino)-cyclopropane carbonsäure 4-[3-chlor-2-(5-methyl-[1,3,4]oxadiazol-2-yl)-indol-1-yl]-benzylamid
3-Methoxy-isoxazol-5-carbonsäure (1-{4-[3-chlor-2-(1-methyl-1H-tetrazol-5-yl)-indol-1-yl]-benzylcarbamoyl}-cyclopropyl)-amid
1-(2,2,2-Trifluor-acetylamino)-cyclopropane carbonsäure 4-[3-chlor-2-(1-methyl-1H-tetrazol-5-yl)-indol-1-yl]-benzylamid
3-Methoxy-isoxazol-5-carbonsäure (1-{4-[3-chlor-2-(5-methyl-[1,2,4]oxadiazol-3-yl)-indol-1-yl]-benzylcarbamoyl}-cyclopropyl)-amid
3-Chlor-1-[4-({[1-(2,2,2-trifluor-acetylamino)-cyclopropanecarbonyl]-amino}-methyl)-phenyl]-1H-indole-2-carbonsäure methylester
3-Chlor-1-{4-[({1-[(3-methoxy-isoxazol-5-carbonyl)-amino]-cyclopropane-carbonyl}-amino)-methyl]-phenyl}-1H-indole-2-carbonsäure methylester
1-(2,2,2-Trifluor-acetylamino)-cyclopropane carbonsäure 4-[3-chlor-5-fluor-2-(2-methyl-2H-tetrazol-5-yl)-indol-1-yl]-benzylamid
3-Methoxy-isoxazol-5-carbonsäure (1-{4-[3-chlor-5-fluor-2-(2-methyl-2H-tetrazol-5-yl)-indol-1-yl]-benzylcarbamoyl}-cyclopropyl)-amid
1-{4-[({1-[(3-Methoxy-isoxazol-5-carbonyl)-amino]-cyclopropanecarbonyl}-amino)-methyl]-phenyl}-1H-indole-2-carbonsäure methylester
3-Methoxy-isoxazol-5-carbonsäure (1-{4-[3-chlor-2-(2-methyl-2H-tetrazol-5-yl)-indol-1-yl]-2-fluorbenzylcarbamoyl}-cyclopropyl)-amid
3-Methoxy-isoxazol-5-carbonsäure(1-{4-[3-chlor-2-(1,3-oxazol-5-yl)-indol-1-yl]-benzylcarbamoyl}-cyclopropyl)-amid
1-(2,2,2-Trifluor-acetylamino)-cyclopropane carbonsäure 4-[3,5-dichlor-2-(2-methyl-2H-tetrazol-5-yl)-indol-1-yl]-benzylamid
3-Methoxy-isoxazol-5-carbonsäure (1-{4-[3,5-dichlor-2-(2-methyl-2H-tetrazol-5-yl)-indol-1-yl]-benzylcarbamoyl}-cyclopropyl)-amid
1-(2,2,2-Trifluor-acetylamino)-cyclopropane carbonsäure 4-[3-chlor-5-fluor-2-(2-methyl-2H-tetrazol-5-yl)-indol-1-yl]-2-fluor-benzylamid
3-Methoxy-isoxazol-5-carbonsäure (1-{4-[3-chlor-5fluor-2-(2-methyl-2H-tetrazol-5-yl)-indol-1-yl]-2-fluor-benzylcarbamoyl}-cyclopropyl)-amid
3-Methoxy-isoxazol-5-carbonsäure (1-{4-[3-cyano-2-(5-methyl-[1,2,4]oxadiazol-3-yl)-indol-1-yl]-benzylcarbamoyl}-cyclopropyl)-amid
1-(2,2,2-Trifluor-acetylamino)-cyclopropane carbonsäure 4-[3,5-dichlor-2-(2-methyl-2H-tetrazol-5-yl)-indol-1-yl]-2-fluor-benzylamid
1-(2,2,2-Trifluor-acetylamino)-cyclopropane carbonsäure 4-[3-chlor-2-(5-methyl-[1,2,4]oxadiazol-3-yl)-indol-1-yl]-benzylamid
5-Cyclopropyl-isoxazol-3-carbonsäure (1-{4-[3-chlor-2-(2-methyl-2H-tetrazol-5-yl)-indol-1-yl]-benzylcarbamoyl}-cyclopropyl)-amid
5-Methyl-pyrazine-2-carbonsäure (1-{4-[3-chlor-2-(2-methyl-2H-tetrazol-5-yl)-indol-1-yl]-benzylcarbamoyl}-cyclopropyl)-amid
5-Methyl-isoxazol-3-carbonsäure (1-{4-[3-chlor-2-(2-methyl-2H-tetrazol-5-yl)-indol-1-yl]-benzylcarbamoyl}-cyclopropyl)-amid
2-Chlor-N-(1-{4-[3-chlor-2-(2-methyl-2H-tetrazol-5-yl)-indol-1-yl]-benzylcarbamoyl}-cyclopropyl)-nikotinamid
N-(1-{4-[3-chlor-2-(2-methyl-2H-tetrazol-5-yl)-indol-1-yl]-benzylcarbamoyl}-cyclopropyl)-2-fluor-isonikotinamid
1-(3,3,3,-Trifluor-propionylamino)-cyclopropane carbonsäure 4-[3-chlor-2-(2-methyl-2H-tetrazol-5-yl)-indol-1-yl]-benzylamid
N-(1-{4-[3-chlor-2-(2-methyl-2H-tetrazol-5-yl)-indol-1-yl]-benzylcarbamoyl}-cyclopropyl)-5-trifluormethyl-nikotinamid
N-(1-{4-[3-chlor-2-(2-methyl-2H-tetrazol-5-yl)-indol-1-yl]-benzylcarbamoyl}-cyclopropyl)-3-fluor-benzamid
3-Chlor-1-[4-(1-{[1-(2,2,2-trifluor-acetylamino)-cyclopropanecarbonyl]-amino}-ethyl)-phenyl]-1H-indole-2-carbonsäure methylamid
1-(2,2,2-Trifluor-acetylamino)-cyclopropane carbonsäure (1-{4-[3-chlor-2-(pyrrolidine-1-carbonyl)-indol-1-yl]-phenyl}-ethyl)-amid
3-Chlor-1-{4-[({1-[(3-methoxy-isoxazol-5-carbonyl)-amino]-cyclopropane-carbonyl}-amino)-methyl]-phenyl}-1H-indole-2-carbonsäure ethylester
3-Chlor-1-{4-[({1-[(3-methoxy-isoxazol-5-carbonyl)-amino]-cyclopropane-carbonyl}-amino)-methyl]-phenyl}-1H-indole-2-carbonsäure dimethylamid
1-(2,2,2-Trifluor-acetylamino)-cyclopropane carbonsäure 4-[2-(2-methyl-2H-tetrazol-5-yl)-indol-1-yl]-benzylamid
3-Methoxy-isoxazol-5-carbonsäure (1-{4-[3-chlor-5-fluor-2-(5-methyl-[1,3,4]oxadiazol-2-yl)-indol-1-yl]-benzylcarbamoyl}-cyclopropyl)-amid
3-Methoxy-isoxazol-5-carbonsäure (1-{4-[3-chlor-2-(5-methyl-oxazol-2-yl)-indol-1-yl]-benzylcarbamoyl}-cyclopropyl)-amid
3-Methoxy-isoxazol-5-carbonsäure {1-[4-(3-chlor-2-cyano-indol-1-yl)-benzyl-carbamoyl]-cyclopropyl}-amid
(R)-1-(2,2,2-Trifluor-acetylamino)-cyclopropane carbonsäure (1-{4-[3-chlor-2-(5-methyl-[1,2,4]oxadiazol-3-yl)-indol-1-yl]-phenyl}-ethyl)-amid
(R)-3-Methoxy-isoxazol-5-carbonsäure [1-(1-{4-[3-chlor-2-(5-methyl-[1,2,4]oxadiazol-3-yl)-indol-1-yl]-phenyl}-ethylcarbamoyl)-cyclopropyl]-amid
3-Propyl-isoxazol-5-carbonsäure (1-{4-[3-chlor-2-(5-methyl-[1,2,4]oxadiazol-3-yl)-indol-1-yl]-benzylcarbamoyl}-cyclopropyl)-amid
3-Methoxy-isoxazol-5-carbonsäure {1-[4-(5-chlor-2-cyano-indol-1-yl)-benzylcarbamoyl]-cyclopropyl}-amid
N-{1-[4-(2-Cyano-5-methoxy-indol-1-yl)-benzylcarbamoyl]-cyclopropyl}-5-trifluormethyl-nikotinamid
N-{1-[4-(2-Cyano-5-fluor-indol-1-yl)-benzylcarbamoyl]-cyclopropyl}-5-trifluormethyl-nikotinamid
N-(1-{4-[5-Fluor-2-(5-methyl-[1,2,4]oxadiazol-3-yl)-indol-1-yl]-benzylcarbamoyl}-cyclopropyl)-5-trifluormethyl-nikotinamid
3-Methoxy-isoxazol-5-carbonsäure (1-{4-[5-fluor-2-(5-methyl-[1,2,4]oxadiazol-3-yl)-indol-1-yl]-benzylcarbamoyl}-cyclopropyl)-amid
3-Methoxy-isoxazol-5-carbonsäure {1-[4-(2-cyano-5-methoxy-indol-1-yl)-benzylcarbamoyl]-cyclopropyl}-amid
3-Methoxy-isoxazol-5-carbonsäure {1-[4-(2-cyano-5-fluor-indol-1-yl)-benzylcarbamoyl]-cyclopropyl}-amid
N-(1-{4-[3-Chlor-5-methoxy-2-(3-methyl-[1,2,4]oxadiazol-5-yl)-indol-1-yl]-benzyl-carbamoyl}-cyclopropyl)-5-trifluormethyl-nikotinamid
3-Methoxy-isoxazol-5-carbonsäure (1-{4-[3-chlor-5-methoxy-2-(3-methyl-[1,2,4]oxadiazol-5-yl)-indol-1-yl]-benzylcarbamoyl}-cyclopropyl)-amid
3-Methoxy-isoxazol-5-carbonsäure {1-[4-(3-chlor-2-cyano-5-methoxy-indol-1-yl)-benzylcarbamoyl]-cyclopropyl}-amid
3-Methoxy-isoxazol-5-carbonsäure (1-{4-[5-chlor-2-(5-methyl-[1,2,4]oxadiazol-3-yl)-indol-1-yl]-benzylcarbamoyl}-cyclopropyl)-amid
3-Chlor-1-[3-fluor-4-({[1-(2,2,2-trifluor-acetylamino)-cyclopropanecarbonyl]-amino}-methyl)-phenyl]-1H-indole-2-carbonsäure methylester
3-Chlor-1-{3-fluor-4-[({1-[(3-methoxy-isoxazol-5-carbonyl)-amino]-cyclo-propanecarbonyl}-amino)-methyl]-phenyl}-1H-indole-2-carbonsäure methylester
3-Methoxy-isoxazol-5-carbonsäure [1-({5-[2-(3-methyl-[1,2,4]oxadiazol-5-yl)-indol-1-yl]-pyridin-2-ylmethyl}-carbamoyl)-cyclopropyl]-amid
3-Methoxy-isoxazol-5-carbonsäure [1-({5-[2-(5-methyl-[1,2,4]oxadiazol-3-yl)-indol-1-yl]-pyridin-2-ylmethyl}-carbamoyl)-cyclopropyl]-amid
3-Methoxy-isoxazol-5-carbonsäure [1-({5-[3-chlor-2-(3-methyl-[1,2,4]oxadiazol-5-yl)-indol-1-yl]-pyridin-2-ylmethyl}-carbamoyl)-cyclopropyl]-amid
3-Methoxy-isoxazol-5-carbonsäure [1-({5-[3-chlor-5-fluor-2-(2-methyl-2H-tetrazol-5-yl)-indol-1-yl]-pyridin-2-ylmethyl}-carbamoyl)-cyclopropyl]-amid
3-Methoxy-isoxazol-5-carbonsäure [1-({5-[3-chlor-2-(2-methyl-2H-tetrazol-5-yl)-indol-1-yl]-pyridin-2-ylmethyl}-carbamoyl)-cyclopropyl]-amid
3-Methoxy-isoxazol-5-carbonsäure [1-({5-[5-fluor-2-(5-methyl-[1,2,4]oxadiazol-3-yl)-indol-1-yl]-pyridin-2-ylmethyl}-carbamoyl)-cyclopropyl]-amid
3-Methoxy-isoxazol-5-carbonsäure [1-({5-[3-chlor-5-fluor-2-(5-methyl-[1,2,4]oxadiazol-3-yl)-indol-1-yl]-pyridin-2-ylmethyl}-carbamoyl)-cyclopropyl]-amid
1-(2,2,2-Trifluor-acetylamino)-cyclopropane carbonsäure 4-[3-chlor-5-methoxy-2-(5-methyl-[1,2,4]oxadiazol-3-yl)-indol-1-yl]-benzylamid
3-Methoxy-isoxazol-5-carbonsäure (1-{4-[3-chlor-5-methoxy-2-(5-methyl-[1,2,4]oxadiazol-3-yl)-indol-1-yl]-benzylcarbamoyl}-cyclopropyl)-amid
1-(2,2,2-Trifluor-acetylamino)-cyclopropane carbonsäure 4-[3,5-dichlor-2-(5-methyl-[1,2,4]oxadiazol-3-yl)-indol-1-yl]-benzylamid
3-Methoxy-isoxazol-5-carbonsäure (1-{4-[3,5-dichlor-2-(5-methyl-[1,2,4]oxadiazol-3-yl)-indol-1-yl]-benzylcarbamoyl}-cyclopropyl)-amid
3-Methoxy-isoxazol-5-carbonsäure (1-{4-[3-chlor-5-fluor-2-(5-methyl-[1,2,4]oxadiazol-3-yl)-indol-1-yl]-benzylcarbamoyl}-cyclopropyl)-amid
3-Chlor-4-fluor-1-{4-[({1-[(3-methoxy-isoxazol-5-carbonyl)-amino]-cyclo-propanecarbonyl}-amino)-methyl]-phenyl}-1H-indole-2-carbonsäure methyl ester
3-Chlor-4-fluor-1-[4-({[1-(2,2,2-trifluor-acetylamino)-cyclopropanecarbonyl]-amino}-methyl)-phenyl]-1H-indole-2-carbonsäure methylester
2-Chlor-N-(1-{4-[3,5-dichlor-2-(5-methyl-[1,2,4]oxadiazol-3-yl)-indol-1-yl]-benzylcarbamoyl}-cyclopropyl)-nikotinamid
N-(1-{4-[3,5-Dichlor-2-(5-methyl-[1,2,4]oxadiazol-3-yl)-indol-1-yl]-benzylcarbamoyl}-cyclopropyl)-3-fluor-benzamid
5-Methyl-pyrazine-2-carbonsäure (1-{4-[3,5-dichlor-2-(5-methyl-[1,2,4]oxadiazol-3-yl)-indol-1-yl]-benzylcarbamoyl}-cyclopropyl)-amid
5-Methyl-isoxazol-3-carbonsäure (1-{4-[3,5-dichlor-2-(5-methyl-[1,2,4]oxadiazol-3-yl)-indol-1-yl]-benzylcarbamoyl}-cyclopropyl)-amid
N-(1-{4-[3,5-Dichlor-2-(5-methyl-[1,2,4]oxadiazol-3-yl)-indol-1-yl]-benzylcarbamoyl}-cyclopropyl)-2-fluor-isonikotinamid
5-Cyclopropyl-isoxazol-3-carbonsäure (1-{4-[3,5-dichlor-2-(5-methyl-[1,2,4]oxadiazol-3-yl)-indol-1-yl]-benzylcarbamoyl}-cyclopropyl)-amid
N-(1-{4-[3,5-Dichlor-2-(5-methyl-[1,2,4]oxadiazol-3-yl)-indol-1-yl]-benzylcarbamoyl}-cyclopropyl)-5-trifluormethyl-nikotinamid
2-Methylsulfanyl-pyrimidine-5-carbonsäure (1-{4-[3-chlor-2-(5-methyl-[1,2,4]oxadiazol-3-yl)-indol-1-yl]-benzylcarbamoyl}-cyclopropyl)-amid
1-(3,3,3-Trifluor-propionylamino)-cyclopropane carbonsäure 4-[5-methoxy-2-(3-methyl-[1,2,4]oxadiazol-5-yl)-indol-1-yl]-benzylamid
N-(1-{4-[5-Methoxy-2-(3-methyl-[1,2,4]oxadiazol-5-yl)-indol-1-yl]-benzylcarbamoyl}-cyclopropyl)-5-trifluormethyl-nikotinamid
3-Methoxy-isoxazol-5-carbonsäure (1-{4-[5-methoxy-2-(3-methyl-[1,2,4]oxadiazol-5-yl)-indol-1-yl]-benzylcarbamoyl}-cyclopropyl)-amid
1-(3,3,3-Trifluor-propionylamino)-cyclopropane carbonsäure 4-[2-(5-methyl-[1,2,4]oxadiazol-3-yl)-indol-1-yl]-benzylamid
N-(1-{4-[2-(5-Methyl-[1,2,4]oxadiazol-3-yl)-indol-1-yl]-benzylcarbamoyl}-cyclopropyl)-5-trifluormethyl-nikotinamid
N-(1-{4-[2-(3-Methyl-[1,2,4]oxadiazol-5-yl)-indol-1-yl]-benzylcarbamoyl}-cyclopropyl)-5-trifluormethyl-nikotinamid
N-{1-[4-(2-Cyano-indol-1-yl)-benzylcarbamoyl]-cyclopropyl}-5-trifluormethyl-nikotinamid
2-Chlor-N-(1-{4-[3-chlor-5-fluor-2-(5-methyl-[1,2,4]oxadiazol-3-yl)-indol-1-yl]-benzylcarbamoyl}-cyclopropyl)-nikotinamid
N-(1-{4-[3-Chlor-5-fluor-2-(5-methyl-[1,2,4]oxadiazol-3-yl)-indol-1-yl]-benzylcarbamoyl}-cyclopropyl)-3-fluor-benzamid
5-Methyl-pyrazine-2-carbonsäure (1-{4-[3-chlor-5-fluor-2-(5-methyl-[1,2,4]oxadiazol-3-yl)-indol-1-yl]-benzylcarbamoyl}-cyclopropyl)-amid
5-Methyl-isoxazol-3-carbonsäure (1-{4-[3-chlor-5-fluor-2-(5-methyl-[1,2,4]oxadiazol-3-yl)-indol-1-yl]-benzylcarbamoyl}-cyclopropyl)-amid
N-(1-{4-[3-Chlor-5-fluor-2-(5-methyl-[1,2,4]oxadiazol-3-yl)-indol-1-yl]-benzylcarbamoyl}-cyclopropyl)-2-fluor-isonikotinamid
5-Cyclopropyl-isoxazol-3-carbonsäure (1-{4-[3-chlor-5-fluor-2-(5-methyl-[1,2,4]oxadiazol-3-yl)-indol-1-yl]-benzylcarbamoyl}-cyclopropyl)-amid
N-(1-{4-[3-Chlor-5-fluor-2-(5-methyl-[1,2,4]oxadiazol-3-yl)-indol-1-yl]-benzylcarbamoyl}-cyclopropyl)-5-trifluormethyl-nikotinamid
N-(1-{4-[3-Chlor-2-(5-methyl-[1,2,4]oxadiazol-3-yl)-indol-1-yl]-benzylcarbamoyl}-cyclopropyl)-2-fluor-3-trifluormethyl-benzamid
N-(1-{4-[3-Chlor-2-(5-methyl-[1,2,4]oxadiazol-3-yl)-indol-1-yl]-benzylcarbamoyl}-cyclopropyl)-3-fluor-4-trifluormethyl-benzamid
N-(1-{4-[3-Chlor-2-(5-methyl-[1,2,4]oxadiazol-3-yl)-indol-1-yl]-benzylcarbamoyl}-cyclopropyl)-3-fluor-5-trifluormethyl-benzamid
N-(1-{4-[3-Chlor-2-(5-methyl-[1,2,4]oxadiazol-3-yl)-indol-1-yl]-benzylcarbamoyl}-cyclopropyl)-2-fluor-nikotinamid
N-(1-{4-[3-Chlor-2-(5-methyl-[1,2,4]oxadiazol-3-yl)-indol-1-yl]-benzylcarbamoyl}-cyclopropyl)-2-fluor-5-trifluormethyl-benzamid
N-(1-{4-[3-Chlor-2-(5-methyl-[1,2,4]oxadiazol-3-yl)-indol-1-yl]-benzylcarbamoyl}-cyclopropyl)-3-fluor-isonikotinamid
N-(1-{4-[3-Chlor-2-(5-methyl-[1,2,4]oxadiazol-3-yl)-indol-1-yl]-benzylcarbamoyl}-cyclopropyl)-4-fluor-3-trifluormethyl-benzamid
N-(1-{4-[3-Chlor-2-(5-methyl-[1,2,4]oxadiazol-3-yl)-indol-1-yl]-benzylcarbamoyl}-cyclopropyl)-6-fluor-nikotinamid
N-{1-[4-(5-Chlor-2-cyano-indol-1-yl)-benzylcarbamoyl]-cyclopropyl}-5-trifluormethyl-nikotinamid
1-(3,3,3-Trifluor-propionylamino)-cyclopropane carbonsäure 4-[5-fluor-2-(3-methyl-[1,2,4]oxadiazol-5-yl)-indol-1-yl]-benzylamid
N-(1-{4-[5-Fluor-2-(3-methyl-[1,2,4]oxadiazol-5-yl)-indol-1-yl]-benzylcarbamoyl}-cyclopropyl)-5-trifluormethyl-nikotinamid
3-Methoxy-isoxazol-5-carbonsäure (1-{4-[5-fluor-2-(3-methyl-[1,2,4]oxadiazol-5-yl)-indol-1-yl]-benzylcarbamoyl}-cyclopropyl)-amid
2-Chlor-N-(1-{4-[3-chlor-2-(5-methyl-[1,2,4]oxadiazol-3-yl)-indol-1-yl]-2-fluor-benzylcarbamoyl}-cyclopropyl)-nikotinamid
N-(1-{4-[3-Chlor-2-(5-methyl-[1,2,4]oxadiazol-3-yl)-indol-1-yl]-2-fluor-benzylcarbamoyl}-cyclopropyl)-3-fluor-benzamid
5-Methyl-pyrazine-2-carbonsäure (1-{4-[3-chlor-2-(5-methyl-[1,2,4]oxadiazol-3-yl)-indol-1-yl]-2-fluor-benzylcarbamoyl}-cyclopropyl)-amid
5-Methyl-isoxazol-3-carbonsäure (1-{4-[3-chlor-2-(5-methyl-[1,2,4]oxadiazol-3-yl)-indol-1-yl]-2-fluor-benzylcarbamoyl}-cyclopropyl)-amid
1-(3,3,3-Trifluor-propionylamino)-cyclopropane carbonsäure 4-[3-chlor-2-(5-methyl-[1,2,4]oxadiazol-3-yl)-indol-1-yl]-2-fluor-benzylamid
N-(1-{4-[3-Chlor-2-(5-methyl-[1,2,4]oxadiazol-3-yl)-indol-1-yl]-2-fluor-benzylcarbamoyl}-cyclopropyl)-2-fluor-isonikotinamid
5-Cyclopropyl-isoxazol-3-carbonsäure (1-{4-[3-chlor-2-(5-methyl-[1,2,4]oxadiazol-3-yl)-indol-1-yl]-2-fluor-benzylcarbamoyl}-cyclopropyl)-amid
N-(1-{4-[3-Chlor-2-(5-methyl-[1,2,4]oxadiazol-3-yl)-indol-1-yl]-2-fluor-benzylcarbamoyl}-cyclopropyl)-5-trifluormethyl-nikotinamid
1-(3,3,3-Trifluor-propionylamino)-cyclopropane carbonsäure 4-[3-chlor-2-(3-methyl-[1,2,4]oxadiazol-5-yl)-indol-1-yl]-benzylamid
N-(1-{4-[3-Chlor-2-(3-methyl-[1,2,4]oxadiazol-5-yl)-indol-1-yl]-benzylcarbamoyl}-cyclopropyl)-2-fluor-isonikotinamid
5-Cyclopropyl-isoxazol-3-carbonsäure (1-{4-[3-chlor-2-(3-methyl-[1,2,4]oxadiazol-5-yl)-indol-1-yl]-benzylcarbamoyl}-cyclopropyl)-amid
N-(1-{4-[3-Chlor-2-(3-methyl-[1,2,4]oxadiazol-5-yl)-indol-1-yl]-benzylcarbamoyl}-cyclopropyl)-5-trifluormethyl-nikotinamid
2-Fluor-N-(1-{4-[5-methoxy-2-(3-methyl-[1,2,4]oxadiazol-5-yl)-indol-1-yl]-benzylcarbamoyl}-cyclopropyl)-nikotinamid
N-(1-{4-[3-Chlor-5-fluor-2-(5-methyl-[1,2,4]oxadiazol-3-yl)-indol-1-yl]-benzylcarbamoyl}-cyclopropyl)-2-fluor-nikotinamid
N-(1-{4-[3-Chlor-5-methoxy-2-(3-methyl-[1,2,4]oxadiazol-5-yl)-indol-1-yl]-benzylcarbamoyl}-cyclopropyl)-2-fluor-nikotinamid
N-(1-{4-[3-Chlor-2-(2-methyl-2H-tetrazol-5-yl)-indol-1-yl]-benzylcarbamoyl}-cyclopropyl)-2-fluor-nikotinamid
2-Fluor-N-(1-{4-[5-fluor-2-(5-methyl-[1,2,4]oxadiazol-3-yl)-indol-l-yl]-benzylcarbamoyl}-cyclopropyl)-nikotinamid
1-Methyl-1H-pyrazole-4-carbonsäure (1-{4-[3-chlor-2-(5-methyl-[1,2,4]oxadiazol-3-yl)-indol-1-yl]-benzylcarbamoyl}-cyclopropyl)-amid
2-Oxo-imidazolidine-4-carbonsäure (1-{4-[3-chlor-2-(5-methyl-[1,2,4]oxadiazol-3-yl)-indol-1-yl]-benzylcarbamoyl}-cyclopropyl)-amid
3-Ethyl-isoxazol-5-carbonsäure (1-{4-[3-chlor-2-(5-methyl-[1,2,4]oxadiazol-3-yl)-indol-1-yl]-benzylcarbamoyl}-cyclopropyl)-amid
1-(3,3,3-Trifluor-propionylamino)-cyclopropane carbonsäure 4-[5-methoxy-2-(5-methyl-[1,3,4]oxadiazol-2-yl)-indol-1-yl]-benzylamid
N-(1-{4-[5-Methoxy-2-(5-methyl-[1,3,4]oxadiazol-2-yl)-indol-1-yl]-benzylcarbamoyl}-cyclopropyl)-5-trifluormethyl-nikotinamid
3-Methoxy-isoxazol-5-carbonsäure (1-{4-[5-methoxy-2-(5-methyl-[1,3,4]oxadiazol-2-yl)-indol-1-yl]-benzylcarbamoyl}-cyclopropyl)-amid
1-(3,3,3-Trifluor-propionylamino)-cyclopropane carbonsäure 4-[5-chlor-2-(5-methyl-[1,2,4]oxadiazol-3-yl)-indol-1-yl]-benzylamid
N-(1-{4-[5-Chlor-2-(5-methyl-[1,2,4]oxadiazol-3-yl)-indol-1-yl]-benzylcarbamoyl}-cyclopropyl)-5-trifluormethyl-nikotinamid
5-Methyl-pyrazine-2-carbonsäure (1-{4-[3-chlor-5-methoxy-2-(5-methyl-[1,2,4]oxadiazol-3-yl)-indol-1-yl]-benzylcarbamoyl}-cyclopropyl)-amid
N-(1-{4-[3-Chlor-5-methoxy-2-(5-methyl-[1,2,4]oxadiazol-3-yl)-indol-1-yl]-benzylcarbamoyl}-cyclopropyl)-5-trifluormethyl-nikotinamid
5-Methyl-pyrazine-2-carbonsäure (1-{2-fluor-4-[5-fluor-2-(5-methyl-[1,2,4]oxadiazol-3-yl)-indol-1-yl]-benzylcarbamoyl}-cyclopropyl)-amid
N-(1-{2-Fluor-4-[5-fluor-2-(5-methyl-[1,2,4]oxadiazol-3-yl)-indol-1-yl]-benzylcarbamoyl}-cyclopropyl)-5-trifluormethyl-nikotinamid
3-Methoxy-isoxazol-5-carbonsäure (1-{2-fluor-4-[5-fluor-2-(5-methyl-[1,2,4]oxadiazol-3-yl)-indol-1-yl]-benzylcarbamoyl}-cyclopropyl)-amid
Isoxazol-5-carbonsäure (1-{4-[3-chlor-2-(5-methyl-[1,2,4]oxadiazol-3-yl)-indol-1-yl]-benzylcarbamoyl}-cyclopropyl)-amid
5-Methyl-isoxazol-3-carbonsäure (1-{4-[3-chlor-2-(5-methyl-[1,2,4]oxadiazol-3-yl)-indol-1-yl]-benzylcarbamoyl}-cyclopropyl)-amid
Tetrahydro-furan-2-carbonsäure (1-{4-[3-chlor-2-(5-methyl-[1,2,4]oxadiazol-3-yl)-indol-1-yl]-benzylcarbamoyl}-cyclopropyl)-amid
Furan-3-carbonsäure (1-{4-[3-chlor-2-(5-methyl-[1,2,4]oxadiazol-3-yl)-indol-1-yl]-benzylcarbamoyl}-cyclopropyl)-amid
2-Methyl-cyclopropanecarbonsäure (1-{4-[3-chlor-2-(5-methyl-[1,2,4]oxadiazol-3-yl)-indol-1-yl]-benzylcarbamoyl}-cyclopropyl)-amid
1-Pent-4-ynoylamino-cyclopropanecarbonsäure 4-[3-chlor-2-(5-methyl-[1,2,4]oxadiazol-3-yl)-indol-1-yl]-benzylamid 1-(4-Methyl-pentanoylamino)-cyclopropane carbonsäure 4-[3-chlor-2-(5-methyl-[1,2,4]oxadiazol-3-yl)-indol-1-yl]-benzylamid
1H-Pyrazole-4-carbonsäure (1-{4-[3-chlor-2-(5-methyl-[1,2,4]oxadiazol-3-yl)-indol-1-yl]-benzylcarbamoyl}-cyclopropyl)-amid
1-Cyano-cyclopropanecarbonsäure (1-{4-[3-chlor-2-(5-methyl-[1,2,4]oxadiazol-3-yl)-indol-1-yl]-benzylcarbamoyl}-cyclopropyl)-amid
Cyclobutane carbonsäure (1-{4-[3-chlor-2-(5-methyl-[1,2,4]oxadiazol-3-yl)-indol-1-yl]-benzylcarbamoyl}-cyclopropyl)-amid
Cyclopentane carbonsäure (1-{4-[3-chlor-2-(5-methyl-[1,2,4]oxadiazol-3-yl)-indol-1-yl]-benzylcarbamoyl}-cyclopropyl)-amid
Furan-2-carbonsäure (1-{4-[3-chlor-2-(5-methyl-[1,2,4]oxadiazol-3-yl)-indol-1-yl]-benzylcarbamoyl}-cyclopropyl)-amid
N-(1-{4-[3-Chlor-2-(5-methyl-[1,2,4]oxadiazol-3-yl)-indol-1-yl]-benzylcarbamoyl}-cyclopropyl)-3-cyano-benzamid 1-(2-Thiophen-3-yl-acetylamino)-cyclopropane carbonsäure 4-[3-chlor-2-(5-methyl-[1,2,4]oxadiazol-3-yl)-indol-1-yl]-benzylamid
1,5-Dimethyl-1H-pyrazole-3-carbonsäure (1-{4-[3-chlor-2-(5-methyl-[1,2,4]oxadiazol-3-yl)-indol-1-yl]-benzylcarbamoyl}-cyclopropyl)-amid
4-Methyl-thiazole-5-carbonsäure (1-{4-[3-chlor-2-(5-methyl-[1,2,4]oxadiazol-3-yl)-indol-1-yl]-benzylcarbamoyl}-cyclopropyl)-amid
2,4-Dimethyl-thiazole-5-carbonsäure (1-{4-[3-chlor-2-(5-methyl-[1,2,4]oxadiazol-3-yl)-indol-1-yl]-benzylcarbamoyl}-cyclopropyl)-amid
1-(2-Acetylamino-3-hydroxy-propionylamino)-cyclopropanecarbonsäure 4-[3-chlor-2-(5-methyl-[1,2,4]oxadiazol-3-yl)-indol-1-yl]-benzylamid
5-Methyl-1H-pyrazole-3-carbonsäure (1-{4-[3-chlor-2-(5-methyl-[1,2,4]oxadiazol-3-yl)-indol-1-yl]-benzylcarbamoyl}-cyclopropyl)-amid
2-Chlor-N-(1-{4-[3-chlor-2-(5-methyl-[1,2,4]oxadiazol-3-yl)-indol-1-yl]-benzylcarbamoyl}-cyclopropyl)-nikotinamid
N-(1-{4-[3-Chlor-2-(5-methyl-[1,2,4]oxadiazol-3-yl)-indol-1-yl]-benzylcarbamoyl}-cyclopropyl)-6-methyl-nikotinamid
N-(1-{4-[3-Chlor-2-(5-methyl-[1,2,4]oxadiazol-3-yl)-indol-1-yl]-benzylcarbamoyl}-cyclopropyl)-2-methyl-nikotinamid
2-Chlor-N-(1-{4-[3-chlor-2-(5-methyl-[1,2,4]oxadiazol-3-yl)-indol-1-yl]-benzylcarbamoyl}-cyclopropyl)-6-methyl-nikotinamid
6-Bromo-pyridine-2-carbonsäure (1-{4-[3-chlor-2-(5-methyl-[1,2,4]oxadiazol-3-yl)-indol-1-yl]-benzylcarbamoyl}-cyclopropyl)-amid
N-(1-{4-[3-Chlor-2-(5-methyl-[1,2,4]oxadiazol-3-yl)-indol-1-yl]-benzylcarbamoyl}-cyclopropyl)-nikotinamid
Quinoline-3-carbonsäure (1-{4-[3-chlor-2-(5-methyl-[1,2,4]oxadiazol-3-yl)-indol-1-yl]-benzylcarbamoyl}-cyclopropyl)-amid
6-Oxo-1,6-dihydro-pyridine-3-carbonsäure (1-{4-[3-chlor-2-(5-methyl-[1,2,4]oxadiazol-3-yl)-indol-1-yl]-benzylcarbamoyl}-cyclopropyl)-amid
5-Methyl-pyrazine-2-carbonsäure (1-{4-[3-chlor-2-(5-methyl-[1,2,4]oxadiazol-3-yl)-indol-1-yl]-benzylcarbamoyl}-cyclopropyl)-amid
6-Oxo-1,6-dihydro-pyridine-2-carbonsäure (1-{4-[3-chlor-2-(5-methyl-[1,2,4]oxadiazol-3-yl)-indol-1-yl]-benzylcarbamoyl}-cyclopropyl)-amid
1-(3,3,3-Trifluor-propionylamino)-cyclopropanecarbonsäure 4-[3-chlor-2-(5-methyl-[1,2,4]oxadiazol-3-yl)-indol-1-yl]-benzylamid
N-(1-{4-[3-Chlor-2-(5-methyl-[1,2,4]oxadiazol-3-yl)-indol-1-yl]-benzylcarbamoyl}-cyclopropyl)-2-fluor-isonikotinamid
5-Cyclopropyl-isoxazol-3-carbonsäure (1-{4-[3-chlor-2-(5-methyl-[1,2,4]oxadiazol-3-yl)-indol-1-yl]-benzylcarbamoyl}-cyclopropyl)-amid
Pyrimidine-5-carbonsäure (1-{4-[3-chlor-2-(5-methyl-[1,2,4]oxadiazol-3-yl)-indol-1-yl]-benzylcarbamoyl}-cyclopropyl)-amid
Thiophene-3-carbonsäure (1-{4-[3-chlor-2-(5-methyl-[1,2,4]oxadiazol-3-yl)-indol-1-yl]-benzylcarbamoyl}-cyclopropyl)-amid
5-Oxo-pyrrolidine-2-carbonsäure (1-{4-[3-chlor-2-(5-methyl-[1,2,4]oxadiazol-3-yl)-indol-1-yl]-benzylcarbamoyl}-cyclopropyl)-amid
1-(3-Furan-3-yl-acryloylamino)-cyclopropane carbonsäure 4-[3-chlor-2-(5-methyl-[1,2,4]oxadiazol-3-yl)-indol-1-yl]-benzylamid
N-(1-{4-[3-Chlor-2-(5-methyl-[1,2,4]oxadiazol-3-yl)-indol-1-yl]-benzylcarbamoyl}-cyclopropyl)-4-cyano-benzamid
1-(3-Furan-2-yl-acryloylamino)-cyclopropane carbonsäure 4-[3-chlor-2-(5-methyl-[1,2,4]oxadiazol-3-yl)-indol-1-yl]-benzylamid
Thiophene-2-carbonsäure (1-{4-[3-chlor-2-(5-methyl-[1,2,4]oxadiazol-3-yl)-indol-1-yl]-benzylcarbamoyl}-cyclopropyl)-amid
5-Methylsulfanyl-thiophene-2-carbonsäure (1-{4-[3-chlor-2-(5-methyl-[1,2,4]oxadiazol-3-yl)-indol-1-yl]-benzylcarbamoyl}-cyclopropyl)-amid
[1,2,3]Thiadiazole-4-carbonsäure (1-{4-[3-chlor-2-(5-methyl-[1,2,4]oxadiazol-3-yl)-indol-1-yl]-benzylcarbamoyl}-cyclopropyl)-amid
N-(1-{4-[3-Chlor-2-(5-methyl-[1,2,4]oxadiazol-3-yl)-indol-1-yl]-benzylcarbamoyl}-cyclopropyl)-3-methoxy-benzamid
N-(1-{4-[3-Chlor-2-(5-methyl-[1,2,4]oxadiazol-3-yl)-indol-1-yl]-benzylcarbamoyl}-cyclopropyl)-3-iodo-benzamid
N-(1-{4-[3-Chlor-2-(5-methyl-[1,2,4]oxadiazol-3-yl)-indol-1-yl]-benzylcarbamoyl}-cyclopropyl)-3-trifluormethyl-benzamid
N-(1-{4-[3-Chlor-2-(5-methyl-[1,2,4]oxadiazol-3-yl)-indol-1-yl]-benzylcarbamoyl}-cyclopropyl)-3-fluor-benzamid
N-(1-{4-[3-Chlor-2-(5-methyl-[1,2,4]oxadiazol-3-yl)-indol-1-yl]-benzylcarbamoyl}-cyclopropyl)-3-methyl-benzamid
N-(1-{4-[3-Chlor-2-(5-methyl-[1,2,4]oxadiazol-3-yl)-indol-1-yl]-benzylcarbamoyl}-cyclopropyl)-5-trifluormethyl-nikotinamid
3-Methyl-isoxazol-4-carbonsäure (1-{4-[3-chlor-2-(5-methyl-[1,2,4]oxadiazol-3-yl)-indol-1-yl]-benzylcarbamoyl}-cyclopropyl)-amid
Quinoline-3-carbonsäure (1-{4-[3-chlor-2-(5-methyl-[1,3,4]oxadiazol-2-yl)-indol-1-yl]-benzylcarbamoyl}-cyclopropyl)-amid
1-(3,3,3-Trifluor-propionylamino)-cyclopropane carbonsäure 4-[3-chlor-2-(5-methyl-[1,3,4]oxadiazol-2-yl)-indol-1-yl]-benzylamid
Pyrimidine-5-carbonsäure (1-{4-[3-chlor-2-(5-methyl-[1,3,4]oxadiazol-2-yl)-indol-1-yl]-benzylcarbamoyl}-cyclopropyl)-amid
2-Methyl-pyrimidine-5-carbonsäure (1-{4-[3-chlor-2-(5-methyl-[1,2,4]oxadiazol-3-yl)-indol-1-yl]-benzylcarbamoyl}-cyclopropyl)-amid
3-Amino-N-(1-{4-[3-chlor-2-(5-methyl-[1,2,4]oxadiazol-3-yl)-indol-1-yl]-benzylcarbamoyl}-cyclopropyl)-benzamid Hydrochlorid
3-Chlor-1-[4-({[1-(2,2,2-trifluor-acetylamino)-cyclopropanecarbonyl]-amino}-methyl)-phenyl]-1H-indole-2-carbonsäure-amid
3-Chlor-1-{4-[({1-[(3-methoxy-isoxazol-5-carbonyl)-amino]-cyclopropane-carbonyl}-amino)-methyl]-phenyl}-1H-indole-2-carbonsäure-amid
1-(2,2,2-Trifluor-acetylamino)-cyclopropane carbonsäure 4-[3-chlor-5-fluor-2-(5-methyl-[1,2,4]oxadiazol-3-yl)-indol-1-yl]-benzylamid
1-(2,2,2-Trifluor-acetylamino)-cyclopropane carbonsäure ((R)-1-{4-[3-chlor-2-(5-methyl-[1,2,4]oxadiazol-3-yl)-indol-1-yl]-2-fluor-phenyl}-ethyl)-amid
3-Methoxy-isoxazol-5-carbonsäure [1-((R)-1-{4-[3-chlor-2-(5-methyl-[1,2,4]oxadiazol-3-yl)-indol-1-yl]-2-fluor-phenyl}-ethylcarbamoyl)-cyclopropyl]-amid
3-Methoxy-isoxazol-5-carbonsäure (1-{4-[3-chlor-2-(5-methyl-[1,2,4]oxadiazol-3-yl)-indol-1-yl]-2-fluor-benzylcarbamoyl}-cyclopropyl)-amid
3-Methoxy-isoxazol-5-carbonsäure (1-{4-[3-chlor-2-(3-methyl-[1,2,4]oxadiazol-5-yl)-indol-1-yl]-benzylcarbamoyl}-cyclopropyl)-amid
1-(2,2,2-Trifluor-acetylamino)-cyclopropane carbonsäure 4-[3-chlor-2-(3-methyl-[1,2,4]oxadiazol-5-yl)-indol-1-yl]-benzylamid
3-Methoxy-isoxazol-5-carbonsäure (1-{4-[2-(5-methyl-[1,2,4]oxadiazol-3-yl)-indol-1-yl]-benzylcarbamoyl}-cyclopropyl)-amid
3-Methoxy-isoxazol-5-carbonsäure (1-{4-[2-(3-methyl-[1,2,4]oxadiazol-5-yl)-indol-1-yl]-benzylcarbamoyl}-cyclopropyl)-amid
3-Methoxy-isoxazol-5-carbonsäure (1-{4-[3-chlor-5-fluor-2-(5-methyl-[1,2,4]oxadiazol-3-yl)-indol-1-yl]-2-fluor-benzylcarbamoyl}-cyclopropyl)-amid
3-Methoxy-isoxazol-5-carbonsäure (1-{4-[3,5-difluor-2-(5-methyl-[1,2,4]oxadiazol-3-yl)-indol-1-yl]-benzylcarbamoyl}-cyclopropyl)-amid
1-(2,2,2-Trifluor-acetylamino)-cyclopropane carbonsäure 4-[3,5-difluor-2-(5-methyl-[1,2,4]oxadiazol-3-yl)-indol-1-yl]-benzylamid
3-Methoxy-isoxazol-5-carbonsäure (1-{4-[5-fluor-3-methyl-2-(5-methyl-[1,2,4]oxadiazol-3-yl)-indol-1-yl]-benzylcarbamoyl}-cyclopropyl)-amid und
3-Methoxy-isoxazol-5-carbonsäure (1-{4-[3-chlor-2-(4-methyl-5-oxo-4,5-dihydro-[1,2,4]oxadiazol-3-yl)-indol-1-yl]-benzylcarbamoyl}-cyclopropyl)-amid und optische Antipoden oder Racemate und/oder Salze davon.

3. Verfahren zur Herstellung von Verbindungen der Formel (I) gemäß Anspruch 1 **dadurch gekennzeichnet, daß**
a) Umsetzen eines Indol Derivats der Formel (II) 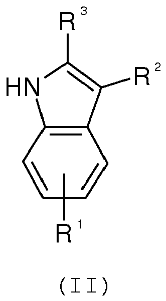
- worin die Bedeutung von R¹, R² und R³ wie oben für die Formel (I) in Anspruch 1 beschrieben sind - mit einem a Boronsäure der Formel (III): 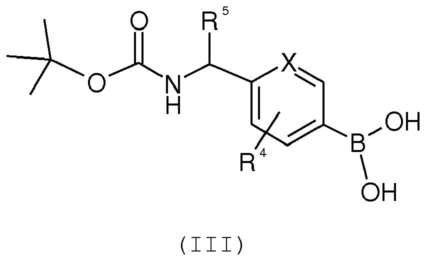
- worin die Bedeutung von R⁴ und R⁵ wie oben für die Formel (I) in Anspruch 1 beschrieben sind - dann die so erhaltenen Indol Derivate der Formel (IV) 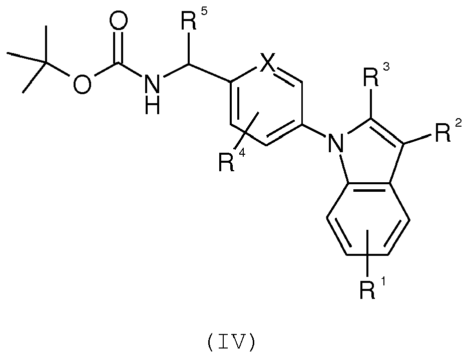
- worin die Bedeutung von R¹, R², R³, R⁴ und R⁵ wie oben für die Formel (I) in Anspruch 1 beschrieben sind - entschützt wird um ein Amin Derivat der Formel (V) zu ergeben: 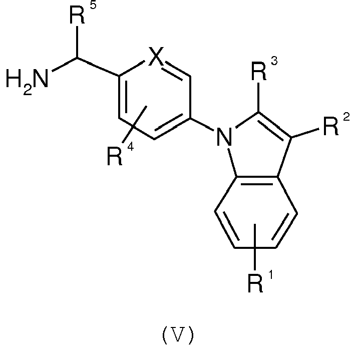
- worin die Bedeutung von R¹, R², R³, R⁴ und R⁵ wie oben für die Formel (I) in Anspruch 1 beschrieben sind - schließlich diese letztere wird mit einem Säurederivat der Formel (VI) umsetzt 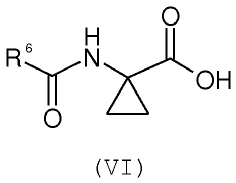
- worin die Bedeutung von R⁶ wie oben für die Formel (I) in Anspruch 1 beschrieben ist; oder
b) Umsetzen eines Indol Derivats der Formel (II) 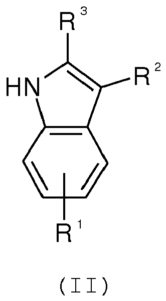
- worin die Bedeutung von R¹, R² and R³ wie oben für die Formel (I) in Anspruch 1 beschrieben sind - mit einem a Boronsäure der Formel (VII) 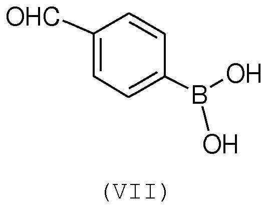 dann die so erhaltenen Indol Derivat der Formel (VIII) 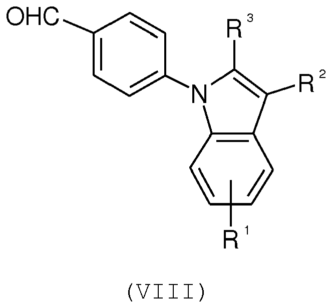
- worin die Bedeutung von R¹, R² und R³ wie oben für die Formel (I) in Anspruch 1 beschrieben sind - wird mit hydroxylamine hydrochlorid umsetzt um Indol Derivat der Formel (IX) zu ergeben 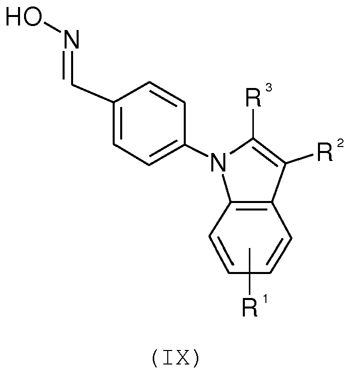
- worin die Bedeutung von R¹, R² und R³ wie oben für die Formel (I) in Anspruch 1 beschrieben sind - diese letztere wird hydriert um ein Amin-Derivat der Formel (X) zu liefern: 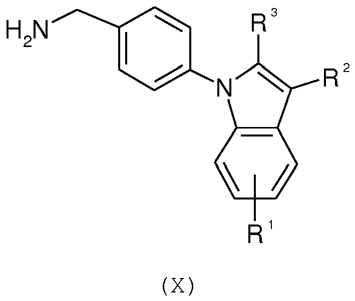
- worin die Bedeutung von R¹, R² und R³ wie oben für die Formel (I) in Anspruch 1 beschrieben sind - schließlich diese letztere wird mit einem Säurederivat der Formel (VI) umsetzt: 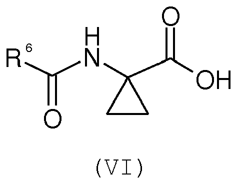
- worin die Bedeutung von R⁶ wie oben für die Formel (I) in Anspruch 1 beschrieben ist; oder
c) Umsetzen eines Amin-Derivats der Formel (V) oder Formel (X) - erhaltenen nach der Verfahren das in dem Verfahren a) und in dem Verfahren b) ausgeführt ist - mit Aminsäure Derivat der Formel (XI) 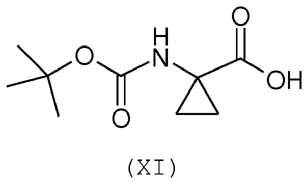 und das so erhaltenen Indol Derivate der Formel (XII) 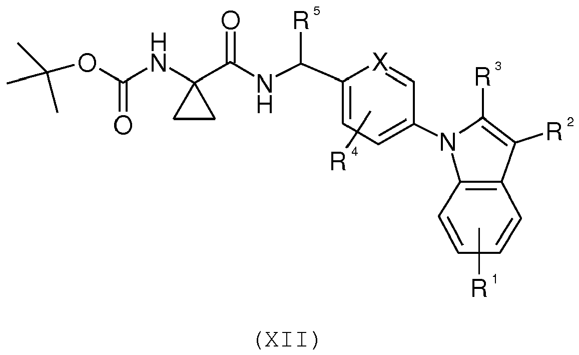
- worin die Bedeutung von R¹, R², R³, R⁴ und R⁵ wie oben für die Formel (I) in Anspruch 1 beschrieben sind -wird entschützt um ein Aminderivat der Formel (XIII) zu ergeben: 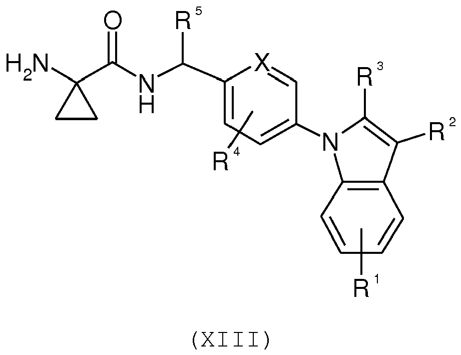
- worin die Bedeutung von R¹, R², R³, R⁴ und R⁵ wie oben für die Formel (I) in Anspruch 1 beschrieben sind - schließlich diese letztere wird umsetzt mit einem Säurederivat der Formel (XIV): 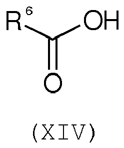
- worin die Bedeutung von R⁶ wie oben für die Formel (I) in Anspruch 1 beschrieben ist; und gegebenenfalls das Indol Derivat der Formel (I) das in Verfahren a) oder b) oder c) erhalten wird und optische Antipode oder Racemate und/oder Salze davon kann in eine andere Verbindung der Formel (I) und optische Antipode oder Racemate und/oder Salze davon umgewandelt werden durch Einführen neuer Substituenten und/oder Modifizieren oder Entfernen der existierenden.

4. Pharmazeutische Zusammensetzung, umfassend eine therapeutische wirksame Menge einer Verbindung der Formel (I) gemäß Anspruch 1 oder optische Antipoden oder Racemate und/oder Salze davon als Wirkstoffe und pharmazeutisch annehmbare Hilfsstoffe.

5. Verfahren zur Herstellung der Pharmazeutischen Zusammensetzung mit selektivem Bradykinin-B1-Rezeptor-Antagonisten-Effekt **dadurch gekennzeichnet, dass** die therapeutische wirksame Menge einer Verbindung der Formel (I) gemäß Anspruch 1 und optische Antipoden oder Racemate und/oder Salze davon als Wirkstoffe und pharmazeutisch annehmbare Hilfsstoffe gemischt werden.

6. Verbindung der Formel (I) gemäß Anspruch 1 und optische Antipoden oder Racemate und/oder Salze davon für Verwendung als selektiver Bradykinin-B1-Rezeptor-Antagonist.

7. Verbindung der Formel (I) gemäß Anspruch 1 und optische Antipoden oder Racemate und/oder Salze davon für Verwendung bei der Behandlung oder Prävention von Störungen assoziierten mit Bradykinin-B1-Rezeptor Aktivität.

8. Verbindung gemäß Anspruch 7 wobei die Störung ausgewählt ist aus der Gruppe von Schmerzen und Entzündungen, einschließlich somatischer Muskel-Skelett-Schmerzen, Rückenschmerzen, sich wiederholende Bewegung Störungen, myofasziale Schmerzsyndrom, Arthritis - einschließlich Arthrose, rheumatoider Arthritis und Gicht - Fibromyalgie, Eingeweideschmerzen, entzündliche Hauterkrankungen und Hautverletzungen, Komplikationen im Zusammenhang mit Diabetes, wie Neuropathie, Nephropathie, Vaskulopathie, Retinopathie, Tumorschmerzen und entzündlicher Darmerkrankung.

9. Verwendung von Verbindung der Formel (I) gemäß Anspruch 1 und optische Antipoden oder Racemate und/oder Salze oder Hydrats oder Solvats davon für die Herstellung eines Medikaments zur Prävention und / oder Behandlung eines Zustands, der die Inhibierung eines Bradykinin Rezeptors erfordert.

10. Verwendung gemäß Anspruch 9, wobei der Bradykinin-Receptor ein Bradykinin B1 Rezeptor ist.

## Revendications

1. Dérivés d'indole de formule (I) 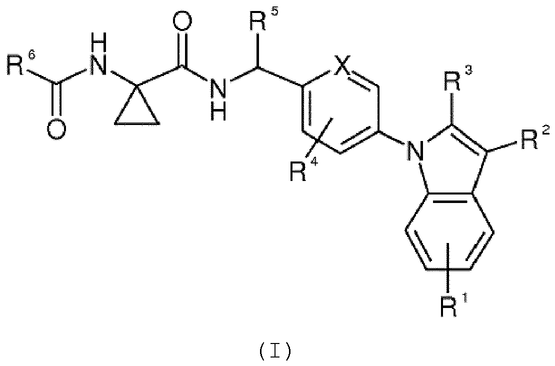 dans laquelle
R¹ est un atome d'hydrogène, un atome d'halogène ou un groupe alcoxy en C₁ à C₄ ;
R² est un atome d'hydrogène, un atome d'halogène, un groupe alkyle en C₁ à C₄ ou un groupe cyano ;
R³ est choisi parmi (1) -COOR ; (2) -CN ; (3) -CONR^{a}R^{b} ; 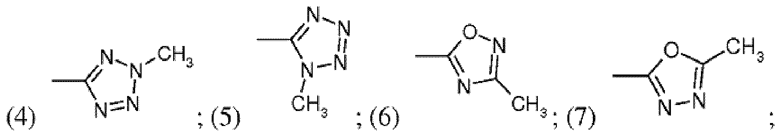 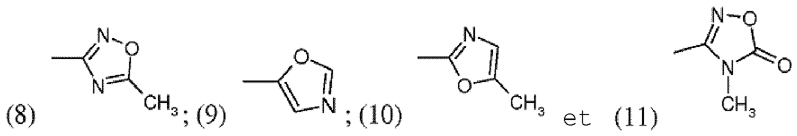
R⁴ est un atome d'hydrogène ou un atome d'halogène ;
R⁵ est un atome d'hydrogène ou un groupe alkyle en C₁ à C₄ ;
R⁶ est choisi parmi
(1) un cycloalkyle en C₃ à C₆ ;
(2) un cycle aromatique à cinq ou six chaînons éventuellement substitué, portant deux atomes d'azote ;
(3) un pyridyle éventuellement substitué ;
(4) un cycle aromatique à cinq chaînons éventuellement substitué, portant un hétéroatome choisi parmi O et S ;
(5) - CF₃ ; (6) -CH₂-CF₃ ; (7) -CH₂-CH₂-C≡CH ; (8) -CH₂-CH₂-CH(CH₃)₂ ; 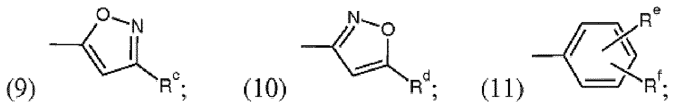 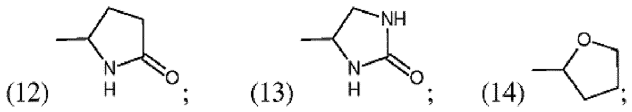 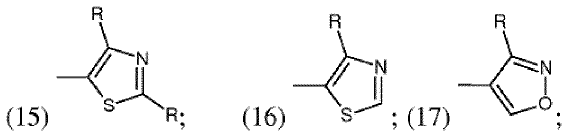 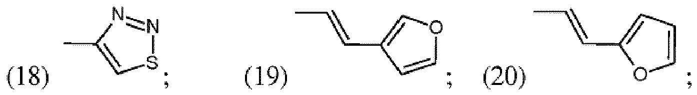 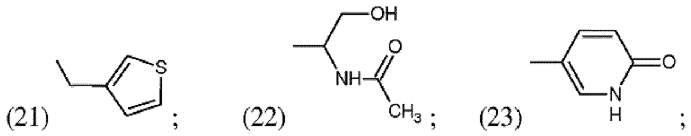 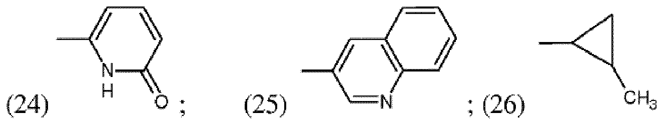 et 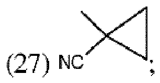
X est -CH- ou un atome d'azote ;
R est un groupe alkyle en C₁ à C₄ ;
R^{a} et R^{b}, indépendamment l'un de l'autre, sont un atome d'hydrogène ; un groupe alkyle en C₁ à C₄ ou un groupe pyrrolidin-1-yle ;
R^{c} est un atome d'hydrogène ; ou un groupe alkyle en C₁ à C₄ ou alcoxy en C₁ à C₄ ;
R^{d} est un atome d'hydrogène ; ou un groupe alkyle en C₁ à C₄ ou cycloalkyle en C₃ à C₆ ;
R^{e} et R^{f}, indépendamment l'un de l'autre, sont un atome d'hydrogène ; un atome d'halogène ; ou un groupe alcoxy en C₁ à C₄ ; -CF₃ ; -CN ou -NH₂ ;
et leurs antipodes optiques ou racémates et/ou sels.

2. Composé selon la revendication 1, choisi dans l'ensemble constitué par les suivants :
4-[3-chloro-2-(2-méthyl-2H-tétrazol-5-yl)indol-1-yl]benzylamide d'acide 1-(2,2,2-trifluoroacétylamino)cyclopropanecarboxylique
(1-{4-[3-chloro-2-(2-méthyl-2H-tétrazol-5-yl)indol-1-yl]benzylcarbamoyl}cyclopropyl)amide d'acide 3-méthoxyisoxazole-5-carboxylique
(1-{4-[2-(2-méthyl-2H-tétrazol-5-yl)indol-1-yl]benzylcarbamoyl}cyclopropyl)amide d'acide 3-méthoxyisoxazole-5-carboxylique
4-[3-chloro-2-(2-méthyl-2H-tétrazol-5-yl)indol-1-yl]-2-fluorobenzylamide d'acide 1-(2,2,2-trifluoroacétylamino)cyclopropanecarboxylique
3-méthoxy-N-[1-({4-[3-chloro-2-(5-méthyl-1,3,4-oxadiazol-2-yl)-1H-indol-1-yl]benzyl}carbamoyl)cyclopropyl]isoxazole-5-carboxamide
4-[3-chloro-2-(5-méthyl-[1,3,4]oxadiazol-2-yl)indol-1-yl]benzylamide d'acide 1-(2,2,2-trifluoroacétylamino)cyclopropanecarboxylique
(1-{4-[3-chloro-2-(1-méthyl-1H-tétrazol-5-yl)indol-1-yl]benzylcarbamoyl}cyclopropyl)amide d'acide 3-méthoxyisoxazole-5-carboxylique
4-[3-chloro-2-(1-méthyl-1H-tétrazol-5-yl)indol-1-yl]benzylamide d'acide 1-(2,2,2-trifluoroacétylamino)cyclopropanecarboxylique
(1-{4-[3-chloro-2-(5-méthyl-[1,2,4]oxadiazol-3-yl)indol-1-yl]benzylcarbamoyl}cyclopropyl)amide d'acide 3-méthoxyisoxazole-5-carboxylique
ester méthylique d'acide 3-chloro-1-[4-({[1-(2,2,2-trifluoroacétylamino)cyclopropanecarbonyl]amino}méthyl)-phényl]-1H-indole-2-carboxylique
ester méthylique d'acide 3-chloro-1-{4-[({1-[(3-méthoxyisoxazole-5-carbonyl)amino]cyclopropanecarbonyl}amino)méthyl]phényl}-1H-indole-2-carboxylique
4-[3-chloro-5-fluoro-2-(2-méthyl-2H-tétrazol-5-yl)indol-1-yl]benzylamide d'acide 1-(2,2,2-trifluoroacétylamino)cyclopropanecarboxylique
(1-{4-[3-chloro-5-fluoro-2-(2-méthyl-2H-tétrazol-5-yl)indol-1-yl]benzylcarbamoyl}cyclopropyl)amide d'acide 3-méthoxyisoxazole-5-carboxylique
ester méthylique d'acide 1-{4-[({1-[(3-méthoxyisoxazole-5-carbonyl)amino]cyclopropanecarbonyl}amino)méthyl]phényl}-1H-indole-2-carboxylique
(1-{4-[3-chloro-2-(2-méthyl-2H-tétrazol-5-yl)indol-1-yl]-2-fluorobenzylcarbamoyl}cyclopropyl)amide d'acide 3-méthoxyisoxazole-5-carboxylique
(1-{4-[3-chloro-2-(1,3-oxazol-5-yl)indol-1-yl]benzylcarbamoyl}cyclopropyl)amide d'acide 3-méthoxyisoxazole-5-carboxylique
4-[3,5-dichloro-2-(2-méthyl-2H-tétrazol-5-yl)indol-1-yl]benzylamide d'acide 1-(2,2,2-trifluoroacétylamino)cyclopropanecarboxylique
(1-{4-[3,5-dichloro-2-(2-méthyl-2H-tétrazol-5-yl)indol-1-yl]benzylcarbamoyl}cyclopropyl)amide d'acide 3-méthoxyisoxazole-5-carboxylique
4-[3-chloro-5-fluoro-2-(2-méthyl-2H-tétrazol-5-yl)indol-1-yl]-2-fluorobenzylamide d'acide 1-(2,2,2-trifluoroacétylamino)cyclopropanecarboxylique
(1-{4-[3-chloro-5-fluoro-2-(2-méthyl-2H-tétrazol-5-yl)indol-1-yl]-2-fluorobenzylcarbamoyl}cyclopropyl)amide d'acide 3-méthoxyisoxazole-5-carboxylique
(1-{4-[3-cyano-2-(5-méthyl-[1,2,4]oxadiazol-3-yl)indol-1-yl]benzylcarbamoyl}cyclopropyl)amide d'acide 3-méthoxyisoxazole-5-carboxylique
4-[3,5-dichloro-2-(2-méthyl-2H-tétrazol-5-yl)indol-1-yl]-2-fluorobenzylamide d'acide 1-(2,2,2-trifluoroacétylamino)cyclopropanecarboxylique
4-[3-chloro-2-(5-méthyl-[1,2,4]oxadiazol-3-yl)indol-1-yl]benzylamide d'acide 1-(2,2,2-trifluoroacétylamino)cyclopropanecarboxylique
(1-{4-[3-chloro-2-(2-méthyl-2H-tétrazol-5-yl)indol-1-yl]benzylcarbamoyl}cyclopropyl)amide d'acide 5-cyclopropylisoxazole-3-carboxylique
(1-{4-[3-chloro-2-(2-méthyl-2H-tétrazol-5-yl)indol-1-yl]benzylcarbamoyl}cyclopropyl)amide d'acide 5-méthyl-pyrazine-2-carboxylique
(1-{4-[3-chloro-2-(2-méthyl-2H-tétrazol-5-yl)indol-1-yl]benzylcarbamoyl}cyclopropyl)amide d'acide 5-méthylisoxazole-3-carboxylique
2-chloro-N-(1-{4-[3-chloro-2-(2-méthyl-2H-tétrazol-5-yl)indol-1-yl]benzylcarbamoyl}cyclopropyl)nicotinamide
N-(1-{4-[3-chloro-2-(2-méthyl-2H-tétrazol-5-yl)indol-1-yl]benzylcarbamoyl}cyclopropyl)-2-fluoroisonicotinamide
4-[3-chloro-2-(2-méthyl-2H-tétrazol-5-yl)indol-1-yl]benzylamide d'acide 1-(3,3,3-trifluoropropionylamino)cyclopropanecarboxylique
N-(1-{4-[3-chloro-2-(2-méthyl-2H-tétrazol-5-yl)indol-1-yl]benzylcarbamoyl}cyclopropyl)-5-trifluorométhylnicotinamide
N-(1-{4-[3-chloro-2-(2-méthyl-2H-tétrazol-5-yl)indol-1-yl]benzylcarbamoyl}cyclopropyl)-3-fluorobenzamide
méthylamide d'acide 3-chloro-1-[4-(1-{[1-(2,2,2-trifluoroacétylamino)cyclopropanecarbonyl]amino}éthyl)-phényl]-1H-indole-2-carboxylique
(1-{4-[3-chloro-2-(pyrrolidine-1-carbonyl)indol-1-yl]phényl}éthyl)amide d'acide 1-(2,2,2-trifluoroacétylamino)cyclopropanecarboxylique
ester éthylique d'acide 3-chloro-1-{4-[({1-[(3-méthoxyisoxazole-5-carbonyl)amino]cyclopropanecarbonyl}amino)méthyl]phényl}-1H-indole-2-carboxylique
diméthylamide d'acide 3-chloro-1-{4-[({1-[(3-méthoxyisoxazole-5-carbonyl)amino]cyclopropanecarbonyl}amino)méthyl]phényl}-1H-indole-2-carboxylique
4-[2-(2-méthyl-2H-tétrazol-5-yl)indol-1-yl]benzylamide d'acide 1-(2,2,2-trifluoroacétylamino)cyclopropanecarboxylique
(1-{4-[3-chloro-5-fluoro-2-(5-méthyl-[1,3,4]oxadiazol-2-yl)indol-1-yl]benzylcarbamoyl}cyclopropyl)amide d'acide 3-méthoxyisoxazole-5-carboxylique
(1-{4-[3-chloro-2-(5-méthyloxazol-2-yl)indol-1-yl]benzylcarbamoyl}cyclopropyl)amide d'acide 3-méthoxyisoxazole-5-carboxylique
{1-[4-(3-chloro-2-cyano-indol-1-yl)benzylcarbamoyl]cyclopropyl}amide d'acide 3-méthoxyisoxazole-5-carboxylique
(1-{4-[3-chloro-2-(5-méthyl-[1,2,4]oxadiazol-3-yl)indol-1-yl]phényl}éthyl)amide d'acide (R)-1-(2,2,2-trifluoroacétylamino)cyclopropanecarboxylique
[1-(1-{4-[3-chloro-2-(5-méthyl-[1,2,4]oxadiazol-3-yl)indol-1-yl]phényl}éthylcarbamoyl)cyclopropyl]amide d'acide (R)-3-méthoxyisoxazole-5-carboxylique
(1-{4-[3-chloro-2-(5-méthyl-[1,2,4]oxadiazol-3-yl)indol-1-yl]benzylcarbamoyl}cyclopropyl)amide d'acide 3-propylisoxazole-5-carboxylique
{1-[4-(5-chloro-2-cyano-indol-1-yl)benzylcarbamoyl]cyclopropyl}amide d'acide 3-méthoxyisoxazole-5-carboxylique
N-{1-[4-(2-cyano-5-méthoxyindol-1-yl)benzylcarbamoyl]cyclopropyl}-5-trifluorométhylnicotinamide
N-{1-[4-(2-cyano-5-fluoroindol-1-yl)benzylcarbamoyl]cyclopropyl}-5-trifluorométhylnicotinamide
N-(1-{4-[5-fluoro-2-(5-méthyl-[1,2,4]oxadiazol-3-yl)indol-1-yl]benzylcarbamoyl}cyclopropyl)-5-trifluorométhylnicotinamide
(1-{4-[5-fluoro-2-(5-méthyl-[1,2,4]oxadiazol-3-yl)indol-1-yl]benzylcarbamoyl}cyclopropyl)amide d'acide 3-méthoxyisoxazole-5-carboxylique
{1-[4-(2-cyano-5-méthoxyindol-1-yl)benzylcarbamoyl]cyclopropyl}amide d'acide 3-méthoxyisoxazole-5-carboxylique
{1-[4-(2-cyano-5-fluoroindol-1-yl)benzylcarbamoyl]cyclopropyl}amide d'acide 3-méthoxyisoxazole-5-carboxylique
N-(1-{4-[3-chloro-5-méthoxy-2-(3-méthyl-[1,2,4]oxadiazol-5-yl)indol-1-yl]benzylcarbamoyl}cyclopropyl)-5-trifluorométhylnicotinamide
(1-{4-[3-chloro-5-méthoxy-2-(3-méthyl-[1,2,4]oxadiazol-5-yl)indol-1-yl]benzylcarbamoyl}cyclopropyl)amide d'acide 3-méthoxyisoxazole-5-carboxylique
{1-[4-(3-chloro-2-cyano-5-méthoxyindol-1-yl)benzylcarbamoyl]cyclopropyl}amide d'acide 3-méthoxyisoxazole-5-carboxylique
(1-{4-[5-chloro-2-(5-méthyl-[1,2,4]oxadiazol-3-yl)indol-1-yl]benzylcarbamoyl}cyclopropyl)amide d'acide 3-méthoxyisoxazole-5-carboxylique
ester méthylique d'acide 3-chloro-1-[3-fluoro-4-({[1-(2,2,2-trifluoroacétylamino)cyclopropanecarbonyl]amino}-méthyl)phényl]-1H-indole-2-carboxylique
ester méthylique d'acide 3-chloro-1-{3-fluoro-4-[({1-[(3-méthoxyisoxazole-5-carbonyl)amino]cyclopropanecarbonyl}amino)méthyl]phényl}-1H-indole-2-carboxylique
[1-({5-[2-(3-méthyl-[1,2,4]oxadiazol-5-yl)indol-1-yl]pyridin-2-ylméthyl}carbamoyl)cyclopropyl]amide d'acide 3-méthoxyisoxazole-5-carboxylique
[1-({5-[2-(5-méthyl-[1,2,4]oxadiazol-3-yl)indol-1-yl]pyridin-2-ylméthyl}carbamoyl)cyclopropyl]amide d'acide 3-méthoxyisoxazole-5-carboxylique
[1-({5-[3-chloro-2-(3-méthyl-[1,2,4]oxadiazol-5-yl)indol-1-yl]pyridin-2-ylméthyl}carbamoyl)cyclopropyl]amide d'acide 3-méthoxyisoxazole-5-carboxylique
[1-({5-[3-chloro-5-fluoro-2-(2-méthyl-2H-tétrazol-5-yl)indol-1-yl]pyridin-2-ylméthyl}carbamoyl)cyclopropyl]amide d'acide 3-méthoxyisoxazole-5-carboxylique
[1-({5-[3-chloro-2-(2-méthyl-2H-tétrazol-5-yl)indol-1-yl]pyridin-2-ylméthyl}carbamoyl)cyclopropyl]amide d'acide 3-méthoxyisoxazole-5-carboxylique
[1-({5-[5-fluoro-2-(5-méthyl-[1,2,4]oxadiazol-3-yl)indol-1-yl]pyridin-2-ylméthyl}carbamoyl)cyclopropyl]amide d'acide 3-méthoxyisoxazole-5-carboxylique
[1-({5-[3-chloro-5-fluoro-2-(5-méthyl-[1,2,4]oxadiazol-3-yl)indol-1-yl]pyridin-2-ylméthyl}carbamoyl)cyclopropyl]amide d'acide 3-méthoxyisoxazole-5-carboxylique
4-[3-chloro-5-méthoxy-2-(5-méthyl-[1,2,4]oxadiazol-3-yl)indol-1-yl]benzylamide d'acide 1-(2,2,2-trifluoroacétylamino)cyclopropanecarboxylique
(1-{4-[3-chloro-5-méthoxy-2-(5-méthyl-[1,2,4]oxadiazol-3-yl)indol-1-yl]benzylcarbamoyl}cyclopropyl)amide d'acide 3-méthoxyisoxazole-5-carboxylique
4-[3,5-dichloro-2-(5-méthyl-[1,2,4]oxadiazol-3-yl)indol-1-yl]benzylamide d'acide 1-(2,2,2-trifluoroacétylamino)cyclopropanecarboxylique
(1-{4-[3,5-dichloro-2-(5-méthyl-[1,2,4]oxadiazol-3-yl)indol-1-yl]benzylcarbamoyl}cyclopropyl)amide d'acide 3-éthoxyisoxazole-5-carboxylique
(1-{4-[3-chloro-5-fluoro-2-(5-méthyl-[1,2,4]oxadiazol-3-yl)indol-1-yl]benzylcarbamoyl}cyclopropyl)amide d'acide 3-méthoxyisoxazole-5-carboxylique
ester méthylique d'acide 3-chloro-4-fluoro-1-{4-[({1-[(3-méthoxyisoxazole-5-carbonyl)amino]cyclopropanecarbonyl}amino)méthyl]phényl}-1H-indole-2-carboxylique
ester méthylique d'acide 3-chloro-4-fluoro-1-[4-({[1-(2,2,2-trifluoroacétylamino)cyclopropanecarbonyl]amino}-méthyl)phényl]-1H-indole-2-carboxylique
2-chloro-N-(1-{4-[3,5-dichloro-2-(5-méthyl-[1,2,4]oxadiazol-3-yl)indol-1-yl]benzylcarbamoyl}cyclopropyl)nicotinamide
N-(1-{4-[3,5-dichloro-2-(5-méthyl-[1,2,4]oxadiazol-3-yl)indol-1-yl]benzylcarbamoyl}cyclopropyl)-3-fluorobenzamide
(1-{4-[3,5-dichloro-2-(5-méthyl-[1,2,4]oxadiazol-3-yl)indol-1-yl]benzylcarbamoyl}cyclopropyl)amide d'acide 5-méthylpyrazine-2-carboxylique
(1-{4-[3,5-dichloro-2-(5-méthyl-[1,2,4]oxadiazol-3-yl)indol-1-yl]benzylcarbamoyl}cyclopropyl)amide d'acide 5-méthylisoxazole-3-carboxylique
N-(1-{4-[3,5-dichloro-2-(5-méthyl-[1,2,4]oxadiazol-3-yl)indol-1-yl]benzylcarbamoyl}cyclopropyl)-2-fluoroisonicotinamide
(1-{4-[3,5-dichloro-2-(5-méthyl-[1,2,4]oxadiazol-3-yl)indol-1-yl]benzylcarbamoyl}cyclopropyl)amide d'acide 5-cyclopropylisoxazole-3-carboxylique
N-(1-{4-[3,5-dichloro-2-(5-méthyl-[1,2,4]oxadiazol-3-yl)indol-1-yl]benzylcarbamoyl}cyclopropyl)-5-trifluorométhylnicotinamide
(1-{4-[3-chloro-2-(5-méthyl-[1,2,4]oxadiazol-3-yl)indol-1-yl]benzylcarbamoyl}cyclopropyl)amide d'acide 2-méthylsulfanylpyrimidine-5-carboxylique
4-[5-méthoxy-2-(3-méthyl-[1,2,4]oxadiazol-5-yl)indol-1-yl]benzylamide d'acide 1-(3,3,3-trifluoropropionylamino)cyclopropanecarboxylique
N-(1-{4-[5-méthoxy-2-(3-méthyl-[1,2,4]oxadiazol-5-yl)indol-1-yl]benzylcarbamoyl}cyclopropyl)-5-trifluorométhylnicotinamide
(1-{4-[5-méthoxy-2-(3-méthyl-[1,2,4]oxadiazol-5-yl)indol-1-yl]benzylcarbamoyl}cyclopropyl)amide d'acide 3-méthoxyisoxazole-5-carboxylique
4-[2-(5-méthyl-[1,2,4]oxadiazol-3-yl)indol-1-yl]benzylamide d'acide 1-(3,3,3-trifluoropropionylamino)cyclopropanecarboxylique
N-(1-{4-[2-(5-méthyl-[1,2,4]oxadiazol-3-yl)indol-1-yl]benzylcarbamoyl}cyclopropyl)-5-trifluorométhylnicotinamide
N-(1-{4-[2-(3-méthyl-[1,2,4]oxadiazol-5-yl)indol-1-yl]benzylcarbamoyl}cyclopropyl)-5-trifluorométhylnicotinamide
N-{1-[4-(2-cyanoindol-1-yl)benzylcarbamoyl]cyclopropyl}-5-trifluorométhylnicotinamide
2-chloro-N-(1-{4-[3-chloro-5-fluoro-2-(5-méthyl-[1,2,4]oxadiazol-3-yl)indol-1-yl]benzylcarbamoyl}cyclopropyl)nicotinamide
N-(1-{4-[3-chloro-5-fluoro-2-(5-méthyl-[1,2,4]oxadiazol-3-yl)indol-1-yl]benzylcarbamoyl}cyclopropyl)-3-fluorobenzamide
(1-{4-[3-chloro-5-fluoro-2-(5-méthyl-[1,2,4]oxadiazol-3-yl)indol-1-yl]benzylcarbamoyl}cyclopropyl)amide d'acide 5-méthylpyrazine-2-carboxylique
(1-{4-[3-chloro-5-fluoro-2-(5-méthyl-[1,2,4]oxadiazol-3-yl)indol-1-yl]benzylcarbamoyl}cyclopropyl)amide d'acide 5-méthylisoxazole-3-carboxylique
N-(1-{4-[3-chloro-5-fluoro-2-(5-méthyl-[1,2,4]oxadiazol-3-yl)indol-1-yl]benzylcarbamoyl}cyclopropyl)-2-fluoroisonicotinamide
(1-{4-[3-chloro-5-fluoro-2-(5-méthyl-[1,2,4]oxadiazol-3-yl)indol-1-yl]benzylcarbamoyl}cyclopropyl)amide d'acide 5-cyclopropylisoxazole-3-carboxylique
N-(1-{4-[3-chloro-5-fluoro-2-(5-méthyl-[1,2,4]oxadiazol-3-yl)indol-1-yl]benzylcarbamoyl}cyclopropyl)-5-trifluorométhylnicotinamide
N-(1-{4-[3-chloro-2-(5-méthyl-[1,2,4]oxadiazol-3-yl)indol-1-yl]benzylcarbamoyl}cyclopropyl)-2-fluoro-3-trifluorométhylbenzamide
N-(1-{4-[3-chloro-2-(5-méthyl-[1,2,4]oxadiazol-3-yl)indol-1-yl]benzylcarbamoyl}cyclopropyl)-3-fluoro-4-trifluorométhylbenzamide
N-(1-{4-[3-chloro-2-(5-méthyl-[1,2,4]oxadiazol-3-yl)indol-1-yl]benzylcarbamoyl}cyclopropyl)-3-fluoro-5-trifluorométhylbenzamide
N-(1-{4-[3-chloro-2-(5-méthyl-[1,2,4]oxadiazol-3-yl)indol-1-yl]benzylcarbamoyl}cyclopropyl)-2-fluoronicotinamide
N-(1-{4-[3-chloro-2-(5-méthyl-[1,2,4]oxadiazol-3-yl)indol-1-yl]benzylcarbamoyl}cyclopropyl)-2-fluoro-5-trifluorométhylbenzamide
N-(1-{4-[3-chloro-2-(5-méthyl-[1,2,4]oxadiazol-3-yl)indol-1-yl]benzylcarbamoyl}cyclopropyl)-3-fluoroisonicotinamide
N-(1-{4-[3-chloro-2-(5-méthyl-[1,2,4]oxadiazol-3-yl)indol-1-yl]benzylcarbamoyl}cyclopropyl)-4-fluoro-3-trifluorométhylbenzamide
N-(1-{4-[3-chloro-2-(5-méthyl-[1,2,4]oxadiazol-3-yl)indol-1-yl]benzylcarbamoyl}cyclopropyl)-6-fluoronicotinamide
N-{1-[4-(5-chloro-2-cyanoindol-1-yl)benzylcarbamoyl]cyclopropyl}-5-trifluorométhylnicotinamide
4-[5-fluoro-2-(3-méthyl-[1,2,4]oxadiazol-5-yl)indol-1-yl]benzylamide d'acide 1-(3,3,3-trifluoropropionylamino)cyclopropanecarboxylique
N-(1-{4-[5-fluoro-2-(3-méthyl-[1,2,4]oxadiazol-5-yl)indol-1-yl]benzylcarbamoyl}cyclopropyl)-5-trifluorométhylnicotinamide
(1-{4-[5-fluoro-2-(3-méthyl-[1,2,4]oxadiazol-5-yl)indol-1-yl]benzylcarbamoyl}cyclopropyl)amide d'acide 3-méthoxyisoxazole-5-carboxylique
2-chloro-N-(1-{4-[3-chloro-2-(5-méthyl-[1,2,4]oxadiazol-3-yl)indol-1-yl]-2-fluorobenzylcarbamoyl}cyclopropyl)nicotinamide
N-(1-{4-[3-chloro-2-(5-méthyl-[1,2,4]oxadiazol-3-yl)indol-1-yl]-2-fluorobenzylcarbamoyl}cyclopropyl)-3-fluorobenzamide
(1-{4-[3-chloro-2-(5-méthyl-[1,2,4]oxadiazol-3-yl)indol-1-yl]-2-fluorobenzylcarbamoyl}cyclopropyl)amide d'acide 5-méthylpyrazine-2-carboxylique
(1-{4-[3-chloro-2-(5-méthyl-[1,2,4]oxadiazol-3-yl)indol-1-yl]-2-fluorobenzylcarbamoyl}cyclopropyl)amide d'acide 5-méthylisoxazole-3-carboxylique
4-[3-chloro-2-(5-méthyl-[1,2,4]oxadiazol-3-yl)indol-1-yl]-2-fluorobenzylamide d'acide 1-(3,3,3-trifluoropropionylamino)cyclopropanecarboxylique
N-(1-{4-[3-chloro-2-(5-méthyl-[1,2,4]oxadiazol-3-yl)indol-1-yl]-2-fluorobenzylcarbamoyl}cyclopropyl)-2-fluoroisonicotinamide
(1-{4-[3-chloro-2-(5-méthyl-[1,2,4]oxadiazol-3-yl)indol-1-yl]-2-fluorobenzylcarbamoyl}cyclopropyl)amide d'acide 5-cyclopropylisoxazole-3-carboxylique
N-(1-{4-[3-chloro-2-(5-méthyl-[1,2,4]oxadiazol-3-yl)indol-1-yl]-2-fluorobenzylcarbamoyl}cyclopropyl)-5-trifluorométhylnicotinamide
4-[3-chloro-2-(3-méthyl-[1,2,4]oxadiazol-5-yl)indol-1-yl]benzylamide d'acide 1-(3,3,3-trifluoropropionylamino)cyclopropanecarboxylique
N-(1-{4-[3-chloro-2-(3-méthyl-[1,2,4]oxadiazol-5-yl)indol-1-yl]benzylcarbamoyl}cyclopropyl)-2-fluoroisonicotinamide
(1-{4-[3-chloro-2-(3-méthyl-[1,2,4]oxadiazol-5-yl)indol-1-yl]benzylcarbamoyl}cyclopropyl)amide d'acide 5-cyclopropylisoxazole-3-carboxylique
N-(1-{4-[3-chloro-2-(3-méthyl-[1,2,4]oxadiazol-5-yl)indol-1-yl]benzylcarbamoyl}cyclopropyl)-5-trifluorométhylnicotinamide
2-fluoro-N-(1-{4-[5-méthoxy-2-(3-méthyl-[1,2,4]oxadiazol-5-yl)indol-1-yl]benzylcarbamoyl}cyclopropyl)nicotinamide
N-(1-{4-[3-chloro-5-fluoro-2-(5-méthyl-[1,2,4]oxadiazol-3-yl)indol-1-yl]benzylcarbamoyl}cyclopropyl)-2-fluoronicotinamide
N-(1-{4-[3-chloro-5-méthoxy-2-(3-méthyl-[1,2,4]oxadiazol-5-yl)indol-1-yl]benzylcarbamoyl}cyclopropyl)-2-fluoronicotinamide
N-(1-{4-[3-chloro-2-(2-méthyl-2H-tétrazol-5-yl)indol-1-yl]benzylcarbamoyl}cyclopropyl)-2-fluoronicotinamide
2-fluoro-N-(1-{4-[5-fluoro-2-(5-méthyl-[1,2,4]oxadiazol-3-yl)indol-1-yl]benzylcarbamoyl}cyclopropyl)nicotinamide
(1-{4-[3-chloro-2-(5-méthyl-[1,2,4]oxadiazol-3-yl)indol-1-yl]benzylcarbamoyl}cyclopropyl)amide d'acide 1-méthyl-1H-pyrazole-4-carboxylique
(1-{4-[3-chloro-2-(5-méthyl-[1,2,4]oxadiazol-3-yl)indol-1-yl]benzylcarbamoyl}cyclopropyl)amide d'acide 2-oxoimidazolidine-4-carboxylique
(1-{4-[3-chloro-2-(5-méthyl-[1,2,4]oxadiazol-3-yl)indol-1-yl]benzylcarbamoyl}cyclopropyl)amide d'acide 3-éthylisoxazole-5-carboxylique
4-[5-méthoxy-2-(5-méthyl-[1,3,4]oxadiazol-2-yl)indol-1-yl]benzylamide d'acide 1-(3,3,3-trifluoropropionylamino)cyclopropanecarboxylique
N-(1-{4-[5-méthoxy-2-(5-méthyl-[1,3,4]oxadiazol-2-yl)indol-1-yl]benzylcarbamoyl}cyclopropyl)-5-trifluorométhylnicotinamide
(1-{4-[5-méthoxy-2-(5-méthyl-[1,3,4]oxadiazol-2-yl)indol-1-yl]benzylcarbamoyl}cyclopropyl)amide d'acide 3-méthoxyisoxazole-5-carboxylique
4-[5-chloro-2-(5-méthyl-[1,2,4]oxadiazol-3-yl)indol-1-yl]benzylamide d'acide 1-(3,3,3-trifluoropropionylamino)cyclopropanecarboxylique
N-(1-{4-[5-chloro-2-(5-méthyl-[1,2,4]oxadiazol-3-yl)indol-1-yl]benzylcarbamoyl}cyclopropyl)-5-trifluorométhylnicotinamide
(1-{4-[3-chloro-5-méthoxy-2-(5-méthyl-[1,2,4]oxadiazol-3-yl)indol-1-yl]benzylcarbamoyl}cyclopropyl)amide d'acide 5-méthyl-pyrazine-2-carboxylique
N-(1-{4-[3-chloro-5-méthoxy-2-(5-méthyl-[1,2,4]oxadiazol-3-yl)indol-1-yl]benzylcarbamoyl}cyclopropyl)-5-trifluorométhylnicotinamide
(1-{2-fluoro-4-[5-fluoro-2-(5-méthyl-[1,2,4]oxadiazol-3-yl)indol-1-yl]benzylcarbamoyl}cyclopropyl)amide d'acide 5-méthylpyrazine-2-carboxylique
N-(1-{2-fluoro-4-[5-fluoro-2-(5-méthyl-[1,2,4]oxadiazol-3-yl)indol-1-yl]benzylcarbamoyl}cyclopropyl)-5-trifluorométhylnicotinamide
(1-{2-fluoro-4-[5-fluoro-2-(5-méthyl-[1,2,4]oxadiazol-3-yl)indol-1-yl]benzylcarbamoyl}cyclopropyl)amide d'acide 3-méthoxyisoxazole-5-carboxylique
(1-{4-[3-chloro-2-(5-méthyl-[1,2,4]oxadiazol-3-yl)indol-1-yl]benzylcarbamoyl}cyclopropyl)amide d'acide isoxazole-5-carboxylique
(1-{4-[3-chloro-2-(5-méthyl-[1,2,4]oxadiazol-3-yl)indol-1-yl]benzylcarbamoyl}cyclopropyl)amide d'acide 5-méthylisoxazole-3-carboxylique
(1-{4-[3-chloro-2-(5-méthyl-[1,2,4]oxadiazol-3-yl)indol-1-yl]benzylcarbamoyl}cyclopropyl)amide d'acide tétrahydrofurane-2-carboxylique
(1-{4-[3-chloro-2-(5-méthyl-[1,2,4]oxadiazol-3-yl)indol-1-yl]benzylcarbamoyl}cyclopropyl)amide d'acide furane-3-carboxylique
(1-{4-[3-chloro-2-(5-méthyl-[1,2,4]oxadiazol-3-yl)indol-1-yl]benzylcarbamoyl}cyclopropyl)amide d'acide 2-méthylcyclopropanecarboxylique
4-[3-chloro-2-(5-méthyl-[1,2,4]oxadiazol-3-yl)indol-1-yl]benzylamide d'acide 1-pent-4-ynoylaminocyclopropanecarboxylique
4-[3-chloro-2-(5-méthyl-[1,2,4]oxadiazol-3-yl)indol-1-yl]benzylamide d'acide 1-(4-méthylpentanoylamino)cyclopropanecarboxylique
(1-{4-[3-chloro-2-(5-méthyl-[1,2,4]oxadiazol-3-yl)indol-1-yl]benzylcarbamoyl}cyclopropyl)amide d'acide 1H-pyrazole-4-carboxylique
(1-{4-[3-chloro-2-(5-méthyl-[1,2,4]oxadiazol-3-yl)indol-1-yl]benzylcarbamoyl}cyclopropyl)amide d'acide 1-cyanocyclopropanecarboxylique
(1-{4-[3-chloro-2-(5-méthyl-[1,2,4]oxadiazol-3-yl)indol-1-yl]benzylcarbamoyl}cyclopropyl)amide d'acide cyclobutanecarboxylique
(1-{4-[3-chloro-2-(5-méthyl-[1,2,4]oxadiazol-3-yl)indol-1-yl]benzylcarbamoyl}cyclopropyl)amide d'acide cyclopentanecarboxylique
(1-{4-[3-chloro-2-(5-méthyl-[1,2,4]oxadiazol-3-yl)indol-1-yl]benzylcarbamoyl}cyclopropyl)amide d'acide furane-2-carboxylique
N-(1-{4-[3-chloro-2-(5-méthyl-[1,2,4]oxadiazol-3-yl)indol-1-yl]benzylcarbamoyl}cyclopropyl)-3-cyanobenzamide
4-[3-chloro-2-(5-méthyl-[1,2,4]oxadiazol-3-yl)indol-1-yl]benzylamide d'acide 1-(2-thiophén-3-ylacétylamino)cyclopropanecarboxylique
(1-{4-[3-chloro-2-(5-méthyl-[1,2,4]oxadiazol-3-yl)indol-1-yl]benzylcarbamoyl}cyclopropyl)amide d'acide 1,5-diméthyl-1H-pyrazole-3-carboxylique
(1-{4-[3-chloro-2-(5-méthyl-[1,2,4]oxadiazol-3-yl)indol-1-yl]benzylcarbamoyl}cyclopropyl)amide d'acide 4-méthylthiazole-5-carboxylique
(1-{4-[3-chloro-2-(5-méthyl-[1,2,4]oxadiazol-3-yl)indol-1-yl]benzylcarbamoyl}cyclopropyl)amide d'acide 2,4-diméthylthiazole-5-carboxylique
4-[3-chloro-2-(5-méthyl-[1,2,4]oxadiazol-3-yl)indol-1-yl]benzylamide d'acide 1-(2-acétylamino-3-hydroxypropionylamino)cyclopropanecarboxylique
(1-{4-[3-chloro-2-(5-méthyl-[1,2,4]oxadiazol-3-yl)indol-1-yl]benzylcarbamoyl}cyclopropyl)amide d'acide 5-méthyl-1H-pyrazole-3-carboxylique
2-chloro-N-(1-{4-[3-chloro-2-(5-méthyl-[1,2,4]oxadiazol-3-yl)indol-1-yl]benzylcarbamoyl}cyclopropyl)nicotinamide
N-(1-{4-[3-chloro-2-(5-méthyl-[1,2,4]oxadiazol-3-yl)indol-1-yl]benzylcarbamoyl}cyclopropyl)-6-méthylnicotinamide
N-(1-{4-[3-chloro-2-(5-méthyl-[1,2,4]oxadiazol-3-yl)indol-1-yl]benzylcarbamoyl}cyclopropyl)-2-méthylnicotinamide
2-chloro-N-(1-{4-[3-chloro-2-(5-méthyl-[1,2,4]oxadiazol-3-yl)indol-1-yl]benzylcarbamoyl}cyclopropyl)-6-méthylnicotinamide
(1-{4-[3-chloro-2-(5-méthyl-[1,2,4]oxadiazol-3-yl)indol-1-yl]benzylcarbamoyl}cyclopropyl)amide d'acide 6-bromopyridine-2-carboxylique
N-(1-{4-[3-chloro-2-(5-méthyl-[1,2,4]oxadiazol-3-yl)indol-1-yl]benzylcarbamoyl}cyclopropyl)nicotinamide
(1-{4-[3-chloro-2-(5-méthyl-[1,2,4]oxadiazol-3-yl)indol-1-yl]benzylcarbamoyl}cyclopropyl)amide d'acide quinoline-3-carboxylique
(1-{4-[3-chloro-2-(5-méthyl-[1,2,4]oxadiazol-3-yl)indol-1-yl]benzylcarbamoyl}cyclopropyl)amide d'acide 6-oxo-1,6-dihydropyridine-3-carboxylique
(1-{4-[3-chloro-2-(5-méthyl-[1,2,4]oxadiazol-3-yl)indol-1-yl]benzylcarbamoyl}cyclopropyl)amide d'acide 5-méthylpyrazine-2-carboxylique
(1-{4-[3-chloro-2-(5-méthyl-[1,2,4]oxadiazol-3-yl)indol-1-yl]benzylcarbamoyl}cyclopropyl)amide d'acide 6-oxo-1,6-dihydropyridine-2-carboxylique
4-[3-chloro-2-(5-méthyl-[1,2,4]oxadiazol-3-yl)indol-1-yl]benzylamide d'acide 1-(3,3,3-trifluoropropionylamino)cyclopropanecarboxylique
N-(1-{4-[3-chloro-2-(5-méthyl-[1,2,4]oxadiazol-3-yl)indol-1-yl]benzylcarbamoyl}cyclopropyl)-2-fluoroisonicotinamide
(1-{4-[3-chloro-2-(5-méthyl-[1,2,4]oxadiazol-3-yl)indol-1-yl]benzylcarbamoyl}cyclopropyl)amide d'acide 5-cyclopropylisoxazole-3-carboxylique
(1-{4-[3-chloro-2-(5-méthyl-[1,2,4]oxadiazol-3-yl)indol-1-yl]benzylcarbamoyl}cyclopropyl)amide d'acide pyrimidine-5-carboxylique
(1-{4-[3-chloro-2-(5-méthyl-[1,2,4]oxadiazol-3-yl)indol-1-yl]benzylcarbamoyl}cyclopropyl)amide d'acide thiophène-3-carboxylique
(1-{4-[3-chloro-2-(5-méthyl-[1,2,4]oxadiazol-3-yl)indol-1-yl]benzylcarbamoyl}cyclopropyl)amide d'acide 5-oxopyrrolidine-2-carboxylique
4-[3-chloro-2-(5-méthyl-[1,2,4]oxadiazol-3-yl)indol-1-yl]benzylamide d'acide 1-(3-furan-3-ylacryloylamino)cyclopropanecarboxylique
N-(1-{4-[3-chloro-2-(5-méthyl-[1,2,4]oxadiazol-3-yl)indol-1-yl]benzylcarbamoyl}cyclopropyl)-4-cyanobenzamide
4-[3-chloro-2-(5-méthyl-[1,2,4]oxadiazol-3-yl)indol-1-yl]benzylamide d'acide 1-(3-furan-2-ylacryloylamino)cyclopropanecarboxylique
(1-{4-[3-chloro-2-(5-méthyl-[1,2,4]oxadiazol-3-yl)indol-1-yl]benzylcarbamoyl}cyclopropyl)amide d'acide thiophène-2-carboxylique
(1-{4-[3-chloro-2-(5-méthyl-[1,2,4]oxadiazol-3-yl)indol-1-yl]benzylcarbamoyl}cyclopropyl)amide d'acide 5-méthylsulfanylthiophène-2-carboxylique
(1-{4-[3-chloro-2-(5-méthyl-[1,2,4]oxadiazol-3-yl)indol-1-yl]benzylcarbamoyl}cyclopropyl)amide d'acide [1,2,3]thiadiazole-4-carboxylique
N-(1-{4-[3-chloro-2-(5-méthyl-[1,2,4]oxadiazol-3-yl)indol-1-yl]benzylcarbamoyl}cyclopropyl)-3-méthoxybenzamide
N-(1-{4-[3-chloro-2-(5-méthyl-[1,2,4]oxadiazol-3-yl)indol-1-yl]benzylcarbamoyl}cyclopropyl)-3-iodobenzamide
N-(1-{4-[3-chloro-2-(5-méthyl-[1,2,4]oxadiazol-3-yl)indol-1-yl]benzylcarbamoyl}cyclopropyl)-3-trifluorométhylbenzamide
N-(1-{4-[3-chloro-2-(5-méthyl-[1,2,4]oxadiazol-3-yl)indol-1-yl]benzylcarbamoyl}cyclopropyl)-3-fluorobenzamide
N-(1-{4-[3-chloro-2-(5-méthyl-[1,2,4]oxadiazol-3-yl)indol-1-yl]benzylcarbamoyl}cyclopropyl)-3-méthylbenzamide
N-(1-{4-[3-chloro-2-(5-méthyl-[1,2,4]oxadiazol-3-yl)indol-1-yl]benzylcarbamoyl}cyclopropyl)-5-trifluorométhylnicotinamide
(1-{4-[3-chloro-2-(5-méthyl-[1,2,4]oxadiazol-3-yl)indol-1-yl]benzylcarbamoyl}cyclopropyl)amide d'acide 3-méthylisoxazole-4-carboxylique
(1-{4-[3-chloro-2-(5-méthyl-[1,3,4]oxadiazol-2-yl)indol-1-yl]benzylcarbamoyl}cyclopropyl)amide d'acide quinoline-3-carboxylique
4-[3-chloro-2-(5-méthyl-[1,3,4]oxadiazol-2-yl)indol-1-yl]benzylamide d'acide 1-(3,3,3-trifluoropropionylamino)cyclopropanecarboxylique
(1-{4-[3-chloro-2-(5-méthyl-[1,3,4]oxadiazol-2-yl)indol-1-yl]benzylcarbamoyl}cyclopropyl)amide d'acide pyrimidine-5-carboxylique
(1-{4-[3-chloro-2-(5-méthyl-[1,2,4]oxadiazol-3-yl)indol-1-yl]benzylcarbamoyl}cyclopropyl)amide d'acide 2-méthylpyrimidine-5-carboxylique
chlorhydrate de 3-amino-N-(1-{4-[3-chloro-2-(5-méthyl-[1,2,4]oxadiazol-3-yl)indol-1-yl]benzylcarbamoyl}cyclopropyl)benzamide
amide d'acide 3-chloro-1-[4-({[1-(2,2,2-trifluoro-acétylamino)cyclopropanecarbonyl]amino}méthyl)phényl]-1H-indole-2-carboxylique
amide d'acide 3-chloro-1-{4-[({1-[(3-méthoxyisoxazole-5-carbonyl)amino]cyclopropanecarbonyl}amino)méthyl]phényl}-1H-indole-2-carboxylique
4-[3-chloro-5-fluoro-2-(5-méthyl-[1,2,4]oxadiazol-3-yl)indol-1-yl]benzylamide d'acide 1-(2,2,2-trifluoroacétylamino)cyclopropanecarboxylique
((R)-1-{4-[3-chloro-2-(5-méthyl-[1,2,4]oxadiazol-3-yl)indol-1-yl]-2-fluorophényl}éthyl)amide d'acide 1-(2,2,2-trifluoroacétylamino)cyclopropanecarboxylique
[1-((R)-1-{4-[3-chloro-2-(5-méthyl-[1,2,4]oxadiazol-3-yl)indol-1-yl]-2-fluorophényl}éthylcarbamoyl)cyclopropyl]amide d'acide 3-méthoxyisoxazole-5-carboxylique
(1-{4-[3-chloro-2-(5-méthyl-[1,2,4]oxadiazol-3-yl)indol-1-yl]-2-fluorobenzylcarbamoyl}cyclopropyl)amide d'acide 3-méthoxyisoxazole-5-carboxylique
(1-{4-[3-chloro-2-(3-méthyl-[1,2,4]oxadiazol-5-yl)indol-1-yl]benzylcarbamoyl}cyclopropyl)amide d'acide 3-méthoxyisoxazole-5-carboxylique
4-[3-chloro-2-(3-méthyl-[1,2,4]oxadiazol-5-yl)indol-1-yl]benzylamide d'acide 1-(2,2,2-trifluoroacétylamino)cyclopropanecarboxylique
(1-{4-[2-(5-méthyl-[1,2,4]oxadiazol-3-yl)indol-1-yl]benzylcarbamoyl}cyclopropyl)amide d'acide 3-méthoxyisoxazole-5-carboxylique
(1-{4-[2-(3-méthyl-[1,2,4]oxadiazol-5-yl)indol-1-yl]benzylcarbamoyl}cyclopropyl)amide d'acide 3-méthoxyisoxazole-5-carboxylique
(1-{4-[3-chloro-5-fluoro-2-(5-méthyl-[1,2,4]oxadiazol-3-yl)indol-1-yl]-2-fluorobenzylcarbamoyl}cyclopropyl)amide d'acide 3-méthoxyisoxazole-5-carboxylique
(1-{4-[3,5-difluoro-2-(5-méthyl-[1,2,4]oxadiazol-3-yl)indol-1-yl]benzylcarbamoyl}cyclopropyl)amide d'acide 3-méthoxyisoxazole-5-carboxylique
4-[3,5-difluoro-2-(5-méthyl-[1,2,4]oxadiazol-3-yl)indol-1-yl]benzylamide d'acide 1-(2,2,2-trifluoroacétylamino)cyclopropanecarboxylique
(1-{4-[5-fluoro-3-méthyl-2-(5-méthyl-[1,2,4]oxadiazol-3-yl)indol-1-yl]benzylcarbamoyl}cyclopropyl)amide d'acide 3-méthoxyisoxazole-5-carboxylique et
(1-{4-[3-chloro-2-(4-méthyl-5-oxo-4,5-dihydro-[1,2,4]oxadiazol-3-yl)indol-1-yl]benzylcarbamoyl}cyclopropyl)amide d'acide 3-méthoxyisoxazole-5-carboxylique
et leurs antipodes optiques ou racémates et/ou sels.

3. Procédé pour préparer les composés de formule (I) tels que revendiqués dans la revendication 1, **caractérisé par**
a) la réaction d'un dérivé d'indole de formule (II) 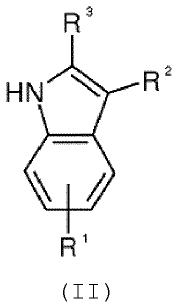 dans laquelle la signification de R¹, R² et R³ est telle que décrite à propos de la formule (I) dans la revendication 1, avec un acide boronique de formule (III) 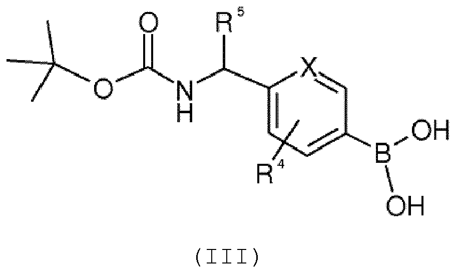 dans laquelle la signification de R⁴ et R⁵ est telle que décrite à propos de la formule (I) dans la revendication 1, puis le dérivé d'indole ainsi obtenu de formule (IV) 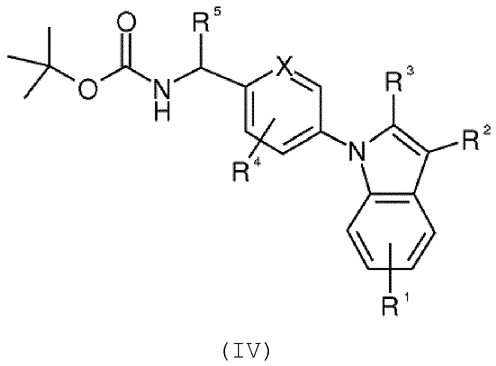 dans laquelle la signification de R¹, R², R³, R⁴ et R⁵ est telle que décrite à propos de la formule (I) dans la revendication 1, est déprotégé pour engendrer un dérivé d'amine de formule (V) 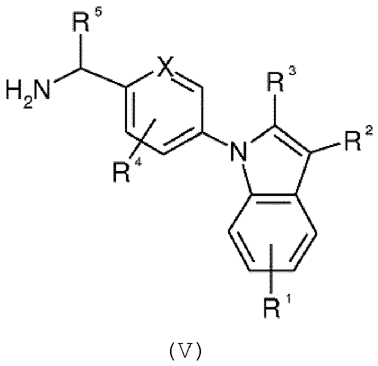 dans laquelle la signification de R¹, R², R³, R⁴ et R⁵ est telle que décrite à propos de la formule (I) dans la revendication 1, finalement ce dernier est mis à réagir avec un dérivé d'acide de formule (VI) 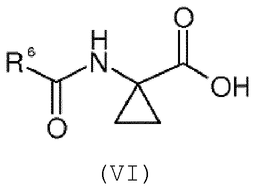 dans laquelle la signification de R⁶ est telle que décrite à propos de la formule (I) dans la revendication 1 ;
ou
b) la réaction d'un dérivé d'indole de formule (II) 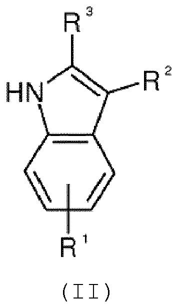 dans laquelle la signification de R¹, R² et R³ est telle que décrite à propos de la formule (I) dans la revendication 1, avec un acide boronique de formule (VII) 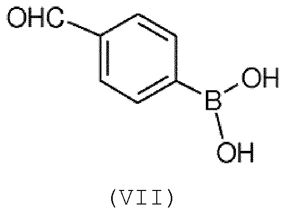 puis le dérivé d'indole ainsi obtenu de formule (VIII) 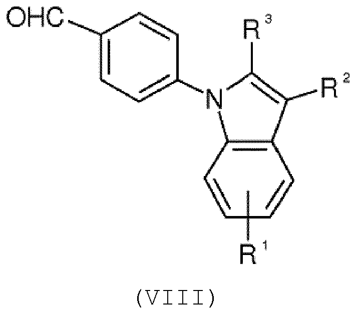 dans laquelle la signification de R¹, R² et R³ est telle que décrite à propos de la formule (I) dans la revendication 1,
est mis à réagir avec du chlorhydrate d'hydroxylamine pour engendrer le dérivé d'indole de formule (IX) 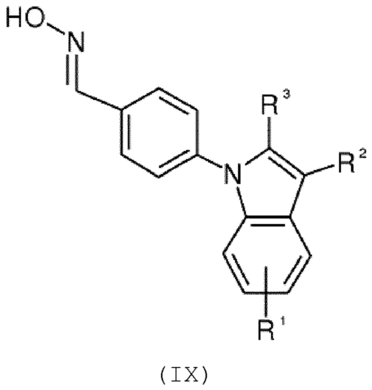 dans laquelle la signification de R¹, R² et R³ est telle que décrite à propos de la formule (I) dans la revendication 1,
ce dernier est hydrogéné pour fournir un dérivé d'amine de formule (X) 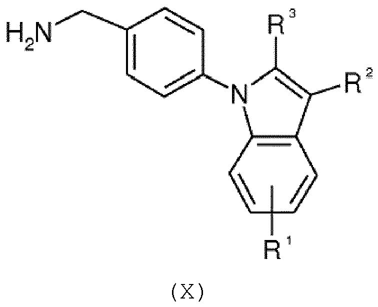 dans laquelle la signification de R¹, R² et R³ est telle que décrite à propos de la formule (I) dans la revendication 1, finalement ce dernier est mis à réagir avec un dérivé d'acide de formule (VI) 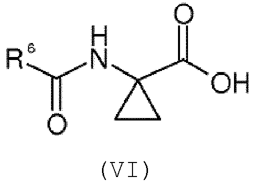 dans laquelle la signification de R⁶ est telle que décrite à propos de la formule (I) dans la revendication 1 ou
c) la réaction des dérivés d'amine de formule (V) ou de formule (X), obtenus conformément au procédé décrit dans le procédé a) et le procédé b), avec le dérivé d'acide aminé de formule (XI) 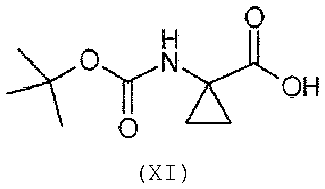 et le dérivé d'indole ainsi obtenu de formule (XII) 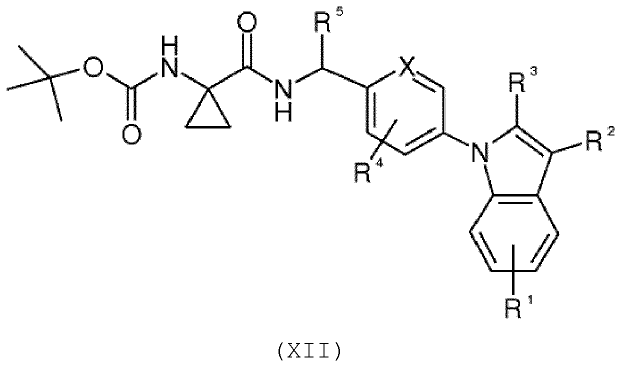 dans laquelle la signification de R¹, R², R³, R⁴ et R⁵ est telle que décrite à propos de la formule (I) dans la revendication 1, est déprotégé pour engendrer un dérivé d'amine de formule (XIII) 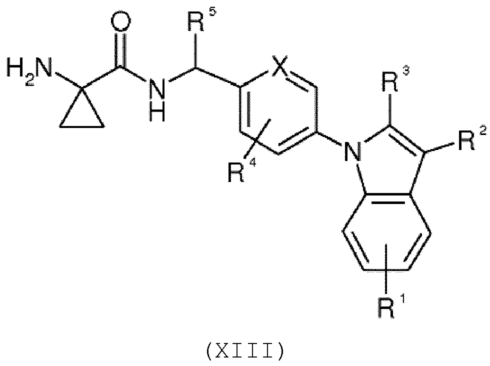
dans laquelle la signification de R¹, R², R³, R⁴ et R⁵ est telle que décrite à propos de la formule (I) dans la revendication 1, finalement ce dernier est mis à réagir avec un dérivé d'acide de formule (XIV) 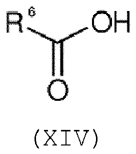 dans laquelle la signification de R⁶ est telle que décrite à propos de la formule (I) dans la revendication 1 ;
et éventuellement un dérivé d'indole de formule (I) obtenu dans le procédé a) ou b) ou c) et ses antipodes optiques ou racémates et/ou sels peuvent être transformés en un autre composé de formule (I) et ses antipodes optiques ou racémates et/ou sels par introduction de nouveaux substituants et/ou modification ou élimination des substituants existants.

4. Composition pharmaceutique comprenant une quantité efficace du point de vue thérapeutique d'un composé de formule (I) tel que revendiqué dans la revendication 1 et d'antipodes optiques ou racémates et/ou sels de celui-ci, à titre d'agents actifs, et des matériaux auxiliaires pharmaceutiquement acceptables.

5. Procédé pour fabriquer une composition pharmaceutique ayant un effet antagoniste sélectif du récepteur de bradykinine B1, **caractérisé par** le mélange d'une quantité efficace du point de vue thérapeutique d'un composé de formule (I) tel que revendiqué dans la revendication 1 et d'antipodes optiques ou racémates et/ou sels de celui-ci, à titre d'agents actifs, et de matériaux auxiliaires.

6. Composé de formule (I) selon la revendication 1 et ses antipodes optiques ou racémates et/ou sels, pour utilisation en tant qu'antagoniste sélectif du récepteur de bradykinine B1.

7. Composé de formule (I) selon la revendication 1 et ses antipodes optiques ou racémates et/ou sels, pour utilisation dans le traitement ou la prévention de troubles associés à une activité du récepteur de bradykinine B1.

8. Composé selon la revendication 7, dans lequel le trouble est choisi dans l'ensemble constitué par une douleur et une inflammation, y compris une douleur de l'appareil locomoteur, une lombalgie, des troubles liés à un mouvement répétitifs, le syndrome algique myofascial, l'arthrite (y compris l'arthrose, la polyarthrite rhymatoïde et la goutte), la fibromyalgie, une douleur viscérale, les troubles cutanés inflammatoires et les lésions cutanées, les complications associées au diabète telles qu'une neuropathie, une néphropathie, une vasculopathie, une rétinopathie, une douleur liée à un cancer, et une maladie intestinale inflammatoire.

9. Utilisation d'un composé de formule (I) selon la revendication 1 ou d'antipodes optiques ou racémates ou d'un sel ou hydrate ou solvate pharmaceutiquement acceptable de ceux-ci pour la fabrication d'un médicament destiné à prévenir et/ou traiter un état qui requiert l'inhibition d'un récepteur de bradykinine.

10. Utilisation selon la revendication 9, dans laquelle le récepteur de bradykinine est le récepteur de bradykinine B1.
